# EUROPEAN PATENT APPLICATION

(11) **EP 4 501 912 A1**
(43) Date of publication of application: **05.02.2025**
(21) Application number: 23778176.0
(22) Date of filing: 28.03.2023
(51) Int. Cl.: C07D 215/54, C07D 215/42, C07D 215/22, C07D 471/04, A61K 31/4375, A61K 31/4706, A61P 35/00

(54) **NOVEL HETEROCYCLIC COMPOUND**

(30) Priority: 29.03.2022 CN 202210327578; 11.07.2022 CN 202210811870; 14.10.2022 CN 202211264066; 22.11.2022 CN 202211470947; 06.01.2023 CN 202310021006
(71) Applicant: Shouyao Holdings (Beijing) Co., Ltd., Beijing 100176 (CN)
(72) Inventor: LOU, Zhenbang, Beijing 100195 (CN); HU, Wenqi, Beijing 100195 (CN); ZHANG, Quanhao, Beijing 100195 (CN); WANG, Yeliu, Beijing 100195 (CN); SHANG, Xianxing, Beijing 100195 (CN); LIU, Shuang, Beijing 100195 (CN); SHI, Junfen, Beijing 100195 (CN); WANG, He, Beijing 100195 (CN)
(74) Representative: f & e patent
(86) International application number: PCT/CN2023/084314
(87) International publication number: WO 2023/185811

(57) **Abstract**

The present invention relates to a novel heterocyclic compound; specifically, the present invention provides a compound of formula (1) or a pharmaceutically acceptable salt, solvate, polymorph or tautomer thereof, a pharmaceutical composition comprising the same, and the use of the same in the manufacture of a medicament for the treatment of a disease related to MAT2A.

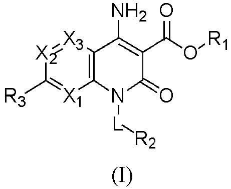

## Description

### Cross Reference

This application claims the priority of Chinese Patent Application No. 202210327578.2, filed on March 29, 2022, entitled "NOVEL HETEROCYCLIC COMPOUND", Chinese Patent Application No. 202210811870.1, filed on July 11, 2022, entitled "NOVEL HETEROCYCLIC COMPOUND", and Chinese Patent Application No. 202211264066.2, filed on October 14, 2022, entitled "NOVEL HETEROCYCLIC COMPOUND". Priority is claimed to Chinese Patent Application No. 202211470947.X, filed November 22, 2022, entitled "NOVEL HETEROCYCLIC COMPOUND", and Chinese Patent Application No. 202310021006.6, filed January 6, 2023, entitled "NOVEL HETEROCYCLIC COMPOUND", the entire disclosures of which are incorporated herein by reference in their entirety.

### Technical Field

The present invention relates to a novel heterocyclic compound or pharmaceutically acceptable salts, solvates, polymorphs, or isomers thereof, pharmaceutical compositions containing the same, and their use as MAT2A inhibitors.

### Background Art

Methylthioadenosine phosphorylase (MTAP) is an enzyme present in all normal tissues that catalyzes the production of Methylthioribose-1-phosphate (MTR-1P) and adenine from methylthioadenosine (MTA). The MTAP gene is located in the 9p21 region of the human chromosome, and p15INK2A (also called CDKN2A) and p15INK4B are also located in this region. Approximately 15% of human cancers have a homozygous deletion of the MTAP gene, e.g. primary leukemia, glioma, melanoma, pancreatic cancer, non-small cell lung cancer (NSCLC), bladder cancer, etc., and are often accompanied by a common deletion of the tumor suppressor gene CDKN2A. This co-deletion results in a poor prognosis of invasive tumors and a lack of effective molecular-targeted therapies.

Methionine adenosyltransferase (MAT), also known as S-adenosylmethionine synthetase, participates in the rate-limiting step of the methionine cycle and catalyzes the synthesis of S-adenosylmethionine (SAM) from methionine and ATP. SAM is the major methyl donor in cells and is used to methylate a variety of substrates, such as DNA, RNA, and proteins, involved in a variety of biological processes. Methionine adenosyltransferase 2A (MAT2A) is one of the MAT family protein members, besides, the MAT family also has two members, MAT1A and MAT2B. MAT1A is specifically expressed in the liver, while MAT2A is widely distributed. Both MAT1A and MAT2A can catalyze the synthesis of SAM, but their catalytic kinetics and regulatory properties are different. MAT2B has no catalytic ability and acts as a regulatory subunit to form a dimer with MAT2A so that MAT2A undergoes a conformational change and regulates the activity of MAT2A by increasing the affinity of MAT2A for methionine and SAM.

MAT2A was identified as a synthetic lethal target in MTAP deficient cancers. It was found that in a shRNA screen of approximately 400 cancer cell lines, knockdown of MAT2A resulted in loss of viability of more MTAP deficient cells than MTAP-expressing cells. In addition, induced knockdown of the MAT2A protein reduced tumor growth in vivo, and these results suggest that MAT2A inhibitors may provide a therapeutic approach for cancer patients with MTAP deficiency. Further studies have found that inhibition of MAT2A in MTAP deficient cells inhibits the activity of the protein arginine N-methyltransferase 5 (PRMT5). PRMT5 uses SAM as a methyl donor and can methylate many proteins, including histones and non-histones, involved in gene transcription and translation, cell proliferation, and other processes. MTA, a substrate of MTAP, is a potent inhibitor of PRMT5, and a large amount of MTA accumulates in MTAP deficient tumor cells, resulting in decreased catalytic activity of PRMT5. MTAP deficient cells are more dependent on the production of SAM and thus on the activity of MAT2A than cells expressing MTAP. Inhibition of MAT2A activity decreases intracellular SAM concentrations and selectively decreases PRMT5 activity in MTAP deficient cells. Thus, targeting MAT2A specifically inhibits the proliferation of MTAP deficient tumor cells by inhibiting PRMT5 activity, resulting in a synthetic lethal effect.

MAT2A inhibitors may provide a new treatment for cancer patients with MTAP deficiency, which is an innovative field and has broad development space. WO2018039972, WO2020123395, etc. disclose certain MAT2A inhibitor compounds.

This patent describes a class of 4-aminoquinoline derivatives having good inhibitory activity against MAT2A and also against MTAP deficient tumor cells. Furthermore, these compounds have excellent oral bioavailability, plasma protein adsorption, pharmacokinetic characteristics, and in vivo efficacy in animals; with lower toxicity, CYP-inhibition, etc.

### Summary of the Invention

The present invention provides an MAT2A inhibitor, which is a compound of general formula (I) or a pharmaceutically acceptable salt, solvate, polymorph or tautomer thereof. The present invention also provides a compound of general formula (I) or a pharmaceutically acceptable salt, solvate, polymorph or tautomer thereof, a pharmaceutical composition comprising such compound, a method of treating a disease related to MAT2A using such compound or composition thereof, and the use of such compound or composition thereof in the manufacture of a medicament for the treatment of a disease related to MAT2A.

In one aspect, the present invention provides a compound of Formula (I) or a pharmaceutically acceptable salt, solvate, polymorph or tautomer thereof, 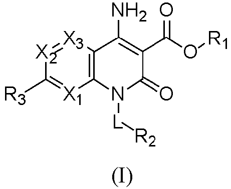 wherein,
L is bond or CH₂, CH₂ can be optionally substituted with C₁₋₆ alkyl or 3-8 membered cycloalkyl,
X₁, X₂ and X₃ are each independently selected from N and CR₄,
R₁ is selected from C₁₋₆ alkyl, 3-8 membered cycloalkyl, 3-8 membered heterocycloalkyl, 6-10 membered aryl and 5-12 membered heteroaryl, the alkyl, cycloalkyl, heterocycloalkyl, aryl, or heteroaryl can be optionally substituted with halogen, -CN, -OH, -NH₂, C₁₋₆ alkyl, 3-8 membered cycloalkyl, 3-8 membered heterocycloalkyl, 6-10 membered aryl, 5-12 membered heteroaryl, -O-C₁₋₆ alkyl, or -N(R)-C₁₋₆ alkyl,
R₂ is 3-8 membered carbocycle, 3-8 membered heterocycle, benzene ring, or 5-6 membered heteroaromatic ring, said carbocycle, heterocycle, benzene ring and heteroaromatic ring can be optionally fused with 3-8 membered carbocycle, 3-8 membered heterocycle, benzene ring, or 5-6 membered heteroaromatic ring, said carbocycle, heterocycle, benzene ring, or heteroaromatic ring can be optionally substituted with 1-3 R₁₂,
R₁₂ are each independently selected from halogen, NO₂, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -(CO)-C₁₋₆ alkyl, -(CO)-C₃₋₈ cycloalkyl, -COOH, -(CO)-O-C₁₋₆ alkyl, -(CO)-NH₂, -(CO)-NH-C₁₋₆ alkyl, -O-C₁₋₆ alkyl and -N(R)-C₁₋₆ alkyl, the alkyl, cycloalkyl and heterocycloalkyl can be optionally substituted with halogen, -CN, -OH, -NH₂, -NH-(CO)-O-*tert*-butyl, =N-OH, C₁₋₆ alkyl, 3-8 membered cycloalkyl, 3-8 membered heterocycloalkyl, -O-C₁₋₆ alkyl, or -N(R)-C₁₋₆ alkyl,
R₃ is selected from H, halogen, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl, -S-C₁₋₆ alkyl, -NH(R), -N(R)-C₁₋₆ alkyl, -N(R)-(3-8 membered cycloalkyl), -N(R)-(3-8 membered heterocycloalkyl) and 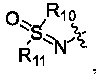 the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl,
R₄ are each independently selected from H, halogen, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl and -N(R)-C₁₋₆ alkyl, the alkyl, cycloalkyl and heterocycloalkyl can be optionally substituted with halogen, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl, or -N(R)-C₁₋₆ alkyl,
R are each independently selected from H, C₁₋₆ alkyl, 3-8 membered cycloalkyl and 3-8 membered heterocycloalkyl, the alkyl, cycloalkyl and heterocycloalkyl can be optionally substituted with halogen, -CN, -OH, -NH₂, C₁₋₆ alkyl, 3-8 membered cycloalkyl, or 3-8 membered heterocycloalkyl,
R₁₀ and R₁₁ are each independently selected from C₁₋₆ alkyl and 3-8 membered cycloalkyl, the alkyl and cycloalkyl can be optionally substituted with halogen, -CN, -OH, or -NH₂,
with the proviso that the following compound is not included: 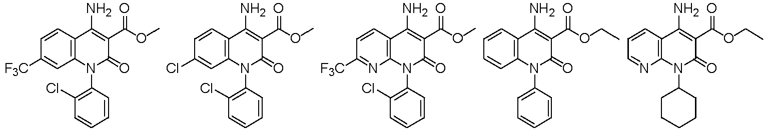 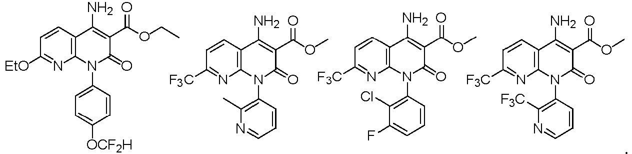
In some embodiments, R₃ is selected from H, halogen, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl, -NH(R), -N(R)-C₁₋₆ alkyl, -N(R)-(3-8 membered cycloalkyl), -N(R)-(3-8 membered heterocycloalkyl) and 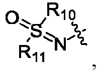 the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl, R, R₁₀ and R₁₁ are as defined above;
In some embodiments, R₃ is selected from H, halogen, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl, -N(R)-C₁₋₆ alkyl and 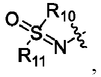 the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl, R, R₁₀ and R₁₁ are as defined above;
In some embodiments, R₁₂ are each independently selected from halogen, NO₂, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -(CO)-C₁₋₆ alkyl, -(CO)-C₃₋₈ cycloalkyl, -COOH, -(CO)-O-C₁₋₆ alkyl, -(CO)-NH₂, -(CO)-NH-C₁₋₆ alkyl, -O-C₁₋₆ alkyl and -N(R)-C₁₋₆ alkyl, the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl, R is as defined above;
In some embodiments, R₁₂ are each independently selected from halogen, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl and -N(R)-C₁₋₆ alkyl, the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl,
R are each independently selected from C₁₋₆ alkyl, 3-8 membered cycloalkyl and 3-8 membered heterocycloalkyl, the alkyl, cycloalkyl and heterocycloalkyl can be optionally substituted with halogen, -CN, -OH, -NH₂, C₁₋₆ alkyl, 3-8 membered cycloalkyl, or 3-8 membered heterocycloalkyl;
In some embodiments, L is bond;
In some embodiments, X₂ and X₃ are CH, X₁ is N or CH;
In some embodiments, X₁, X₂ and X₃ are CH;
In some embodiments, R₁ is selected from C₁₋₆ alkyl, 3-8 membered cycloalkyl and 3-8 membered heterocycloalkyl, the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, 6-10 membered aryl, 5-12 membered heteroaryl, -O-C₁₋₆ alkyl, or -N(R)-C₁₋₆ alkyl, R is as defined above;
In some embodiments, R₁ is selected from C₁₋₆ alkyl, 3-8 membered cycloalkyl and 3-8 membered heterocycloalkyl, preferably C₁₋₆ alkyl, more preferably methyl;
In some embodiments, R₃ is selected from H, halogen, CF₃, -CN, -OH, -NH₂, C₁₋₆ alkyl, C₂₋₆ alkenyl, C₂₋₆ alkynyl and 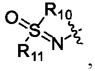 R₁₀ and R₁₁ are as defined above;
In some embodiments, R₂ is benzene ring or 5-6 membered heteroaromatic ring, the benzene ring and heteroaromatic ring can be optionally fused with 3-8 membered carbocycle, 3-8 membered heterocycle, benzene ring, or 5-6 membered heteroaromatic ring, said carbocycle, heterocycle, benzene ring, or heteroaromatic ring can be optionally substituted with 1-3 R₁₂, R₁₂ is as defined above;
In some embodiments, R₄ are each independently selected from H, halogen, -CN, -OH, -NH₂, C₁₋₆ alkyl, 3-8 membered cycloalkyl, 3-8 membered heterocycloalkyl, -O-C₁₋₆ alkyl and -N(R)-C₁₋₆ alkyl, the alkyl, cycloalkyl and heterocycloalkyl can be optionally substituted with halogen, -CN, -OH, or -NH₂;
In some embodiments, R₄ are each independently selected from H, halogen, -CN, -OH, -NH₂, C₁₋₆ alkyl, 3-8 membered cycloalkyl, 3-8 membered heterocycloalkyl, -O-C₁₋₆ alkyl and -N(R)-C₁₋₆ alkyl, preferably H, halogen, -CN, -OH, -NH₂, or C₁₋₆ alkyl;
In some embodiments, R is selected from C₁₋₆ alkyl, 3-8 membered cycloalkyl and 3-8 membered heterocycloalkyl, preferably C₁₋₆ alkyl;
In some embodiments, R₁₀ and R₁₁ are each independently selected from C₁₋₆ alkyl and 3-8 membered cycloalkyl, preferably C₁₋₆ alkyl;
In some embodiments of the present invention, the compound of the present invention is selected from the following: 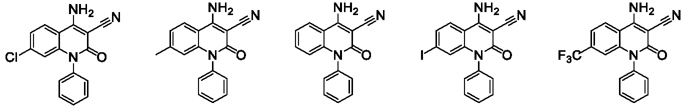 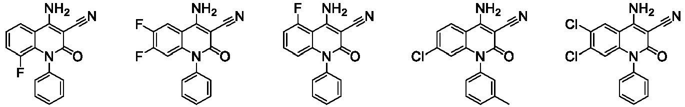 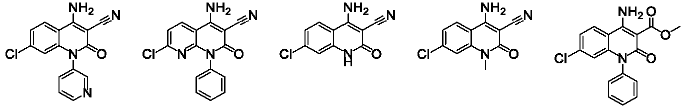 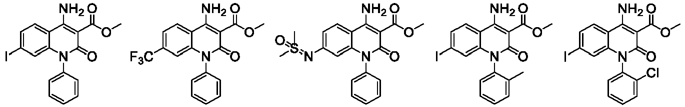 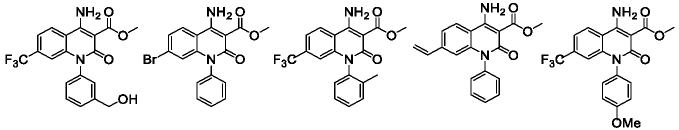 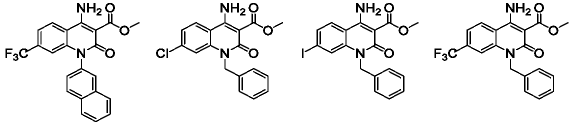 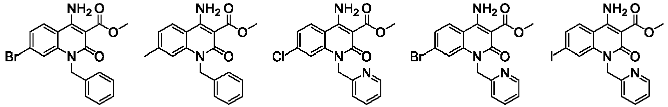 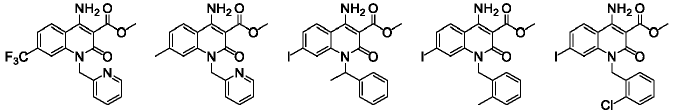 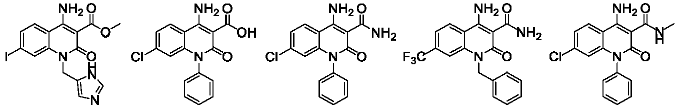 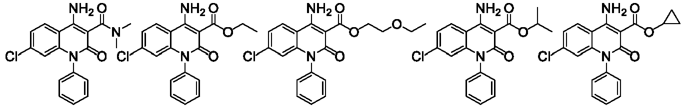 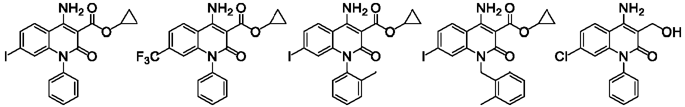 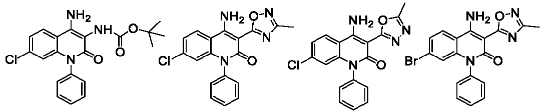 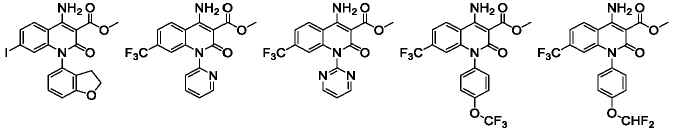 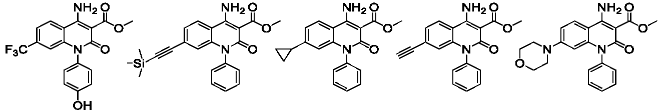 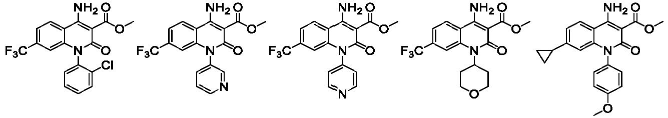 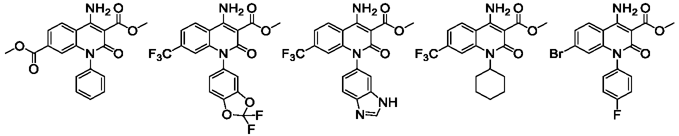 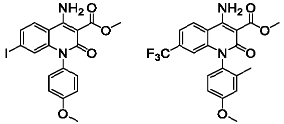 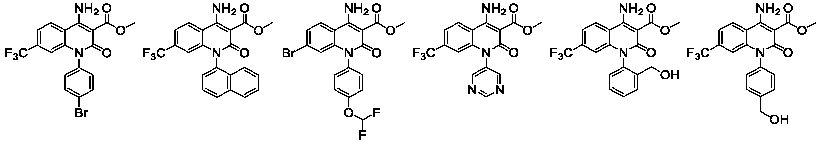 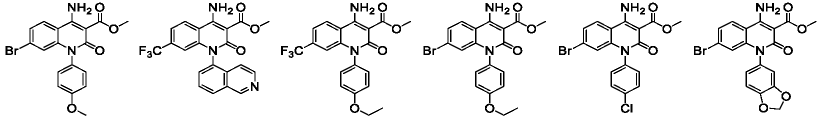 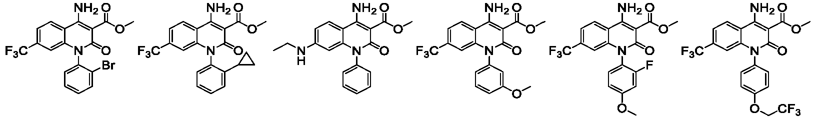 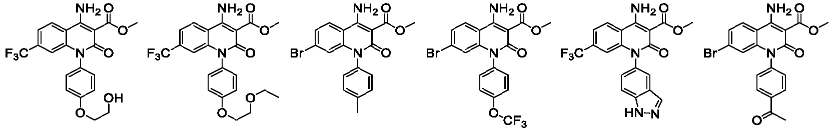 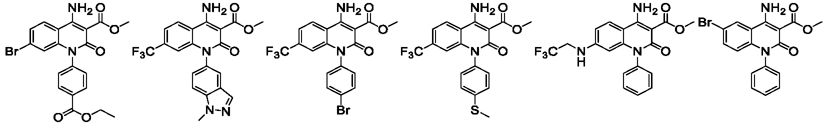 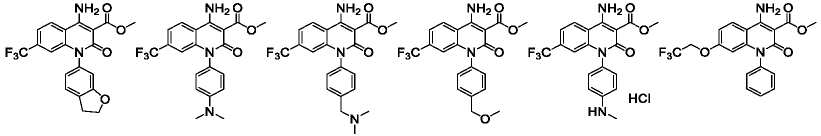 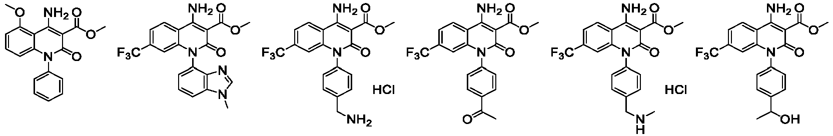 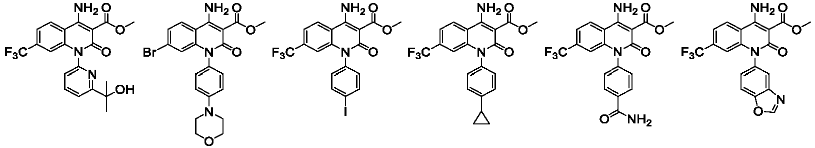 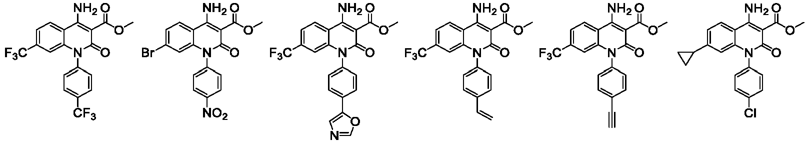 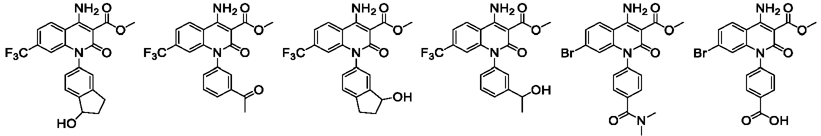 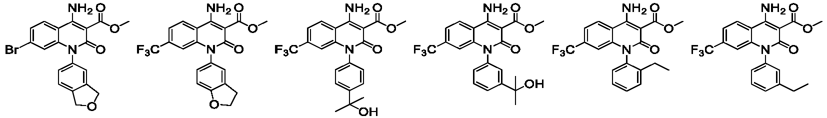 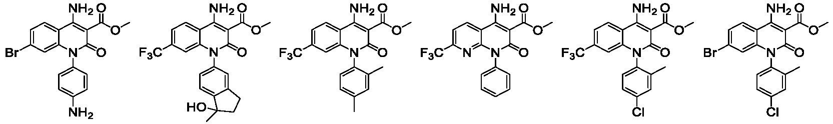 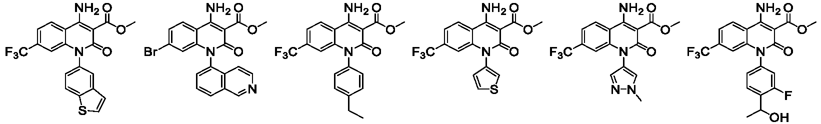 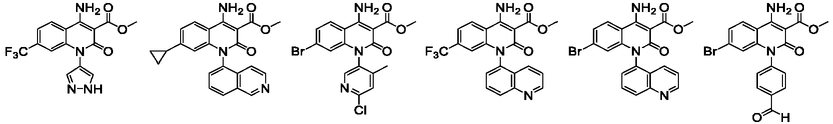 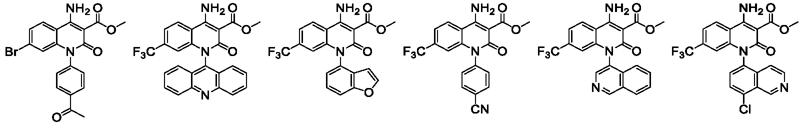 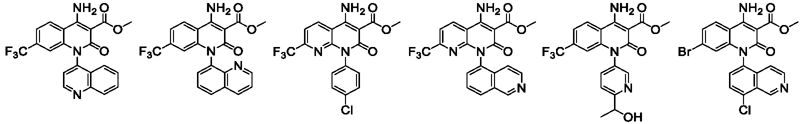 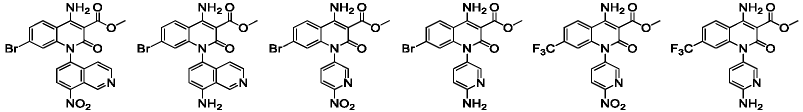 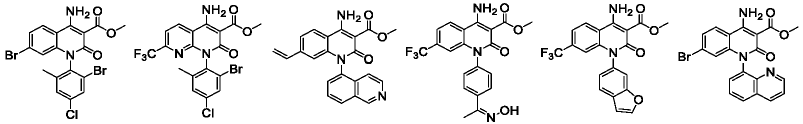 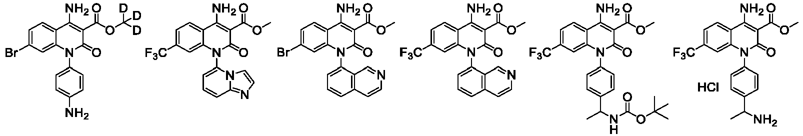 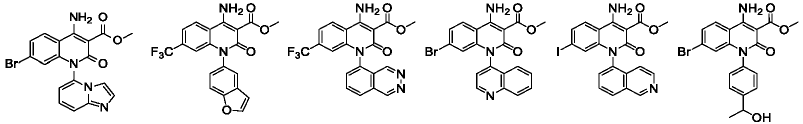 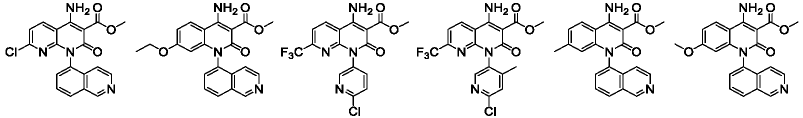 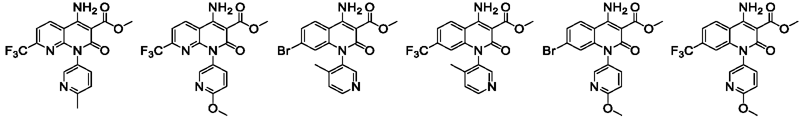 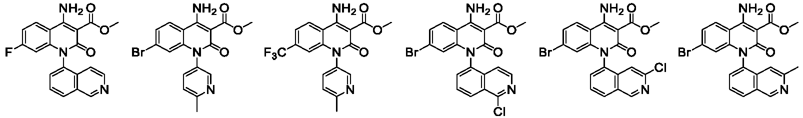 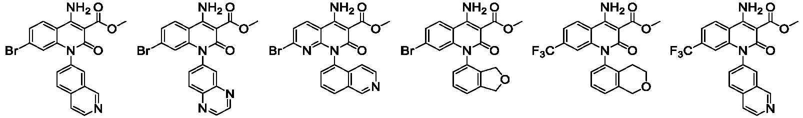 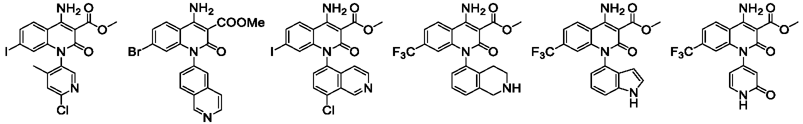 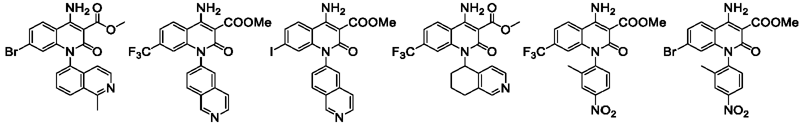 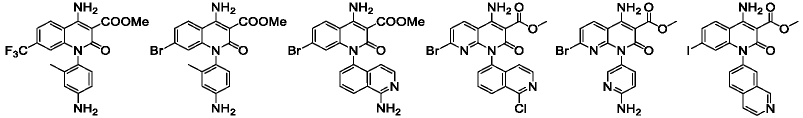 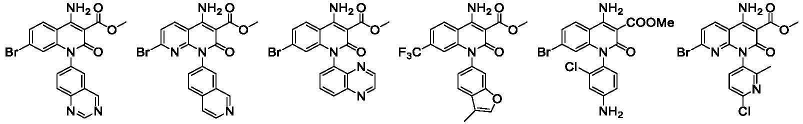 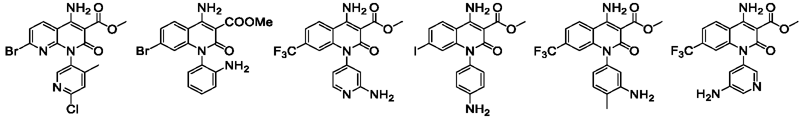 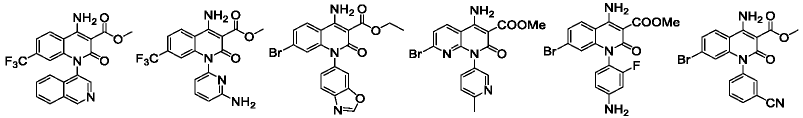 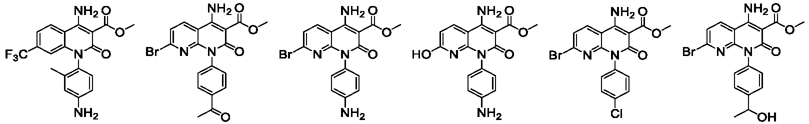 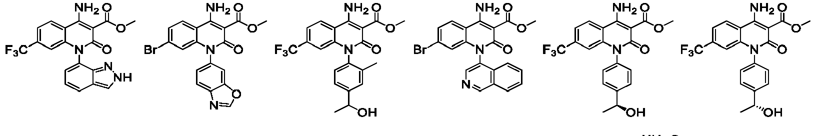 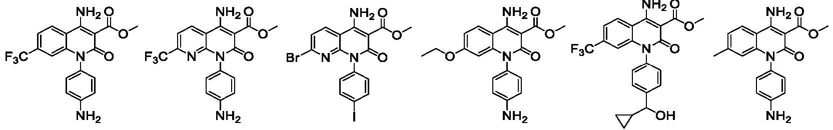 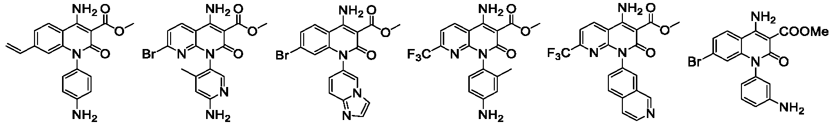 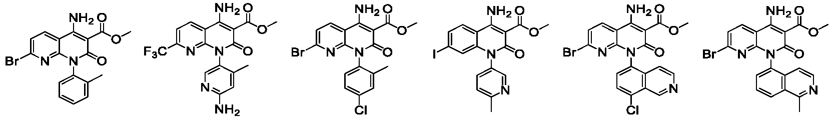 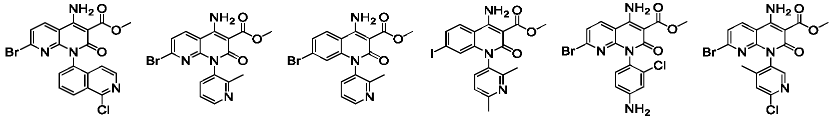 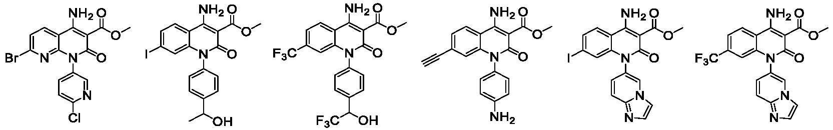 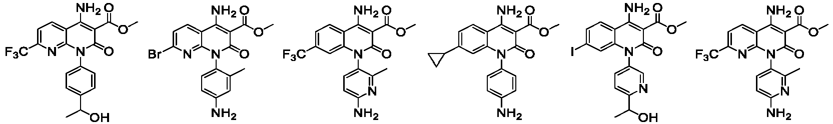 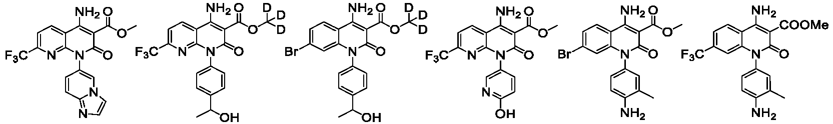 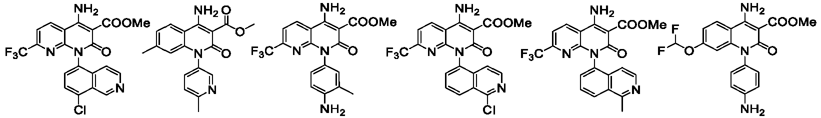 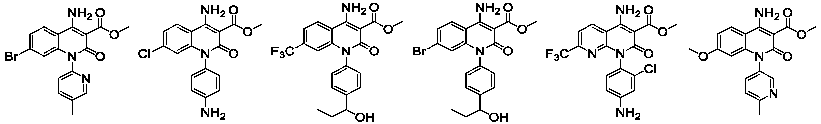 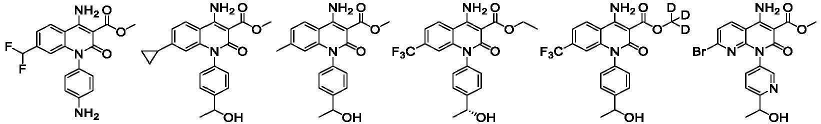 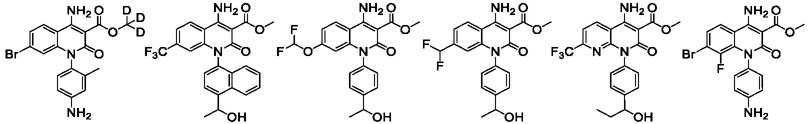 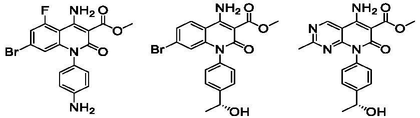 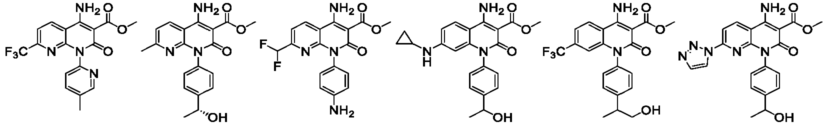 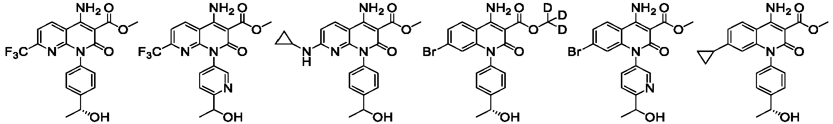 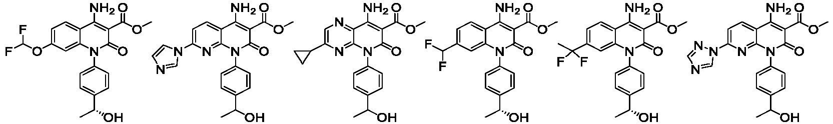 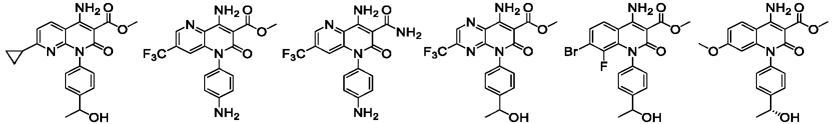 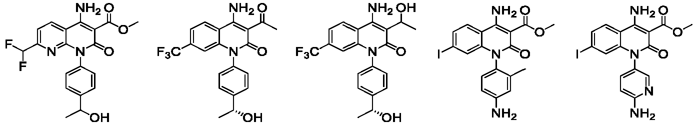 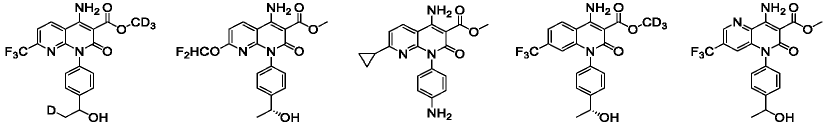 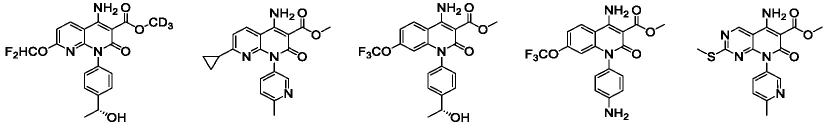 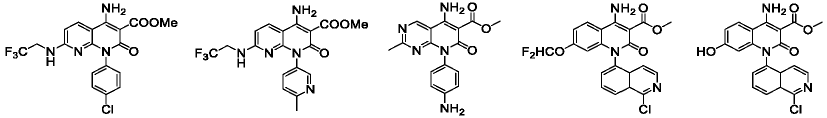 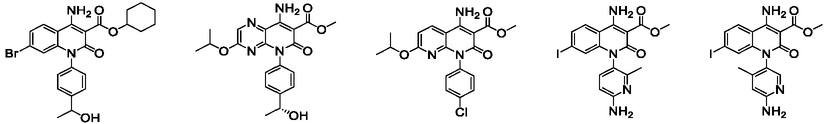 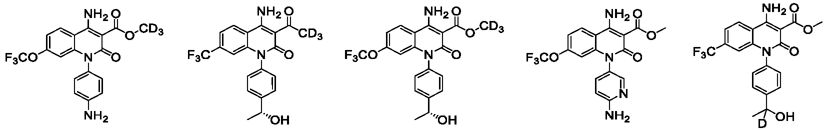 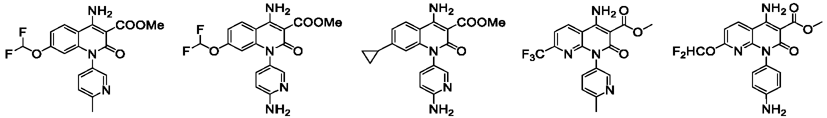 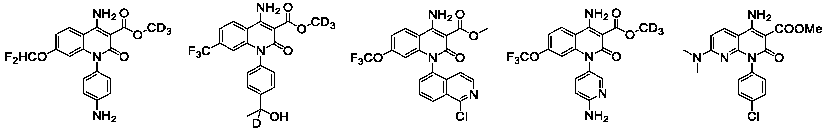 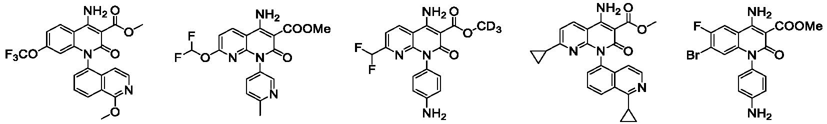 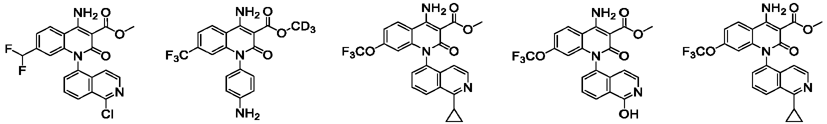 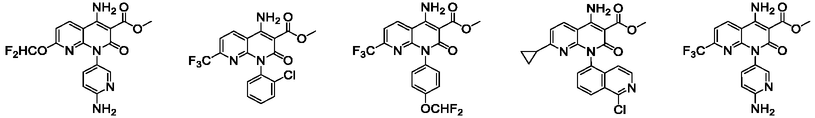 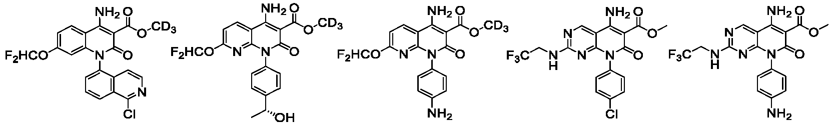 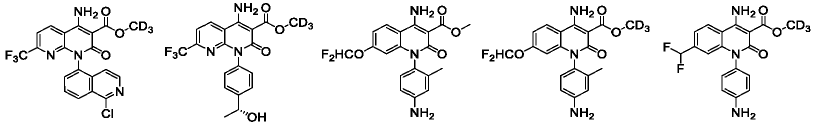 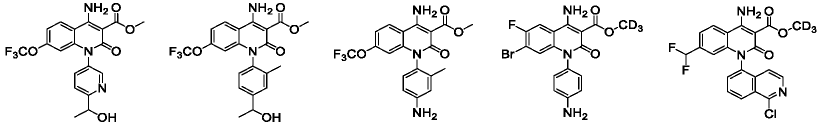 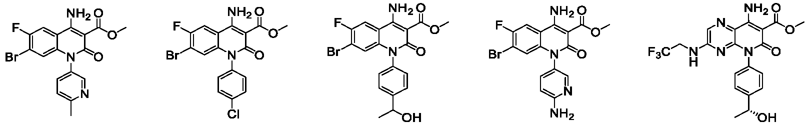 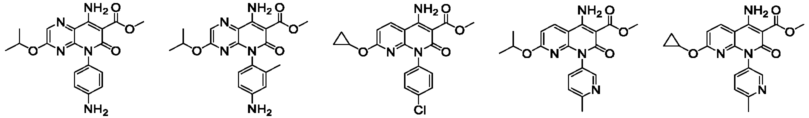 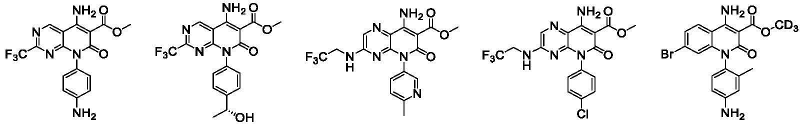 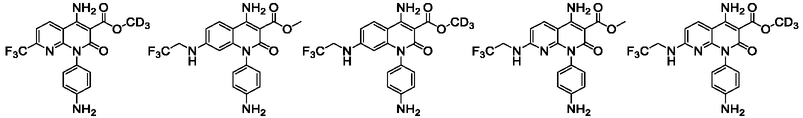 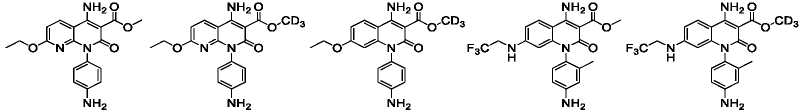 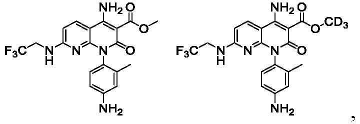 or a pharmaceutically acceptable salt, solvate, polymorph or tautomer thereof.

In another aspect, the present invention provides a pharmaceutical composition comprising the compound of Formula (I) or a pharmaceutically acceptable salt, solvate, polymorph or tautomer thereof. In some embodiments, the pharmaceutical composition of the present invention also comprise a pharmaceutically acceptable excipient.

In another aspect, the present invention provides a method of treating a disease related to MAT2A comprising administering to a subject an effective amount of a compound of Formula (I) or a pharmaceutically acceptable salt, solvate, polymorph or tautomer thereof, or a pharmaceutical composition thereof;

In another aspect, the present invention provides a use of a compound of Formula (I) or a pharmaceutically acceptable salt, solvate, polymorph or tautomer thereof, or a pharmaceutical composition thereof in the manufacture of a medicament for the treatment of a disease related to MAT2A.

In some embodiments of the present invention, the disease related to MAT2A is tumor, preferably solid tumor, more preferably non-small cell lung cancer, esophageal cancer, pancreatic cancer, or bladder cancer.

### Detailed Description of the Invention

Exemplary embodiments utilizing the principles of the present invention are set forth in the detailed description that follows. The features and advantages of the present invention may be better understood with reference to the following summary of the invention.

It is understood that the scope of the various aspects of the invention is to be determined by the following claims and that methods and structures within the scope of these claims and their equivalents are to be embraced therein.

Unless defined otherwise, all technical and scientific terms used herein have the same meaning as commonly understood by one of ordinary skill in the art to which the claimed subject matter belongs. All patents, patent applications, and publications cited herein are incorporated by reference in their entirety unless otherwise indicated.

It is to be understood that both the foregoing general description and the following detailed description are exemplary and explanatory and are not restrictive of any inventive subject matter. The use of the singular also includes the plural unless specifically stated otherwise. The use of "or", and "alternatively" means "and/or" unless stated otherwise. Furthermore, the use of the term "comprise" as well as other forms, such as "include", "have", and "contain", is not limiting.

### Certain chemical terms

The terms "optional", "optional" or "optionally" mean that the subsequently described event or circumstance may or may not occur and that the description includes instances where said event or circumstance occurs and instances in which it does not. For example, "optionally substituted alkyl" means "unsubstituted alkyl" or "substituted alkyl". Also, optionally substituted groups may be unsubstituted (e.g. -CH₂CH₃), fully substituted (e.g. -CF₂CF₃), monosubstituted (e.g. -CH₂CH₂F), or any level between mono-and fully substituted (e.g. -CH₂CHF₂, -CF₂CH₃, -CFHCHF₂, and the like). It will be understood by those skilled in the art that for any group containing one or more substituents, no substitution or substitution pattern is introduced that is sterically impossible and/or synthetically non-feasible.

Unless otherwise indicated, conventional methods within the skill of the art are employed, such as, for example, mass spectrometry, nuclear magnetic resonance, high-performance liquid chromatography, infrared and ultraviolet/visible spectroscopy, and pharmacological methods. Unless specific definitions are set forth, the nomenclatures used in connection with, and the laboratory procedures and techniques of, analytical chemistry, organic synthetic chemistry, and pharmaceutical and medicinal chemistry described herein are those known in the art. Standard techniques can be used in chemical syntheses, chemical analyses, pharmaceutical preparation, formulation, delivery, and treatment of patients. For example, the reaction and purification can be carried out using the manufacturer's instructions for the use of the kit, or in a manner known in the art or the present invention. The techniques and methods described above can generally be performed according to conventional methods well known in the art, in light of the description in the numerous general and more specific documents cited and discussed in this specification. In this specification, groups and substituents thereof may be selected by one skilled in the art to provide stable moieties and compounds.

When a substituent is described by a conventional chemical formula written from left to right, the substituent also includes chemically equivalent substituents that result when the structural formula is written from right to left. For example, -CH₂O-is equivalent to-OCH₂-.

As used herein, the terms "group" and "chemical group" refer to a particular moiety or functional group of a molecule. Chemical groups are often considered chemical entities embedded in or appended to a molecule.

Some of the chemical groups named herein may represent the total number of carbon atoms with a shorthand notation. For example, C₁₋₆ alkyl describes an alkyl group, as defined below, having a total of from 1 to 6 carbon atoms. The total number of carbon atoms indicated by the shorthand notation does not include carbon atoms on possible substituents.

The term "halogen", "halo" or "halide" refers to bromine, chlorine, fluorine, or iodine.

The compounds of the invention may contain one or more (e.g. one, two, three, or four) isotopic substitutions. For example, in the compounds, H may be in any isotopic form, including ¹H, ²H (D or deuterium), and ³H (T or tritium); C may be in any isotopic form, including ¹²C, ¹³C, and ¹⁴C; O can be in any isotopic form, including ¹⁶O and ¹⁸O, and the like.

The terms "aroma", "aromatic ring", "aromatic", "aromaticity", and "aromatic cyclic", as used herein, refer to a planar ring or ring portion of a ring having a delocalized electron conjugate system containing 4n + 2 electrons, where n is an integer. The aromatic ring may be formed from 5, 6, 7, 8, 9, or more than 9 atoms. Aromatic compounds may be optionally substituted and may be monocyclic or fused-ring polycyclic.

The term "heteroatom" or "hetero" as used herein alone or as part of another component refers to an atom other than carbon and hydrogen. Heteroatoms are independently selected from the group consisting of oxygen, nitrogen, sulfur, phosphorus, silicon, selenium, and tin, but are not limited to these atoms. In embodiments where two or more heteroatoms are present, the two or more heteroatoms may be the same as each other, or some or all of the two or more heteroatoms may be different from each other.

The term "fused" or "fused ring" as used herein, alone or in combination, refers to a cyclic structure in which two or more rings share one or more bonds.

The term "spiro" or "spirocycle" as used herein, alone or in combination, refers to a cyclic structure in which two or more rings share one or more atoms.

The term "alkyl" as used herein, alone or in combination, refers to an optionally substituted straight-chain or optionally substituted branched-chain monovalent saturated hydrocarbon having 1 to 12 carbon atoms, preferably 1 to 8 carbon atoms, more preferably 1 to 6 carbon atoms, attached to the rest of the molecule by a single bond, e.g. methyl, ethyl, n-propyl, isopropyl, n-butyl, isobutyl, sec-butyl, tert-butyl, n-pentyl, n-hexyl, n-heptyl, 2-methylhexyl, 3-methylhexyl, n-octyl, n-nonyl, n-decyl and the like.

The term "alkenyl" as used herein, alone or in combination, refers to an optionally substituted straight-chain or optionally substituted branched-chain monovalent hydrocarbon radical having one or more C=C double bonds and having from 2 to about 10 carbon atoms, more preferably from 2 to about 6 carbon atoms. The double bonds in these groups may be in either the cis or trans configuration and are understood to encompass both isomers. Examples include but are not limited to, ethenyl (CH=CH₂), 1-propenyl (CH₂CH=CH₂), isopropenyl (C(CH₃)=CH₂), butenyl, 1,3-butadienyl, and the like. Where a numerical range of alkenyl as defined herein appears, e.g. "C₂-C₆ alkenyl" or "C₂₋₆ alkenyl" refers to an alkenyl group that may consist of 2 carbon atoms, 3 carbon atoms, 4 carbon atoms, 5 carbon atoms, or 6 carbon atoms, where no numerical range is designated.

The term "alkynyl" as used herein, alone or in combination, refers to an optionally substituted straight or branched chain monovalent hydrocarbon radical having one or more C≡C triple bonds and having from 2 to about 10 carbon atoms, more preferably from 2 to about 6 carbon atoms. Examples include but are not limited to, ethynyl, 2-propynyl, 2-butynyl, 1,3-butadiynyl, and the like. Whenever there is a numerical range for an alkynyl group as defined herein, e.g. "C₂-C₆ alkynyl" or "C₂₋₆ alkynyl", it is intended that the alkynyl group may consist of 2 carbon atoms, 3 carbon atoms, 4 carbon atoms, 5 carbon atoms or 6 carbon atoms, and it is intended that the alkynyl group herein also covers instances where no numerical range is designated.

The term "aryl" refers to an all-carbon monocyclic or fused ring having a fully conjugated pi-electron system having 6 to 14 carbon atoms, preferably 6 to 12 carbon atoms, most preferably 6 carbon atoms. Aryl groups can be unsubstituted or substituted with one or more substituents, examples of which include, but are not limited to, alkyl, alkyloxy, aryl, aralkyl, amino, halo, hydroxy, sulfonyl, sulfinyl, phosphoryl, and heteroalicyclyl. Non-limiting examples of unsubstituted aryl groups include but are not limited to, phenyl, naphthyl, and anthracenyl.

The term "heteroaryl" or "heteroaromatic ring" refers to a monocyclic or fused ring of 5 to 12 ring atoms having 5, 6, 7, 8, 9, 10, 11, or 12 ring atoms containing 1, 2, 3, or 4 ring atoms selected from N, O, S, the remaining ring atoms being C, and having a fully conjugated pi-electron system. The heteroaryl or heteroaromatic rings may be unsubstituted or substituted, including, but not limited to, alkyl, alkyloxy, aryl, aralkyl, amino, halogen, hydroxy, cyano, nitro, carbonyl, and heteroalicyclyl. Non-limiting examples of unsubstituted heteroaryl groups include but are not limited to, pyrrolyl, furanyl, thienyl, imidazolyl, oxazolyl, pyrazolyl, pyridinyl, pyrimidinyl, pyrazinyl, quinolinyl, isoquinolinyl, tetrazolyl, triazinyl.

The term "cycloalkyl", as used herein, alone or in combination, refers to an optionally substituted monovalent saturated hydrocarbon ring containing from 3 to about 15 ring-forming carbon atoms or from 3 to about 10 ring-forming carbon atoms and may also include other non-ring-forming carbon atoms as substituents (e.g. methylcyclopropyl).

The term "carbocycle" refers to a structure covalently closed by carbon, which may be saturated or partially unsaturated. Carbocycles may be formed from 3, 4, 5, 6, 7, 8, 9, or more than 9 atoms. The term carbocycle differs from heterocycle in that the ring backbone of the heterocycle comprises at least one atom different from carbon. As used herein, a "carbocycle" may be a single ring or multiple rings including spiro rings, fused rings, and bridged rings. Carbocycles may be optionally substituted. A "carbocycle" herein preferably comprises about 5 to about 20, or 5 to 10, or 5-8, or 5-6 skeletal ring-forming atoms.

The terms "heterocycle", "heterocycloalkyl", as used herein, alone or in combination, refer to an aliphatic heterocycle that may be saturated or partially unsaturated. Where the number of carbon atoms in a heterocycle is indicated herein (e.g. a C₃₋₆ heterocycle), at least one non-carbon atom (heteroatom) must be present in the ring. For example, the designation "C₃₋₆ heterocycle" refers only to the number of carbon atoms in the ring and does not refer to the total number of atoms in the ring. A designation such as "4-6 membered heterocycle" refers to the total number of atoms contained in the ring (i.e. a four, five, or six-membered ring wherein at least one atom is a carbon atom, at least one atom is a heteroatom, and the remaining 2-4 atoms are carbon atoms or heteroatoms). For heterocycles having two or more heteroatoms, the two or more heteroatoms can be the same or different from each other. As used herein, a "heterocycle" may be a single ring or multiple rings including spiro rings, fused rings, and bridged rings. Heterocycles may be optionally substituted. "heterocycle" herein preferably comprises about 5 to about 20, or 5 to 10, or 5-8, or 5-6 skeletal ring-forming atoms.

As used herein, the term "polymorph" or "polymorphism" means that the compounds of the present invention have multiple crystal lattice morphologies. Some of the compounds of the invention may have more than one crystalline form, and the invention encompasses all polymorphs or mixtures thereof.

Intermediate compounds of the compounds of the invention and polymorphs thereof are also within the scope of the invention.

Unless otherwise specified, olefinic double bonds contained in the compounds of the present invention include E and Z isomers.

It will be appreciated that the compounds of the present invention may contain asymmetric centers. These asymmetric centers may independently be in the R or S configuration. It will be apparent to those skilled in the art that some of the compounds of the present invention may also exhibit cis-trans isomerism. It will be understood that the compounds of the present invention include their individual geometric isomers and stereoisomers as well as mixtures thereof, including racemic mixtures. These isomers can be separated from their mixtures by the implementation or modification of known methods, for example, chromatographic techniques and recrystallization techniques, or they can be prepared separately from the appropriate isomers of their intermediates.

As used herein, the term "pharmaceutically acceptable salts" includes both acid and base addition salts.

"Pharmaceutically acceptable acid addition salt" refers to those salts which retain the biological effectiveness and properties of the free bases of the compounds and which are not biologically or otherwise undesirable, formed with inorganic acids such as, but not limited to, hydrochloric acid, hydrobromic acid, sulfuric acid, nitric acid, phosphoric acid and the like, or organic acids such as, but not limited to, acetic acid, 2,2-dichloroacetic acid, adipic acid, alginic acid, ascorbic acid, aspartic acid, benzenesulfonic acid, benzoic acid, decanoic acid, hexanoic acid, carbonic acid, cinnamic acid, citric acid and the like. "Pharmaceutically acceptable base salt" refers to those salts that retain the biological effectiveness and properties of the free acids of the compounds and which are not biologically or otherwise undesirable. These salts are prepared by reacting the free acid with an inorganic or organic base. Salts generated by reactions with inorganic bases include but are not limited to, sodium, potassium, lithium, ammonium, calcium, magnesium, iron, zinc, copper, manganese, aluminum salts, and the like. Preferred inorganic salts are ammonium, sodium, potassium, calcium, and manganese salts.

Salts forming organic bases include but are not limited to, primary, secondary, tertiary, cyclic amines and the like, such as ammonia, isopropylamine, trimethylamine, diethylamine, triethylamine, tripropylamine, ethanolamine, diethanolamine, ethanolamine, dicyclohexylamine, ethylenediamine, purine, piperazine, piperidine, choline, caffeine and the like. Particularly preferred organic bases are isopropylamine, diethylamine, ethanolamine, trimethylamine, dicyclohexylamine, choline, and caffeine.

Often crystallizations produce solvates of the compounds of the invention. As used herein, the term "solvate" refers to an assembly of one or more molecules of a compound of the invention with one or more molecules of a solvent.

The solvent may be water, in which case the solvate is a hydrate. Also, organic solvents are possible. Thus, the compounds of the present invention may exist as a hydrate, including a monohydrate, dihydrate, hemihydrate, trihydrate, tetrahydrate, and the like, as well as the corresponding solvated forms. The compounds of the present invention may be true solvates, but in other cases the compounds of the present invention may only occasionally retain water or a mixture of water with some other solvent. The compounds of the present invention can be reacted in a solvent or precipitated or crystallized in a solvent. Solvates of the compounds of the present invention are also included within the scope of the present invention.

As used herein, the term "pharmaceutical composition" refers to a formulation incorporating a compound of the present invention and a medium generally accepted in the art for the delivery of biologically active compounds to mammals, such as humans. Such media include all pharmaceutically acceptable carriers.

The term "acceptable" with respect to a formulation, composition, or ingredient, as used herein, means having no persistent detrimental effect on the general health of the main body being treated.

The term "pharmaceutically acceptable", as used herein, refers to a material (e.g. a carrier or diluent) that does not interfere with the biological activity or properties of the compounds of this invention and is relatively nontoxic, i.e. the material may be administered to an individual without causing undesirable biological reactions or interacting in an undesirable manner with any of the components of the composition in which it is contained.

"Pharmaceutically acceptable carrier" includes, but is not limited to, adjuvants, carriers, excipients, adjuvants, deodorants, diluents, preservatives, dyes/colorants, flavor enhancers, surfactants and wetting agents, dispersants, suspending agents, stabilizers, isotonic agents, solvents, or emulsifiers that have been approved by the relevant governmental agency to be used with humans and domestic animals.

The terms "main body", "patient", "subject", or "individual" refer to an individual suffering from a disease, disorder, or condition, and the like, including mammals and non-mammals. Examples of mammals include but are not limited to, any member of the Mammalian class: humans, non-human primates (e.g. chimpanzees and other apes and monkeys); livestock such as cattle, horses, sheep, goats, and swine; domestic animals such as rabbits, dogs and cats; laboratory animals, including rodents, such as rats, mice and guinea pigs, and the like. Examples of non-human mammals include but are not limited to, birds, fish, and the like. In one embodiment of the methods and compositions provided herein, the mammal is a human.

The term "treatment" refers to the treatment of a disease or condition associated with a mammal, particularly a human, including
(i) preventing a mammal, particularly a mammal that has previously been exposed to a disease or condition but has not yet been diagnosed as having it, from developing the corresponding disease or condition;
(ii) inhibiting the disease or disorder, i.e. controlling its development;
(iii) relieving the disease or condition, i.e. causing regression of the disease or condition;
(iv) alleviating the symptoms caused by the disease or disorder.

The terms "disease" and "condition" may be used interchangeably or in a different sense, because certain specific diseases or conditions do not have a known causative agent (so the cause of the disease is unknown), and therefore cannot be considered as diseases but only as unwanted conditions or syndromes, more or less specific symptoms of which have been confirmed by clinical investigators.

The terms "effective amount", "therapeutically effective amount", or "pharmaceutically effective amount" refer to an amount of at least one agent or compound that, upon administration, is sufficient to relieve to some extent one or more of the symptoms of the disease or condition being treated. The result may be reduction and/or alleviation of the signs, symptoms, or causes of a disease, or any other desired alteration of a biological system. For example, an "effective amount" for treatment is the amount of the composition comprising a compound disclosed herein required to provide a clinically significant condition-alleviating effect. An effective amount in any individual case may be determined using techniques, such as a dose escalation assay.

The terms "administrate", "administrating", "administration", and the like refer to a method capable of delivering a compound or composition to a desired site for biological action. These methods include but are not limited to, oral routes, duodenal routes, parenteral injection (including intravenous, subcutaneous, intraperitoneal, intramuscular, intraarterial injection or infusion), topical administration, and rectal administration. In preferred embodiments, the compounds and compositions discussed herein are administered orally.

The anti-cancer treatments described herein may be useful as monotherapy or may involve, in addition to the administration of a compound of Formula (I), conventional surgery, radiotherapy, or chemotherapy; or a combination of such additional therapies. Such conventional surgery, radiotherapy, or chemotherapy may be administered simultaneously, sequentially, or separately with the compound of formula (I) for the treatment.

### Preparation of Compounds of the Invention

The following reaction schemes illustrate methods for preparing compounds of the invention.

It is to be understood that in the following description, combinations of substituent groups and/or variables of the depicted formulae are permissible only if stable compounds are formed.

It will also be appreciated by those skilled in the art that in the schemes described below, functional groups of intermediate compounds may need to be protected by suitable protecting groups. These functional groups include hydroxyl, amino, sulfhydryl, and carboxyl groups. Suitable hydroxy protecting groups include trialkylsilyl or diarylalkylsilyl (e.g. tert-butylmethylsilyl, tert-butyldiphenylsilyl or trimethylsilyl), tetrahydropyranyl, benzyl and the like. Suitable amino, amidino, and guanidine-protecting groups include tert-butoxycarbonyl, benzyloxycarbonyl, and the like. Suitable protecting groups for mercapto include -C(O)-R" (R" represents alkyl, aryl or arylalkyl), p-methoxybenzyl, trityl, and the like. Suitable carboxy protecting groups include alkyl, aryl, or arylalkyl esters. Protecting groups may be added or removed by standard techniques known to those skilled in the art.

### Examples

The following non-limiting examples are merely illustrative and do not limit the invention in any way.

Unless otherwise indicated, temperatures are in degrees Celsius. Reagents were purchased from commercial suppliers such as Sinopharm Chemical Reagents Beijing Co. Ltd., Alfa Aesar, or J&K Scientific Ltd. and were used as received without further purification unless otherwise noted.

Unless otherwise stated, the following reactions were carried out at room temperature in anhydrous solvents, under a positive pressure of nitrogen or argon, or using a drying tube; the reaction flask was fitted with a rubber septum to allow the addition of substrates and reagents via syringe; glassware was oven dried and/or heat dried.

Unless otherwise stated, 200-300 mesh silica gel from Qingdao Marine Chemical Plant was used for column chromatography purification; Preparative thin-layer chromatography Silica gel precast plates (HSGF254) produced by Yantai Chemical Industry Research Institute were used for preparative thin-layer chromatography; MS was determined by Thermo LCQ Fleet type (ESI) liquid chromatography-mass spectrometry; SGW-3 automatic polarimeter and Shanghai Shenguang Instrument and Meter Co. Ltd. were used for polarimetry.

Nuclear magnetic data (¹H NMR) were run on a Varian instrument at 400 MHz. The solvents used for NMR data were CDCl₃, CD₃OD, D₂O, DMSO-d6, etc. based on tetramethylsilane (0.00 ppm) or residual solvent (CDCl₃: 7.26 ppm; CD₃OD: 3.31 ppm; D₂O: 4.79 ppm; d6- DMSO: 2.50 ppm). When peak form diversity is indicated, the following abbreviations denote different peak forms: S (singlet), d (doublet), t (triplet), q (quartet), m (multiplet), br (broadened), dd (doublet of doublets), dt (doublet of triplets). Coupling constants are given in Hertz (Hz).

### Synthetic method

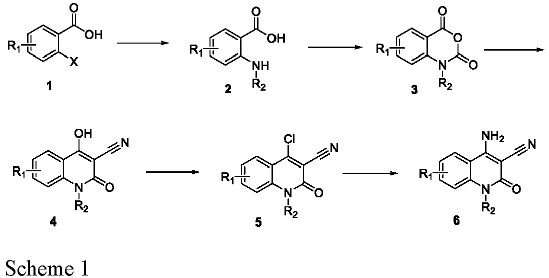

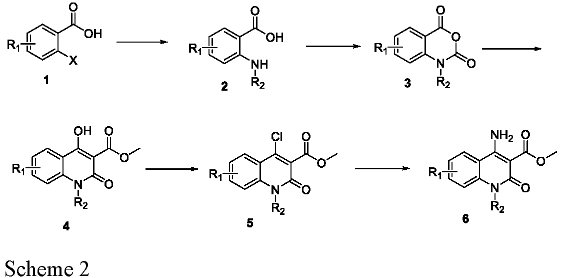

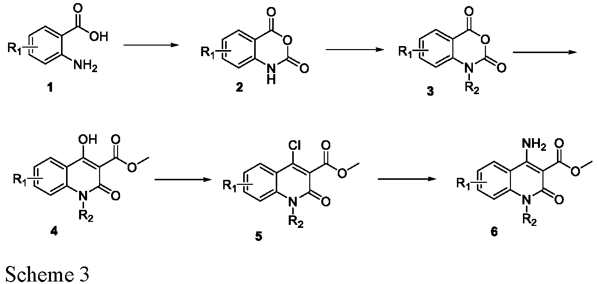

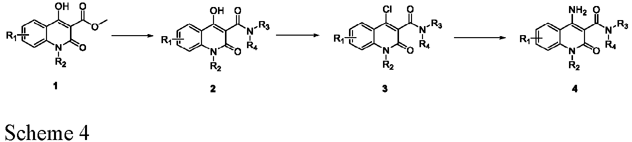

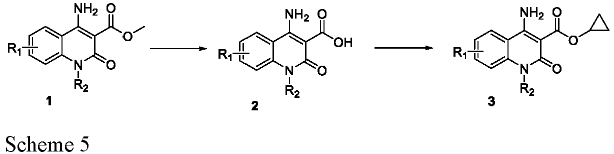

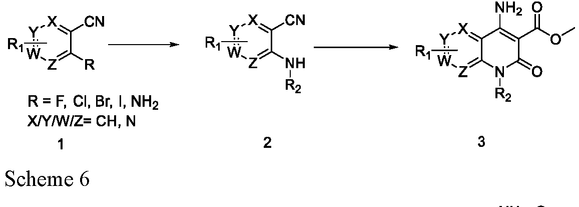

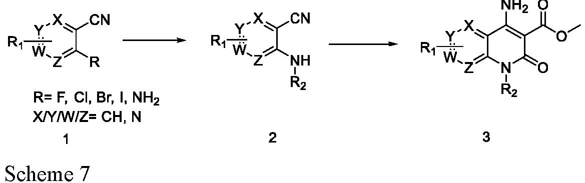

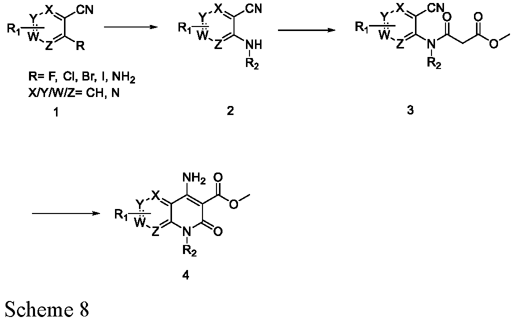

The present examples can be prepared according to the routes described in Schemes 1-8. Examples 1-14 can be prepared according to the route outlined in Scheme 1, examples 15, 16, 17, 19, 20, 22, 23, 25, and 26 can be prepared according to the route outlined in Scheme 2, examples 27-40 can be prepared according to the route outlined in Scheme 3, examples 44 and 45 can be prepared according to the route outlined in Scheme 4, and Examples 49-53 can be prepared according to the route outlined in Scheme 5.

In Scheme 1, starting material 1 is subjected to a Ullmann reaction with an amine in the presence of a copper catalyst and a suitable base to generate intermediate 2; intermediate 2 is reacted with triphosgene to generate intermediate 3; intermediate 3 is reacted with ethyl cyanoacetate in the presence of a suitable base to generate intermediate 4; the intermediate 4 is chlorinated with phosphorus oxychloride to generate intermediate 5; intermediate 5 is substituted with ammonia methanol solution to generate product 6.

In Scheme 2, starting material 1 is subjected to a Ullmann reaction with an amine in the presence of a copper catalyst and a suitable base to generate intermediate 2; intermediate 2 is reacted with triphosgene to generate intermediate 3; intermediate 3 is reacted with dimethyl malonate in the presence of a suitable base to generate intermediate 4; the intermediate 4 is chlorinated with phosphorus oxychloride to generate intermediate 5; intermediate 5 is substituted with ammonia methanol solution to generate product 6.

In Scheme 3, starting material 1 is reacted with triphosgene to generate intermediate 2; intermediate 2 is subjected to a Mitsunobu reaction with an alcohol to generate intermediate 3; intermediate 3 is reacted with dimethyl malonate in the presence of a suitable base to generate intermediate 4; the intermediate 4 is chlorinated with phosphorus oxychloride to generate intermediate 5; intermediate 5 is substituted with ammonia methanol solution to generate product 6.

In Scheme 4, starting material 1 is subjected to an aminolysis reaction with an amine to generate intermediate 2; the intermediate 2 is chlorinated with phosphorus oxychloride to generate intermediate 3; intermediate 3 is substituted with ammonia methanol solution to generate product 4.

In Scheme 5, starting material 1 is subjected to a hydrolysis reaction in the presence of a suitable base to generate intermediate 2; Condensation of intermediate 2 with cyclopropanol gives product 3.

In Scheme 6, starting material 1 is subjected to a coupling reaction with a halide or an amine in the presence of a palladium catalyst, a suitable ligand, and a suitable base to generate intermediate 2; intermediate 2 is subjected to tandem cyclization with dimethyl malonate under the action of the base to generate product 3.

In Scheme 7, starting material 1 is subjected to a substitution reaction with an amine or halide in the presence of a suitable base to generate intermediate 2; intermediate 2 is subjected to tandem cyclization with dimethyl malonate under the action of the base to generate product 3.

In Scheme 8, starting material 1 is subjected to a substitution reaction with an amine or halide in the presence of a suitable base to generate intermediate 2; intermediate 2 is subjected to substitution with methyl 3-chloro-3-oxopropanoate under the action of a base to generate intermediate 3; the intermediate 3 is subjected to a tandem cyclization reaction under the action of a base to generate product 4.

### Example 1. 4-atnino-7-chloro-2-oxo-1-phenyl-1,2-dihydroquinolin- 3- carbonitrile

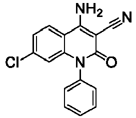

According to the preparation route of scheme 1, the specific synthetic route is as follow:

### Step 1: 4-chloro-2-(phenylamino)benzoic acid (Intermediate 1-2)

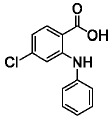

Under nitrogen protection, 2,4-dichlorobenzoic acid (1.90 g), copper powder (64 mg), copper(I) oxide (72 mg), and potassium carbonate (1.66 g) were added into a three-neck flask, to the mixture was added *N*,*N*-dimethylformamide (50 mL), the mixture was stirred at room temperature for 30 minutes, then a solution of aniline (1.12 g) in *N*,*N*-dimethylformamide (5 mL) was slowly added, and the mixture was heated to 130 °C and stirred for 12 hours. The reaction solution was cooled to room temperature and filtered, to the filtrate were added 3 mol/L hydrochloric acid (10 mL) and water (100 mL), the mixture was extracted with dichloromethane (100 mL*3), the extract was dried over anhydrous sodium sulphate and filtered, the filtrate was concentrated under reduced pressure, and the residue was purified through column chromatography (petroleum ether/ethyl acetate = 5:1) to obtain the target compound 1-2 (1.24 g).

### Step 2: 7-chloro-1-phenyl-2,4-dihydro-1H-3,1-benzo-oxazine-2,4-dione (Intermediate 1-3)

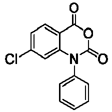

While being cooled by an ice-bath, Intermediate **1-2** (1.24 g) was dissolved in dichloromethane (30 mL), *N*,*N*-diisopropylethylamine (1.29 g) was added, triphosgene (1.78 g) was slowly added, and the mixture was stirred for 30 minutes while being cooled by an ice-bath, then stirred at room temperature for 6 hours. The reaction solution was concentrated under reduced pressure, and the residue was purified through column chromatography (eluted by dichloromethane) to obtain the target compound **1-3** (1.23 g).

### Step 3: 7-chloro-4-hydroxy-2-oxo-1-phenyl-1,2-dihydroquinolin-3-carbonitrile (Intermediate 1-4)

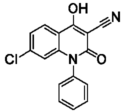

While being cooled by an ice-bath, Intermediate **1-3** (1.23 g) and cyano ethyl acetate (945 mg) were dissolved in dry *N,N-*dimethylformamide (20 mL), sodium hydride (540 mg) was slowly added, the temperature rised to room temperature and the mixture was stirred overnight. To the reaction solution were added 3 mol/L hydrochloric acid (10 mL) and water (40 mL), the mixture was filtered, and the filter cake was washed with water (30 mL*3) to obtain the target compound **1-4** (912 mg).

### Step 4: 4,7-dichloro-2-oxo-1-phenyl-1,2-dihydroquinolin-3-carbonitrile (Intermediate 1-5)

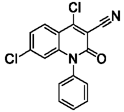

To Intermediate **1-4** (900 mg) was added phosphoryl trichloride (20 mL), and the mixture was stirred at 100 °C for 12 hours. The reaction solution was concentrated under reduced pressure, to the residue was added an aqueous solution of saturated sodium bicarbonate (30 mL), the mixture was extracted with dichloromethane (100 mL*3), the extract was dried over anhydrous sodium sulphate and filtered, the filtrate was concentrated under reduced pressure, and the residue was purified through column chromatography (dichloromethane/methanol = 20:1) to obtain the target compound **1-5** (858 mg).

### Step 5: 4-amino-7-chloro-2-oxo-1-phenyl-1,2-dihydroquinolin-3-carbonitrile (Example 1)

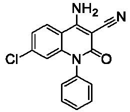

To Intermediate **1-5** (850 mg) were added methanol (20 mL) and 7 mol/L ammonia methanol solution (10 mL), and the mixture was heated to 50 °C and stirred for 12 hours. The reaction solution was concentrated under reduced pressure, and the residue was purified through column chromatography (dichloromethane/methanol = 10:1) to obtain the product (704 mg).

¹H NMR (400 MHz, DMSO-*d₆*) δ 8.27 (d, *J=* 8.8 Hz, 1H), 7.95-8.26 (brs, 2H), 7.57-7.60 (m, 2H), 7.50-7.54 (m, 1H), 7.28-7.33 (m, 3H), 6.33 (d, *J* = 2.0 Hz, 1H).

### Example 2. 4-atnino-7-methyl-2-oxo-1-phenyl-1,2-dihydroquinolin-3- carbonitrile

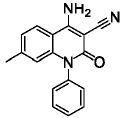

According to the preparation route of scheme 1, 2-bromo-4-methylbenzoic acid and aniline were used as the starting material to obtain the product with reference to the preparation method of Example 1.

¹H NMR (400 MHz, DMSO-*d₆*) δ 8.12 (d, *J =* 8.4 Hz, 1H), 7.76-8.06 (brs, 2H), 7.54-7.60 (m, 2H), 7.48-7.52 (m, 1H), 7.22-7.26 (m, 2H), 7.06 (dd, *J* = 8.4 Hz, 1.2 Hz, 1H), 6.22 (s, 1H), 2.18 (s, 3H).

### Example 3. 4-amino-2-oxo-1-phenyl-1,2-dihydroquinolin-3-carbonitrile

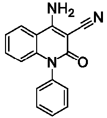

According to the preparation route of scheme 1, 2-bromobenzoic acid and aniline were used as the starting material to obtain the product with reference to the preparation method of Example 1.

¹H NMR (400 MHz, DMSO-*d₆*) δ 8.23 (d, *J =* 7.2 Hz, 1H), 7.84-8.15 (brs, 2H), 7.54-7.58 (m, 2H), 7.44-7.51 (m, 2H), 7.19-7.27 (m, 3H), 6.44 *(d, J=* 8.8 Hz, 1H).

### Example 4. 4-amino-7-iodo-2-oxo-1-phenyl-1,2-dihydroquinolin-3-carbonitrile

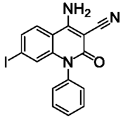

According to the preparation route of scheme 1, 2-bromo-4-iodobenzoic acid and aniline were used as the starting material to obtain the product with reference to the preparation method of Example 1.

¹H NMR (400 MHz, DMSO-*d₆*) δ 8.20 (d, *J =* 8.4 Hz, 1H), 8.00-8.15 (brs, 2H), 7.57-7.59 (m, 2H), 7.52-7.54 (m, 1H), 7.27-7.30 (m, 3H), 6.33 (d, *J* = 2.0 Hz, 1H).

### Example 5. 4-atnino-7-trifluoromethyl-2-oxo-1-phenyl-1,2-dihydroquinolin-3- carbonitrile

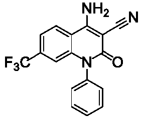

According to the preparation route of scheme 1, 2-bromo-4-trifluoromethylbenzoic acid and aniline were used as the starting material to obtain the product with reference to the preparation method of Example 1.

¹H NMR (400 MHz, CDCl₃) δ 8.48 (d, *J =* 8.8 Hz, 1H), 8.00-8.10 (brs, 2H), 7.52-7.62 (m, 4H), 7.33 (d, *J* = 8.4 Hz, 2H), 6.61 (s, 1H).

### Example 6. 4-amino-8-fluoro-2-oxo-1-phenyl-1,2-dihydroquinolin-3- carbonitrile

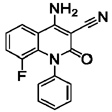

According to the preparation route of scheme 1, 2-bromo-3-fluorobenzoic acid and aniline were used as the starting material to obtain the product with reference to the preparation method of Example 1.

¹H NMR (400 MHz, DMSO-*d₆*) δ 7.98-8.26 (m, 3H), 7.34-7.43 (m, 4H), 7.21-7.28 (m, 3H).

### Example 7. 4-amino-6,7-difluoro-2-oxo-1-phenyl-1,2-dihydroquinolin-3- carbonitrile

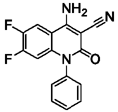

According to the preparation route of scheme 1, 2-bromo-4,5-difluorobenzoic acid and aniline were used as the starting material to obtain the product with reference to the preparation method of Example 1.

¹H NMR (400 MHz, CD₃OD+CDCl₃) δ 7.96 (dd, *J* = 11.2 Hz, 8.4 Hz, 1H), 7.48-7.57 (m, 3H), 7.16-7.18 (m, 2H), 6.40 (dd, *J* = 12.0 Hz, 6.8 Hz, 1H).

### Example 8. 4-amino-5-fluoro-2-oxo-1-phenyl-1,2-dihydroquinolin-3- carbonitrile

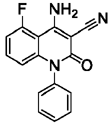

According to the preparation route of scheme 1, 2-bromo-5-fluorobenzoic acid and aniline were used as the starting material to obtain the product with reference to the preparation method of Example 1.

¹H NMR (400 MHz, CD₃OD+CDCl₃) δ 7.46-7.56 (m, 3H), 7.32-7.38 (m, 1H), 7.14-7.18 (m, 2H), 6.93 (dd, *J =* 13.2 Hz, 8.0 Hz, 1H), 6.43 (d, *J =* 9.2 Hz, 1H).

### Example 9. 4-atnino-7-chloro-1-(3-methylphenyl)-2-oxo-1,2-dihydroquinolin-3- carbonitrile

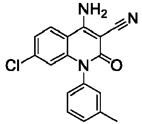

According to the preparation route of scheme 1, 2,4-dichlorobenzoic acid and 3-methylaniline were used as the starting material to obtain the product with reference to the preparation method of Example 1.

¹H NMR (400 MHz, DMSO-*d₆*) δ 8.26 (d, *J =* 8.8 Hz, 1H), 7.96-8.21 (brs, 2H), 7.46 (t, *J =* 7.6 Hz, 1H), 7.30-7.34 (m, 2H), 7.10 (s, 1H), 7.08 (d, *J =* 8.0 Hz, 1H), 6.37 (d, *J =* 1.6 Hz, 1H), 2.47 (s, 3H).

### Example 10. 4-amino-6,7-dichloro-2-oxo-1-phenyl-1,2-dihydroquinolin-3- carbonitrile

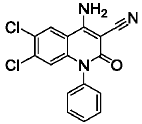

According to the preparation route of scheme 1, 4,5-dichlorobenzoic acid and aniline were used as the starting material to obtain the product with reference to the preparation method of Example 1.

¹H NMR (400 MHz, DMSO-*d₆*) δ 8.60 (s, 1H), 7.94-8.38 (brs, 2H), 7.51-7.61 (m, 3H), 7.30 (d, *J* = 7.2 Hz, 2H), 6.49 (s, 1H).

### Example 11. 4-atnino-7-chloro-2-oxo-1-(pyridin-3-yl)-1,2-dihydroquinolin-3- carbonitrile

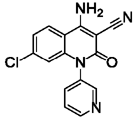

According to the preparation route of scheme 1, 2,4-dichlorobenzoic acid and 3-aminopyridine were used as the starting material to obtain the product with reference to the preparation method of Example 1.

¹H NMR (400 MHz, DMSO-*d₆*) δ 8.72 (d, *J* = 4.8 Hz, 1.6 Hz, 1H), 8.53 (d, *J* = 2.4 Hz, 1H), 8.29 (d, *J* = 8.8 Hz, 1H), 8.01-8.28 (brs, 2H), 7.83-7.86 (m, 1H), 7.64 (dd, *J* = 8.8 Hz, 4.8 Hz, 1H), 7.36 (dd, *J* = 8.8 Hz, 2.0 Hz, 1H), 6.39 (d, *J* = 2.0 Hz, 1H).

### Example 12. 4-amino-7-chloro-2-oxo-1-phenyl-1,2-dihydro-1,8-naphthyridine-3- carbonitrile

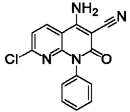

According to the preparation route of scheme 1, 2,6-dichloronicotinic acid and aniline were used as the starting material to obtain the product with reference to the preparation method of Example 1.

¹H NMR (400 MHz, CD₃OD+CDCl₃) δ 8.22 (d, *J* = 8.4 Hz, 1H), 7.34-7.43 (m, 3H), 7.07-7.10 (m, 3H).

### Example 13. 4-amino-7-chloro-2-oxo-1,2-dihydroquinolin-3-carbonitrile

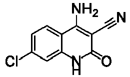

According to the preparation route of scheme 1, 2-amino-4-chlorobenzoic acid was used as Intermediate 2 to obtain the product with reference to the preparation route of Example 1 (from step 2 on).

¹H NMR (400 MHz, DMSO-*d₆*) δ 11.31 (s, 1H), 8.12 (d, *J* = 8.4 Hz, 1H), 7.78-8.08 (brs, 2H), 7.20-7.23 (m, 2H).

### Example 14. 4-atnino-7-chloro-1-methyl-2-oxo-1,2-dihydroquinolin-3- carbonitrile

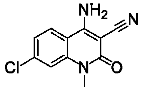

According to the preparation route of scheme 1, 4-chloro-2-methylaminobenzoic acid was used as Intermediate 2 to obtain the product with reference to the preparation route of Example 1 (from step 2 on).

¹H NMR (400 MHz, DMSO-*d₆*) δ 8.20 (d, *J* = 8.4 Hz, 1H), 7.80-8.04 (brs, 2H), 7.53 (d, *J* = 2.0 Hz, 1H), 7.32 (dd, *J* = 8.4 Hz, 2.0 Hz, 1H), 3.46 (s, 3H).

### Example 15. methyl 4-amino-7-chloro-2-oxo-1-phenyl-1,2-dihydroquinoline-3- carboxylate

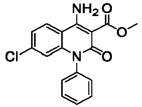

According to the preparation route of scheme 2:

### Step 1: 4-chloro-2-(phenylamino)benzoic acid (Intermediate 1-2)

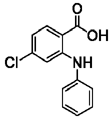

According to step 1 of Example 1.

### Step 2: 7-chloro-1-phenyl-2,4-dihydro-1H-3,1-benzo-oxazine-2,4-dione (Intermediate 1-3)

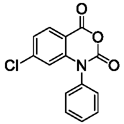

According to step 2 of Example 12.

### Step 3: methyl 7-chloro-4-hydroxy-2-oxo-1-phenyl-1,2-dihydroquinoline-3-carboxylate (Intermediate 15-4)

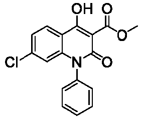

Intermediate 1-3 (1.23 g) and dimethyl malonate (763 mg) were dissolved in dry *N,N*-dimethylformamide (20 mL), sodium hydride (540 mg) was added slowly while cooled with ice bath, the temperature rised to room temperature and the mixture was stirred overnight. To the reaction solution were added 3 mol/L hydrochloric acid (10 mL) and water (40 mL), the mixture was filtered, and the filter cake was washed with water (30 mL*3) to obtain the target compound 15-4 (937 mg).

### Step 4: methyl 7-chloro-4-chloro-2-oxo-1-phenyl-1,2-dihydroquinoline-3- carboxylate (Intermediate 15-5)

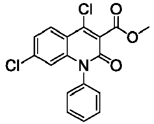

To Intermediate **15-4** (937 mg) was added phosphoryl trichloride (20 mL), and the mixture was heated to 100 °C and stirred for 12 hours. The reaction solution was cooled to room temperature and concentrated under reduced pressure, to the residue was added an aqueous solution of saturated sodium bicarbonate (30 mL), the mixture was extracted with dichloromethane (100 mL*3), the extract was dried over anhydrous sodium sulphate and filtered, the filtrate was concentrated under reduced pressure, and the residue was purified through column chromatography (dichloromethane/methanol = 20:1) to obtain the target compound **15-5** (911 mg).

### Step 5: methyl 4-amino-7-chloro-2-oxo-1-phenyl-1,2-dihydroquinoline-3-carboxylate (Example 15)

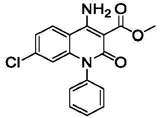

To Intermediate **15-5** (900 mg) were added methanol (20 mL) and 7 mol/L ammonia methanol solution (10 mL), and the mixture was heated to 50 °C and stirred for 12 hours. The reaction solution was concentrated under reduced pressure, and the residue was purified through column chromatography (dichloromethane/methanol = 10:1) to obtain the product (804 mg).

¹H NMR (400 MHz, CD₃OD+CDCl₃) δ 7.95 (d, *J* = 8.8 Hz, 1H), 7.52-7.56 (m, 2H), 7.45-7.49 (m, 1H), 7.17-7.19 (m, 2H), 7.14 (dd, *J* = 8.8 Hz, 1.6 Hz, 1H), 6.49 (d, *J* = 1.6 Hz, 1H), 3.81 (s, 3H).

### Example 16. methyl 4-amino-7-iodo-2-oxo-1-phenyl-1,2-dihydroquinoline-3- carboxylate

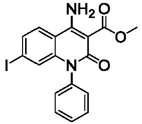

According to the preparation route of scheme 2, 2-bromo-4-iodobenzoic acid and aniline were used as the starting material to obtain the product with reference to the preparation route of Example 15.

¹H NMR (400 MHz, DMSO-*d₆*) δ 8.36 (s, 2H), 7.97 (d, *J* = 8.4 Hz, 1H), 7.48-7.59 (m, 4H), 7.22-7.25 (m, 2H), 6.65 (d, *J* = 1.6 Hz, 1H), 3.68 (s, 3H).

### Example 17. methyl 4-amino-7-trifluoromethyl-2-oxo-1-phenyl-1,2-dihydroquinoline-3-carboxylate

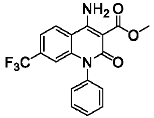

According to the preparation route of scheme 2, 2-bromo-4-trifluoromethylbenzoic acid and aniline were used as the starting material to obtain the product with reference to the preparation route of Example 2.

¹H NMR (400 MHz, DMSO-*d₆*) δ 8.46 (d, *J =* 8.8 Hz, 1H), 8.40 (s, 2H), 7.50-7.60 (m, 4H), 7.27-7.29 (m, 2H), 6.55 (s, 1H), 3.71 (s, 3H).

### Example 18. methyl 4-amino-7-((dimethyl(oxo)-λ⁶⁻sulfanyfidene)amino)-2-oxo-1-phenyl-1,2-dihydroquinoline-3 -carboxylate

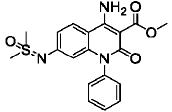

Under nitrogen protection, to a mixture of Example 15 (80 mg), S,S-dimethyl sulfoximine (35 mg), 4,5-bis(diphenylphosphino)-9,9-dimethylxanthene (21 mg), cesium carbonate (121 mg) and Tris(dibenzylideneacetone) dipalladium(0) (10 mg) was added 1.4-dioxane (10 mL), and the mixture was heated to 100 °C and stirred for 12 hours. The reaction solution was cooled to room temperature and filtered, water (100 mL) was added, the mixture was extracted with dichloromethane (100 mL*3), the extract was dried over anhydrous sodium sulphate and filtered, the filtrate was concentrated under reduced pressure, and the residue was purified through column chromatography (dichloromethane/methanol = 20:1) to obtain the product (2 mg).

¹H NMR (400 MHz, CDCl₃) δ 7.48-7.53 (m, 3H), 7.41-7.45 (m, 1H), 7.20-7.23 (m, 2H), 6.94 (dd, *J =* 8.8 Hz, 2.0 Hz, 1H), 6.16 (d, *J* = 2.0 Hz, 1H), 3.87 (s, 3H), 3.08 (s, 6H).

### Example 19. methyl 4-atnino-7-iodo-1-(2-methylphenyl)-2-oxo-1,2-dihydroquinoline-3-carboxylate

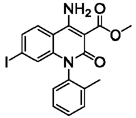

According to the preparation route of scheme 2, 2-bromo-4-iodobenzoic acid and 2-methylaniline were used as the starting material to obtain the product with reference to the preparation route of Example 15.

¹H NMR (400 MHz, DMSO-*d₆*) δ 8.42 (s, 2H), 8.02 (d, *J* = 9.2 Hz, 1H), 7.55 (dd, *J* = 8.4 Hz, 1.2 Hz, 1H), 7.35-7.46 (m, 3H), 7.13 (dd, *J* = 7.2 Hz, 1.2 Hz, 1H), 6.56 (d, *J* = 1.2 Hz, 1H), 3.69 (s, 3H), 1.87 (s, 3H).

### Example 20. methyl 4-amino-7-iodo-1-(2-chlorophenyl)-2-oxo-1,2-dihydroquinoline-3-carboxylate

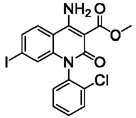

According to the preparation route of scheme 2, 2-bromo-4-iodobenzoic acid and 2-chloroaniline were used as the starting material to obtain the product with reference to the preparation route of Example 15.

¹H NMR (400 MHz, DMSO-*d₆*) δ 8.53 (s, 2H), 8.03 (d, *J =* 8.8 Hz, 1H), 7.71-7.73 (m, 1H), 7.54-7.59 (m, 3H), 7.42-7.45 (m, 1H), 6.55 (s, 1H), 3.69 (s, 3H).

### Example 21. methyl 4-amino-1-(3-(hydroxymethyl)phenyl)-2-oxo-7-(trifluoromethyl)-1,2-dihydroquinoline-3 -carboxylate

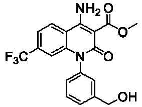

### Step 1: 2-((3-(dimethoxymethyl)phenyl)amino)-4-(trifluoromethyl)benzoic acid

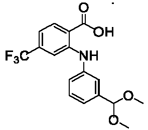

Under nitrogen protection, 2-amino-4-(trifluoromethyl)benzoic acid (1.0 g), 1-bromo-3-(dimethoxymethyl)benzene (2.23 g), copper powder (31 mg), copper oxide (35 mg) and potassium carbonate (808 mg) were mixed in *N*,*N*-dimethylformamide (20 mL), and the mixture was heated to 150 °C and stirred overnight. The reaction solution was cooled to room temperature and filtered through celite, the filtrate was acidified with 1 mol/L hydrochloric acid to pH 6~7, the mixture was extracted with dichloromethane, the extract was dried over anhydrous sodium sulphate and filtered, the filtrate was concentrated under reduced pressure, and the residue was purified through column chromatography (100% ethyl acetate ) to obtain the product ( 352 mg).

### Step 2: 1-(3-(dimethoxymethyl)phenyl)-7-(trifluoromethyl)-2H-benzo[d][1,3]oxazine-2,4 (1H)-dione

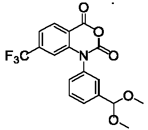

2-((3-(Dimethoxymethyl)phenyl)amino)-4-(trifluoromethyl)benzoic acid (310 mg) and N,N diisopropylethylamine (258 mg) were dissolved in dichloromethane (10 mL), triphosgene (327 mg) was added, and the mixture was stirred at room temperature overnight. Water was added to quench the reaction, the organic phase was separated, the aqueous phase was extracted with dichloromethane, the extracts were combined, dried over anhydrous sodium sulphate and filtered, and the filtrate was concentrated under reduced pressure to obtain the product (271 mg), which was used directly in the next step.

### Step 3: methyl 1-(3-(dimethoxymethyl)phenyl)-4-hydroxy-2-oxo-7-(trifluoromethyl)-1,2-dihydroquinoline-3 -carboxylate

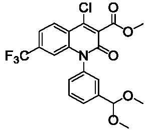

Under nitrogen protection, 1-(3-(dimethoxymethyl)phenyl)-7-(trifluoromethyl)-2H-benzo[d] [1,3]oxazine-2,4(1*H*)-dione (271 mg) and 1,3-dimethyl malonate (188 mg) were dissolved in *N,N*-dimethylformamide (5 mL), the mixture was cooled to 0 °C, sodium *tert*-butoxide (137 mg) was added portionwise, and the mixture was stirred at room temperature overnight. Water was added to quench the reaction, citric acid (500 mg) was added, a solid precipitated, and the precipitate was filtered and collected to obtain the product (198 mg).

### Step 4: methyl 4-chloro-1-(3-formylphenyl)-2-oxo-7-(trifluoromethyl)-1,2-dihydro quinoline-3 -carboxylate

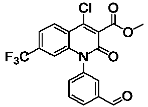

Methyl 1-(3-(dimethoxymethyl)phenyl)-4-hydroxy-2-oxo-7-(trifluoromethyl)-1,2-dihydro quinoline-3-carboxylate (190 mg) was dissolved in phosphoryl trichloride (10 mL), and the mixture was heated to reflux, stirred for 2 hours, cooled to room temperature and concentrated under reduced pressure to obtain a solid as the product (148 mg).

### Step 5: methyl 4-chloro-1-(3-(hydroxymethyl)phenyl)-2-oxo-7-(trifluoromethyl)-1,2-dihydro quinoline-3 -carboxylate

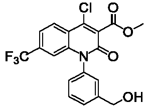

Methyl 4-chloro-1-(3 -formylphenyl)-2-oxo-7-(trifluoromethyl)-1,2-dihydroquinoline-3 - carboxylate (140 mg) was dissolved in methanol (10 mL), the mixture was cooled to 0 °C, sodium boronhydride (15 mg) was added portionwise, and the mixture was stirred at room temperature for 4 hours. Water was added to quench the reaction, the mixture was extracted with dichloromethane, the extract was dried over anhydrous sodium sulphate and filtered, the filtrate was concentrated under reduced pressure, and the residue was purified through column chromatography (100% ethyl acetate ) to obtain the product (119 mg).

### Step 6: methyl 4-amino-1-(3-(hydroxymethyl)phenyl)-2-oxo-7-(trifluoro methyl)-1,2-dihydro quinoline-3 -carboxylate

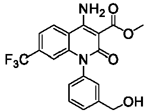

Methyl 4-chloro-1-(3-(hydroxymethyl)phenyl)-2-oxo-7-(trifluoromethyl)-1,2-dihydro quinoline-3-carboxylate (110 mg) was dissolved in a 7 mol/L ammonia methanol solution (5 mL), and the mixture was heated to reflux, stirred overnight, cooled to room temperature and concentrated under reduced pressure to obtain the product (18 mg).

¹H NMR (400 MHz, CDCl₃) δ 7.82 (d, *J* = 8.4 Hz, 1H), 7.60-7.69 (brs, 3H), 7.50-7.57 (m, 3H), 7.39 (d, *J* = 8.8 Hz, 1H), 7.14 (d, *J* = 7.2 Hz, 1H), 6.85 (s, 1H), 4.76 (s, 2H), 3.89 (s, 3H).

### Example 22. methyl 4-amino-7-bromo-2-oxo-1-phenyl-1,2-dihydroquinoline-3- carboxylate

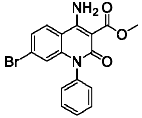

According to the preparation route of scheme 2, 2,4-dibromobenzoic acid and aniline were used as the starting material to obtain the product with reference to the preparation route of Example 15.

¹H NMR (400 MHz, DMSO-*d₆*) δ 8.37 (s, 2H), 8.16 (d, *J =* 8.8 Hz, 1H), 7.56-7.59 (m, 2H), 7.48-7.52 (m, 1H), 7.40 (dd, *J =* 8.8 Hz, 2.0 Hz, 1H), 7.24 (d, *J =* 7.6 Hz, 2H), 6.44 (d, *J =* 2.0 Hz, 1H), 3.69 (s, 3H).

### Example 23. methyl 4-amino-7-trifluoromethyl-1-(2-methylphenyl)-2-oxo-1,2-dihydro quinoline-3 -carboxylate

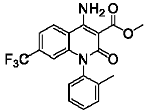

According to the preparation route of scheme 2, 2-bromo-4-trifluoromethylbenzoic acid and 2-methylaniline were used as the starting material to obtain the product with reference to the preparation route of Example 15.

¹H NMR (400 MHz, CDCl₃) δ 7.54-8.10 (m, 3H), 7.34-7.41 (m, 4H), 7.12 *(d, J=* 7.6 Hz, 1H), 6.73 (s, 1H), 3.90 (s, 3H), 2.00 (s, 3H).

### Example 24. methyl 4-amino-7-vinyl-2-oxo-1-phenyl-1,2-dihydroquinoline-3- carboxylate

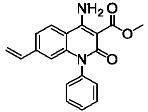

Under nitrogen protection, to a mixture of Example 15 (50 mg), potassium vinyltrifluoroborate (48 mg), 1,1'-bis(diphenylphosphino)ferrocene-palladium(II)dichloride (13 mg) and cesium carbonate (85 mg) were added 1.4-dioxane (10 mL) and water (2 mL), and the mixture was heated to 120 °C and stirred for 12 hours. The reaction solution was cooled to room temperature and filtered, water (100 mL) was added, the mixture was extracted with dichloromethane (100 mL*3), the extract was dried over anhydrous sodium sulphate and filtered, the filtrate was concentrated under reduced pressure, and the residue was purified through column chromatography (dichloromethane/methanol = 20:1) to obtain the product (22 mg).

¹H NMR (400 MHz, CDCl₃) δ 7.60 (d, *J* = 8.8 Hz, 1H), 7.52-7.56 (m, 2H), 7.45-7.49 (m, 1H), 7.27 (dd, *J* = 8.8 Hz, 1.6 Hz, 1H), 7.22-7.25 (m, 2H), 6.54 (dd, *J* = 17.6 Hz, 10.8 Hz, 1H), 6.52 (d, *J =* 1.6 Hz, 1H), 5.68 (d, *J* = 17.6 Hz, 1H), 5.31 *(d, J=* 10.8 Hz, 1H), 3.88 (s, 3H).

### Example 25. methyl 4-amino-1-(4-methoxyphenyl)-2-oxo-7-(trifluoromethyl)-1,2-dihydro quinoline-3-carboxylate

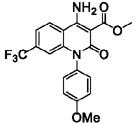

According to the preparation route of scheme 2, 2-bromo-4-trifluoromethylbenzoic acid and 4-methoxyaniline were used as the starting material to obtain the product with reference to the preparation route of Example 15.

¹H NMR (400 MHz, DMSO-*d₆*) δ 8.44 (d, *J* = 8.4 Hz, 1H), 8.34 (s, 2H), 7.53 (d, *J* = 8.0 Hz, 1H), 7.18 *(d, J=* 8.8 Hz, 2H), 7.10 *(d, J=* 8.8 Hz, 2H), 6.64 (s, 1H), 3.82 (s, 3H), 3.71 (s, 3H).

### Example 26. methyl 4-amino-1-(naphthalen-2-yl)-2-oxo-7-(trifluoromethyl)-1,2-dihydro quinoline-3 -carboxylate

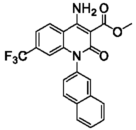

According to the preparation route of scheme 2, 2-bromo-4-trifluoromethylbenzoic acid and naphthalen-2-amine were used as the starting material to obtain the product with reference to the preparation route of Example 15.

¹H NMR (400 MHz, CDCl₃) δ 8.04 (d, *J* = 8.4 Hz, 1H), 7.94 (d, *J* = 8.0 Hz, 1H), 7.85 (d, *J* = 7.6 Hz, 1H), 7.79 (d, *J =* 8.8 Hz, 1H), 7.77 (s, 1H), 7.52-7.60 (m, 2H), 7.39 (d, *J =* 8.4 Hz, 1H), 7.29 (dd, *J* = 8.8 Hz, 1.6 Hz, 1H), 6.89 (s, 1H), 3.90 (s, 3H).

### Example 27. methyl 4-amino-1-benzyl-7-chloro-2-oxo-1,2-dihydroquinoline-3- carboxylate

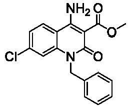

Referring to the preparation route of Scheme 3, with specific synthetic route as follow:

### Step 1: 7-chloro-2,4-dihydro-1H-3,1-benzo-oxazine-2,4-dione (Intermediate 27-2)

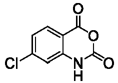

2-amino-4-chlorobenzoic acid **27-1** (3.0 g) was dissolved in dry tetrahydrofuran (30 mL), triphosgene (2.084 g) was added, and the mixture was stirred at room temperature overnight. The mixture was filtered, and the filter cake was washed with tetrahydrofuran (10 mL*3) to obtain the target compound **27-2** (1.42 g).

### Step 2: 1-benzyl-7-chloro-2,4-dihydro-1H-3,1-benzo-oxazine-2,4-dione (Intermediate 27-3)

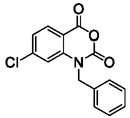

While being cooled with an ice-bath, triphenylphosphine (1.132 g) was dissolved in dichloromethane (10 mL), a solution of diisopropyl azodicarboxylate (872 mg) in dichloromethane (3 mL) was added slowly, the mixture was stirred for 30 minutes, Intermediate **27-2** (800 mg) was added, the mixture was stirred for 5 minutes, and a solution of benzyl alcohol (434 mg) in dichloromethane (5 mL) was added. The mixture was stirred at room temperature overnight and filtered, and the filter cake was washed with dichloromethane (10 mL*3) to obtain the target compound **27-3** (749 mg).

### Step 3: methyl 1-benzyl-7-chloro-4-hydroxy-2-oxo-1,2-dihydroquinoline-3-carboxylate (Intermediate 27-4)

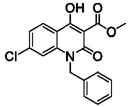

While being cooled with an ice-bath, Intermediate **7-3** (200 mg) and dimethyl malonate (111 mg) were dissolved in dry *N*,*N*-dimethylformamide (20 mL), sodium hydride (84 mg) was added slowly, and the mixture was stirred at room temperature overnight. 3 Mol/L hydrochloric acid (10 mL) and water (40 mL) were added, the mixture was filtered, and the filter cake was washed with water (30 mL*3) to obtain the target compound **27-4** (165 mg).

### Step 4: methyl 1-benzyl-7-chloro-4-chloro-2-oxo-1,2-dihydroquinoline-3-carboxylate (Intermediate 27-5)

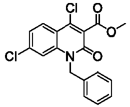

To Intermediate **27-4** (160 mg) was added phosphoryl trichloride (10 mL), and the mixture was heated to 100 °C and stirred for 12 hours. The reaction solution was cooled to room temperature and concentrated under reduced pressure, to the residue was added an aqueous solution of saturated sodium bicarbonate (20 mL), the mixture was extracted with dichloromethane (30 mL*3), the extract was dried over anhydrous sodium sulphate and filtered, the filtrate was concentrated under reduced pressure, and the residue was purified through column chromatography (dichloromethane/methanol = 20:1) to obtain the target compound **27-5** (128 mg).

### Step 5: methyl 4-amino-1-benzyl-7-chloro-2-oxo-1,2-dihydroquinoline-3-carboxylate (Example 27)

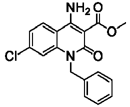

To Intermediate **27-5** (120 mg) were added methanol (20 mL) and 7 mol/L ammonia methanol solution (10 mL), and the mixture was heated to 50 °C and stirred for 12 hours. The reaction solution was cooled to room temperature and concentrated under reduced pressure, and the residue was purified through column chromatography (dichloromethane/methanol = 10:1) to obtain the product (52 mg).

¹H NMR (400 MHz, CDCl₃) δ 7.56 (d, *J* = 8.8 Hz, 1H), 7.20-7.31 (m, 6H), 7.13 (dd, *J* = 8.8 Hz, 2.0 Hz, 1H), 544 (s, 2H), 3.95 (s, 3H).

### Example 28. methyl 4-amino-1-benzyl-7-iodo-2-oxo-1,2-dihydroquinoline-3- carboxylate

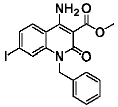

According to the preparation route of scheme 3, 2-amino-4-iodobenzoic acid and benzyl alcohol were used as the starting material to obtain the product with reference to the preparation route of Example 27.

¹H NMR (400 MHz, CD₃OD+CDCl₃) δ 7.58-7.64 (m, 1H), 7.53 (s, 1H), 7.45 (d, *J* = 8.4 Hz, 1H), 7.24-7.28 (m, 2H), 7.14-7.20 (m, 3H), 5.38 (s, 2H), 3.85 (s, 3H).

### Example 29. methyl 4-atnino-1-benzyl-2-oxo-7-(trifluoromethyl)-1,2-dihydroquinoline-3-carboxylate

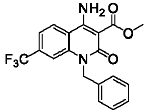

According to the preparation route of scheme 3, 2-amino-4-trifluoromethylbenzoic acid and benzyl alcohol were used as the starting material to obtain the product with reference to the preparation route of Example 27.

¹H NMR (400 MHz, CD₃OD+CDCl₃) δ 8.10 (d, *J* = 8.8 Hz, 1H), 7.42 (s, 1H), 7.36 (d, *J =* 8.8 Hz, 1H), 7.23-7.27 (m, 2H), 7.17-7.19 (m, 3H), 5.46 (s, 2H), 3.88 (s, 3H).

### Example 30. methyl 4-amino-1-benzyl-7-bromo-2-oxo-1,2-dihydroquinoline-3- carboxylate

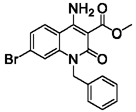

According to the preparation route of scheme 3, 2-amino-4-bromobenzoic acid and benzyl alcohol were used as the starting material to obtain the product with reference to the preparation route of Example 27.

¹H NMR (400 MHz, CDCl₃) δ 7.40-7.72 (m, 3H), 7.38 (d, *J* = 2.0 Hz, 1H), 7.19-7.32 (m, 6H), 5.43 (s, 2H), 3.95 (s, 3H).

### Example 31. methyl 4-amino-1-benzyl-7-methyl-2-oxo-1,2-dihydroquinoline- 3-carboxylate

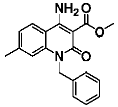

According to the preparation route of scheme 3, 2-amino-4-methylbenzoic acid and benzyl alcohol were used as the starting material to obtain the product with reference to the preparation route of Example 27.

¹H NMR (400 MHz, CDCl₃) δ 7.52 (d, *J* = 7.2 Hz, 1H), 7.17-7.29 (m, 5H), 6.99 (s, 1H), 6.97 (d, *J* = 8.8 Hz, 1H), 5.46 (s, 2H), 3.93 (s, 3H), 2.33 (s, 3H).

### Example 32. methyl 4-atnino-7-chloro-2-oxo-1-(pyridin-2-ylmethyl)-1,2-dihydroquinoline-3-carboxylate

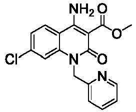

According to the preparation route of scheme 3, 2-amino-4-chlorobenzoic acid and 2-hydroxymethylpyridine were used as the starting material to obtain the product with reference to the preparation route of Example 27.

¹H NMR (400 MHz, CDCl₃) δ 8.56 (d, *J =* 4.8 Hz, 1H), 7.56-7.92 (m, 4H), 7.43 (d, *J =* 1.6 Hz, 1H), 7.15-7.21 (m, 2H), 7.10 (dd, *J* = 8.4 Hz, 2.0 Hz, 1H), 5.54 (s, 2H), 3.93 (s, 3H).

### Example 33. methyl 4-atnino-7-bromo-2-oxo-1-(pyridin-2-ylmethyl)-1,2-dihydroquinoline-3-carboxylate

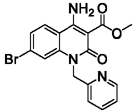

According to the preparation route of scheme 3, 2-amino-4-bromobenzoic acid and 2-hydroxymethylpyridine were used as the starting material to obtain the product with reference to the preparation route of Example 27.

¹H NMR (400 MHz, CDCl₃) δ 8.57 (d, *J =* 4.4 Hz, 1H), 7.52-7.85 (m, 4H), 7.49 (d, *J =* 8.8 Hz, 1H), 7.25 (dd, *J =* 8.8 Hz, 1.6 Hz, 1H), 7.21 (d, *J =* 8.4 Hz, 1H), 7.15-7.18 (m, 1H), 5.54 (s, 2H), 3.93 (s, 3H).

### Example 34. methyl 4-atnino-7-iodo-2-oxo-1-(pyridin-2-ylmethyl)-1,2-dihydroquinoline-3-carboxylate

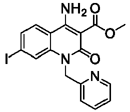

According to the preparation route of scheme 3, 2-amino-4-iodobenzoic acid and 2-hydroxymethylpyridine were used as the starting material to obtain the product with reference to the preparation route of Example 27.

¹H NMR (400 MHz, CD₃OD+CDCl₃) δ 8.52 (d, *J =* 4.0 Hz, 1H), 7.61-7.67 (m, 2H), 7.60 (s, 1H), 7.48-7.52 (m, 1H), 7.23-7.26 (m, 1H), 7.06-7.11 (m, 1H), 5.50 (s, 2H), 3.85 (s, 3H).

### Example 35. methyl 4-amino-2-oxo-1-(pyridin-2-ylmethyl)-7-(trifluoromethyl)-1,2-dihydro quinoline-3 -carboxylate

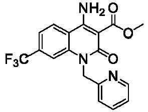

According to the preparation route of scheme 3, 2-amino-4-trifluoromethylbenzoic acid and 2-hydroxymethylpyridine were used as the starting material to obtain the product with reference to the preparation route of Example 27.

¹H NMR (400 MHz, CD₃OD+CDCl₃) δ 8.42 (d, *J* = 4.8 Hz, 1H), 7.97 (d, *J* = 7.6 Hz, 1H), 7.47-7.54 (m, 2H), 7.30 *(d, J=* 7.6 Hz, 1H), 7.05-7.12 (m, 2H), 5.49 (s, 2H), 3.81 (s, 3H).

### Example 36. methyl 4-atnino-7-methyl-2-oxo-1-(pyridin-2-ylmethyl)-1,2-dihydroquinoline-3-carboxylate

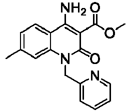

According to the preparation route of scheme 3, 2-amino-4-methylbenzoic acid and 2-hydroxymethylpyridine were used as the starting material to obtain the product with reference to the preparation route of Example 27.

¹H NMR (400 MHz, CDCl₃) δ 8.57 (d, *J* = 4.4 Hz, 1H), 7.30-7.92 (m, 4H), 7.12-7.17 (m, 3H), 6.99 (d, *J* = 8.0 Hz, 1H), 5.58 (s, 2H), 3.95 (s, 3H), 2.35 (s, 3H).

### Example 37. methyl 4-atnino-7-iodo-2-oxo-1-(1-phenylethyl)-1,2-dihydroquinoline-3-carboxylate

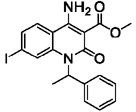

According to the preparation route of scheme 3, 2-amino-4-iodobenzoic acid and 1-phenylethan-1-ol were used as the starting material to obtain the product with reference to the preparation route of Example 27.

¹H NMR (400 MHz, CDCl₃) δ 7.47-7.68 (brs, 2H), 7.45 (d, *J =* 1.2 Hz, 1H), 7.21-7.36 (m, 7H), 7.10 (q, *J* = 7.2 Hz, 1H), 3.95 (s, 3H), 1.84 *(d, J=* 7.6 Hz, 3H).

### Example 38. methyl 4-amino-7-iodo-1-((2-methylphenyl)methyl)-2-oxo-1,2-dihydro quinoline-3 -carboxylate

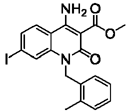

According to the preparation route of scheme 3, 2-amino-4-iodobenzoic acid and 2-methylbenzyl alcohol were used as the starting material to obtain the product with reference to the preparation route of Example 27.

¹H NMR (400 MHz, DMSO-*d₆*) δ 8.24 (s, 2H), 7.95 (d, *J* = 8.4 Hz, 1H), 7.55 (dd, *J* = 8.4 Hz, 1.2 Hz, 1H), 7.83 (d, *J =* 1.2 Hz, 1H), 7.22 (d, *J* = 7.2 Hz, 1H), 7.11 (t, *J =* 7.6 Hz, 1H), 6.99 (t, *J* = 7.6 Hz, 1H), 6.43 (d, *J =* 7.6 Hz, 1H), 5.27 (s, 2H), 3.71 (s, 3H), 2.39 (s, 3H).

### Example 39. methyl 4-amino-1-((2-chlorophenyl)methyl)-7-iodo-2-oxo-1,2-dihydroquinoline-3 -carboxylate

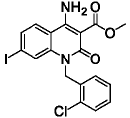

According to the preparation route of scheme 3, 2-amino-4-iodobenzoic acid and 2-chlorobenzyl alcohol were used as the starting material to obtain the product with reference to the preparation route of Example 27.

¹H NMR (400 MHz, DMSO-*d₆*) δ 8.31 (s, 2H), 7.97 (d, *J* = 8.4 Hz, 1H), 7.57 (dd, *J* = 8.4 Hz, 1.2 Hz, 1H), 7.52 (d, *J =* 8.0 Hz, 1H), 7.40 (d, *J =* 1.2 Hz, 1H), 7.27 (td, *J =* 7.6 Hz, 1.2 Hz, 1H), 7.19 (td, *J* = 7.6 Hz, 1.2 Hz, 1H), 6.66 (dd, *J* = 8.0 Hz, 1.2 Hz, 1H), 5.36 (s, 2H), 3.71 (s, 3H).

### Example 40. methyl 4-amino-1-(3H-imidazol-4-ylmethyl)-7-iodo-2-oxo-1,2-dihydroquinoline-3 -carboxylate

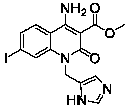

According to the preparation route of scheme 3, 2-amino-4-iodobenzoic acid and 4-(hydroxymethyl)imidazole were used as the starting material to obtain the product with reference to the preparation route of Example 27.

¹H NMR (400 MHz, DMSO-*d₆*) δ 12.80-13.50 (brs, 1H), 8.22 (s, 2H), 8.00-8.09 (m, 1H), 7.96 (s, 1H), 7.92 (d, *J* = 8.8 Hz, 1H), 7.55 (d, *J* = 8.4 Hz, 1H), 7.06 (s, 1H), 5.24 (s, 2H), 3.72 (s, 3H).

### Example 41. 4-amino-7-chloro-2-oxo-1-phenyl-1,2-dihydroquinolin-3- carboxylic acid

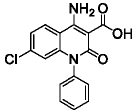

Example 15 (100 mg) was dissolved in tetrahydrofuran (10 mL), a solution of lithium hydroxide (22 mg) in water (3 mL) was added, and the mixture was heated to 70 °C and stirred overnight. The mixture was cooled to room temperature and concentrated under reduced pressure, to the residue was added water (30 mL), the mixture was washed with dichloromethane (20 mL*3), then 3 mol/L hydrochloric acid (10 mL) was added, a white solid precipitated, the mixture was filtered, and the filter cake was washed with water to obtain the product (78 mg).

¹H NMR (400 MHz, CD₃OD+CDCl₃) δ 8.07 (d, *J* = 8.8 Hz, 1H), 7.54-7.63 (m, 3H), 7.22-7.27 (m, 3H), 6.61 (s, 1H).

### Example 42. 4-amino-7-chloro-2-oxo-1-phenyl-1,2-dihydroquinolin-3- carboxamide

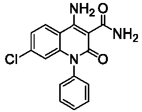

To Intermediate 15-5 (850 mg) were added methanol (20 mL) and 7 mol/L ammonia methanol solution (10 mL), and the mixture was heated to 50 °C and stirred for 12 hours. The reaction solution was cooled to room temperature and concentrated under reduced pressure, and the residue was purified through column chromatography (dichloromethane/methanol = 10:1) to obtain the product (43 mg).

¹H NMR (400 MHz, CD₃OD+CDCl₃) δ 7.97 (d, *J* = 8.4 Hz, 1H), 7.57-7.60 (m, 2H), 7.50-7.54 (m, 1H), 7.19-7.22 (m, 2H), 7.17 (dd, *J* = 9.2 Hz, 2.0 Hz, 1H), 6.50 (d, *J =* 2.0 Hz, 1H).

### Example 43. 4-amino-1 -benzyl-2-oxo-7-(trifluoromethyl)-1,2-dihydroquinolin-3 - carboxamide

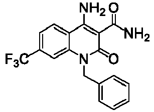

The product was obtained according to the preparation route of Example 29.

¹H NMR (400 MHz, DMSO-*d₆*) δ 10.78-11.38 (brs, 1H), 9.64 (s, 1H), 8.30-8.60 (m, 2H), 7.51-7.58 (m, 2H), 7.43 (d, J = 2.8 Hz, 1H), 7.27-7.30 (m, 2H), 7.16-7.22 (m, 3H), 5.54 (s, 2H).

### Example 44. 4-amino-7-chloro-N-methyl-2-oxo-1-phenyl-1,2-dihydroquinolin-3-carboxamide

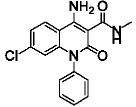

According to the preparation route of scheme 3, and the specific synthetic route is as follow:

### Step 1: 7-chloro-4-hydroxy-N-methyl-2-oxo-1-phenyl-1,2-dihydroquinolin-3-carboxamide (Intermediate 44-1)

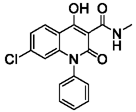

Methyl 7-chloro-4-hydroxy-2-oxo-1-phenyl-1,2-dihydroquinoline-3-carboxylate (Intermediate 1-5) (200 mg) was dissolved in acetonitrile (20 mL), methylamine hydrochloride (300 mg) was added, and the mixture was heated to 90 °C and stirred overnight. The mixture was cooled to room temperature and filtered, the filtrate was concentrated under reduced pressure, water (30 mL) was added, the mixture was extracted with dichloromethane (30 mL*3), the extract was dried over anhydrous sodium sulphate and filtered, and the filtrate was concentrated under reduced pressure to obtain the target compound **44-1** (188 mg).

### Step 2: 7-chloro-4-chloro-N-methyl-2-oxo-1-phenyl-1,2-dihydroquinolin-3- carboxamide (Intermediate 44-2)

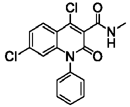

To Intermediate **44-1** (180 mg) was added phosphoryl trichloride (10 mL), and the mixture was heated to 100 °C and stirred for 12 hours. The reaction solution was cooled to room temperature and concentrated under reduced pressure, to the residue was added an aqueous solution of saturated sodium bicarbonate (30 mL), the mixture was extracted with dichloromethane (30 mL*3), the extract was dried over anhydrous sodium sulphate and filtered, the filtrate was concentrated under reduced pressure, and the residue was purified through column chromatography (dichloromethane/methanol = 20:1) to obtain the target compound **44-2** (112 mg).

### Step 3: 4-amino-7-chloro-N-methyl-2-oxo-1-phenyl-1,2-dihydroquinolin-3-carboxamide (Example 44)

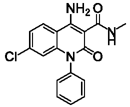

To Intermediate **44-2** (100 mg) were added methanol (5 mL) and 7 mol/L ammonia methanol solution (5 mL), and the mixture was heated to 50 °C and stirred for 12 hours. The reaction solution was cooled to room temperature and concentrated under reduced pressure, and the residue was purified through column chromatography (dichloromethane/methanol = 10:1) to obtain the product (81 mg).

¹H NMR (400 MHz, DMSO-*d₆*) δ 10.80-11.20 (brs, 1H), 10.02 (q, *J* = 4.4 Hz, 1H), 8.16-8.42 (m, 2H), 7.57-7.60 (m, 2H), 7.50-7.54 (m, 1H), 7.34 (dd, *J =* 8.4 Hz, 1.6 Hz, 1H), 7.29 (d, *J* = 7.6 Hz, 2H), 6.33 (d, *J* = 1.2 Hz, 1H), 2.72 (d, *J* = 4.4 Hz, 3H).

### Example 45. 4-amino-7-chloro-N,N-dimethyl-2-oxo-1-phenyl-1,2-dihydroquinolin-3 - carboxamide

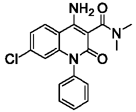

According to the preparation route of Example 44, methylamine hydrochloride in step 1 was replaced by a solution of 1 mol/L dimethylamine in tetrahydrofuran to obtain the product (62 mg).

¹H NMR (400 MHz, CDCl₃) δ 7.47-7.58 (m, 4H), 7.20 (d, *J* = 7.2 Hz, 2H), 7.15 (dd, *J* = 8.4 Hz, 2.0 Hz, 1H), 6.64 (d, *J* = 1.6 Hz, 1H), 5.76 (s, 2H), 3.09 (s, 3H), 3.02 (s, 3H).

### Example 46. ethyl 4-amino-7-chloro-2-oxo-1-phenyl-1,2-dihydroquinoline- 3-carboxylate

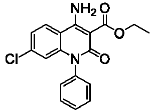

According to the preparation route of Example 1, dimethyl malonate in step 3 was replaced by diethyl malonate to obtain the product.

¹H NMR (400 MHz, CDCl₃) δ 7.51-7.84 (m, 5H), 7.46-7.50 (m, 1H), 7.21-7.23 (m, 2H), 7.13 (dd, *J* = 8.8 Hz, 2.0 Hz, 1H), 6.56 (d, *J* = 2.0 Hz, 1H), 4.36 (q, *J* = 7.2 Hz, 2H), 1.37 (t, *J* = 7.2 Hz, 3H).

### Example 47. 2-ethoxyethyl 4-amino-7-chloro-2-oxo-1-phenyl-1,2-dihydroquinolin-3-carboxylate

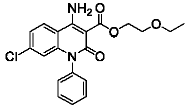

To Example 45 (100 mg) were added acetonitrile (20 mL) and 2-ethoxyethanol (1 mL), and the mixture was heated to 90 °C and stirred overnight. The reaction solution was cooled to room temperature and concentrated under reduced pressure, and the residue was purified through column chromatography (dichloromethane/methanol = 10:1) to obtain the product (11 mg).

¹H NMR (400 MHz, CDCl₃) δ 7.40-7.60 (m, 6H), 7.20-7.22 (m, 2H), 7.13 (dd, *J =* 8.8 Hz, 1.6 Hz, 1H), 6.55 (d, *J* = 2.0 Hz, 1H), 4.45 (t, *J* = 5.6 Hz, 2H), 3.75 (t, *J* = 5.6 Hz, 2H), 3.53 (q, *J* = 7.2 Hz, 2H), 1.17 (t, *J* = 7.2 Hz, 3H).

### Example 48. propan-2-yl 4-amino-7-chloro-2-oxo-1-phenyl-1,2-dihydroquinolin-3-carboxylate

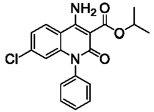

To Example 45 (100 mg) were added acetonitrile (20 mL) and isopropanol (1 mL), and the mixture was heated to 90 °C and stirred overnight. The reaction solution was cooled to room temperature and concentrated under reduced pressure, and the residue was purified through column chromatography (dichloromethane/methanol = 10:1) to obtain the product (14 mg).

¹H NMR (400 MHz, DMSO-*d₆*) δ 8.29 (s, 2H), 8.23 (d, *J* = 8.8 Hz, 1H), 7.55-7.59 (m, 2H), 7.48-7.52 (m, 1H), 7.25-7.28 (m, 3H), 6.27 (d, *J* = 2.0 Hz, 1H), 5.02-5.10 (m, 1H), 1.21 (d, *J* = 6.0 Hz, 6H).

### Example 49. cyclopropyl 4-amino-7-chloro-2-oxo-1-phenyl-1,2-dihydroquinolin-3-carboxylate

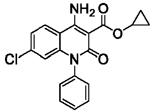

Example 41 (50 mg) was dissolved in *N*,*N*-dimethylformamide (5 mL), 2-(7-aza-*1H-*benzotriazole-1-yl)-1,1,3,3-tetramethyluronium hexafluorophosphate (91 mg) and *N*,*N*-diisopropyl ethylamine (62 mg) were added, and the mixture was heated to 60 °C and stirred overnight. The reaction solution was cooled to room temperature, water (20 mL) was added, the mixture was extracted with dichloromethane (30 mL*3), the extract was dried over anhydrous sodium sulphate and filtered, the filtrate was concentrated under reduced pressure, and the residue was purified through column chromatography (dichloromethane/methanol = 20:1) to obtain the product (8 mg).

¹H NMR (400 MHz, DMSO-*d₆*) δ 8.47 (s, 2H), 8.25 (d, *J* = 8.8 Hz, 1H), 7.55-7.58 (m, 2H), 7.48-7.51 (m, 1H), 7.26 (dd, *J* = 9.2 Hz, 2.0 Hz, 1H), 7.24 (d, *J=* 8.0 Hz, 2H), 6.26 (d, *J* = 2.0 Hz, 1H), 4.15-4.19 (m, 1H), 0.66-0.71 (m, 2H), 0.60-0.64 (m, 2H).

### Example 50. cyclopropyl 4-amino-7-iodo-2-oxo-1-phenyl-1,2-dihydroquinolin-3-carboxylate

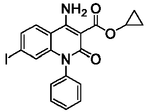

According to the preparation route of scheme 4, and the specific synthetic route is as follow:

### Step 1: 4-amino-7-iodo-2-oxo-1-phenyl-1,2-dihydroquinolin-3-carboxylic acid (Intermediate 50-1)

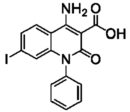

According to the preparation route of Example 41, the final product of Example 1 was replaced by the final product of Example 16 to obtain the target compound **50-1** (80 mg).

### Step 2: cyclopropyl 4-amino-7-iodo-2-oxo-1-phenyl-1,2-dihydroquinolin-3-carboxylate (Example 50)

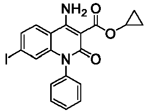

According to the preparation route of Example 49, Intermediate **50-1** was used as the starting material to obtain the product (10 mg).

¹H NMR (400 MHz, DMSO-*d₆*) δ 8.45 (s, 2H), 7.98 (d, *J =* 8.8 Hz, 1H), 7.49-7.58 (m, 4H), 7.21-7.25 (m, 2H), 6.63 (d, *J =* 1.6 Hz, 1H), 4.14-4.20 (m, 1H), 0.66-0.72 (m, 2H), 0.58-0.65 (m, 2H).

### Example 51. cyclopropyl 4-amino-2-oxo-1-phenyl-7-(trifluoromethyl)-1,2-dihydroquinolin-3-carboxylate

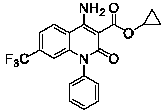

According to the preparation route of scheme 4, Example 17 was used as the starting material to obtain the product with reference to the preparation route of Example 50 (12 mg).

¹H NMR (400 MHz, DMSO-*d₆*) δ 8.48 (s, 2H), 8.46 (d, *J =* 8.8 Hz, 1H), 7.49-7.60 (m, 4H), 7.27 (d, *J* = 6.8 Hz, 2H), 6.54 (s, 1H), 4.17-4.22 (m, 1H), 0.68-0.73 (m, 2H), 0.61-0.65 (m, 2H).

### Example 52. cyclopropyl 4-amino-7-iodo-1-(2-methylphenyl)-2-oxo-1,2-dihydroquinolin-3-carboxylate

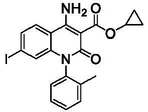

According to the preparation route of scheme 4, Example 19 was used as the starting material to obtain the product 52 with reference to the preparation route of Example 50 (10 mg).

¹H NMR (400 MHz, CD₃OD+CDCl₃) δ 7.63 (d, *J =* 8.8 Hz, 1H), 7.47 (dd, *J =* 8.8 Hz, 1.2 Hz, 1H), 7.29-7.41 (m, 3H), 7.04 (d, *J=* 7.6 Hz, 1H), 6.74 (d, *J=* 1.6 Hz, 1H), 4.15-4.22 (m, 1H), 1.93 (s, 3H), 0.80-0.84 (m, 2H), 0.67-0.71 (m, 2H).

### Example 53. cyclopropyl 4-amino-7-iodo-1-((2-methylphenyl)methyl)-2-oxo-1,2-dihydro quinolin-3 -carboxylate

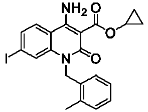

According to the preparation route of scheme 4, Example 38 was used as the starting material to obtain the product with reference to the preparation route of Example 50.

¹H NMR (400 MHz, CDCl₃) δ 7.42-7.78 (m, 3H), 7.31-7.34 (m, 2H), 7.19 (d, *J* = 7.6 Hz, 1H), 7.12 (t, *J* = 7.6 Hz, 1H), 7.01 (t, *J* = 7.6 Hz, 1H), 6.63 (d, *J* = 8.0 Hz, 1H), 5.32 (s, 2H), 4.25-4.30 (m, 1H), 2.44 (s, 3H), 0.91-0.94 (m, 2H), 0.76-0.81 (m, 2H).

### Example 54. 4-amino-7-chloro-3-(hydroxymethyl)-1-phenyl-1,2-dihydroquinolin-2-one

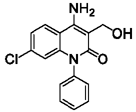

Under nitrogen protection and while being cooled by an ice-bath, Example 15 (100 mg) was dissolved in dry tetrahydrofuran (20 mL), a solution of 1 mol/L lithium aluminum hydride in toluene (0.9 mL) was added slowly, and the mixture was stirred for 30 minutes while being cooled by a ice-bath, and stirred at room temperature overnight. The reaction solution was poured into ice-water (30 mL), the mixture was extracted with ethyl acetate (30 mL*3), the extract was dried over anhydrous sodium sulphate and filtered, the filtrate was concentrated under reduced pressure, and the residue was purified through column chromatography (dichloromethane/methanol = 20:1) to obtain the product (52 mg).

¹H NMR (400 MHz, DMSO-*d₆*) δ 8.09 (d, *J* = 8.4 Hz, 1H), 7.55-7.59 (m, 2H), 7.48-7.52 (m, 1H), 7.20-7.24 (m, 3H), 6.66 (s, 2H), 6.31 (d, *J* = 1.6 Hz, 1H), 4.73 (t, *J* = 5.2 Hz, 1H), 4.52 (d, *J* = 5.6 Hz, 2H).

### Example 55. tert-butyl (4-amino-2-oxo-1-phenyl-7-((trimethylsilyl)ethynyl)-1,2-dihydroquinolin-3 -yl)carbamate

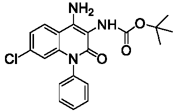

The final product of Example 41 (150 mg) was dissolved in tert-butanol (10 mL), diphenylphosphoryl azide (176 mg) and di-tert-butyl dicarbonate (94 mg) were added, and the mixture was heated to 100 °C and stirred overnight. The mixture was cooled to room temperature, water (20 mL) was added, the mixture was extracted with dichloromethane (30 mL*3), the extract was dried over anhydrous sodium sulphate and filtered, the filtrate was concentrated under reduced pressure, and the residue was purified through column chromatography (petroleum ether/ethyl acetate = 4:1) to obtain the product (28 mg).

¹H NMR (400 MHz, CDCl₃) δ 7.93 (d, *J* = 8.4 Hz, 1H), 7.55-7.59 (m, 2H), 7.48-7.52 (m, 1H), 7.24-7.26 (m, 2H), 7.14 (dd, *J* = 8.4 Hz, 2.0 Hz, 1H), 6.58 (d, *J* = 2.0 Hz, 1H), 1.59 (s, 9H).

### Example 56. 4-amino-7-chloro-3-(3-methyl-1,2,4-oxadiazol-5-yl)-1-phenyl-1,2-dihydro quinolin-2-one

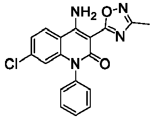

The final product of Example 15 (20 mg) was dissolved in 1,4-dioxane (5 mL), *N*-hydroxyacetamide (9 mg) and cesium carbonate (20 mg) wee added, and the mixture was heated to 110 °C and stirred for 40 hours. The mixture was cooled to room temperature, water was added, the mixture was extracted with dichloromethane, the extract was dried over anhydrous sodium sulphate and filtered, the filtrate was concentrated under reduced pressure, and the residue was purified through column chromatography (dichloromethane/methanol = 20:1) to obtain the product (11 mg).

¹H NMR (400 MHz, DMSO-*d₆*) δ 8.56-9.75 (brs, 2H), 8.34 (d, *J* = 8.8 Hz, 1H), 7.59-7.63 (m, 2H), 7.52-7.56 (m, 1H), 7.37 (dd, *J* = 8.8 Hz, 2.0 Hz, 1H), 7.32 (d, *J* = 8.0 Hz, 2H), 6.35 (d, *J* = 2.0 Hz, 1H), 2.41 (s, 3H).

### Example 57. 4-amino-7-chloro-3-(5-methyl-1,3,4-oxadiazol-2-yl)-1-phenyl-1,2-dihydroquinolin-2-one

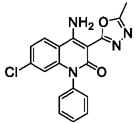

The final product of Example 15 (300 mg) was dissolved in anhydrous methanol (10 mL), a solution of 85% hydrazine hydrate (2 mL) was added, and the mixture was stirred at room temperature for 24 hours. The mixture was filtered, the raw product was dissolved in dichloromethane (5 mL), acetyl chloride (24 mg) was slowly added, and the mixture was stirred at room temperature for 3 hours. 1 Mol/L hydrochloric acid (5 mL) was added, the solution was separated, the organic phase was dried over anhydrous sodium sulphate, the mixture was filtered, the filtrate was concentrated under reduced pressure, the residue was separated through column chromatography (dichloromethane/methanol = 10:1) to obtain a white solid, which was dissolved in acetonitrile (6 mL), phosphoryl trichloride (6 drops) and catalytical amount of 4-dimethylaminopyridine were added, the mixture was refluxed overnight, cooled to room temperature and concentrated under reduced pressure, and the residue was separated through column chromatography (dichloromethane/methanol = 20:1) to obtain the product (74 mg).

¹H NMR (400 MHz, DMSO-*d₆*) δ 8.54-8.80 (brs, 2H), 8.31 (d, *J* = 8.8 Hz, 1H), 7.58-7.62 (m, 2H), 7.51-7.55 (m, 1H), 7.34 (d, *J* = 8.8 Hz, 1H), 7.31 (d, *J* = 7.6 Hz, 2H), 6.36 (s, 1H), 2.48 (s, 3H).

### Example 58. 4-amino-7-bromo-3-(3-methyl-1,2,4-oxadiazol-5-yl)-1-phenyl-1,2-dihydroquinolin-2-one

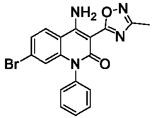

The final product of Example 22 was used as the starting material to obtain the product with reference to the preparation route of Example 56.

¹H NMR (400 MHz, DMSO-*d₆*) δ 8.50-9.72 (brs, 2H), 8.25 (d, *J=* 8.8 Hz, 1H), 7.59-7.63 (m, 2H), 7.52-7.55 (m, 1H), 7.49 (dd, *J =* 8.8 Hz, 1.6 Hz, 1H), 7.32 (d, *J =* 7.6 Hz, 2H), 6.50 (d, *J =* 2.0 Hz, 1H), 2.41 (s, 3H).

### Example 59. methyl 4-amino-1-(2,3-dihydro-1-benzofuran-4-yl)-7-iodo-2-oxo-1,2-dihydro quinoline-3 -carboxylate

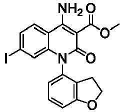

According to the preparation route of scheme 2, 2-bromo-4-iodobenzoic acid and 2,3-dihydro-1-benzofuran-4-amine were used as the starting material to obtain the product with reference to the preparation route of Example 15.

¹H NMR (400 MHz, DMSO-*d₆*) δ 8.42 (s, 2H), 8.01 (d, *J* = 8.6 Hz, 1H), 7.59 (dd, *J* = 8.5, 1.7 Hz, 1H), 7.29 (t, *J =* 7.9 Hz, 1H), 6.91 (d, *J =* 8.0 Hz, 1H), 6.81 (d, *J =* 1.6 Hz, 1H), 6.69 (d, *J =* 7.8 Hz, 1H), 4.54 (t, *J =* 8.8 Hz, 2H), 3.72 (s, 3H), 2.87 (dt, *J =* 17.1, 8.8 Hz, 1H), 2.75 (dt, *J =* 16.3, 8.6 Hz, 1H).

### Example 60. methyl 4-atnino-2-oxo-1-(pyridin-2-yl)-7-(trifluoromethyl)-1,2-dihydroquinoline-3 -carboxylate

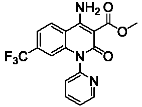

According to the preparation route of scheme 2, 2-bromopyridine and 2-amino-4-(trifluoromethyl)benzoic acid were used as the starting material to obtain the product with reference to the preparation route of Example 15.

¹H NMR (400 MHz, CD₃OD) δ 8.74 (s, 1H), 8.34 (d, *J =* 9.2 Hz, 1H), 8.16 (t, *J =* 8.4 Hz, 1H), 7.66 (d, *J =* 6.8 Hz, 1H), 7.52-7.56 (m, 2H), 6.58 (s, 1H), 3.82 (s, 3H).

### Example 61. methyl 4-atnino-2-oxo-1-(pyrimidin-2-yl)-7-(trifluoromethyl)-1,2-dihydroquinoline-3 -carboxylate

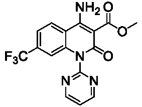

According to the preparation route of scheme 2, 2-bromopyrimidine and 2-amino-4-(trifluoromethyl)benzoic acid were used as the starting material to obtain the product with reference to the preparation route of Example 15.

¹H NMR (400 MHz, CD₃OD) δ 9.27 (d, *J* = 8.8 Hz, 1H), 8.40 (d, *J* = 8.4 Hz, 1H), 8.34 (dd, *J* = 7.2 Hz, 2.0 Hz, 1H), 7.82-8.07 (m, 2H), 6.54 (t, *J =* 7.2 Hz, 1H), 3.85 (s, 3H).

### Example 62. methyl 4-amino-7-(trifluoromethyl)-1-(4-(trifluoromethoxy)phenyl)-2-oxo-1,2-dihydroquinoline-3 -carboxylate

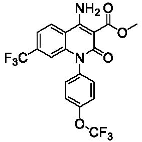

According to the preparation route of scheme 2, 2-bromo-4-trifluoromethylbenzoic acid and 4-trifluoromethoxyaniline were used as the starting material to obtain the product with reference to the preparation route of Example 2.

¹H NMR (400 MHz, DMSO-*d₆*) δ 8.46-8.48 (m, 3H), 7.55-7.59 (m, 3H), 7.45 (d, *J =* 8.4 Hz, 2H), 6.56 (s, 1H), 3.72 (s, 3H).

### Example 63. methyl 4-atnino-1-(4-(difluoromethoxy)phenyl)-2-oxo-7-(trifluoromethyl)-1,2-dihydroquinoline-3 -carboxylate

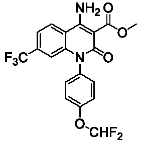

According to the preparation route of scheme 2, 2-bromo-4-trifluoromethylbenzoic acid and 4-difluoromethoxyaniline were used as the starting material to obtain the product with reference to the preparation route of Example 2.

¹H NMR (400 MHz, DMSO-*d₆*) δ 8.46 (d, *J* = 8.8 Hz, 1H), 8.41 (s, 2H), 7.56 (dd, *J =* 8.8 Hz, 1.6 Hz, 1H), 7.37 (t, *J* = 73.6 Hz, 1H), 7.37 (s, 4H), 6.60 (s, 1H), 3.72 (s, 3H).

### Example 64. methyl 4-amino-1-(4-hydroxyphenyl)-2-oxo-7-(trifluoromethyl)-1,2-dihydro quinoline-3 -carboxylate

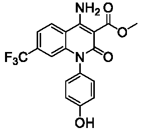

While being cooled with an ice-bath, Example 25 (50 mg) was dissolved in dichloromethane (10 mL), boron tribromide (1 mL) was added slowly, and the mixture was stirred for 2 hours. The reaction solution was filtered, to the filtrate was added water (20 mL), the mixture was extracted with dichloromethane (20 mL*3), the extract was dried over anhydrous sodium sulphate and filtered, the filtrate was concentrated under reduced pressure, and the residue was purified through column chromatography (dichloromethane/methanol = 15:1) to obtain the product (32 mg).

¹H NMR (400 MHz, DMSO-*d₆*) δ 10.12 (s, 1H), 9.15 (s, 1H), 8.61 (d, *J* = 8.8 Hz, 1H), 7.74 (d, *J* = 8.8 Hz, 1.2 Hz, 1H), 7.31-7.35 (m, 2H), 7.14-7.17 (m, 2H), 6.79 (s, 1H), 3.84 (s, 3H).

### Example 65. methyl 4-amino-7-((trimethylsilyl)ethynyl)-2-oxo-1-phenyl-1,2-dihydro quinoline-3 -carboxylate

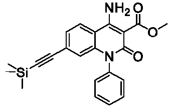

Under nitrogen protection, Example 16 (50 mg), ethynyltrimethylsilane (48 mg), dichloro[bis(diphenylphosphinophenyl)ether]palladium(II) (8 mg), triethylamine (60 mg) and tetrahydrofuran (5 mL) were heated to 90 °C in a sealed tube and reacted for 12 hours. The reaction solution was cooled to room temperature and filtered, the filtrate was concentrated under reduced pressure, and the residue was purified through column chromatography (dichloromethane/ methanol = 25:1) to obtain the product (55 mg).

¹H NMR (400 MHz, CDCl₃) δ 7.42-7.84 (m, 6H), 7.21-7.24 (m, 3H), 6.67 (d, *J* = 1.2 Hz, 1H), 3.88 (s, 3H), 0.19 (s, 9H).

### Example 66. methyl 4-amino-7-cyclopropyl-2-oxo-1-phenyl-1,2-dihydroquinoline-3-carboxylate

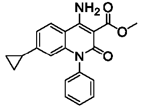

Under nitrogen protection, a solution of Example 16 (50 mg), cyclopropylboronic acid (31 mg), 1,1'-bis(diphenylphosphino)ferrocene-palladium(II)dichloride (9 mg) and sodium carbonate (64 mg) dissolved in 1.4-dioxane (10 mL) and water (2 mL) was heated to 90 °C and stirred for 12 hours. The reaction solution was cooled to room temperature and filtered, to the filtrate was added water (100 mL), the mixture was extracted with dichloromethane (100 mL*3), the extract was dried over anhydrous sodium sulphate and filtered, the filtrate was concentrated under reduced pressure, and the residue was purified through column chromatography (dichloromethane/ methanol = 25:1) to obtain the product (18 mg).

¹H NMR (400 MHz, CDCl₃) δ 7.34-7.74 (m, 6H), 7.22-7.24 (m, 2H), 6.80 *(d, J=* 7.2 Hz, 1H), 6.32 (s, 1H), 3.90 (s, 3H), 1.72-1.80 (m, 1H), 0.94-0.99 (m, 2H), 0.58-0.63 (m, 2H).

### Example 67. methyl 4-amino-7-ethynyl-2-oxo-1-phenyl-1,2-dihydroquinoline-3- carboxylate

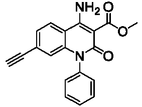

Example 65 (53 mg) was dissolved in methanol (10 mL), tetrabutylammonium fluoride trihydrate (135 mg) was added, and the mixture was heated to 60 °C and stirred for 3 hours. The mixture was cooled to room temperature and concentrated under reduced pressure, and the residue was purified through column chromatography (dichloromethane/methanol = 20:1) to obtain the product (22 mg).

¹H NMR (400 MHz, CDCl₃) δ 7.75 (d, *J* = 8.8 Hz, 1H), 7.47-7.51 (m, 2H), 7.40-7.44 (m, 1H), 7.18 (dd, *J* = 8.8 Hz, 1.6 Hz, 1H), 7.13-7.15 (m, 2H), 6.61 (s, 1H), 3.79 (s, 3H), 3.12 (s, 1H).

### Example 68. methyl 4-amino-7-morpholino-2-oxo-1-phenyl-1,2-dihydroquinoline-3-carboxylate

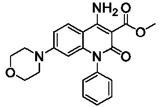

Under nitrogen protection, Example 16 (50 mg), morpholine (52 mg), (2-dicyclohexylphosphino-2',6'-diisopropoxy-1,1'-biphenyl)[2-(2'-amino-1,1'-biphenyl)]palladiu m (II) methanesulfonate (10 mg), cesium carbonate (79 mg) and 1,4-dioxane (10 mL) were heated in a sealed tube to 110 °C and reacted for 12 hours. The reaction solution was cooled to room temperature and filtered, the filtrate was concentrated under reduced pressure, and the residue was purified through column chromatography (dichloromethane/methanol = 20:1) to obtain the product (20 mg).

¹H NMR (400 MHz, CDCl₃) δ 7.49-7.54 (m, 3H), 7.42-7.46 (m, 1H), 7.22-7.24 (m, 2H), 6.72 (dd, *J =* 8.8 Hz, 2.4 Hz, 1H), 5.83 (d, *J =* 2.4 Hz, 1H), 3.87 (s, 3H), 3.72-3.74 (m, 4H), 3.02-3.04 (m, 4H).

### Example 69. methyl 4-amino-1-(2-chlorophenyl)-2-oxo-7-(trifluoromethyl)-1,2-dihydro quinoline-3 -carboxylate

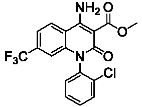

According to the preparation route of scheme 2, 2-bromo-4-trifluoromethylbenzoic acid and 2-chloroaniline were used as the starting material to obtain the product with reference to the preparation route of Example 15.

¹H NMR (399 MHz, DMSO-*d₆*) δ 8.59 (s, 2H), 8.54 (d, *J =* 8.5 Hz, 1H), 7.82-7.72 (m, 1H), 7.66-7.57 (m, 3H), 7.55-7.48 (m, 1H), 6.46 (d, *J* = 1.7 Hz, 1H), 3.75 (s, 3H).

### Example 70. methyl 4-amino-2-oxo-1-(pyridin-3-yl)-7-(trifluoromethyl)-1,2-dihydro quinoline-3 -carboxylate

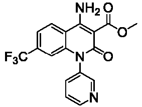

According to the preparation route of scheme 2, 3-bromopyridine and 2-amino-4-(trifluoromethyl)benzoic acid were used as the starting material to obtain the product with reference to the preparation route of Example 15.

¹H NMR (400 MHz, CD₃OD) δ 8.74 (d, *J =* 4.0 Hz, 1H), 8.53 (s, 1H), 8.35 (d, *J =* 8.4 Hz, 1H), 7.86-7.89 (m, 1H), 7.71 (dd, *J =* 8.4 Hz, 4.8 Hz, 1H), 7.55 (dd, *J =* 8.4 Hz, 1.6 Hz, 1H), 6.72(s, 1H), 3.82 (s, 3H).

### Example 71. methyl 4-atnino-2-oxo-1-(pyridin-4-yl)-7-(trifluoromethyl)-1,2-dihydroquinoline-3 -carboxylate

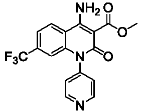

According to the preparation route of scheme 2, 4-bromopyridine and 2-amino-4-(trifluoromethyl)benzoic acid were used as the starting material to obtain the product with reference to the preparation route of Example 15.

¹H NMR (400 MHz, CDCl₃) δ 8.72 (d, *J =* 5.6 Hz, 2H), 8.17 (d, *J =* 8.8 Hz, 1H), 7.39-7.44 (brs, 2H), 7.29 *(d, J=* 8.2 Hz, 1H), 7.13 *(d, J=* 5.6 Hz, 2H), 6.60 (s, 1H), 3.75 (s, 3H).

### Example 72. methyl 4-amino-2-oxo-1-(3,4,5,6-tetrahydro-2H-pyran-4-yl)-7-(trifluoromethyl)-1,2-dihydroquinoline-3 -carboxylate

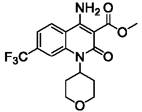

According to the preparation route of scheme 2, 2-bromo-4-trifluoromethylbenzoic acid and 4-aminotetrahydropyran were used as the starting material to obtain the product with reference to the preparation route of Example 15.

¹H NMR (400 MHz, DMSO-*d₆*) δ 8.37 (d, *J =* 8.5 Hz, 1H), 7.99 (s, 2H), 7.90 (s, 1H), 7.57 (d, *J* = 8.5 Hz, 1H), 4.92 (s, 1H), 3.95 (dd, *J =* 11.4, 4.5 Hz, 2H), 3.76 (s, 3H), 3.53 (td, *J* = 11.8, 2.0 Hz, 2H), 2.74 (qd, *J* = 12.2, 4.7 Hz, 2H), 1.56 (dd, *J* = 13.1, 4.0 Hz, 2H).

### Example 73. methyl 4-atnino-7-cyclopropyl-1-(4-methoxyphenyl)-2-oxo-1-phenyl-1,2-dihydroquinoline-3 -carboxylate

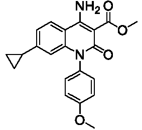

Example 79 was used as starting material to obtain the product (10 mg) with the reaction route reference to the synthetic method of Example 66.

¹H NMR (400 MHz, CDCl₃) δ 7.51 (d, *J* = 8.4 Hz, 1H), 7.13 (d, *J* = 8.8 Hz, 2H), 7.04 (d, *J* = 8.8 Hz, 2H), 6.75 (d, *J =* 8.4 Hz, 1H), 6.38 (d, *J =* 1.2 Hz, 1H), 3.87 (s, 6H), 1.74-1.80 (m, 1H), 0.93-0.98 (m, 2H), 0.59-0.63 (m, 2H).

### Example 74. methyl 4-atnino-7-(methoxycarbonyl)-2-oxo-1-phenyl-1,2-dihydroquinoline-3-carboxylate

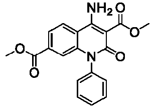

Example 16 (50 mg), 1,1'-bis(diphenylphosphino)ferrocene-palladium(II)dichloride (9 mg) and triethylamine (60 mg) were dissolved in methanol (15 mL), carbon monoxide was purged into the mixture three times, and the mixture was heated to 60 °C and stirred for 12 hours. The reaction solution was cooled to room temperature and filtered, the filtrate was concentrated under reduced pressure, and the residue was purified through column chromatography (dichloromethane/ methanol = 20:1) to obtain the product (20 mg).

¹H NMR (400 MHz, CDCl₃) δ 7.79 (dd, *J =* 8.8 Hz, 1.6 Hz, 1H), 7.71 (d, *J =* 8.8 Hz, 1H), 7.54-7.58 (m, 2H), 7.47-7.51 (m, 1H), 7.28 (d, *J =* 1.6 Hz, 1H), 7.22-7.24 (m, 2H), 3.90 (s, 3H), 3.83 (s, 3H).

### Example 75. methyl 4-amino-1-(2,2-difluorobenzo[d][1,3]dioxol-5-yl)-2-oxo-7-(trifluoro methyl)-1,2-dihydroquinoline-3-carboxylate

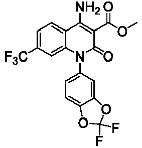

According to the preparation route of scheme 2, 2-iodo-4-(trifluoromethyl)benzoic acid and 2,2-difluorobenzo[d][1,3]dioxol-5-amine were used as the starting material to obtain the product with reference to the preparation route of Example 15.

¹H NMR (400 MHz, DMSO-*d₆*) δ 8.41-8.52 (m, 3H), 7.56-7.61 (m, 2H), 7.52 (s, 1H), 7.17 (d, *J* = 7.6 Hz, 1H), 6.69 (s, 1H), 3.71 (s, 3H).

### Example 76. methyl 4-amino-1-(3H-benzo[d]imidazol-5-yl)-2-oxo-7-(trifluoromethyl)-1,2-dihydroquinoline-3 -carboxylate

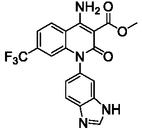

According to the preparation route of scheme 2, 2-amino-4-(trifluoromethyl)benzoic acid and 6-bromo-*1H*-benzo[d]imidazole were used as the starting material to obtain the product with reference to the preparation route of Example 15.

¹H NMR (400 MHz, DMSO-*d₆*) δ 12.44-13.12 (brs, 1H), 8.46 (d, *J =* 8.8 Hz, 1H), 8.37 (s, 3H), 7.75 (d, *J* = 8.8 Hz, 1H), 7.52-7.54 (m, 2H), 7.04 (dd, *J* = 8.4 Hz, 2.0 Hz, 1H), 6.60 (s, 1H), 3.71 (s, 3H).

### Example 77. methyl 4-atnino-1-cyclohexyl-2-oxo-7-(trifluoromethyl)-1,2-dihydroquinoline-3-carboxylate

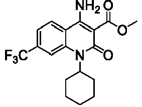

According to the preparation route of scheme 2, 2-iodo-4-(trifluoromethyl)benzoic acid and cyclohexanamine were used as the starting material to obtain the product with reference to the preparation route of Example 15.

¹H NMR (400 MHz, DMSO-d₆) δ 8.33 (d, *J =* 8.4 Hz, 1H), 7.94 (s, 2H), 7.78 (s, 1H), 7.52 (d, *J =* 8.4 Hz, 1H), 4.48-4.86 (m, 1H), 3.72 (s, 3H), 2.33-2.46 (m, 2H), 1.74-1.84 (m, 2H), 1.56-1.70 (m, 3H), 1.35-1.50 (m, 2H), 1.08-1.22 (m, 1H).

### Example 78. methyl 4-atnino-7-bromo-1-(4-fluorophenyl)-2-oxo-1,2-dihydroquinoline-3-carboxylate

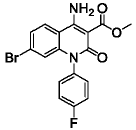

According to the preparation route of scheme 2, 2,4-dibromobenzoic acid and 4-fluoroaniline were used as the starting material to obtain the product with reference to the preparation route of Example 15.

¹H NMR (400 MHz, DMSO-d₆) δ 8.43 (s, 2H), 8.19 (d, *J* = 8.8 Hz, 1H), 7.47-7.39 (m, 3H), 7.37-7.30 (m, 2H), 6.50 *(d, J=* 1.9 Hz, 1H), 3.71 (s, 3H).

### Example 79. methyl 4-atnino-7-iodo-1-(4-methoxyphenyl)-2-oxo-1,2-dihydroquinoline-3-carboxylate

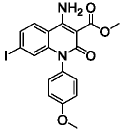

According to the preparation route of scheme 2, 2-bromo-4-iodobenzoic acid and 4-methoxyaniline were used as the starting material to obtain the product with reference to the preparation route of Example 15.

¹H NMR (399 MHz, DMSO-d₆) δ 8.34 (s, 2H), 7.98 (d, *J* = 8.5 Hz, 1H), 7.55 (d, *J* = 8.6 Hz, 1H), 7.14 (q, *J* = 8.9 Hz, 4H), 6.75 (d, *J* = 1.6 Hz, 1H), 3.85 (s, 3H), 3.71 (s, 3H).

### Example 80. methyl 4-amino-1-(4-methoxy-2-methylphenyl)-2-oxo-7-(trifluoromethyl)-1,2-dihydroquinoline-3 -carboxylate

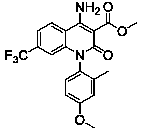

According to the preparation route of scheme 2, 2-bromo-4-trifluoromethylbenzoic acid and 4-methoxy-2-methylaniline were used as the starting material to obtain the product with reference to the preparation route of Example 15.

¹H NMR (400 MHz, DMSO-d₆) δ 8.46 (d, *J* = 8.4 Hz, 1H), 8.40 (s, 2H), 7.56 (d, *J* = 9.6 Hz, 1H), 7.08 (d, *J =* 8.4 Hz, 1H), 7.02 (d, *J* = 2.8 Hz, 1H), 6.92 (dd, *J =* 8.8 Hz, 2.8 Hz, 1H), 6.53 (s, 1H), 3.81 (s, 3H), 3.72 (s, 3H), 1.83 (s, 3H).

### Example 81. methyl 4-amino-1-(4-bromophenyl)-2-oxo-7-(trifluoromethyl)-1,2-dihydro quinoline-3 -carboxylate

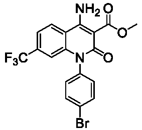

### Step 1: 2-((4-bromophenyl)amino)-4-(trifluoromethyl)benzonitrile

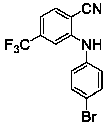

Under nitrogen protection, 2-amino-4-trifluoromethylbenzonitrile (372 mg), 1-bromo-4-iodobenzene (566 mg), palladium (II) acetate (55 mg), 4,5-bis(diphenylphosphino)-9,9-dimethylxanthene (231 mg) and cesium carbonate (1.30 g) were dissolved in 1,4-dioxane (10 mL), and the mixture was heated to 130 °C and stirred for 12 hours. The mixture was cooled to room temperature and filtered, the filtrate was concentrated under reduced pressure, and the residue was purified through column chromatography (petroleum ether/ethyl acetate = 5:1) to obtain the target compound (489 mg).

### Step 2: methyl 4-amino-1-(4-bromophenyl)-2-oxo-7-(trifluoromethyl)-1,2-dihydroquinoline-3-carboxylate

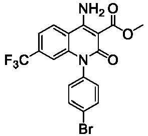

A mixture of 2-((4-bromophenyl)amino)-4-(trifluoromethyl)benzonitrile (100 mg), dimethyl malonate (210 mg), *N*,*N*-dimethylformamide (10 mL) and sodium *tert*-butoxide (290 mg) was heated to 90 °C and stirred for 12 hours. The mixture was cooled to room temperature, water (100 mL) was added, the mixture was extracted with dichloromethane (100 mL*3), the extract was dried over anhydrous sodium sulphate and filtered, the filtrate was concentrated under reduced pressure, and the residue was purified through column chromatography (dichloromethane/ methanol = 10:1) to obtain the target compound (12 mg).

¹H NMR (400 MHz, DMSO-d₆) δ 8.46 (d, *J =* 8.0 Hz, 1H), 8.43 (s, 2H), 7.78 (d, *J =* 8.8 Hz, 2H), 7.57 (d, *J* = 8.4 Hz, 1.6 Hz, 1H), 7.28 (d, *J =* 8.8 Hz, 2H), 6.59 (s, 1H), 3.71 (s, 3H).

### Example 82. methyl 4-amino-1-(naphthalen-1-yl)-2-oxo-7-(trifluoromethyl)-1,2-dihydroquinoline-3 -carboxylate

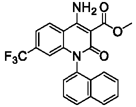

According to the preparation route of scheme 2, 2-fluoro-4-trifluoromethylbenzoic acid and naphthalen-1-amine were used as the starting material to obtain the product with reference to the preparation route of Example 15.

¹H NMR (400 MHz, DMSO-d₆) δ 8.50-8.58 (m, 3H), 8.12 (d, *J =* 8.0 Hz, 1H), 8.09 (d, *J =* 7.6 Hz, 1H), 7.70 (t, *J =* 8.0 Hz, 1H), 7.52-7.59 (m, 3H), 7.45 (t, *J =* 7.6 Hz, 1H), 7.30 (d, *J =* 8.0 Hz, 1H), 6.35 (s, 1H), 3.71 (s, 3H).

### Example 83. methyl 4-amino-7-bromo-1-(4-((difluoromethyl)oxy)phenyl)-2-oxo-1,2-dihydroquinoline-3 -carboxylate

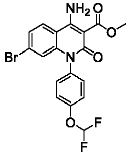

According to the preparation route of scheme 2, 2,4-dibromobenzoic acid and 4-(difluoromethoxy)aniline were used as the starting material to obtain the product with reference to the preparation route of Example 15.

¹H NMR (400 MHz, DMSO-d₆) δ 8.39 (s, 2H), 8.16 *(d, J=* 8.4 Hz, 1H), 7.41 (dd, *J* = 8.8 Hz, 2.0 Hz, 1H), 7.36 (t, *J =* 74.0 Hz, 1H), 7.31-7.37 (m, 4H), 6.49 (d, *J =* 1.6 Hz, 1H), 3.69 (s, 3H).

### Example 84. methyl 4-amino-2-oxo-1-(pyrimidin-5-yl)-7-(trifluoromethyl)-1,2-dihydro quinoline-3 -carboxylate

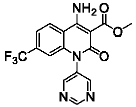

According to the preparation route of scheme 6, 5-bromopyrimidine and 2-amino-4-(trifluoromethyl)benzonitrile were used as the starting material to obtain the product with reference to the preparation route of Example 81.

¹H NMR (400 MHz, DMSO₋*d₆*) δ 9.33 (s, 1H), 8.89 (s, 2H), 8.61 (s, 2H), 8.50 (d, *J =* 8.4 Hz, 1H), 7.63 (d, *J* = 9.2 Hz, 1H), 6.73 (s, 1H), 3.73 (s, 3H).

### Example 85. methyl 4-amino-2-oxo-1-(2-(hydroxymethyl)phenyl)-7-(trifluoromethyl)-1,2-dihydroquinoline-3 -carboxylate

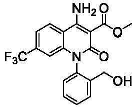

According to the preparation route of scheme 6, 2-bromobenzene methanol and 2-amino-4-(trifluoromethyl)benzonitrile were used as the starting material to obtain the product with reference to the preparation route of Example 81.

¹H NMR (400 MHz, CDCl₃) δ 7.95 (d, *J =* 8.4 Hz, 1H), 7.80-8.20 (brs, 2H), 7.47-7.53 (m, 2H), 7.31 (d, *J =* 8.4 Hz, 1H), 7.25 (s, 1H), 7.12 (d, *J =* 6.8 Hz, 1H), 6.80 (s, 1H), 4.70 (s, 2H), 3.82 (s, 3H), 2.75-3.30 (brs, 1H).

### Example 86. methyl 4-amino-2-oxo-1-(4-(hydroxymethyl)phenyl)-7-(trifluoromethyl)-1,2-dihydroquinoline-3 -carboxylate

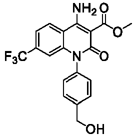

According to the preparation route of scheme 6, 4-bromobenzene methanol and 2-amino-4-(trifluoromethyl)benzonitrile were used as the starting material to obtain the product with reference to the preparation route of Example 81.

¹H NMR (400 MHz, DMSO₋*d₆*) δ 8.44 (d, *J* = 8.4 Hz, 1H), 8.36 (s, 2H), 7.50-7.55 (m, 4H), 7.21 (d, *J =* 8.4 Hz, 1H), 6.60 (s, 1H), 5.34 (t, *J =* 6.0 Hz, 1H), 4.60 (d,J = 6.0 Hz, 2H), 3.71 (s, 3H).

### Example 87. methyl 4-atnino-7-bromo-1-(4-methoxyphenyl)-2-oxo-1,2-dihydroquinoline-3-carboxylate

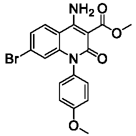

According to the preparation route of scheme 2, 2-iodo-4-bromobenzoic acid and 4-methoxyaniline were used as the starting material to obtain the product with reference to the preparation route of Example 15.

¹H NMR (400 MHz, DMSO-*d₆*) δ 8.32 (s, 2H), 8.14 *(d, J=* 8.8 Hz, 1H), 7.38 (dd, *J* = 8.8 Hz, 2.0 Hz, 1H), 7.08-7.16 (m, 4H), 6.52 (d, *J =* 2.0 Hz, 1H), 3.82 (s, 3H), 3.69 (s, 3H).

### Example 88. methyl 4-amino-1-(isoquinolin-5-yl)-2-oxo-7-(trifluoromethyl)-1,2-dihydroquinoline-3 -carboxylate

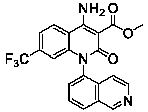

According to the preparation route of scheme 6, 2-bromo-4-trifluoromethylbenzonitrile and 5-amino-isoquinoline were used as the starting material to obtain the product with reference to the preparation route of Example 81.

¹H NMR (400 MHz, DMSO-*d₆*) δ 9.48 (s, 1H), 8.62 (s, 2H), 8.54 (d, *J =* 8.4 Hz, 1H), 8.42 (d, *J* = 6.0 Hz, 1H), 8.33 (d, *J*= 8.4 Hz, 1H), 7.88 (t, *J=* 8.0 Hz, 1H), 7.84 (dd, *J* = 7.6 Hz, 1.6 Hz, 1H), 7.58 (d, *J =* 8.0 Hz, 1H), 7.24 (d, *J =* 6.0 Hz, 1H), 6.33 (s, 1H), 3.70 (s, 3H).

### Example 89. methyl 4-amino-1-(4-ethoxyphenyl)-2-oxo-1,2-dihydroquinoline-3-carboxylate

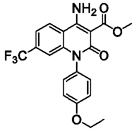

According to the preparation route of scheme 2, 2-bromo-4-trifluoromethylbenzoic acid and 4-ethoxyaniline were used as the starting material to obtain the product with reference to the preparation route of Example 15.

¹H NMR (400 MHz, DMSO-*d₆*) δ 8.43 (d, *J =* 8.4 Hz, 1H), 8.34 (s, 2H), 7.53 (d, *J =* 8.8 Hz, 1H), 7.16 (d, *J =* 9.2 Hz, 2H), 7.08 (d, *J =* 9.2 Hz, 2H), 6.64 (s, 1H), 4.09 (q, *J =* 6.8 Hz, 2H), 3.71 (s, 3H), 1.35 (t, *J* = 6.8 Hz, 3H).

### Example 90. methyl 4-atnino-7-bromo-1-(4-ethoxyphenyl)-2-oxo-1,2-dihydroquinoline-3-carboxylate

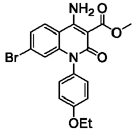

According to the preparation route of scheme 2, 2,4-dibromobenzoic acid and 4-ethoxyaniline were used as the starting material to obtain the product with reference to the preparation route of Example 15.

¹H NMR (400 MHz, DMSO-*d₆*) δ 8.32 (s, 2H), 8.15 *(d, J=* 8.8 Hz, 1H), 7.38 (dd, *J* = 8.8 Hz, 2.0 Hz, 1H), 7.13 (d, *J* = 9.2 Hz, 2H), 7.07 (d, *J* = 8.8 Hz, 2H), 6.51 (d, *J* = 2.0 Hz, 1H), 4.09 (q, *J* = 7.2 Hz, 2H), 3.69 (s, 3H), 1.35 (t, *J* = 7.2 Hz, 3H).

### Example 91. methyl 4-atnino-7-bromo-1-(4-chlorophenyl)-2-oxo-1,2-dihydroquinoline-3-carboxylate

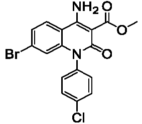

According to the preparation route of scheme 2, 2,4-dibromobenzoic acid and 4-chloroaniline were used as the starting material to obtain the product with reference to the preparation route of Example 15.

¹H NMR (400 MHz, DMSO-*d₆*) δ 8.41 (s, 2H), 8.17 *(d, J=* 8.4 Hz, 1H), 7.63 *(d, J=* 8.4 Hz, 2H), 7.41 (d, *J* = 8.4 Hz, 2.0 Hz, 1H), 7.31 (d, *J* = 8.4 Hz, 2H), 6.49 (d, *J* = 1.6 Hz, 1H), 3.69 (s, 3H).

### Example 92. methyl 4-amino-1-(benzo[d](1,3)dioxol-5-yl)-7-bromo-2-oxo-1,2-dihydroquinoline-3 -carboxylate

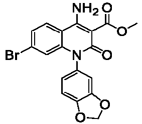

According to the preparation route of scheme 2, 2,4-dibromobenzoic acid and benzo[d][1,3] dioxol-5-amine were used as the starting material to obtain the product with reference to the preparation route of Example 15.

¹H NMR (400 MHz, DMSO-*d₆*) δ 8.33 (s, 2H), 8.14 *(d, J=* 8.8 Hz, 1H), 7.39 (dd, *J* = 8.4 Hz, 2.0 Hz, 1H), 7.06 (d, *J =* 8.0 Hz, 1H), 6.86 (d, *J* = 2.0 Hz, 1H), 6.68 (dd, *J=* 8.0 Hz, 2.0 Hz, 1H), 6.60 (d, *J* = 2.0 Hz, 1H), 6.13 (s, 2H), 3.69 (s, 3H).

### Example 93. methyl 4-atnino-1-(2-bromophenyl)-2-oxo-7-(trifluoromethyl)-1,2-dihydroquinoline-3 -carboxylate

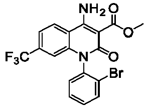

According to the preparation route of scheme 6, 2-amino-4-trifluoromethylbenzonitrile and 1-bromo-2-iodobenzene were used as the starting material to obtain the product with reference to the preparation route of Example 81.

¹H NMR (400 MHz, CDCl₃) δ 7.83 (d, *J* = 8.8 Hz, 1H), 7.79 (dd, *J* = 8.4 Hz, 1.2 Hz, 1H), 7.58-7.80 (brs, 2H), 7.52 (td, *J* = 8.0 Hz, 1.2 Hz, 1H), 7.37-7.43 (m, 2H), 7.32 (dd, *J* = 8.0 Hz, 1.6 Hz, 1H), 6.69 (s, 1H), 3.90 (s, 3H).

### Example 94. methyl 4-atnino-1-(2-cyclopropylphenyl)-2-oxo-7-(trifluoromethyl)-1,2-dihydroquinoline-3 -carboxylate

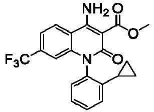

Under nitrogen protection, a mixture of methyl 4-amino-1-(2-bromophenyl)-2-oxo-7-(trifluoro methyl)-1,2-dihydroquinoline-3-carboxylate (30 mg), cyclopropylboronic acid (31 mg), 1,1'-bis(diphenylphosphino)ferrocene-palladium(II)dichloride dichloromethane complex (5 mg), sodium carbonate (37 mg), 1,4-dioxane (6 mL) and water (2 mL) was heated to 90 °C and stirred for 12 hours. The mixture was cooled to room temperature and filtered, to the filtrate was added water (100 mL), the mixture was extracted with dichloromethane (100 mL*3), the extract was dried over anhydrous sodium sulphate and filtered, the filtrate was concentrated under reduced pressure, and the residue was purified through column chromatography (dichloromethane/ methanol = 20:1) to obtain the product (12 mg).

¹H NMR (400 MHz, CDCl₃) δ 7.78 (d, *J* = 8.8 Hz, 1H), 7.55-7.98 (brs, 2H), 7.38-7.43 (m, 2H), 7.34 (td, *J* = 7.6 Hz, 1.6 Hz, 1H), 7.15 (dd, *J* = 7.6 Hz, 1.2 Hz, 1H), 7.09 (dd, *J* = 7.6 Hz, 1.6 Hz, 1H), 6.77 (s, 1H), 3.91 (s, 3H), 1.43-1.50 (m, 1H), 0.79-0.89 (m, 1H), 0.67-0.75 (m, 1H), 0.48-0.55 (m, 1H), 0.29-0.36 (m, 1H).

### Example 95. methyl 4-atnino-7-(ethylamino)-2-oxo-1-phenyl-1,2-dihydroquinoline-3-carboxylate

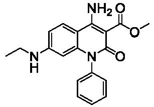

Under nitrogen protection, a mixture of methyl 4-amino-7-iodo-2-oxo-1-phenyl-1,2-dihydroquinoline-3-carboxylate (30 mg), copper(I) iodide(3 mg), L-proline(2 mg), *N*,*N*'-dimethyl formamide (3 mL) and a solution of 2.0 mol/L ethylamine in tetrahydrofuran (1.4 mL) was added into a sealed tube, the mixture was reacted at 60 °C for 12 hours and concentrated under reduced pressure, and the residue was purified through column chromatography ( ethyl acetate / methanol = 30:1) to obtain the product (11 mg).

¹H NMR (400 MHz, CDCl₃) δ 7.48-7.52 (m, 2H), 7.40-7.44 (m, 2H), 7.21 (d, *J* = 7.6 Hz, 2H), 6.41 (dd, *J* = 8.4 Hz, 2.0 Hz, 1H), 5.51 (d, *J* = 1.6 Hz, 1H), 3.98-4.04 (brs, 1H), 3.82 (s, 3H), 2.93-3.00 (m, 2H), 1.13 (t, *J* = 6.8 Hz, 3H).

### Example 96. methyl 4-amino-1-(3-methoxyphenyl)-2-oxo-7-(trifluoromethyl)-1,2-dihydroquinoline-3-carboxylate

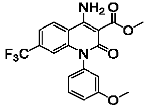

According to the preparation route of scheme 6, 2-amino-4-trifluoromethylbenzonitrile and 1-bromo-3-methoxybenzene were used as the starting material to obtain the product with reference to the preparation route of Example 81.

¹H NMR (400 MHz, CDCl₃) δ 7.78 (d, *J* = 8.0 Hz, 1H), 7.56-7.94 (brs, 2H), 7.46 (t, *J* = 8.0 Hz, 1H), 7.39 (dd, *J* = 8.4 Hz, 1.2 Hz, 1H), 7.04 (dd, *J* = 8.4 Hz, 2.0 Hz, 1H), 6.89 (s, 1H), 6.80 (d, *J* = 7.6 Hz, 1H), 6.76 (t, *J* = 2.0 Hz, 1H), 3.90 (s, 3H), 3.81 (s, 3H).

### Example 97. methyl 4-amino-1-(2-fluoro-4-methoxyphenyl)-2-oxo-7-(trifluoromethyl)-1,2-dihydroquinoline-3 -carboxylate

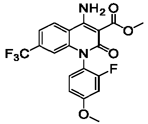

According to the preparation route of scheme 6, 2-amino-4-trifluoromethylbenzonitrile and 1-bromo-2-fluoro-4-methoxybenzene were used as the starting material to obtain the product with reference to the preparation route of Example 81.

¹H NMR (400 MHz, CDCl₃) δ 8.04-8.38 (brs, 1H), 7.95 (dd, *J* = 8.8 Hz, 1H), 7.36 (d, *J* = 8.8 Hz, 1H), 7.09 (t, *J =* 8.8 Hz, 1H), 6.90-6.96 (m, 2H), 6.81 (s, 1H), 3.92 (s, 3H), 3.83 (s, 3H).

### Example 98. methyl 4-amino-2-oxo-1-(4-(2,2,2-trifluoromethylethoxy)phenyl)-7-(trifluoromethyl)-1,2-dihydroquinoline-3 -carboxylate

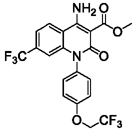

According to the preparation route of scheme 6, 1-bromo-4-(2,2,2-trifluoroethoxy)benzene and 2-amino-4-(trifluoromethyl) benzonitrile were used as the starting material to obtain the product with reference to the preparation route of Example 81.

¹H NMR (400 MHz, DMSO₋*d₆*) δ 8.44 (d, *J =* 8.8 Hz, 1H), 8.36 (s, 2H), 7.54 (d, *J =* 8.4 Hz, 1H), 7.22-7.26 (m, 4H), 6.62 (s, 1H), 4.86 (q, *J =* 8.8 Hz, 2H), 3.72 (s, 3H).

### Example 99. methyl 4-amino-1-(4-(2-hydroxyethoxy)phenyl)-2-oxo-7-(trifluoro methyl)-1,2-dihydroquinoline-3 -carboxylate

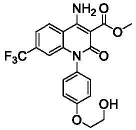

According to the preparation route of scheme 6, 2-(4-bromophenoxy)ethan-1-ol and 2-amino-4-(trifluoromethyl)benzonitrile were used as the starting material to obtain the product with reference to the preparation route of Example 81.

¹H NMR (400 MHz, CDCl₃) δ 7.77 (d, *J* = 8.4 Hz, 1H), 7.40-7.44 (brs, 2H), 7.37 (dd, *J* = 8.4 Hz, 1.2 Hz, 1H), 7.14 (d, *J =* 8.8 Hz, 2H), 7.08 (d, *J* = 8.8 Hz, 2H), 6.90 (s, 1H), 4.16 (t, *J* = 4.4 Hz, 2H), 3.99-4.03 (m, 2H), 3.90 (s, 3H), 2.05 (t, *J =* 6.0 Hz, 1H).

### Example 100. methyl 4-amino-1-(4-(2-ethoxyethoxy)phenyl)-2-oxo-7-(trifluoromethyl)-1,2-dihydroquinoline-3 -carboxylate

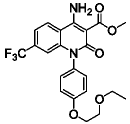

According to the preparation route of scheme 6, 1-bromo-4-(2-ethoxyethoxy)benzene and 2-amino-4-(trifluoromethyl)benzonitrile were used as the starting material to obtain the product with reference to the preparation route of Example 81.

¹H NMR (400 MHz, DMSO_*d₆*) δ 8.43 (d, *J =* 8.4 Hz, 1H), 8.34 (s, 2H), 7.53 (d, *J =* 8.4 Hz, 1H), 7.17 (d, *J =* 8.4 Hz, 2H), 7.11 (d, *J =* 8.4 Hz, 2H), 6.64 (s, 1H), 4.16 (t, *J =* 4.4 Hz, 2H), 3.72 (s, 3H), 3.71-3.73 (m, 2H), 3.52 (q, *J =* 7.2 Hz, 2H), 1.13 (q, *J =* 7.2 Hz, 3H).

### Example 101. methyl 4-atnino-7-bromo-1-(4-methylphenyl)-2-oxo-1,2-dihydroquinoline-3-carboxylate

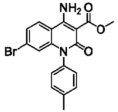

According to the preparation route of scheme 2, 2,4-dibromobenzoic acid and p-toluidine were used as the starting material to obtain the product with reference to the preparation route of Example 15.

¹H NMR (400 MHz, DMSO-*d₆*) δ 8.34 (s, 2H), 8.15 (d, *J =* 8.8 Hz, 1H), 7.35-7.40 (m, 3H), 7.10 (d, *J =* 8.0 Hz, 2H), 6.47 (d, *J =* 2.0 Hz, 1H), 3.69 (s, 3H), 2.39 (s, 3H).

### Example 102. methyl 4-amino-7-bromo-2-oxo-1-(4-((trifluoromethyl)oxy)phenyl)-1,2-dihydroquinoline-3 -carboxylate

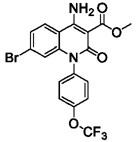

According to the preparation route of scheme 2, 2,4-dibromobenzoic acid and 4-trifluoromethoxyaniline were used as the starting material to obtain the product with reference to the preparation route of Example 15.

¹H NMR (400 MHz, DMSO-*d₆*) δ 8.45 (s, 2H), 8.18 (d, *J =* 8.8 Hz, 1H), 7.57 (d, *J =* 8.4 Hz, 2H), 7.42-7.44 (m, 3H), 6.46 (d, *J =* 2.0 Hz, 1H), 3.69 (s, 3H).

### Example 103. methyl 4-amino-1-(1H-indazol-5-yl)-2-oxo-7-(trifluoromethyl)-1,2-dihydroquinoline-3 -carboxylate

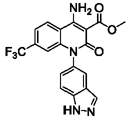

According to the preparation route of scheme 6, 2-amino-4-trifluoromethylbenzonitrile and tert-butyl 5-bromo-*1H*-indazol-1-carboxylate were used as the starting material to obtain the product with reference to the preparation route of Example 81.

¹H NMR (400 MHz, CDCl₃+CD₃OD) δ 8.13 (d, *J =* 8.4 Hz, 1H), 8.05 (s, 1H), 7.67 (d, *J =* 8.8 Hz, 1H), 7.60 (s, 1H), 7.36-7.39 (m, 1H), 7.13 (dd, *J =* 8.8 Hz, 1.6 Hz, 1H), 6.77 (s, 1H), 3.82 (s, 3H).

### Example 104. methyl 1-(4-acetylphenyl)-4-amino-7-bromo-2-oxo-1,2-dihydroquinoline-3-carboxylate

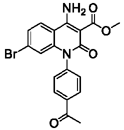

According to the preparation route of scheme 2, 2-iodo-4-bromobenzoic acid and 4-acetylaniline were used as the starting material to obtain the product with reference to the preparation route of Example 15.

¹H NMR (400 MHz, CDCl₃+CD₃OD) δ 8.14 (d, *J =* 8.8 Hz, 2H), 7.88 (d, *J =* 9.2 Hz, 1H), 7.30-7.34 (m, 3H), 6.61 (s, 1H), 3.81 (s, 3H), 2.66 (s, 3H).

### Example 105. methyl 4-amino-7-bromo-1-(4-(ethoxcarbonyl)phenyl)-2-oxo-1,2-dihydroquinoline-3 -carboxylate

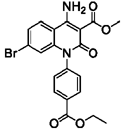

According to the preparation route of scheme 2, 2-iodo-4-bromobenzoic acid and ethyl 4-aminobenzoate were used as the starting material to obtain the product with reference to the preparation route of Example 15.

¹H NMR (400 MHz, DMSO-*d₆*) δ 8.45 (s, 2H), 8.18 *(d, J =* 8.8 Hz, 1H), 8.13 (*d, J =* 8.4 Hz, 2H), 7.41-7.44 (m, 3H), 6.44 (d, *J =* 2.0 Hz, 1H), 4.35 (q, *J =* 7.2 Hz, 2H), 3.69 (s, 3H), 1.34 (t, *J* = 7.2 Hz, 3H).

### Example 106. methyl 4-amino-1-(1H-methyl-1H-indazol-5-yl)-2-oxo-7-(trifluoromethyl)-1,2- dihydroquinoline-3-carboxylate

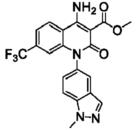

According to the preparation route of scheme 6, 2-amino-4-trifluoromethylbenzonitrile and 5-bromo-1-methyl-*1H*-indazole were used as the starting material to obtain the product with reference to the preparation route of Example 81.

¹H NMR (400 MHz, DMSO-*d₆*) δ 13.85 (s, 1H), 12.56-12.88 (brs, 1H), 8.97 (d, *J =* 8.4 Hz, 1H), 820 (s, 1H), 7.90-7.92 (m, 2H), 7.82 (d, *J =* 8.8 Hz, 1.6 Hz, 1H), 7.43 (dd, *J =* 8.8 Hz, 1.6 Hz, 1H), 6.85 (s, 1H), 4.13 (s, 3H), 3.77 (s, 3H).

### Example 107. methyl 4-amino-7-bromo-1-(4-bromophenyl)-2-oxo-1,2-dihydroquinoline-3-carboxylate

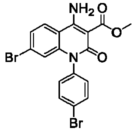

According to the preparation route of scheme 6, 2-amino-4-bromobenzonitrile and 1-bromo-4-iodobenzene were used as the starting material to obtain the product with reference to the preparation route of Example 81.

¹H NMR (400 MHz, DMSO-*d₆*) δ 8.42 (s, 2H), 8.17 (d, *J =* 8.8 Hz, 1H), 7.76 (d, *J* = 8.4 Hz, 2H), 7.42 (dd, *J =* 8.8 Hz, 2.0 Hz, 1H), 7.24 (d, *J =* 8.8 Hz, 2H), 6.49 (d, *J =* 2.0 Hz, 1H), 3.69 (s, 3H).

### Example 108. methyl 4-amino-1-(4-(methylthio)phenyl)-2-oxo-7-(trifluoromethyl)-1,2-dihydroquinoline-3 -carboxylate

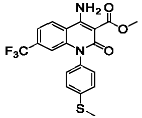

According to the preparation route of scheme 6, 2-amino-4-trifluoromethylbenzonitrile and (4-bromophenyl)(methyl)sulfane were used as the starting material to obtain the product with reference to the preparation route of Example 81.

¹H NMR (400 MHz, CDCl₃) δ 7.77 (d, *J* = 8.4 Hz, 1H), 7.39-7.42 (m, 3H), 7.14 (d, *J* = 8.4 Hz, 2H), 6.91 (s, 1H), 3.90 (s, 3H), 2.54 (s, 3H).

### Example 109. methyl 4-amino-2-oxo-1-phenyl-7-((2,2,2-trifluoroethyl)amino)-1,2-dihydroquinoline-3 -carboxylate

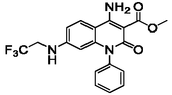

Methyl 4-amino-7-iodo-2-oxo-1-phenyl-1,2-dihydroquinoline-3-carboxylate (30 mg) and trifluoroethylamine (37 mg) were used as the starting material to obtain the product with reference to the preparation route of Example 95.

¹H NMR (400 MHz, CDCl₃) δ 7.43-7.54 (m, 4H), 7.22 (d, *J* = 7.2 Hz, 2H), 6.51 (dd, *J* = 8.8 Hz, 2.4 Hz, 1H), 5.70 (d, *J* = 2.0 Hz, 1H), 4.28 (t, *J* = 6.8 Hz, 1H), 3.86 (s, 3H), 3.58-3.66 (m, 2H).

### Example 110. methyl 4-amino-6-bromo-2-oxo-1-phenyl-1,2-dihydroquinoline- 3-carboxylate

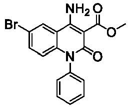

According to the preparation route of scheme 2, 2,5-dibromobenzoic acid and aniline were used as the starting material to obtain the product with reference to the preparation route of Example 15.

¹H NMR (400 MHz, DMSO-*d₆*) δ 8.48 (d, *J =* 2.0 Hz, 1H), 8.33 (s, 2H), 7.54-7.60 (m, 3H), 7.46-7.50 (m, 1H), 7.22 (d, *J =* 7.6 Hz, 2H), 6.32 (d, *J =* 9.2 Hz, 1H), 3.69 (s, 3H).

### Example 111. methyl 4-amino-1-(2,3-dihydro-1-benzofuran-6-yl)-2-oxo-7-(trifluoromethyl)-1,2-dihydroquinoline-3 -carboxylate

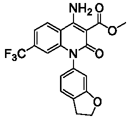

According to the preparation route of scheme 6, 2-amino-4-trifluoromethylbenzonitrile and 6-bromo-2,3-dihydrobenzofuran were used as the starting material to obtain the product with reference to the preparation route of Example 81.

¹H NMR (400 MHz, CDCl₃) δ 7.78 (d, *J =* 8.4 Hz, 1H), 7.38 (d, *J =* 8.0 Hz, 1H), 7.33 (d, *J =* 8.0 Hz, 1H), 6.95 (s, 1H), 6.67 (dd, *J* = 7.6 Hz, 1.6 Hz, 1H), 6.62 (d, *J* = 1.6 Hz, 1H), 4.61-4.72 (m, 2H), 3.89 (s, 3H), 3.26-3.32 (m, 2H).

### Example 112. methyl 4-amino-1-(4-(2-ethoxyethoxy)phenyl)-2-oxo-7-(trifluoromethyl)-1,2-dihydroquinoline-3 -carboxylate

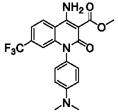

According to the preparation route of scheme 6, 4-bromo-*N,N*-dimethylaniline and 2-amino-4-(trifluoromethyl) benzonitrile were used as the starting material to obtain the product with reference to the preparation route of Example 81.

¹H NMR (400 MHz, CDCl₃) δ 7.75 (d, *J =* 8.8 Hz, 1H),7.60-7.74 (brs, 2H), 7.36 (d, *J =* 8.4 Hz, 1H), 7.00-7.04 (m, 3H), 6.83 (d, *J =* 8.8 Hz, 2H), 3.89 (s, 3H), 3.02 (s, 6H).

### Example 113. methyl 4-amino-1-(4-((dimethylamino)methyl)phenyl)2-oxo-7-(trifluoro methyl)- 1,2-dihydroquinoline-3-carboxylate

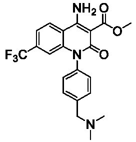

According to the preparation route of scheme 6, 1-(4-bromophenyl)-*N,N*-dimethyl methanamine and 2-amino-4-(trifluoromethyl) benzonitrile were used as the starting material to obtain the product with reference to the preparation route of Example 81.

¹H NMR (400 MHz, CDCl₃) δ 7.77 (d, *J =* 8.4 Hz, 1H),7.60-7.84 (brs, 2H), 7.51 (d, *J =* 8.8 Hz, 2H), 7.38 (dd, *J* = 8.4 Hz, 1.2 Hz, 1H), 7.18 (d, *J =* 8.0 Hz, 2H), 6.85 (s, 1H), 3.90 (s, 3H), 3.52 (s, 2H), 2.29 (s, 6H).

### Example 114. methyl 4-amino-1-(4-(methoxymethyl)phenyl)-2-oxo-7-(trifluoromethyl)-1,2-dihydroquinoline-3-carboxylate

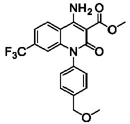

According to the preparation route of scheme 6, 1-bromo-4-(methoxymethyl)benzene and 2-amino-4-(trifluoromethyl)benzonitrile were used as the starting material to obtain the product with reference to the preparation route of Example 81.

¹H NMR (400 MHz, CDCl₃) δ 7.78 (d, *J =* 8.8 Hz, 1H),7.60-8.00 (brs, 2H), 7.53 (d, *J =* 8.0 Hz, 2H), 7.39 (dd, *J* = 8.4 Hz, 1.6 Hz, 1H), 7.21 (dd, *J* = 8.4 Hz, 1.6 Hz, 2H), 6.87 (s, 1H), 4.56 (s, 2H), 3.90 (s, 3H), 3.45 (s, 3H).

### Example 115. methyl 4-atnino-1-(4-(methylamino)phenyl)-2-oxo-7-(trifluoromethyl)-1,2-dihydroquinoline-3-carboxylate hydrochloride

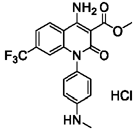

### Step A: N-methyl-N-tert-butoxycarbonyl-4-bromoaniline

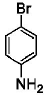

N-methyl-4-bromoaniline (1.85 g) and *N,N*-diisopropylethylamine (2.58 g) were dissolved in dichloromethane (50 mL), di-tert-butyl dicarbonate (3.30 g) was added slowly, the mixture was stirred at room temperature overnight, washed with 1 mol/L hydrochloric acid and brine, dried over anhydrous sodium sulphate and filtered, the filtrate was concentrated under reduced pressure, and the residue was purified through silica gel column chromatography (10% ethyl acetate / petroleum ether) to obtain the product (1.81 g), which was confirmed by MS [M+H]=286.

### Step B: methyl 4-amino-1-(4-((tert-butoxycarbonyl)(methyl)amino)phenyl)-2-oxo-7-(trifluoromethyl)-1,2-dihydroquinoline-3 -carboxylate

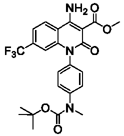

According to the preparation route of scheme 6, *N*-methyl-*N*-*tert*-butoxycarbonyl-4-bromoaniline and 2-amino-4-(trifluoromethyl) benzonitrile were used as the starting material to obtain the product with reference to the preparation route of Example 81, which was confirmed by MS [M+H]=492.

### Step C: methyl 4-amino-1-(4-(methylamino)phenyl)-2-oxo-7-(trifluoromethyl)-1,2-dihydroquinoline-3-carboxylate hydrochloride

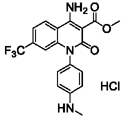

Methyl 4-amino-1-(4-((*tert*-butoxycarbonyl)(methyl)amino)phenyl)-2-oxo-7-(trifluoro methyl)-1,2-dihydroquinoline-3-carboxylate (310 mg) was dissolved in 1,4-dioxane (5 mL), the mixture was cooled to 0 °C, 4 mol/L HCl/1,4-dioxane solution (5 mL) was added, the mixture was stirred at room temperature for 4 hours, and the solution was evaporated to dryness to obtain the product (153 mg).

¹H NMR (400 MHz, DMSO_*d₆*) δ 8.25-8.90 (brs, 2H), 8.35 (d, *J =* 8.0 Hz, 1H), 7.75-7.76 (m, 2H), 7.32-7.50 (brs, 2H), 7.31 (d, *J =* 8.2 Hz, 1H), 7.18 (d, *J =* 8.0 Hz, 2H), 6.63 (s, 1H), 3.73 (s, 3H), 2.90 (s, 3H).

### Example 116. methyl 4-amino-2-oxo-1-phenyl-7-(2,2,2-trifluoroethoxy)-1,2-dihydro quinoline-3 -carboxylate

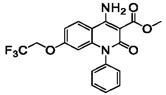

### Step A: 4-bromo-2-(phenylamino)benzonitrile

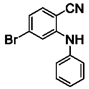

Under nitrogen protection, 2-amino-4-bromobenzonitrile (1.97 g), iodobenzene (3.06 g), tris(dibenzylideneacetone) dipalladium(0) (915 mg), 4,5-bis(diphenylphosphino)-9,9-dimethylxanthene (1.4 g) and cesium carbonate (6.5 g) were dissolved in toluene (50 mL), the mixture was heated to 90 °C, stirred for 12 hours, and filtered through celite, the filtrate was concentrated under reduced pressure, and the residue was purified through column chromatography (petroleum ether/ethyl acetate = 30: 1) to obtain the target compound (1.3 g).

### Step B: 2-(phenylamino)-4-(2,2,2-trifluoroethoxy)benzonitrile

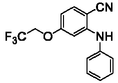

Under nitrogen protection, 4-bromo-2-(phenylamino)benzonitrile (300 mg), trifluoroethanol (550 mg), tris(dibenzylideneacetone) dipalladium(0) (100 mg), 4,5-bis(diphenylphosphino)-9,9-dimethylxanthene (127 mg), cesium carbonate (721 mg) and 1,4-dioxane (20 mL) were added into a sealed tube, the mixture was heated to 90 °C, reacted for 12 hours, and filtered through celite, the filtrate was concentrated under reduced pressure, and the residue was purified through column chromatography (petroleum ether/ethyl acetate = 3:1) to obtain the target compound (120 mg).

### Step C: methyl 4-atnino-2-oxo-1-phenyl-7-(2,2,2-trifluoroethoxy)-1,2-dihydroquinoline-3-carboxylate

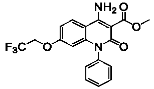

2-(Phenylamino)-4-(2,2,2-trifluoroethoxy)benzonitrile (80 mg) and dimethyl malonate (192 mg) were dissolved in 1,2-dichloroethane (20 mL), tin tetrachloride (344 mg) was added, the mixture was stirred at room temperature for 2 hours, an aqueous solution of saturated sodium bicarbonate (20 mL) was added, lots of solid precipitated, the mixture was filtered through celite, the filtrate was extracted with dichloromethane, the extract was concentrated under reduced pressure, and the residue was purified through column chromatography (dichloromethane/ methanol = 15:1) to obtain the product (41 mg).

¹H NMR (400 MHz, CDCl₃+CD₃OD) δ 7.85 (d, *J* = 8.8 Hz, 1H), 7.49-7.53 (m, 2H), 7.42-7.46 (m, 1H), 7.15-7.17 (m, 2H), 6.77 (dd, *J* = 8.8 Hz, 2.4 Hz, 1H), 5.96 *(d, J* = 2.4 Hz, 1H), 4.16 (q, *J* = 8.0 Hz, 2H), 3.79 (s, 3H).

### Example 117. methyl 4-amino-5-methoxy-2-oxo-1-phenyl-1,2-dihydroquinoline-3-carboxylate

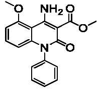

### Step A: 2-methoxy-6-(phenylamino)benzonitrile

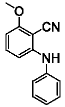

Under nitrogen protection, 2-bromo-6-methoxybenzonitrile (1.0 g), aniline (438 mg), tris(dibenzylideneacetone) dipalladium(0) (430 mg), 4,5-bis(diphenylphosphino)-9,9-dimethylxanthene (543 mg) and cesium carbonate (3.0 g) were dissolved in toluene (50 mL), and the mixture was heated to 110 °C, stirred for 12 hours, and filtered through celite, the filtrate was concentrated under reduced pressure, and the residue was purified through column chromatography (petroleum ether/ethyl acetate = 30: 1) to obtain the target compound (750 mg).

### Step B: methyl 4-amino-5-methoxy-2-oxo-1-phenyl-1,2-dihydroquinoline-3- carboxylate

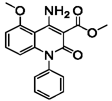

2-Methoxy-6-(phenylamino)benzonitrile (280 mg) and dimethyl malonate (875 mg) were dissolved in 1,2-dichloroethane (20 mL), tin tetrachloride (1.5 g) was added, the mixture was stirred at room temperature for 2 hours, an aqueous solution of saturated sodium bicarbonate (20 mL) was added, lots of solid precipitated, the mixture was filtered through celite, the filtrate was extracted with dichloromethane, the extract was concentrated under reduced pressure, and the residue was purified through column chromatography (dichloromethane/methanol = 15:1) to obtain the product (105 mg).

¹H NMR (400 MHz, CDCl₃) δ 9.64-10.13 (brs, 1H), 8.36-8.82 (brs, 1H), 7.49-7.53 (m, 2H), 7.40-7.45 (m, 1H), 7.18-7.24 (m, 3H), 6.63 (d, *J* = 8.4 Hz, 1H), 6.18 (d, *J* = 8.4 Hz, 1H), 4.03 (s, 3H), 3.86 (s, 3H).

### Example 118. methyl 4-amino-1-(3-methylbenzo[d]imidazol-4-yl)-2-oxo-7-(trifluoromethyl)-1,2-dihydroquinoline-3 -carboxylate

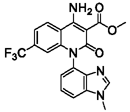

According to the preparation route of scheme 6, 2-amino-4-trifluoromethylbenzonitrile and 4-bromo-1-methyl-*1H*-benzoimidazole were used as the starting material to obtain the product with reference to the preparation route of Example 81.

¹H NMR (400 MHz, DMSO-*d₆*) δ 8.47 (d, *J =* 8.4 Hz, 1H), 8.37 (s, 2H), 8.31 (s, 1H), 7.80 (d, *J =* 8.4 Hz, 1H), 7.58 (d, *J =* 2.0 Hz, 1H), 7.55 (d, *J* = 8.0 Hz, 1H), 7.05 (dd, *J=* 8.8 Hz, 2.0 Hz, 1H), 6.60 (s, 1H), 3.82 (s, 3H), 3.72 (s, 3H).

### Example 119. methyl 4-amino-1-(4-(aminomethyl)phenyl)-2-oxo-7-(trifluoromethyl)-1,2-dihydroquinoline-3-carboxylate hydrochloride

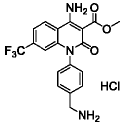

*Tert*-butyl (4-bromobenzyl)carbamate and 2-amino-4-(trifluoromethyl)benzoic acid were used as the starting material to obtain the product with reference to the preparation route of Example 115.

¹H NMR (400 MHz, DMSO₋*d₆*) δ 8.80-8.95 (brs, 5H), 8.32 (d, *J =* 9.2 Hz, 1H), 7.65 (d, *J =* 8.0 Hz, 2H), 7.30 (d, *J =* 7.2 Hz, 1H), 7.19 (d, *J =* 8.0 Hz, 2H), 6.60 (s, 1H), 4.04 (d, d, *J =* 7.2 Hz, 2H), 3.74 (s, 3H).

### Example 120. methyl 4-amino-1-(4-acetylphenyl)-2-oxo-7-(trifluoromethyl)-1,2-dihydroquinoline-3 -carboxylate

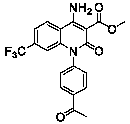

According to the preparation route of scheme 6, 1-(4-bromophenyl)ethan-1-one and 2-amino-4-(trifluoromethyl)benzonitrile were used as the starting material to obtain the product with reference to the preparation route of Example 81.

¹H NMR (400 MHz, DMSO₋*d₆*) δ 8.60-8.95 (brs, 2H), 8.32 (d, *J =* 8.8 Hz, 1H), 8.06 (d, *J =* 8.8 Hz, 2H), 7.27 (d, *J=* 8.4 Hz, 2H), 7.10 (d, *J=* 8.4 Hz, 1H), 6.60 (s, 1H), 3.75 (s, 3H), 2.60 (s, 3H).

### Example 121. methyl 4-amino-1-(4-((methylamino)methyl)phenyl)-2-oxo-7-(trifluoromethyl) -1,2-dihydroquinoline-3-carboxylate hydrochloride

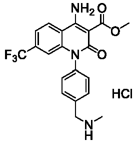

Tert-butyl (4-bromobenzyl)(methyl)carbamate and 2-amino-4-(trifluoromethyl)benzoic acid were used as the starting material to obtain the product with reference to the preparation route of Example 115.

¹H NMR (400 MHz, DMSO_*d₆*) δ 9.05-9.10 (brs, 2H), 8.59-8.70 (brs, 2H), 8.44 (d, *J =* 8.4 Hz, 1H), 7.70 (d, *J =* 8.0 Hz, 2H), 7.39 (d, *J =* 8.4 Hz, 1H), 7.27 (d, *J =* 8.0 Hz, 2H), 6.57 (s, 1H), 4.17-4.20 (m, 2H), 3.72 (s, 3H), 2.62 (s, 3H).

### Example 122. methyl 4-amino-1-(4-(1-hydroxyethyl)phenyl)-2-oxo-7-(trifluoromethyl)-1,2-dihydroquinoline-3 -carboxylate

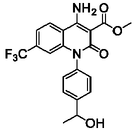

Methyl 4-amino-1-(4-acetylphenyl)-2-oxo-7-(trifluoromethyl)-1,2-dihydroquinoline-3-carboxylate (50 mg) obtained in Example 120 was dissolved in methanol (5 mL), the mixture was cooled to 0 °C, sodium boronhydride (5 mg) was added, the mixture was stirred at room temperature for 6 hours, water (10 mL) was added to quench the reaction, the mixture was extracted with dichloromethane, the extract was washed with brine, dried over anhydrous sodium sulphate and filtered, and the filtrate was concentrated under reduce pressure to obtain the product (34 mg).

¹H NMR (400 MHz, CDCl₃) δ 7.79 (d, *J =* 8.8 Hz, 2H), 7.55-7.61 (m, 3H), 7.41 (d, *J =* 8.8 Hz, 1H), 7.22 (d, *J =* 8.2 Hz, 2H), 6.88 (s, 1H), 5.02 (q, *J =* 6.4 Hz, 1H), 3.89 (s, 3H), 1.95-2.05 (brs, 1H), 1.56 (d, *J =* 6.4 Hz, 3H).

### Example 123. methyl 4-amino-1-(6-(2-hydroxypropan-2-yl)pyridin-2-yl)-2-oxo-7-(trifluoromethyl)-1,2-dihydroquinoline-3 -carboxylate

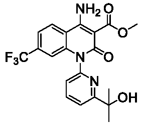

According to the preparation route of scheme 6, 2-(6-bromopyridin-2-yl)propan-2-ol and 2-amino-4-(trifluoromethyl)benzonitrile were used as the starting material to obtain the product with reference to the preparation route of Example 81.

¹H NMR (400 MHz, CDCl₃) δ 7.98 (t, *J =* 8.0 Hz, 1H),7.60-7.84 (brs, 2H), 7.80 (d, *J =* 8.0 Hz, 2H), 7.58 (d, *J =* 8.0 Hz, 1H), 7.43 (d, *J =* 8.4 Hz, 1H), 6.71 (s, 1H), 3.91 (s, 3H), 3.87-3.90 (brs, 1H), 1.55 (d, *J=* 7.2 Hz, 6H).

### Example 124. methyl 4-amino-7-bromo-1-(4-morpholinophenyl)-2-oxo-1,2-dihydro quinoline-3 -carboxylate

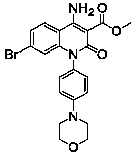

According to the preparation route of scheme 2, 4-bromo-2-iodobenzoic acid and 4-(morpholino-4-yl)aniline were used as the starting material to obtain the product with reference to the preparation route of Example 15.

¹H NMR (400 MHz, DMSO_*d₆*) δ 8.31 (s, 2H), 8.14 (d, *J* = 8.8 Hz, 1H), 7.37 (dd, *J* = 8.4 Hz, 2.4 Hz, 1H), 7.03-7.10 (m, 4H), 6.55 (d, *J* = 2.4 Hz, 1H), 3.74-3.76 (m, 4H), 3.69 (s, 3H), 3.17-3.20 (m, 4H).

### Example 125. methyl 4-atnino-1-(4-iodophenyl)-2-oxo-7-(trifluoromethyl)-1,2-dihydroquinoline-3 -carboxylate

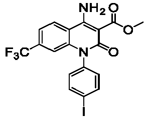

According to the preparation route of scheme 6, 2-amino-4-trifluoromethylbenzonitrile and 1,4-diiodobenzene were used as the starting material to obtain the product with reference to the preparation route of Example 81.

¹H NMR (400 MHz, DMSO_*d₆*) δ 8.46 (d, *J* = 8.8 Hz, 1H), 8.43 (s, 2H), 7.92-7.96 (m, 2H), 7.57 (dd, *J* = 8.4 Hz, 1.6 Hz, 1H), 7.10-7.13 (m, 2H), 6.59 (s, 1H), 3.71 (s, 3H).

### Example 126. methyl 4-atnino-1-(4-cyclopropylphenyl)-2-oxo-7-(trifluoromethyl)-1,2-dihydroquinoline-3 -carboxylate

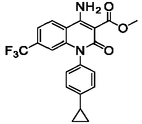

Under nitrogen protection, a mixture of methyl 4-amino-1-(4-iodophenyl)-2-oxo-7-(trifluoro methyl)-1,2-dihydroquinoline-3-carboxylate (50 mg), cyclopropylboronic acid (26 mg), 1,1'-bis(diphenylphosphino)ferrocene-palladium(II)dichloride dichloromethane complex (7 mg), sodium carbonate (53 mg), 1,4-dioxane (6 mL) and water (2 mL) was heated to 90 °C and stirred for 12 hours. The mixture was cooled to room temperature and filtered, to the filtrate was added water (100 mL), the mixture was extracted with dichloromethane (100 mL*3), the extract was dried over anhydrous sodium sulphate and filtered, the filtrate was concentrated under reduced pressure, and the residue was purified through column chromatography (petroleum ether/ethyl acetate = 1:3) to obtain the product (27 mg).

¹H NMR (400 MHz, CDCl₃) δ 7.76 (d, *J* = 8.4 Hz, 1H), 7.49-7.87 (brs, 2H), 7.38 (dd, *J* = 8.4 Hz, 1.2 Hz, 1H), 7.23 (d, *J* = 8.0 Hz, 2H), 7.08 (d, *J* = 8.4 Hz, 2H), 6.90 (s, 1H), 3.89 (s, 3H), 1.94-2.01 (m, 1H), 1.01-1.06 (m, 2H), 0.75-0.79 (m, 2H).

### Example 127. methyl 4-amino-1-((4-carbatnoyl)phenyl)-2-oxo-7-(trifluoromethyl)-1,2-dihydroquinoline-3 -carboxylate

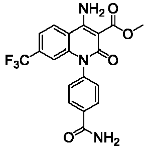

According to the preparation route of scheme 6, 2-amino-4-trifluoromethylbenzonitrile and (4-bromophenyl)methanamine were used as the starting material to obtain the product with reference to the preparation route of Example 81.

¹H NMR (400 MHz, CDCl₃+CD₃OD) δ 8.20 (d, *J =* 8.4 Hz, 1H), 8.05 (d, *J =* 8.0 Hz, 2H), 7.41-7.44 (m, 1H), 7.31 *(d, J=* 8.4 Hz, 2H), 6.71 (s, 1H), 3.83 (s, 3H).

### Example 128. methyl 4-atnino-1-(benzo(2,1-d)(1,3)oxazol-5-yl)-2-oxo-7-(trifluoromethyl)-1,2-dihydroquinoline-3 -carboxylate

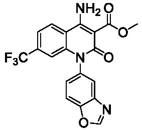

According to the preparation route of scheme 6, 2-amino-4-trifluoromethylbenzonitrile and 5-bromobenzo-oxazole were used as the starting material to obtain the product with reference to the preparation route of Example 81.

¹H NMR (400 MHz, DMSO-*d₆*) δ 8.87 (s, 1H), 8.49 (d, *J =* 8.8 Hz, 1H), 8.45 (s, 2H), 7.97 (d, *J* = 8.4 Hz, 1H), 7.82 (d, *J =* 2.0 Hz, 1H), 7.56 (d, *J =* 8.8 Hz, 1H), 7.35 (dd, *J=* 8.8 Hz, 2.0 Hz, 1H), 6.58 (s, 1H), 3.72 (s, 3H).

### Example 129. methyl 4-atnino-2-oxo-7-(trifluoromethyl)-1-((4-trifluoromethyl)phenyl)-1,2-dihydroquinoline-3 -carboxylate

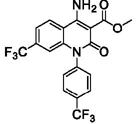

According to the preparation route of scheme 6, 2-amino-4-trifluoromethylbenzonitrile and 1-bromo-4-(trifluoromethyl)benzene were used as the starting material to obtain the product with reference to the preparation route of Example 81.

¹H NMR (400 MHz, CDCl₃+CD₃OD) δ 8.29 (d, *J =* 8.8 Hz, 1H), 7.90 (d, *J =* 8.0 Hz, 2H), 7.44-7.49 (m, 3H), 6.71 (s, 1H), 3.83 (s, 3H).

### Example 130. methyl 4-atnino-2-oxo-7-bromo-1-(4-nitrophenyl)-1,2-dihydroquinoline-3-carboxylate

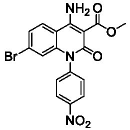

According to the preparation route of scheme 2, 4-bromo-2-iodobenzoic acid and 4-nitroaniline were used as the starting material to obtain the product with reference to the preparation route of Example 15.

¹H NMR (400 MHz, DMSO-*d₆*) δ 8.49 (s, 2H), 8.40 (d, *J =* 8.8 Hz, 2H), 8.19 (d, *J =* 8.4 Hz, 1H), 7.61 (d, *J* = 8.8 Hz, 2H), 7.44 (dd, *J* = 8.8 Hz, 2.0 Hz, 1H), 6.51 (d, *J* = 2.0 Hz, 1H), 3.70 (s, 3H).

### Example 131. methyl 4-amino-1-(4-(1,3-oxazol-5-yl)phenyl)-2-oxo-7-(trifluoro methyl)-1,2-dihydroquinoline-3 -carboxylate

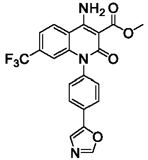

According to the preparation route of scheme 6, 2-amino-4-trifluoromethylbenzonitrile and 5-(4-bromophenyl)-1,3-oxazole were used as the starting material to obtain the product with reference to the preparation route of Example 81.

¹H NMR (400 MHz, DMSO-*d₆*) δ 8.51 (s, 1H), 8.47 (d, *J =* 8.0 Hz, 1H), 8.44 (s, 2H), 7.94 (d, *J =* 8.0 Hz, 2H), 7.82 (s, 1H), 7.57 (d, *J =* 8.0 Hz, 1H), 7.42 (d, *J =* 8.0 Hz, 2H), 6.64 (s, 1H), 3.72 (s, 3H).

### Example 132. methyl 4-amino-2-oxo-7-(trifluoromethyl)-1-(4-vinylphenyl)-1,2-dihydroquinoline-3 -carboxylate

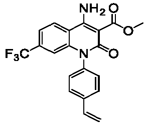

Under nitrogen protection, a mixture of methyl 4-amino-1-(4-iodophenyl)-2-oxo-7-(trifluoromethyl)-1,2-dihydroquinoline-3-carboxylate (50 mg), potassium vinyltrifluoroborate (45 mg), 1,1'-Bis(diphenylphosphino)ferrocene-palladium(II)dichloride dichloromethane complex (7 mg), cesium carbonate (164 mg), 1,4-dioxane (6 mL) and water (2 mL) was heated to 70 °C and stirred for 3 hours. The mixture was cooled to room temperature and filtered, to the filtrate was added water (100 mL), the mixture was extracted with dichloromethane (100 mL*3), the extract was dried over anhydrous sodium sulphate and filtered, the filtrate was concentrated under reduced pressure, and the residue was purified through column chromatography (petroleum ether/ethyl acetate = 1:2) to obtain the product (21 mg).

¹H NMR (400 MHz, CDCl₃) δ 7.77 (d, *J* = 8.4 Hz, 1H), 7.59 (d, *J* = 8.4 Hz, 2H), 7.40 (d, *J =* 8.4 Hz, 1H), 7.19 (d, *J* = 8.4 Hz, 2H), 6.90 (s, 1H), 6.79 (dd, *J* = 17.6 Hz, 11.2 Hz, 1H), 5.84 (d, *J* = 17.6 Hz, 1H), 5.36 (d, *J* = 11.2 Hz, 1H), 3.90 (s, 3H).

### Example 133. methyl 4-amino-1-(4-ethynylphenyl)-2-oxo-7-(trifluoromethyl)-1,2-dihydroquinoline-3 -carboxylate

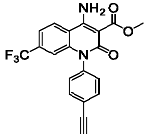

### Step A: methyl 4-amino-2-oxo-7-(trifluoromethyl)-1-(4-(2-(trimethylsilyl)ethynyl) phenyl)-1 ,2-dihydroquinoline-3 -carboxylate

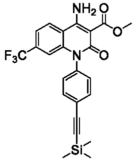

Under nitrogen protection, a mixture of methyl 4-amino-1-(4-iodophenyl)-2-oxo-7-(trifluoro methyl)-1,2-dihydroquinoline-3-carboxylate (50 mg), ethynyltrimethylsilane (39 mg), bis(triphenylphosphine)palladium(II) dichloride (28 mg), copper(I) iodide(7 mg), triethylamine (50 mg) and tetrahydrofuran (6 mL) was heated to 100 °C and stirred for 12 hours. The mixture was cooled to room temperature and filtered, to the filtrate was added water (100 mL), the mixture was extracted with dichloromethane (100 mL*3), the extract was dried over anhydrous sodium sulphate and filtered, the filtrate was concentrated under reduced pressure, and the residue was purified through column chromatography (petroleum ether/ethyl acetate = 1:2) to obtain the product (26 mg).

### Step B: methyl 4-amino-1-(4-ethynylphenyl)-2-oxo-7-(trifluoromethyl)-1,2-dihydroquinoline-3 -carboxylate

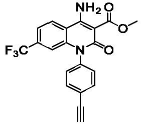

Methyl 4-amino-2-oxo-7-(trifluoromethyl)-1-(4-(2-(trimethylsilyl)ethynyl)phenyl)-1,2-dihydroquinoline-3-carboxylate (25 mg) was dissolved in methanol (10 mL), tetrabutylammonium chloride (51 mg) was added, the mixture was heated to 70 °C, stirred for 2 hours, cooled to room temperature and concentrated under reduced pressure, and the residue was purified through column chromatography (petroleum ether/ethyl acetate = 2:1) to obtain the product (12 mg).

¹H NMR (400 MHz, CDCl₃) δ 7.78 (d, *J* = 8.8 Hz, 1H), 7.69 (d, *J* = 8.4 Hz, 2H), 7.41 (dd, *J* = 8.4 Hz, 1.2 Hz, 1H), 7.21 (d, *J =* 8.8 Hz, 2H), 6.83 (s, 1H), 3.91 (s, 3H), 3.17 (s, 1H).

### Example 134. methyl 4-amino-1-(4-chlorophenyl)-7-cyclopropyl-2-oxo-1,2-dihydro quinoline-3 -carboxylate

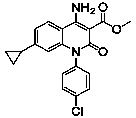

### Step A: 2-amino-4-cyclopropylbenzonitrile

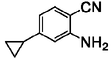

Under nitrogen protection, a mixture of methyl 2-amino-4-bromobenzonitrile (985 mg), cyclopropylboronic acid (516 mg), 1,1'-bis(diphenylphosphino)ferrocene-palladium(II)dichloride dichloromethane complex (365 mg), sodium carbonate (1.06 g), 1,4-dioxane (20 mL) and water (4 mL) was heated to 100 °C and stirred for 12 hours. The mixture was cooled to room temperature and filtered, to the filtrate was added water (100 mL), the mixture was extracted with dichloromethane (100 mL*3), the extract was dried over anhydrous sodium sulphate and filtered, the filtrate was concentrated under reduced pressure, and the residue was purified through column chromatography (petroleum ether/ethyl acetate = 30:1) to obtain the product (550 mg).

### Step B: 2-((4-chlorophenyl)amino)-4-cyclopropylbenzonitrile

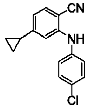

Under nitrogen protection, 2-amino-4-cyclopropylbenzonitrile (550 mg), 1-chloro-4-iodobenzene (1.6 g), tris(dibenzylideneacetone) dipalladium(0) (320 mg), 4,5-bis(diphenyl phosphino)-9,9-dimethylxanthene (402 mg) and cesium carbonate (2.3 g) were mixed in toluene (50 mL), the mixture was reacted at 110 °C for 12 hours and filtered through celite, the filtrate was concentrated under reduced pressure, and the residue was purified through column chromatography (petroleum ether/ethyl acetate = 3:1) to obtain the product (1.03 g).

### Step C: methyl 4-amino-1-(4-chlorophenyl)-7-cyclopropyl-2-oxo-1,2-dihydroquinoline-3-carboxylate

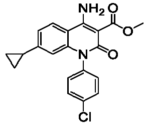

Under nitrogen protection, 2-((4-chlorophenyl)amino)-4-cyclopropylbenzonitrile (150 mg) and dimethyl malonate (392 mg) were dissolved in *N,N*-dimethylformamide (10 mL), sodium *tert*-butoxide (268 mg) was added, the mixture was heated to 90 °C, stirred for 12 hours, and concentrated under reduced pressure, and the residue was purified through column chromatography (petroleum ether/ethyl acetate = 1:3) to obtain the product (108 mg).

¹H NMR (400 MHz, CDCl₃) δ 7.63 (d, *J* = 8.8 Hz, 1H), 7.46 (d, *J* = 8.4 Hz, 2H), 7.11 (d, *J =* 8.4 Hz, 2H), 6.73 (dd, *J* = 8.4 Hz, 1.6 Hz, 1H), 6.22 (d, *J* = 1.2 Hz, 1H), 3.79 (s, 3H), 1.68-1.75 (m, 1H), 0.89-0.94 (m, 2H), 0.53-0.58 (m, 2H).

### Example 135. methyl 4-amino-1-(1-hydroxy-2,3-dihydro-1H-inden-5-yl)-2-oxo-7-(trifluoromethyl)-1,2-dihydroquinoline-3 -carboxylate

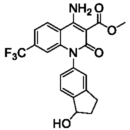

5-Bromo-1-indanone and 2-amino-4-(trifluoromethyl)benzoic acid were used as the starting material to obtain the target product with reference to the preparation route of Example 81 and Example 122.

¹H NMR (400 MHz, CDCl₃) δ 7.79 (d, *J =* 7.2 Hz, 2H),7.56-7.83 (brs, 2H), 7.59 (d, *J =* 8.4 Hz, 1H), 7.40 (d, *J=* 8.8 Hz, 2H), 7.21 (s, 1H), 7.07 (d, *J =* 8.8 Hz, 1H), 6.88 (s, 1H), 5.33 *(t, J=* 6.4 Hz, 1H), 3.89 (s, 3H), 3.71-3.76 (m, 1H), 2.59-2.61 (m, 1H), 2.02-2.07 (m, 1H).

### Example 136. methyl 4-amino-1-(3-acetylphenyl)-2-oxo-7-(trifluoromethyl)-1,2-dihydroquinoline-3 -carboxylate

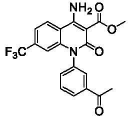

According to the preparation route of scheme 6, 1-(3-bromophenyl)ethan-1-one and 2-amino-4-(trifluoromethyl)benzoic acid were used as the starting material to obtain the product with reference to the preparation route of Example 81.

¹H NMR (400 MHz, CDCl₃) δ 8.11 (d, *J =* 7.6 Hz, 2H), 7.80-7.84 (m, 3H), 7.69 (t, *J =* 8.0 Hz, 1H), 7.46 (d, *J =* 8.4 Hz, 1H), 7.42 (d, *J =* 8.0 Hz, 1H), 6.79 (s, 1H), 3.91 (s, 3H), 2.62 (s, 3H).

### Example 137. methyl 4-amino-1-(3-hydroxy-2,3-dihydro-1H-inden-5-yl)-2-oxo-7-(trifluoromethyl)-1,2-dihydroquinoline-3 -carboxylate

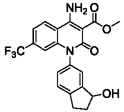

5-Bromo-1-indanone and 2-amino-4-(trifluoromethyl)benzoic acid were used as the starting material to obtain the target product with reference to the preparation route of Example 81 and Example 122.

¹H NMR (400 MHz, CDCl₃) δ 7.80 (dd, *J* = 8.0 Hz, 5.2 Hz, 1H), 7.60-7.85 (brs, 2H), 7.37-7.42 (m, 2H), 7.26 (d, *J*= 8.8 Hz, 1H), 7.10 (dd, *J* = 8.0 Hz, 2.4 Hz, 1H), 6.92 (d, *J* = 14.8 Hz, 1H), 5.27 (t, *J =* 6.0 Hz, 1H), 3.89 (s, 3H), 3.09-3.14 (m, 1H), 2.87-2.95 (m, 2H), 2.56-2.62 (m, 1H), 1.95-2.06 (m, 1H).

### Example 138. methyl 4-amino-1-(3-(1-hydroxyethyl)phenyl)-2-oxo-7-(trifluoromethyl)-1,2-dihydroquinoline-3-carboxylate

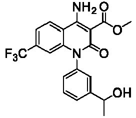

Methyl 4-amino-1-(3-acetylphenyl)-2-oxo-7-(trifluoromethyl)-1,2-dihydroquinoline-3 - carboxylate obtained in Example 136 and sodium boronhydride were used as the starting material to obtain the product with reference to the preparation route of Example 122.

¹H NMR (400 MHz, CDCl₃) δ 7.93 (d, *J =* 8.8 Hz, 1H), 7.70-8.12 (brs, 2H), 7.45-7.56 (m, 2H), 7.30-7.35 (m, 1H), 7.22 (s, 1H), 7.14 (d, *J* = 7.6 Hz, 1H), 6.80 (d, *J* = 6.4 Hz, 1H), 4.93 (quint, *J* = 6.0 Hz, 1H), 3.87 (s, 3H), 2.40-2.60 (brs, 1H), 1.48 (d, *J =* 6.0 Hz, 3H).

### Example 139. methyl 4-amino-7-bromo-1-(4-(dimethylcarbamoyl)phenyl)-2-oxo-1,2-dihydroquinoline-3 -carboxylate

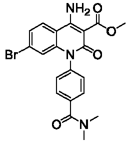

According to the preparation route of scheme 2, 2-iodo-4-bromobenzoic acid and 4-aminobenzoic acid were used as the starting material to obtain the product with reference to the preparation route of Example 15.

¹H NMR (400 MHz, CDCl₃+CD₃OD) δ 7.81 (d, *J =* 8.8 Hz, 1H), 7.58 (d, *J =* 8.4 Hz, 2H), 7.25-7.29 (m, 3H), 6.66 (s, 1H), 3.81 (s, 3H), 3.10 (s, 3H), 3.01 (s, 3H).

### Example 140. methyl 4-amino-7-bromo-1-(4-carboxyphenyl)-2-oxo-1,2-dihydro quinoline-3 -carboxylate

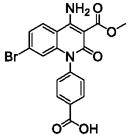

According to the preparation route of scheme 2, 2-iodo-4-bromobenzoic acid and 4-aminobenzoic acid were used as the starting material to obtain the product with reference to the preparation route of Example 15.

¹H NMR (400 MHz, CDCl₃+CD₃OD) δ 8.27 (dd, *J =* 8.4 Hz, 2H), 7.92 (d, *J =* 9.2 Hz, 1H), 7.33-7.38 (m, 3H), 6.68 (d, *J =* 2.0 Hz, 1H), 3.87 (s, 3H).

### Example 141. methyl 4-amino-7-bromo-1-(1,3-dihydro-2-benzofuran-5-yl)-2-oxo-1,2-dihydroquinoline-3 -carboxylate

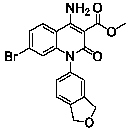

According to the preparation route of scheme 2, 2-iodo-4-bromobenzoic acid and 1,3-dihydroisobenzofuran-5-amine were used as the starting material to obtain the product with reference to the preparation route of Example 15.

¹H NMR (400 MHz, DMSO-*d₆*) δ 8.36 (s, 2H), 8.16 (d, *J =* 8.8 Hz, 1H), 7.49 (d, *J =* 8.0 Hz, 1H), 7.40 (dd, *J=* 8.8 Hz, 2.0 Hz, 1H), 7.19 (s, 1H), 7.13 (dd, *J=* 8.4 Hz, 1.6 Hz, 1H), 6.51 (d,*J* = 2.0 Hz, 1H), 4.99-5.12 (m, 4H), 3.69 (s, 3H).

### Example 142. methyl 4-amino-1-(2,3-dihydro-1-benzofuran-5-yl)-2-oxo-7-(trifluoromethyl)-1,2-dihydroquinoline-3 -carboxylate

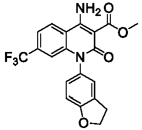

According to the preparation route of scheme 6, 2-amino-4-trifluoromethylbenzonitrile and 5-bromo-2,3-dihydrobenzofuran were used as the starting material to obtain the product with reference to the preparation route of Example 81.

¹H NMR (400 MHz, DMSO-*d₆*) δ 8.43 (d, *J =* 8.4 Hz, 1H), 8.32 (s, 2H), 7.53 (d, *J =* 7.6 Hz, 1H), 7.10 (s, 1H), 6.90-6.96 (m, 2H), 6.70 (s, 1H), 4.62 (t, *J =* 8.8 Hz, 2H), 3.71 (s, 3H), 3.22 (t, *J* = 8.8 Hz, 2H).

### Example 143. methyl 4-amino-1-(4-(2-hydroxypropan-2-yl)phenyl)-2-oxo-7-(trifluoromethyl)-1,2-dihydroquinoline-3 -carboxylate

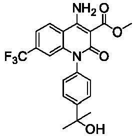

According to the preparation route of scheme 6, 2-(4-bromophenyl)propan-2-ol and 2-amino-4-(trifluoromethyl)benzoic acid were used as the starting material to obtain the product with reference to the preparation route of Example 81.

¹H NMR (400 MHz, CDCl₃) δ 7.79 (d, *J =* 8.8 Hz, 1H), 7.69 (dd, *J =* 6.0 Hz, 2.0 Hz, 2H), 7.55-7.80 (brs, 2H), 7.40 (d, *J =* 8.8 Hz, 1H), 7.19 (dd, *J =* 6.8 Hz, 1.6 Hz, 2H), 6.89 (s, 1H), 3.89 (s, 3H), 1.80-1.93 (brs, 1H), 1.65 (s, 6H).

### Example 144. methyl 4-amino-1-(3-(2-hydroxypropan-2-yl)phenyl)-2-oxo-7-(trifluoro methyl)-1,2-dihydroquinoline-3 -carboxylate

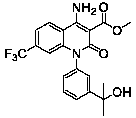

According to the preparation route of scheme 2, 2-(3-bromophenyl)propan-2-ol and 2-amino-4-(trifluoromethyl)benzoic acid were used as the starting material to obtain the product with reference to the preparation route of Example 15.

¹H NMR (400 MHz, CDCl₃) δ 8.40-8.70 (brs, 2H), 8.23 (d, *J* = 8.4 Hz, 1H), 7.54-7.56 (m, 1H), 7.39-7.50 (m, 2H), 7.23-7.27 (m, 1H), 6.98 (d, *J =* 6.8 Hz, 1H), 6.64 (d, *J =* 6.8 Hz, 1H), 4.2-4.40 (brs, 1H), 3.76 (d, *J =* 4.8 Hz, 3H), 1.65 (d, *J* = 4.4 Hz, 6H).

### Example 145. methyl 4-amino-1-(2-ethylphenyl)-2-oxo-7-(trifluoromethyl)-1,2-dihydroquinoline-3 -carboxylate

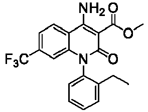

According to the preparation route of scheme 2, 2-bromo-4-trifluoromethylbenzoic acid and 2-ethylaniline were used as the starting material to obtain the product with reference to the preparation route of Example 15.

¹H NMR (400 MHz, DMSO-*d₆*) δ 8.48 (d, *J =* 8.4 Hz, 1H), 8.43 (s, 2H), 7.56 (d, *J =* 8.4 Hz, 1H), 7.46-7.51 (m, 2H), 7.38-7.42 (m, 1H), 7.16 (d, *J =* 8.0 Hz, 1H), 6.47 (s, 1H), 3.72 (s, 3H), 2.13-2.25 (m, 2H), 0.93 (t, *J =* 7.6 Hz, 3H).

### Example 146. methyl 4-amino-1-(3-ethylphenyl)-2-oxo-7-(trifluoromethyl)-1,2-dihydroquinoline-3 -carboxylate

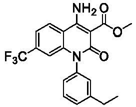

According to the preparation route of scheme 2, 2-bromo-4-trifluoromethylbenzoic acid and 3-ethylaniline were used as the starting material to obtain the product with reference to the preparation route of Example 15.

¹H NMR (400 MHz, DMSO-*d₆*) δ 8.45 (d, *J =* 8.8 Hz, 1H), 8.39 (s, 2H), 7.54 (d, *J =* 8.4 Hz, 1H), 7.49 (t, *J =* 8.0 Hz, 1H), 7.36 (d, *J=* 8.0 Hz, 1H), 7.11 (s, 1H), 7.08 (d, *J =* 7.6 Hz, 1H), 6.58 (s, 1H), 3.71 (s, 3H), 2.65 (q, *J* = 7.6 Hz, 2H), 1.17 (t, *J=* 7.6 Hz, 3H).

### Example 147. methyl 4-amino-1-(4-aminophenyl)-7-bromo-2-oxo-1,2-dihydroquinoline-3-carboxylate

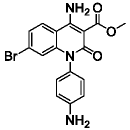

Example 130 (90 mg) was dissolved in acetic acid (15 mL), iron powder (36 mg) was added portionwise, the mixture was stirred at room temperature overnight and filtered, and the filtrate was concentrated under reduced pressure. To the residue were added dichloromethane (50 mL) and an aqueous solution of saturated sodium bicarbonate (100 mL), and the mixture was extracted 3 times with a mixed solvent of dichloromethane and methanol (dichloromethane/methanol =10:1) (50 mL). The extract was concentrated under reduced pressure, and the residue was purified through column chromatography (dichloromethane/methanol =10:1) to obtain the product (54 mg).

¹H NMR (400 MHz, DMSO-*d₆*) δ 8.24 (s, 2H), 8.11 (d, *J =* 8.8 Hz, 1H), 7.35 (dd, *J* = 8.8 Hz, 2.0 Hz, 1H), 6.79 (d, *J =* 8.4 Hz, 2H), 6.67 (d, *J =* 8.4 Hz, 2H), 6.61 (d, *J =* 2.0 Hz, 1H), 5.35 (s, 2H), 3.69 (s, 3H).

### Example 148. methyl 4-amino-1-(1-hydroxy-1-methyl-2,3-dihydro-1H-inden-5- yl)-2-oxo-7-(trifluoromethyl)-1,2-dihydroquinoline-3 -carboxylate

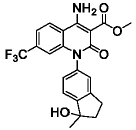

### Step A: 5-bromo-1-methyl-2,3-dihydro-1H-inden-1-ol

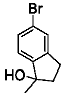

Under nitrogen protection, 5-bromo-1-indanone (2.11 g) was dissolved in anhydrous tetrahydrofuran (50 mL) and cooled to -78 °C, a solution of 1 mol/L methylmagnesium bromide in tetrahydrofuran (50 mL) was added dropwise, and the mixture was stirred at room temperature overnight. Water was added to quench the reaction, the mixture was acidified to pH 5~6 with a solution of saturated ammonium chloride and extracted with dichloromethane, the organic phase was washed with brine, dried over anhydrous sodium sulphate and filtered, and the filtrate was concentrated under reduced pressure to obtain the product (1.8 g), which was used directly in the next step reaction.

### Step B: methyl 4-amino-1-(1-hydroxy-1-methyl-2,3-dihydro-1H-inden-5-yl)-2-oxo-7-(trifluoromethyl)-1,2-dihydroquinoline-3 -carboxylate

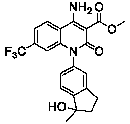

According to the preparation route of scheme 3, 5-bromo-1-methyl-2,3-dihydro-*1H-*inden-1-ol and 2-amino-4-(trifluoromethyl)benzoic acid were used as the starting material to obtain the product with reference to the preparation route of Example 81.

¹H NMR (400 MHz, CD₃OD) δ 8.09-8.13 (m, 1H), 7.47-7.51 (m, 1H), 7.36-7.40 (m, 1H), 6.98-7.03 (m, 2H), 6.80 (s, 1H), 3.82 (d, *J =* 3.2 Hz, 3H), 2.96-3.09 (m, 1H), 2.78-2.88 (m, 1H), 2.21-2.24 (m, 2H),1.54 (d, *J =* 21.6 Hz, 3H).

### Example 149. methyl 4-amino-1-(2,4-dimethylphenyl)-2-oxo-7-(trifluoromethyl)-1,2-dihydroquinoline-3 -carboxylate

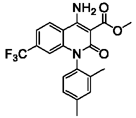

According to the preparation route of scheme 6, 2-amino-4-trifluoromethylbenzonitrile and 1-iodo-2,4-dimethylbenzene were used as the starting material to obtain the product with reference to the preparation route of Example 81.

¹H NMR (400 MHz, CDCl₃) δ 7.81 (d, *J* = 8.4 Hz, 1H), 7.62-8.14 (brs, 2H), 7.35 (d, *J* = 8.4 Hz, 1H), 7.20 (s, 1H), 7.15 (d, *J* = 7.6 Hz, 1H), 6.99 (d, *J* = 7.6 Hz, 1H), 6.76 (s, 1H), 3.90 (s, 3H), 2.39 (s, 3H), 1.95 (s, 3H).

### Example 150. methyl 4-amino-2-oxo-1-phenyl-7-(trifluoromethyl)-1,2-dihydro-1,8-naphthyridine-3 -carboxylate

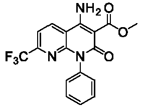

### Step A: 2-(phenylamino)-6-(trifluoromethyl)pyridin-3-carboxylic acid

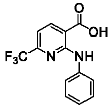

Under nitrogen protection, a mixture of 2-chloro-6-(trifluoromethyl)pyridin-3-carboxylic acid (1.0 g), copper powder (28 mg), copper(I) oxide (32 mg), potassium carbonate (728 mg) and N,N-dimethylformamide (20 mL) was stirred at room temperature for 30 minutes, then a solution of aniline (495 mg) in *N*,*N*-dimethylformamide (5 mL) was slowly added with a syringe, and the mixture was heated to 100 °C and stirred for 12 hours. The mixture was cooled to room temperature and filtered, to the filtrate was added water (200 mL), then 1 mol/L hydrochloric acid (10 mL) was added, lots of solid precipitated, which was filtered to obtain a solid product (700 mg).

### Step B: methyl 4-hydroxy-2-oxo-1-phenyl-7-(trifluoromethyl)-1,2-dihydro-1,8-naphthyridine-3 -carboxylate

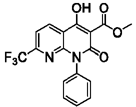

2-(Phenylamino)-6-(trifluoromethyl)pyridin-3-carboxylic acid (200 mg) was dissolved in dichloromethane (30 mL), 1-hydroxybenzotriazole (288 mg) and 1-(3-dimethylaminopropyl)-3-ethylcarbodiimide hydrochloride (163 mg) were added, the mixture was stirred at room temperature for 3 hours, water (30 mL) was added, the mixture was extracted with dichloromethane, the extract was washed with brine, dried over anhydrous sodium sulphate and filtered, the filtrate was concentrated under reduced pressure to obtain a yellow solid, which was dissolved in dry tetrahydrofuran (20 mL), to the solution was added dimethyl malonate (336 mg), then sodium hydride (140 mg) was added, the mixture was stirred at room temperature overnight, saturated ammonium chloride solution (20 mL) was added to quench the reaction, the mixture was extracted with ethyl acetate, the extract was concentrated under reduced pressure, and the residue was purified through column chromatography (ethyl acetate ) to obtain the product (52 mg).

### Step C: methyl 4-chloro-2-oxo-1-phenyl-7-(trifluoromethyl)-1,2-dihydro-1,8-naphthyridine-3-carboxylate

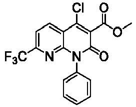

A mixture of methyl 4-hydroxy-2-oxo-1-phenyl-7-(trifluoromethyl)-1,2-dihydro-1,8-naphthyridine-3-carboxylate (52 mg) and phosphoryl trichloride (5 mL) was heated to 100 °C and stirred for 12 hours. The mixture was cooled to room temperature and concentrated under reduced pressure, to the residue was added an aqueous solution of saturated sodium bicarbonate (30 mL), the mixture was extracted with dichloromethane (100 mL*3), the extract was dried over anhydrous sodium sulphate and filtered, the filtrate was concentrated under reduced pressure, and the residue was purified through column chromatography (dichloromethane/methanol = 20:1) to obtain the product (50 mg).

### Step D: methyl 4-amino-2-oxo-1-phenyl-7-(trifluoromethyl)-1,2-dihydro-1,8-naphthyridine-3-carboxylate

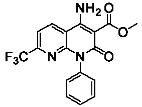

Methyl 4-chloro-2-oxo-1-phenyl-7-(trifluoromethyl)-1,2-dihydro-1,8-naphthyridine-3-carboxylate (50 mg) was added to methanol (10 mL) and 7 mol/L ammonia methanol solution (10 mL), and the mixture was heated to 50 °C and stirred for 12 hours. The reaction solution was concentrated under reduced pressure, and the residue was purified through column chromatography (dichloromethane/methanol = 20:1) to obtain the product (27 mg).

¹H NMR (400 MHz, CDCl₃) δ 8.46 (d, *J* = 8.4 Hz, 1H), 8.24-8.64 (brs, 2H), 7.32-7.43 (m, 4H), 7.10-7.13 (m, 2H), 3.81 (s, 3H).

### Example 151. methyl 4-amino-1-(4-chloro-2-methylphenyl)-2-oxo-7-(trifluoromethyl)-1,2-dihydroquinoline-3 -carboxylate

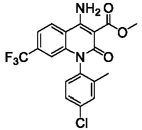

According to the preparation route of scheme 2, 2-bromo-4-trifluoromethylbenzoic acid and 4-chloro-2-methylaniline were used as the starting material to obtain the product with reference to the preparation route of Example 15.

¹H NMR (400 MHz, DMSO-*d₆*) δ 8.45-8.51 (m, 3H), 7.57-7.60 (m, 2H), 7.46 (dd, *J =* 8.0 Hz, 2.4 Hz, 1H), 7.25 (d, *J =* 8.4 Hz, 1H), 6.48 (s, 1H), 3.72 (s, 3H), 1.88 (s, 3H).

### Example 152. methyl 4-amino-7-bromo-1-(4-chloro-2-methylphenyl)-2-oxo-1,2-dihydroquinoline-3 -carboxylate

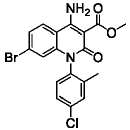

According to the preparation route of scheme 2, 2,4-dibromobenzoic acid and 4-chloro-2-methylaniline were used as the starting material to obtain the product with reference to the preparation route of Example 15.

¹H NMR (400 MHz, DMSO-*d₆*) δ 8.45 (s, 2H), 8.19 (d, *J* = 8.4 Hz, 1H), 7.56 (d, *J* = 2.0 Hz, 1H), 7.41-7.45 (m, 2H), 7.21 (d, *J =* 8.4 Hz, 1H), 6.39 (d, *J* = 2.0 Hz, 1H), 3.69 (s, 3H), 1.88 (s, 3H).

### Example 153. methyl 4-atnino-1-(benzo[b]thiophen-5-yl)-2-oxo-7-(trifluoromethyl)-1,2-dihydroquinoline-3-carboxylate

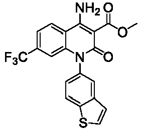

According to the preparation route of scheme 6, 2-amino-4-trifluoromethylbenzonitrile and 5-bromobenzothiofene were used as the starting material to obtain the product with reference to the preparation route of Example 81.

¹H NMR (400 MHz, DMSO-*d₆*) δ 8.47 (d, *J=* 8.0 Hz, 1H), 8.40 (s, 2H), 8.20 (d, *J=* 8.4 Hz, 1H), 7.90 (d, *J* = 5.6 Hz, 1H), 7.82 (d, *J=* 2.0 Hz, 1H), 7.55 (dd, *J* = 8.8 Hz, 2.0 Hz, 1H), 7.50 (d, *J* = 5.6 Hz, 1H), 7.24 (dd, *J=* 8.8 Hz, 2.0 Hz, 1H), 6.61 (s, 1H), 3.71 (s, 3H).

### Example 154. methyl 4-amino-7-bromo-1-(isoquinolin-5-yl)-2-oxo-1,2-dihydroquinoline-3-carboxylate

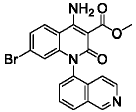

According to the preparation route of scheme 2, 2,4-dibromobenzoic acid and 5-amino-isoquinoline were used as the starting material to obtain the product with reference to the preparation route of Example 15.

¹H NMR (400 MHz, DMSO-*d₆*) δ 9.47 (s, 1H), 8.60 (s, 2H), 8.42 *(d, J=* 6.0 Hz, 1H), 8.31 (d, *J =* 8.0 Hz, 1H), 8.25 (d, *J =* 8.8 Hz, 1H), 7.86 (t, *J =* 8.0 Hz, 1H), 7.79 (d, *J =* 6.4 Hz, 1H), 7.42 (dd, *J =* 8.8 Hz, 2.0 Hz, 1H), 7.20 (d, *J =* 6.0 Hz, 1H), 6.25 (d, *J =* 2.0 Hz, 1H), 3.68 (s, 3H).

### Example 155. methyl 4-amino-1-(4-ethylphenyl)-2-oxo-7-(trifluoromethyl)-1,2-dihydroquinoline-3 -carboxylate

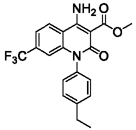

According to the preparation route of scheme 3, 2-amino-4-(trifluoromethyl)benzoic acid and 1-ethyl-4-iodobenzene were used as the starting material to obtain the product with reference to the preparation route of Example 27.

¹H NMR (400 MHz, DMSO-*d₆*) δ 8.44 (d, *J =* 8.4 Hz, 1H), 8.36 (s, 2H), 7.54 (d, *J =* 8.8 Hz, 1H), 7.41 (d, *J =* 8.4 Hz, 2H), 7.16 (d, *J =* 8.4 Hz, 2H), 6.59 (s, 1H), 3.71 (s, 3H), 2.70 (q, *J =* 7.6 Hz, 2H), 1.23 (t, *J =* 7.6 Hz, 3H).

### Example 156. methyl 4-amino-2-oxo-1-(thiophen-3-yl)-7-(trifluoromethyl)-1,2-dihydroquinoline-3 -carboxylate

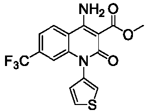

According to the preparation route of scheme 3, 2-amino-4-(trifluoromethyl)benzoic acid and 3-bromothiophene were used as the starting material to obtain the product with reference to the preparation route of Example 27.

¹H NMR (400 MHz, DMSO-*d₆*) δ 8.44 (d, *J =* 8.4 Hz, 1H), 8.39 (s, 2H), 7.77 (dd, *J =* 4.8 Hz, 3.2 Hz, 1H), 7.64 (dd, *J* = 3.2 Hz, 1.2 Hz, 1H), 7.56 (dd, *J =* 8.4 Hz, 1.2 Hz, 1H), 7.03 (dd, *J =* 4.8 Hz, 1.2 Hz, 1H), 6.73 (s, 1H), 3.72 (s, 3H).

### Example 157. methyl 4-amino-1-(1-methyl-1H-pyrazol-4-yl)-2-oxo-7-(trifluoromethyl)-1,2-dihydroquinoline-3 -carboxylate

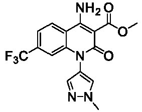

### Step A: 2-((1-methyl-1H-pyrazol-4-yl)amino)-4-(trifluoromethyl)benzonitrile

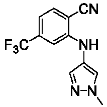

Under nitrogen protection, 2-amino-4-trifluoromethylbenzonitrile (186 mg), 4-iodo-1-methyl-*1H*-pyrazole (236 mg), copper(I) iodide(19 mg), *N¹*,*N²*-dimethylethane-1,2-diamine (28 mg), potassium phosphate (636 mg) and *N,N*-dimethylformamide (50 mL) were heated to 140 °C in a sealed tube and stirred for 12 hours. The mixture was cooled to room temperature and filtered, the filtrate was concentrated under reduced pressure, and the residue was purified through column chromatography (petroleum ether/ethyl acetate = 1:1) to obtain the product (90 mg).

### Step B: methyl 4-amino-1-(1-methyl-1H-pyrazol-4-yl)-2-oxo-7-(trifluoromethyl)-1,2-dihydroquinoline-3 -carboxylate

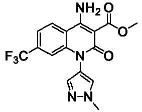

Under nitrogen protection, 2-((1-methyl-*1H-*pyrazol-4-yl)amino)-4-(trifluoromethyl) benzonitrile (90 mg) and dimethyl malonate (160 mg) were dissolved in *N,N*-dimethylformamide (10 mL), sodium *tert*-butoxide (233 mg) was added, the mixture was heated to 90 °C and stirred for 12 hours, cooled to room temperature and concentrated under reduced pressure, and the residue was purified through column chromatography (petroleum ether/ethyl acetate = 1:3) to obtain the product (47 mg).

¹H NMR (400 MHz, CD₃OD) δ 8.29 (d, *J* = 8.4 Hz, 1H), 7.80 (s, 1H), 7.51-7.53 (m, 2H), 7.17 (s, 1H), 4.01 (s, 3H), 3.83 (s, 3H).

### Example 158. methyl 4-amino-1-(3-fluoro-4-(1-hydroxyethyl)phenyl)-2-oxo-7-(trifluoromethyl)-1,2-dihydroquinoline-3 -carboxylate

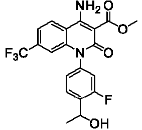

1-(4-Bromo-2-fluorophenyl)ethan-1-one and 2-amino-4-(trifluoromethyl)benzoic acid were used as the starting material to obtain the product with reference to the preparation route of Example 81 and Example 122.

¹H NMR (400 MHz, CDCl₃) δ 7.72-7.81 (m, 2H), 7.65-7.90 (brs, 2H), 7.43 (d, *J =* 6.8 Hz, 1H), 7.06 (d, *J =* 7.6 Hz, 1H), 6.96 (d, *J =* 10.4 Hz, 1H), 6.88 (d, *J =* 2.8 Hz, 1H), 5.21-5.38 (m, 1H), 3.90 (s, 3H), 2.01-2.11 (brs, 1H), 1.60 (d, *J =* 6.8 Hz, 3H).

### Example 159. methyl 4-amino-2-oxo-1-(1H-pyrazol-4-yl)-7-(trifluoromethyl)-1,2-dihydroquinoline-3 -carboxylate

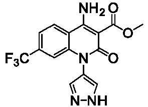

### Step A: methyl 4-amino-2-oxo-1-(1-(tetrahydro-2H-pyran-2-yl)-1H-pyrazol-4-yl)-7-(trifluoromethyl)-1,2-dihydroquinoline-3 -carboxylate

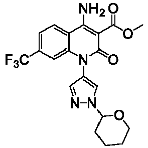

2-Amino-4-trifluoromethylbenzonitrile and 4-iodo-1-(tetrahydro-*2H*-pyran-2-yl)-*1H-*pyrazole were used as the starting material to obtain the product with reference to the preparation route of Example 157 (20 mg).

### Step B: methyl 4-amino-2-oxo-1-(1H-pyrazol-4-yl)-7-(trifluoromethyl)-1,2-dihydroquinoline-3 -carboxylate

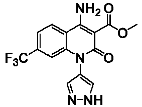

Methyl 4-amino-2-oxo-1-(1-(tetrahydro-2*H*-pyran-2-yl)-*1H*-pyrazol-4-yl)-7-(trifluoro methyl)-1,2-dihydroquinoline-3-carboxylate (20 mg) was dissolved in methanol (10 mg), a solution of 4 mol/L hydrochloric acid in 1,4-dioxane (2 mL) was added, the mixture was stirred at room temperature for 2 hours and concentrated under reduced pressure, and the residue was purified through column chromatography (dichloromethane/methanol = 10:1) to obtain the product (11 mg).

¹H NMR (400 MHz, CD₃OD) δ 8.29 (d, *J* = 8.8 Hz, 1H), 7.87 (s, 1H), 7.61 (s, 1H), 7.52 (d, *J* = 8.8 Hz, 1H), 7.13 (s, 1H), 3.83 (s, 3H).

### Example 160. methyl 4-amino-7-cyclopropyl-1-(isoquinolin-5-yl)-2-oxo-1,2-dihydroquinoline-3 -carboxylate

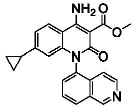

Under nitrogen protection, a mixture of methyl 4-amino-7-bromo-1-(isoquinolin-5-yl)-2-oxo-1,2-dihydroquinoline-3-carboxylate (20 mg), cyclopropylboronic acid (6 mg), 1,1'-bis(diphenylphosphino)ferrocene-palladium(II)dichloride dichloromethane complex (4 mg), cesium fluoride (23 mg), 1,4-dioxane (10 mL) and water (2 mL) was heated to 90 °C and stirred fr 2 hours. The mixture was cooled to room temperature and filtered, to the filtrate was added water (100 mL), the mixture was extracted with dichloromethane (100 mL*3), the extract was dried over anhydrous sodium sulphate and filtered, the filtrate was concentrated under reduced pressure, and the residue was purified through column chromatography (dichloromethane/methanol = 15:1) to obtain the product (13 mg).

¹H NMR (400 MHz, CDCl₃) δ 9.39 (s, 1H), 8.44 (d, *J* = 6.0 Hz, 1H), 8.14 (d, *J* = 8.4 Hz, 1H), 7.76-7.90 (m, 2H), 7.67 (d, *J* = 7.6 Hz, 1H), 7.58 (d, *J* = 8.4 Hz, 1H), 6.77 (d, *J* = 8.4 Hz, 1H), 6.06 (s, 1H), 3.86 (s, 3H), 1.59-1.65 (m, 1H), 0.82-0.90 (m, 2H), 0.44-0.56 (m, 2H).

### Example 161. methyl 4-amino-7-bromo-1-(6-chloro-4-methylpyridin-3-yl)-2-oxo-1,2-dihydroquinoline-3 -carboxylate

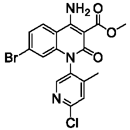

According to the preparation route of scheme 2, 2-iodo-4-bromobenzoic acid and 3-amino-6-chloro-4-methylpyridine were used as the starting material to obtain the product with reference to the preparation route of Example 15.

¹H NMR (400 MHz, DMSO-*d₆*) δ 8.57 (s, 2H), 8.23 (s, 1H), 8.21 (d, *J =* 8.8 Hz, 1H), 7.69 (s, 1H), 7.46 (dd, *J =* 8.8 Hz, 2.0 Hz, 1H), 6.55 (d, *J =* 2.0 Hz, 1H), 3.69 (s, 3H), 1.94 (s, 3H).

### Example 162. methyl 4-amino-2-oxo-1-(quinolin-5-yl)-7-(trifluoromethyl)-1,2-dihydroquinoline-3 -carboxylate

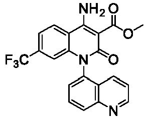

According to the preparation route of scheme 6, 2-amino-4-trifluoromethylbenzonitrile and 5-bromoquinoline were used as the starting material to obtain the product with reference to the preparation route of Example 81.

¹H NMR (400 MHz, DMSO-*d₆*) δ 8.96 (dd, *J =* 4.0 Hz, 1.6 Hz, 1H), 8.62 (s, 2H), 8.54 (d, *J =* 8.4 Hz, 1H), 8.21 (d, *J =* 8.8 Hz, 1H), 7.95 (t, *J=* 8.0 Hz, 1H), 7.81 (d, *J =* 8.4 Hz, 1H), 7.65 (d, *J* = 7.2 Hz, 1H), 7.58 (dd, *J =* 8.4 Hz, 1.6 Hz, 1H), 7.46 (dd, *J =* 8.8 Hz, 4.0 Hz, 1H), 6.36 (s, 1H), 3.70 (s, 3H).

### Example 163. methyl 4-amino-7-bromo-2-oxo-1-(quinolin-5-yl)-1,2-dihydroquinoline-3-carboxylate

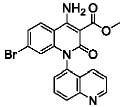

According to the preparation route of scheme 7, 2-fluoro-4-bromobenzonitrile and 5-aminoquinoline were used as the starting material to obtain the product with reference to the preparation route of Example 165.

¹H NMR (400 MHz, DMSO-*d₆*) δ 8.96 (dd, *J =* 4.0 Hz, 1.6 Hz, 1H), 8.59 (s, 2H), 8.24 (d, *J* = 8.8 Hz, 1H), 8.20 (d, *J =* 8.4 Hz, 1H), 7.92-7.96 (m, 1H), 7.77 (d, *J =* 8.4 Hz, 1H), 7.60 (d, *J =* 7.2 Hz, 1H), 7.47 (dd, *J =* 8.8 Hz, 4.0 Hz, 1H), 7.42 (dd, *J =* 8.8 Hz, 2.0 Hz, 1H), 6.27 (d, *J =* 2.0 Hz, 1H), 3.68 (s, 3H).

### Example 164. methyl 4-amino-1-(4-fortnylphenyl)-2-oxo-7-(trifluoromethyl)-1,2-dihydroquinoline-3 -carboxylate

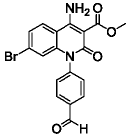

According to the preparation route of scheme 6, 2-amino-4-trifluoromethylbenzonitrile and 1-bromo-4-(trifluoromethyl)benzene were used as the starting material to obtain the product with reference to the preparation route of Example 81.

¹H NMR (400 MHz, DMSO-*d₆*) δ 10.10 (s, 1H), 8.45-8.50 (m, 3H), 8.11 (d, *J =* 8.0 Hz, 2H), 7.55-7.60 (m, 3H), 6.56 (s, 1H), 3.72 (s, 3H).

### Example 165. methyl 1-(4-acetylphenyl)-4-amino-7-bromo-2-oxo-1,2-dihydro quinoline-3 -carboxylate

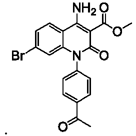

### Step A: 2-((4-acetylphenyl)amino)-4-bromobenzonitrile

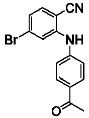

4-Bromo-2-fluorobenzonitrile (200 mg) and 1-(4-aminophenyl)ethan-1-one (135 mg) were dissolved in dimethyl sulfoxide (10 mL), potassium *tert*-butoxide (224 mg) was added, and the mixture was stirred at room temperature for 2 hours. Water (200 mL) was added, lots of solid precipitated, and the mixture was filtered to obtain a solid product (560 mg).

### Step B: methyl 1-(4-acetylphenyl)-4-amino-7-bromo-2-oxo-1,2-dihydroquinoline-3-carboxylate

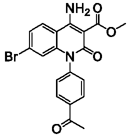

2-((4-Acetylphenyl)amino)-4-bromobenzonitrile (470 mg) and dimethyl malonate (1.0 g) were dissolved in 1,2-dichloroethane (20 mL), tin tetrachloride (1.8 g) was added, and the mixture was stirred at room temperature for 2 hours. An aqueous solution of saturated sodium bicarbonate (20 mL) was added, lots of solid precipitated, the mixture was filtered through celite, the filtrate was extracted with dichloromethane, the extract was concentrated under reduced pressure, and the residue was purified through column chromatography (dichloromethane/methanol = 15:1) to obtain the product (213 mg).

¹H NMR (400 MHz, CD₃OD) δ 8.21 (d, *J* = 8.8 Hz, 2H), 8.01 (d, *J* = 8.8 Hz, 1H), 7.36-7.40 (m, 3H), 6.64 (d, *J* = 2.0 Hz, 1H), 3.82 (s, 3H), 2.69 (s, 3H).

### Example 166. methyl 1-(acridin-9-yl)-4-amino-2-oxo-7-(trifluoromethyl)-1,2-dihydroquinoline-3 -carboxylate

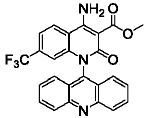

According to the preparation route of scheme 7, 2-bromo-4-(trifluoromethyl)benzonitrile and acridin-9-amine were used as the starting material to obtain the product with reference to the preparation route of Example 165.

¹H NMR (400 MHz, DMSO-*d₆*) δ 8.85 (s, 2H), 8.64 (d, *J =* 8.8 Hz, 1H), 8.31 (d, *J =* 8.8 Hz, 2H), 7.79-7.93 (m, 2H), 7.55-7.64 (m, 5H), 6.10 (s, 1H), 3.68 (s, 3H).

### Example 167. methyl 4-amino-1-(1-benzofuran-4-yl)-2-oxo-7-(trifluoromethyl)-1,2-dihydroquinoline-3 -carboxylate

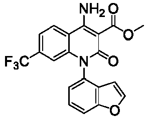

According to the preparation route of scheme 6, 2-amino-4-(trifluoromethyl)benzonitrile and 4-bromobenzofuran were used as the starting material to obtain the product with reference to the preparation route of Example 81.

¹H NMR (400 MHz, CD₃OD) δ 8.35 (d, *J =* 8.0 Hz, 1H), 7.78 (d, *J =* 2.4 Hz, 1H), 7.72 (d, *J* = 8.0 Hz, 1H), 7.49-7.55 (m, 2H), 7.19 (d, *J =* 7.6 Hz, 1H), 6.68 (s, 1H), 6.48 (d, *J =* 2.0 Hz, 1H), 3.83 (s, 3H).

### Example 168. methyl 4-amino-1-(4-cyanophenyl)-2-oxo-7-(trifluoromethyl)-1,2-dihydroquinoline-3 -carboxylate

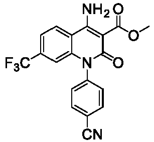

According to the preparation route of scheme 6, 2-bromo-4-trifluoromethylbenzonitrile and 4-aminobenzonitrile were used as the starting material to obtain the product with reference to the preparation route of Example 81.

¹H NMR (400 MHz, DMSO-*d₆*) δ 8.46-8.52 (m, 3H), 8.07 (d, *J=* 8.4 Hz, 2H), 7.56-7.60 (m, 3H), 6.54 (s, 1H), 3.71 (s, 3H).

### Example 169. methyl 4-amino-1-(isoquinolin-4-yl)-2-oxo-7-(trifluoromethyl)-1,2-dihydroquinoline-3 -carboxylate

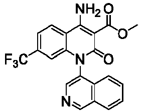

According to the preparation route of scheme 6, 2-bromo-4-trifluoromethylbenzonitrile and 4-amino-isoquinoline were used as the starting material to obtain the product with reference to the preparation route of Example 81.

¹H NMR (400 MHz, DMSO-*d₆*) δ 9.50 (s, 1H), 8.64 (s, 2H), 8.55 (d, *J =* 8.4 Hz, 1H), 8.51 (s, 1H), 8.31 (dd, *J =* 8.4 Hz, 1.6 Hz, 1H), 7.71-7.78 (m, 2H), 7.59 (d, *J =* 8.8 Hz, 1H), 7.41 *(d, J =* 8.0 Hz, 1H), 6.41 (s, 1H), 3.71 (s, 3H).

### Example 170. methyl 4-atnino-1-(8-chloroisoquinolin-5-yl)-2-oxo-7-(trifluoromethyl)-1,2-dihydroquinoline-3 -carboxylate

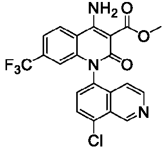

According to the preparation route of scheme 6, 2-bromo-4-trifluoromethylbenzonitrile and 8-chloroisoquinolin-5-amine were used as the starting material to obtain the product with reference to the preparation route of Example 81.

¹H NMR (400 MHz, DMSO-*d₆*) δ 9.66 (s, 1H), 8.67 (s, 2H), 8.54-8.56 (m, 2H), 8.03 (d, *J =* 7.6 Hz, 1H), 7.85 (d, *J* = 8.4 Hz, 1H), 7.59 (d, *J* = 8.8 Hz, 1H), 7.33 (d, *J* = 5.6 Hz, 1H), 6.45 (s, 1H), 3.70 (s, 3H).

### Example 171. methyl 4-amino-2-oxo-1-(quinolin-4-yl)-7-(trifluoromethyl)-1,2-dihydroquinoline-3 -carboxylate

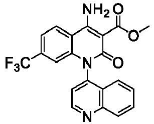

According to the preparation route of scheme 6, 2-bromo-4-trifluoromethylbenzonitrile and 4-aminoquinoline were used as the starting material to obtain the product with reference to the preparation route of Example 81.

¹H NMR (400 MHz, DMSO-*d₆*) δ 9.12 (d, *J =* 4.4 Hz, 1H), 8.67 (s, 2H), 8.56 (d, *J =* 8.8 Hz, 1H), 8.19 (*d, J =* 8.4 Hz, 1H), 7.80-7.84 (m, 1H), 7.64 (*d, J =* 4.4 Hz, 1H), 7.60 (dd, *J* = 8.4 Hz, 1.2 Hz, 1H), 7.52-7.56 (m, 1H), 7.46 (d, *J =* 8.0 Hz, 1H), 6.39 (s, 1H), 3.70 (s, 3H).

### Example 172. methyl 4-amino-2-oxo-1-(quinolin-8-yl)-7-(trifluoromethyl)-1,2-dihydroquinoline-3 -carboxylate

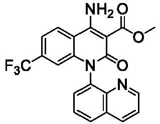

According to the preparation route of scheme 6, 2-bromo-4-trifluoromethylbenzonitrile and 8-aminoquinoline were used as the starting material to obtain the product with reference to the preparation route of Example 81.

¹H NMR (400 MHz, DMSO-*d₆*) δ 8.74 (dd, *J =* 8.4 Hz, 1.6 Hz, 1H), 8.45-8.53 (m, 4H), 8.19 (dd, *J* = 7.2 Hz, 2.4 Hz, 1H), 7.77-7.82 (m, 2H), 7.57 (dd, *J* = 8.0 Hz, 4.0 Hz, 1H), 7.51 (dd,*J* = 8.8 Hz, 1.2 Hz, 1H), 6.36 (s, 1H), 3.69 (s, 3H).

### Example 173. methyl 4-amino-1-(4-chlorophenyl)-2-oxo-7-(trifluoromethyl)-1,2-dihydro-1,8-naphthyridine-3 -carboxylate

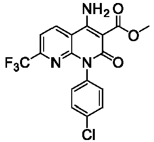

According to the preparation route of scheme 7, 2-chloro-6-(trifluoromethyl)pyridin-3-benzonitrile and 4-chloroaniline were used as the starting material to obtain the product with reference to the preparation route of Example 165.

¹H NMR (400 MHz, CDCl₃+CD₃OD) δ 8.51 (d, *J* = 8.0 Hz, 1H), 7.45 (d, *J* = 8.0 Hz, 1H), 7.39 (d, *J* = 8.4 Hz, 2H), 7.08 (d, *J* = 8.4 Hz, 2H), 3.82 (s, 3H).

### Example 174. methyl 4-amino-1-(isoquinolin-5-yl)-2-oxo-7-(trifluoromethyl)-1,2-dihydro-1,8-naphthyridine-3 -carboxylate

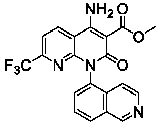

According to the preparation route of scheme 7, 2-chloro-6-(trifluoromethyl)pyridin-3-benzonitrile and isoquinolin-5-amine were used as the starting material to obtain the product with reference to the preparation route of Example 165.

¹H NMR (400 MHz, CDCl₃+CD₃OD) δ 9.05-9.48 (brs, 1H), 8.55 (d, *J* = 8.0 Hz, 1H), 8.14-8.40 (brs, 1H), 8.07 (d, *J* = 8.4 Hz, 1H), 7.71 (t, *J* = 7.6 Hz, 1H), 7.58 (d, *J* = 7.2 Hz, 1H), 7.44 *(d, J* = 8.4 Hz, 1H), 7.11-7.27 (brs, 1H), 3.80 (s, 3H).

### Example 175. methyl 4-amino-1-(6-(1-hydroxyethyl)pyridin-3-yl)-2-oxo-7-(trifluoromethyl)-1,2-dihydroquinoline-3 -carboxylate

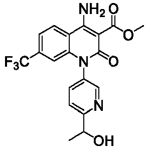

1-(5-Bromopyridin-2-yl)ethan-1-one and 2-amino-4-(trifluoromethyl)benzonitrile were used as the starting material to obtain the product with reference to the preparation route of Example 120 and 122.

¹H NMR (400 MHz, CD₃OD) δ 8.36 (s, 1H), 8.26 (d, *J* = 8.4 Hz, 1H), 7.52-7.80 (m, 2H), 7.46 *(d, J =* 8.8 Hz, 1H), 6.71 (s, 1H), 4.98-4.99 (m, 1H), 3.83 (s, 3H), 1.51-1.56 (m, 3H).

### Example 176. methyl amino-1-(8-chloroisoquinolin-5-yl)-7-bromo-2-oxo-1,2-dihydroquinoline-3 -carboxylate

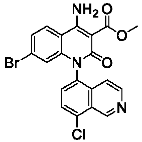

According to the preparation route of scheme 7, 2-fluoro-4-bromobenzonitrile and 5-amino-8-chloro-isoquinoline were used as the starting material to obtain the product with reference to the preparation route of Example 165.

¹H NMR (400 MHz, DMSO-*d₆*) δ 9.65 (s, 1H), 8.63 (s, 2H), 8.55 (d, *J =* 5.6 Hz, 1H), 8.25 (d, *J =* 8.8 Hz, 1H), 8.00 (d, *J =* 7.6 Hz, 1H), 7.80 (d, *J =* 8.0 Hz, 1H), 7.43 (dd, *J =* 8.8 Hz, 2.0 Hz, 1H), 7.29 (d, *J =* 6.0 Hz, 1H), 6.41 (d, *J* = 2.0 Hz, 1H), 3.67 (s, 3H).

### Example 177. methyl 4-amino-1-(8-nitroisoquinolin-5-yl)-7-bromo-2-oxo-1,2-dihydroquinoline-3 -carboxylate

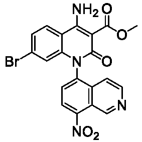

According to the preparation route of scheme 7, 2-amino-4-bromobenzonitrile and 5-bromo-8-nitro-isoquinoline were used as the starting material to obtain the product with reference to the preparation route of Example 165.

¹H NMR (400 MHz, DMSO-*d₆*) δ 9.91 (d, *J =* 1.0 Hz, 1H), 8.73 (s, 2H), 8.68 - 8.61 (m, 2H), 8.31 (d, *J =* 8.8 Hz, 1H), 8.07 (d, *J =* 8.1 Hz, 1H), 7.53 - 7.45 (m, 2H), 6.52 (d, *J* = 1.9 Hz, 1H), 3.72 (s, 3H).

### Example 178. methyl 4-amino-1-(8-aminoisoquinolin-5-yl)-7-bromo-2-oxo-1,2-dihydroquinoline-3-carboxylate

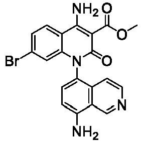

According to the preparation route of Example 147, Example 177 was used as the starting material to obtain the product.

¹H NMR (400 MHz, DMSO-*d₆*) δ 9.59 (s, 1H), 8.46 (s, 2H), 8.25 (d, *J =* 6.4 Hz, 1H), 8.21 (d, *J =* 8.4 Hz, 1H), 7.35-7.39 (m, 2H), 6.90 (d, *J =* 6.0 Hz, 1H), 6.85 (d, *J =* 8.4 Hz, 1H), 6.72 (s, 2H), 6.38 (d, *J =* 2.0 Hz, 1H), 3.67 (s, 3H).

### Example 179. methyl 4-amino-1-(6-nitropyridin-3-yl)-7-bromo-2-oxo-1,2-dihydroquinoline-3-carboxylate

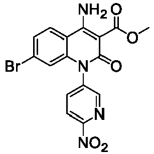

According to the preparation route of scheme 7, 2-amino-4-bromobenzonitrile and 5-fluoro-2-nitropyridine were used as the starting material to obtain the product with reference to the preparation route of Example 165.

¹H NMR (400 MHz, DMSO-*d₆*) δ 8.63 (d, *J =* 2.8 Hz, 1H), 8.58 (s, 2H), 8.50 (d, *J =* 8.4 Hz, 1H), 8.26 (dd, *J =* 8.8 Hz, 2.8 Hz, 1H), 8.19 (d, *J =* 8.8 Hz, 1H), 7.46 (dd, *J =* 8.4 Hz, 1.6 Hz, 1H), 6.71 (d, *J =* 1.6 Hz, 1H), 3.69 (s, 3H).

### Example 180. methyl 4-amino-1-(6-aminopyridin-3-yl)-7-bromo-2-oxo-1,2-dihydroquinoline-3 -carboxylate

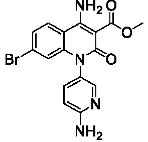

According to the preparation route of Example 147, Example 179 was used as the starting material to obtain the product.

¹H NMR (400 MHz, DMSO-*d₆*) δ 8.33 (s, 2H), 8.13 (d, *J =* 8.8 Hz, 1H), 7.70 (d, *J =* 2.4 Hz, 1H), 7.38 (dd, *J =* 8.4 Hz, 2.0 Hz, 1H), 7.23 (dd, *J =* 8.4 Hz, 2.8 Hz, 1H), 6.67 (d, *J =* 2.0 Hz, 1H), 6.58 (d, *J =* 8.4 Hz, 1H), 6.27 (s, 2H), 3.69 (s, 3H).

### Example 181. methyl 4-atnino-1-(6-nitropyridin-3-yl)-2-oxo-7-(trifluoromethyl)-1,2-dihydroquinoline-3 -carboxylate

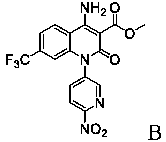

According to the preparation route of scheme 7, 2-amino-4-trifluoromethylbenzonitrile and 5-fluoro-2-nitropyridine were used as the starting material to obtain the product with reference to the preparation route of Example 165.

¹H NMR (400 MHz, DMSO-*d₆*) δ 8.67 (d, *J =* 2.0 Hz, 1H), 8.61 (s, 2H), 8.52 (d, *J =* 8.8 Hz, 1H), 8.49 (d, *J =* 8.8 Hz, 1H), 8.30 (dd, *J =* 8.8 Hz, 2.4 Hz, 1H), 7.62 (d, *J =* 8.4 Hz, 1H), 6.76 (s, 1H), 3.71 (s, 3H).

### Example 182. methyl 4-amino-1-(6-atninopyridin-3-yl)-2-oxo-7-(trifluoromethyl)-1,2-dihydroquinoline-3 -carboxylate

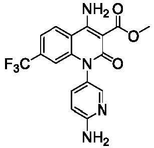

According to the preparation route of Example 147, Example 181 was used as the starting material to obtain the product.

¹H NMR (400 MHz, DMSO-*d₆*) δ 8.43 (d, *J =* 8.4 Hz, 1H), 8.34 (s, 2H), 7.73 (d, *J =* 2.4 Hz, 1H), 7.54 (dd, *J =* 7.6 Hz, 2.4 Hz, 1H), 7.26 (dd, *J =* 8.4 Hz, 2.4 Hz, 1H), 6.77 (s, 1H), 6.57 (d, *J =* 8.4 Hz, 1H), 6.25 (s, 2H), 3.71 (s, 3H).

### Example 183. methyl 4-amino-7-bromo-1-(6-bromo-4-chloro-2-methylphenyl)-2-oxo-1,2-dihydroquinoline-3 -carboxylate

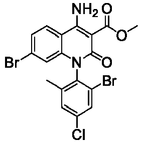

According to the preparation route of scheme 7, 2-fluoro-4-bromobenzonitrile and 6-bromo-4-chloro-2-methylaniline were used as the starting material to obtain the product with reference to the preparation route of Example 165.

¹H NMR (400 MHz, CDCl₃+CD₃OD) δ 7.89 (d, *J =* 8.8 Hz, 1H), 7.58 (d, *J =* 2.0 Hz, 1H), 7.32-7.34 (m, 2H), 6.50 *(d, J=* 1.6 Hz, 1H), 3.81 (s, 3H), 2.02 (s, 3H).

### Example 184. methyl 4-amino-1-(6-bromo-4-chloro-2-methylphenyl)-2-oxo-7-(trifluoromethyl)-1,2-dihydro-1,8-naphthyridine-3-carboxylate

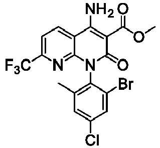

According to the preparation route of scheme 7, 2-chloro-6-(trifluoromethyl)pyridin-3-benzonitrile and 6-bromo-4-chloro-2-methylaniline were used as the starting material to obtain the product with reference to the preparation route of Example 165.

¹H NMR (400 MHz, CDCl₃+CD₃OD) δ 8.69 (d, *J =* 8.4 Hz, 1H), 7.54 (d, *J =* 8.4 Hz, 1H), 7.50 (d, *J =* 2.0 Hz, 1H), 7.27 (d, *J =* 2.0 Hz, 1H), 3.84 (s, 3H), 2.00 (s, 3H).

### Example 185. methyl 4-atnino-1-(isoquinolin-5-yl)-2-oxo-7-vinyl-1,2-dihydroquinoline-3-carboxylate

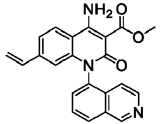

Methyl 4-amino-7-bromo-1-(isoquinolin-5-yl)-2-oxo-1,2-dihydroquinoline-3-carboxylate and potassium vinyltrifluoroborate were used as the starting material to obtain the product with reference to the preparation route of Example 160.

¹H NMR (400 MHz, CDCl₃) δ 9.38 (s, 1H), 8.44 (d, *J* = 5.6 Hz, 1H), 7.77 (t, *J* = 7.6 Hz, 1H), 7.66-7.68 (m, 2H), 7.31 (d, *J* = 8.0 Hz, 1H), 7.23-7.25 (m, 1H), 6.41 (dd, *J* = 17.6 Hz, 11.2 Hz, 1H), 6.25 (s, 1H), 5.59 (d, *J* = 17.6 Hz, 1H), 5.24 (d, *J* = 11.2 Hz, 1H), 3.87 (s, 3H).

### Example 186. methyl 4-amino-1-(4-(1-hydroxyimino)ethyl)phenyl)-2-oxo-7-(trifluoro methyl)-1,2-dihydroquinoline-3 -carboxylate

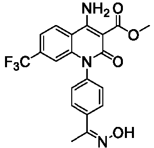

According to the preparation route of scheme 6, 1-(4-bromophenyl)ethan-1-one oxime and 2-amino-4-(trifluoromethyl)benzonitrile were used as the starting material to obtain the product with reference to the preparation route of Example 81.

¹H NMR (400 MHz, CDCl₃) δ 7.83 (d, *J =* 8.8 Hz, 2H), 7.60-7.95 (brs, 2H), 7.78 (d, *J =* 8.4 Hz, 1H), 7.41 (d, *J =* 7.6 Hz, 1H), 7.27 (d, *J =* 8.8 Hz, 2H), 6.87 (s, 1H), 3.90 (s, 3H), 3.60-3.95 (brs, 1H), 2.33 (s, 3H).

### Example 187. methyl 4-amino-1-(1-benzofuran-6-yl)-2-oxo-7-(trifluoromethyl)-1,2-dihydroquinoline-3 -carboxylate

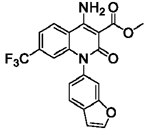

According to the preparation route of scheme 7, 2-amino-4-(trifluoromethyl)benzonitrile and 6-bromo-1-benzofuran were used as the starting material to obtain the product with reference to the preparation route of Example 165.

¹H NMR (400 MHz, DMSO-*d*₆) δ 8.46 (d, *J =* 8.4 Hz, 1H), 8.40 (s, 2H), 8.12 (d, *J =* 2.0 Hz, 1H), 7.83 (d, *J =* 8.4 Hz, 1H), 7.64 (s, 1H), 7.55 (d, *J =* 8.8 Hz, 1H), 7.14 (dd, *J =* 8.4 Hz, 2.0 Hz, 1H), 7.07 (d, *J =* 2.0 Hz, 1H), 6.58 (s, 1H), 3.71 (s, 3H).

### Example 188. methyl 4-amino-7-bromo-2-oxo-1-(quinolin-8-yl)-1,2-dihydroquinoline-3-carboxylate

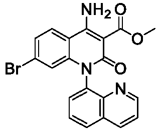

According to the preparation route of scheme 7, 2-fluoro-4-bromobenzonitrile and quinolin-8-amine were used as the starting material to obtain the product with reference to the preparation route of Example 165.

¹H NMR (400 MHz, DMSO-*d*₆) δ 8.75 (dd, *J =* 4.0 Hz, 1.6 Hz, 1H), 8.51 (dd, *J =* 8.0 Hz, 1.6 Hz, 1H), 8.45 (s, 2H), 8.16-8.20 (m, 2H), 7.75-7.80 (m, 2H), 7.58 (dd, *J =* 8.0 Hz, 4.0 Hz, 1H), 7.36 (dd, *J =* 8.8 Hz, 2.0 Hz, 1H), 6.26 (d, *J =* 2.0 Hz, 1H), 3.66 (s, 3H).

### Example 189. methyl-d₃ 4-amino-1-(4-aminophenyl)-7-bromo-2-oxo-1,2-dihydroquinolin-3-carboxylate

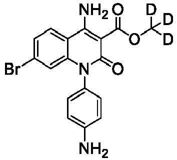

### Step 1: 4-amino-7-bromo-1-(4-nitrophenyl)-2-oxo-1,2-dihydro quinolin- 3-carboxylic acid

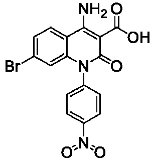

Example 130 (200 mg) and lithium hydroxide (60 mg) were dissolved in a mixed solvent of water (6 mL) and tetrahydrofuran (6 mL), and the mixture was heated to 50 °C and stirred for 11 hours. The mixture was cooled to room temperature, to the reaction solution was added 1 N hydrochloric acid to adjust pH to around 3, and lots of solid precipitated, which was filtered to obtain the product (157 mg).

### Step 2: methyl-d₃ 4-amino-7-bromo-1-(4-nitrophenyl)-2-oxo-1,2-dihydroquinolin-3-carboxylate

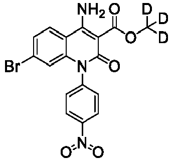

4-Amino-7-bromo-1-(4-nitrophenyl)-2-oxo-1,2-dihydroquinolin-3-carboxylic acid (157 mg), 2-(7-azabenzotriazol-1-yl)-*N,N,N',N'*-tetramethyluronium hexafluorophosphate (380 mg), triethylamine (118 mg) and methanol-*d₄* (1.626 g) were dissolved in *N,N*-dimethylformamide (20 mL), and the mixture was heated to 50 °C and stirred overnight. The mixture was cooled to room temperature, water (100 mL) was added, and lots of solid precipitated, which was filtered to obtain the product (120 mg).

### Step 3: methyl-d₃ 4-amino-1-(4-aminophenyl)-7-bromo-2-oxo-1,2-dihydroquinolin-3-carboxylate

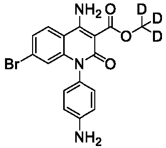

According to the preparation route of Example 147, methyl-*d₃* 4-amino-7-bromo- 1-(4-nitrophenyl)-2-oxo-1,2-dihydroquinolin-3-carboxylate was used as the starting material to obtain the product.

¹H NMR (400 MHz, DMSO-*d*₆) δ 8.24 (s, 2H), 8.11 (d, *J =* 8.8 Hz, 1H), 7.35 (dd, *J =* 8.8 Hz, 2.0 Hz, 1H), 5.80 (d, *J =* 8.4 Hz, 2H), 6.67 (d, *J =* 8.4 Hz, 2H), 6.61 (d, *J =* 5.6 Hz, 1H), 5.35 (s, 2H).

### Example 190. methyl 4-amino-1-(imidazo[3,2-a]pyridin-5-yl)-2-oxo-7-(trifluoro methyl)-1,2-dihydroquinoline-3 -carboxylate

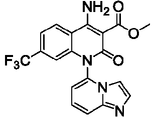

According to the preparation route of scheme 7, 2-fluoro-4-(trifluoromethyl)benzonitrile and imidazo[3,2-a]pyridin-5-amine were used as the starting material to obtain the product with reference to the preparation route of Example 165.

¹H NMR (400 MHz, DMSO-*d*₆) δ 8.84 (s, 2H), 8.56 (d, *J =* 8.8 Hz, 1H), 7.80 (d, *J =* 8.0 Hz, 1H), 7.66 (d, *J =* 7.6 Hz, 1H), 7.59 (s, 1H), 7.56 (s, 1H), 7.45 (dd, *J =* 8.8 Hz, 7.2 Hz, 1H), 7.17 (d, *J =* 7.2 Hz, 1H), 6.49 (s, 1H), 3.71 (s, 3H).

### Example 191. methyl 4-amino-7-bromo-1-(isoquinolin-8-yl)-2-oxo-1,2-dihydroquinoline-3-carboxylate

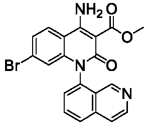

According to the preparation route of scheme 7, 8-aminoquinoline and 4-bromo-2-fluorobenzonitrile were used as the starting material to obtain the product with reference to the preparation route of Example 165.

¹H NMR (400 MHz, CDCl₃) δ 8.85 (s, 1H), 8.56 (d, *J =* 6.0 Hz, 1H), 7.95-8.30 (brs, 2H), 7.97 (d, *J=* 8.8 Hz, 1H), 7.84 (t, *J =* 8.0 Hz, 1H), 7.76 (d, *J =* 6.0 Hz, 1H), 7.61 (d, *J =* 8.8 Hz, 1H), 7.51 (d, *J* = 7.2 Hz, 1H), 7.11 (dd, *J =* 8.8 Hz, 2.0 Hz, 1H), 6.47 (d, *J* = 1.6 Hz, 1H), 3.86 (s, 3H).

### Example 192. methyl 4-amino-1-(isoquinolin-8-yl)-2-oxo-7-(trifluoromethyl)-1,2-dihydro quinoline-3 -carboxylate

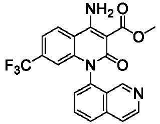

According to the preparation route of scheme 7, 8-amino-isoquinoline and 2-fluoro-4-trifluoromethylbenzonitrile were used as the starting material to obtain the product with reference to the preparation route of Example 165.

¹H NMR (400 MHz, CDCl₃) δ 8.85 (s, 1H), 8.57 (d, *J =* 5.6 Hz, 1H), 7.95-8.20 (brs, 2H), 7.99 (d, *J =* 8.8 Hz, 1H), 7.90 (d, *J =* 8.8 Hz, 1H), 7.84 (d, *J =* 8.0 Hz, 1H), 7.75 (d, *J =* 6.0 Hz, 1H), 7.53 (d, *J =* 7.2 Hz, 1H), 7.22 (d, *J=* 8.8 Hz, 1H), 6.56 (s, 1H), 3.88 (s, 3H).

### Example 193. methyl 4-amino-1-(4-(1-((tert-butoxycarbonyl)amino)ethyl)phenyl)-2-oxo-7-(trifluoromethyl)-1,2-dihydroquinoline-3 -carboxylate

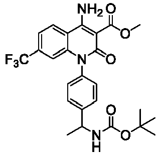

According to the preparation route of scheme 6, *tert*-butyl (1-(4-bromophenyl)ethyl)carbamate and 2-amino-4-(trifluoromethyl) benzonitrile were used as the starting material to obtain the product with reference to the preparation route of Example 81.

¹H NMR (400 MHz, CDCl₃) δ 8.05-8.50 (brs, 2H), 8.01 (d, *J =* 8.8 Hz, 1H), 7.43 (d, *J =* 7.2 Hz, 2H), 7.30 (d, J= 8.4 Hz, 1H), 7.08-7.10 (m, 2H), 6.71 (s, 1H), 5.40-5.61 (m 1H), 4.75-4.83 (m, 1H), 3.76 (s, 3H), 1.39 (d, *J =* 6.8 Hz, 3H), 1.23 (s, 9H).

### Example 194. methyl 4-amino-1-(4-(1-(aminoethyl)phenyl)-2-oxo-7-(trifluoromethyl)-1,2-dihydroquinoline-3-carboxylate hydrochloride

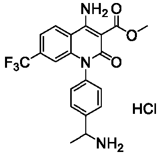

Methyl 4-amino-1-(4-(1-((*tert*-butoxycarbonyl)amino)ethyl)phenyl)-2-oxo-7-(trifluoro methyl)-1,2-dihydroquinoline-3-carboxylate (25 mg) obtained in Example 193 was added to a 4 mol/L HCl/1,4-dioxane solution (5 mL), and the mixture was stirred at room temperature for 2 hours and evaporated to remove the solvent to obtain the product (18 mg).

¹H NMR (400 MHz, DMSO_*d₆*) δ 8.48 (d, *J =* 8.4 Hz, 1H), 8.30-8.60 (brs, 5H), 7.68-7.71 (m, 2H), 7.56 (d, *J =* 8.4 Hz, 1H), 7.35-7.37 (m, 2H), 6.58 (s, 1H), 4.45-4.59 (m, 1H), 3.71 (s, 3H), 1.56 (d, *J =* 6.8 Hz, 3H).

### Example 195. methyl 4-atnino-7-bromo-1-(imidazo(3,2-a)pyridin-5-yl)-2-oxo-1,2-dihydro quinoline-3 -carboxylate

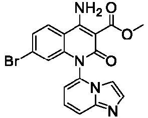

According to the preparation route of scheme 7, 2-fluoro-4-bromobenzonitrile and imidazo(1,2-a)pyridin-5-amine were used as the starting material to obtain the product with reference to the preparation route of Example 165.

¹H NMR (400 MHz, DMSO-*d*₆) δ 8.79 (s, 2H), 8.26 (d, *J =* 8.8 Hz, 1H), 7.80-7.73 (m, 1H), 7.60-7.48 (m, 3H), 7.42 (dd, *J =* 9.1, 7.1 Hz, 1H), 7.11 (d, *J =* 6.9 Hz, 1H), 6.43 (d, *J =* 1.8 Hz, 1H), 3.69 (s, 3H).

### Example 196. methyl 4-amino-1-(6-benzofuran-5-yl)-2-oxo-7-(trifluoromethyl)-1,2-dihydro quinoline-3 -carboxylate

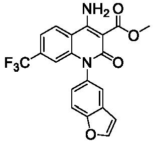

According to the preparation route of scheme 7, 2-chloro-4-(trifluoromethyl)benzonitrile and 5-aminobenzofuran were used as the starting material to obtain the product with reference to the preparation route of Example 165.

¹H NMR (400 MHz, DMSO-*d*₆) δ 8.46 (d, *J =* 8.8 Hz, 1H), 8.38 (s, 2H), 8.11 (d, *J =* 2.4 Hz, 1H), 7.79 (d, *J =* 8.8 Hz, 1H), 7.59 (d, *J =* 2.0 Hz, 1H), 7.54 (d, *J =* 6.8 Hz, 1H), 7.18 (dd, *J =* 8.4 Hz, 2.4 Hz, 1H), 7.03 (d, *J =* 2.4 Hz, 1H), 6.58 (s, 1H), 3.71 (s, 3H).

### Example 197. methyl 4-atnino-1-(phthalazin-5-yl)-7-bromo-2-oxo-1,2-dihydroquinoline-3-carboxylate

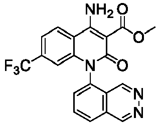

According to the preparation route of scheme 7, 2-chloro-4-bromobenzonitrile and 5-aminophthalazine were used as the starting material to obtain the product with reference to the preparation route of Example 165.

¹H NMR (400 MHz, DMSO-*d*₆) δ 9.84 (d, *J =* 1.5 Hz, 1H), 9.21 (s, 1H), 8.68 (s, 1H), 8.35 (d, *J =* 8.1 Hz, 1H), 8.26 (d, *J =* 8.8 Hz, 1H), 8.21 (t, *J* = 7.8 Hz, 1H), 8.00 (d, *J* = 7.4 Hz, 1H), 7.45 (dd, *J =* 8.8, 1.9 Hz, 1H), 6.36 (d, *J =* 1.9 Hz, 1H), 3.68 (s, 3H).

### Example 198. methyl 4-amino-7-bromo-2-oxo-1-(quinolin-4-yl)-1,2-dihydroquinoline-3-carboxylate

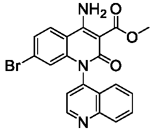

According to the preparation route of scheme 7, 2-fluoro-4-bromobenzonitrile and quinolin-4-amine were used as the starting material to obtain the product with reference to the preparation route of Example 165.

¹H NMR (400 MHz, DMSO-*d*₆) δ 9.48 (s, 1H), 8.60 (s, 2H), 8.46 (s, 1H), 8.30-8.32 (m, 1H), 8.23 *(d, J=* 8.8 Hz, 1H), 7.71-7.78 (m, 2H), 7.42 *(d, J=* 8.4 Hz, 1H), 7.36-7.39 (m, 1H), 6.33 *(d, J* = 1.6 Hz, 1H), 3.68 (s, 3H).

### Example 199. methyl 4-atnino-7-iodo-1-(isoquinolin-5-yl)-2-oxo-1,2-dihydroquinoline-3-carboxylate

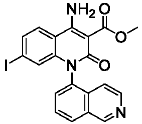

According to the preparation route of scheme 7, 2-fluoro-4-iodobenzonitrile and isoquinolin-5-amine were used as the starting material to obtain the product with reference to the preparation route of Example 165.

¹H NMR (400 MHz, DMSO-*d*₆) δ 9.47 (s, 1H), 8.56 (s, 2H), 8.42 (d, *J =* 6.4 Hz, 1H), 8.31 (d, *J* = 8.0 Hz, 1H), 8.05 (d, *J* = 8.8 Hz, 1H), 7.86 (t, *J =* 8.0 Hz, 1H), 7.77 (dd, *J* = 7.2 Hz, 1.2 Hz, 1H), 7.57 (dd, *J =* 8.4 Hz, 1.6 Hz, 1H), 7.19 (d, *J* = 5.6 Hz, 1H), 6.45 (d, *J* = 1.2 Hz, 1H), 3.67 (s, 3H).

### Example 200. methyl 4-amino-7-bromo-1-(4-(1-hydroxyethyl)phenyl)-2-oxo-1,2-dihydro quinoline-3 -carboxylate

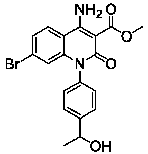

4-Bromo-2-fluorobenzonitrile and 1-(4-aminophenyl)ethan-1-one were used as the starting material to obtain the product with reference to the preparation route of Example 120 and 122.

¹H NMR (400 MHz, CD₃OD+CDCl₃) δ 7.76 (d, *J* = 9.2 Hz, 1H), 7.48-7.54 (m, 2H), 7.26 (dd, *J* = 8.8 Hz, 2.0 Hz, 1H), 7.09-7.12 (m, 2H), 6.68 (d, *J* = 2.0 Hz, 1H), 4.88 (q, *J* = 6.8 Hz, 1H), 3.80 (s, 3H), 1.48 (d, *J* = 6.8 Hz, 3H).

### Example 201. methyl 4-amino-7-chloro-1-(isoquinolin-5-yl)-2-oxo-1,2-dihydro-1,8-naphthyridine-3-carboxylate

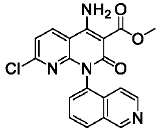

According to the preparation route of scheme 7, 2,6-dichloro-3-cyanopyridine and 5-amino-isoquinoline were used as the starting material to obtain the product with reference to the preparation route of Example 165.

¹H NMR (400 MHz, CD₃OD+CDCl₃) δ 9.24 (s, 1H), 8.29 (d, *J* = 6.0 Hz, 1H), 8.25 (d, *J =* 8.4 Hz, 1H), 8.06 (d, *J* = 8.0 Hz, 1H), 7.71 (t, *J* = 7.6 Hz, 1H), 7.57 (d, *J* = 7.2 Hz, 1H), 7.16 (d, *J* = 6.0 Hz, 1H), 7.10 *(d, J* = 8.4 Hz, 1H), 7.80 (s, 3H).

### Example 202. methyl 4-atnino-7-ethoxy-1-(isoquinolin-5-yl)-2-oxo-1,2-dihydroquinoline-3-carboxylate

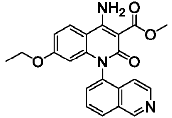

According to the preparation route of scheme 7, 4-ethoxy-2-fluorobenzonitrile and 5-amino-isoquinoline were used as the starting material to obtain the product with reference to the preparation route of Example 165.

¹H NMR (400 MHz, CDCl₃) δ 9.35 (s, 1H), 8.43 (d, *J* = 5.6 Hz, 1H), 8.11 (d, *J* = 8.0 Hz, 1H), 7.76 (t, *J* = 7.6 Hz, 1H), 7.66 (d, *J* = 7.2 Hz, 1H), 7.62 (d, *J* = 8.8 Hz, 1H), 7.24-7.26 (m, 1H), 6.75 (dd, *J* = 8.8 Hz, 2.4 Hz, 1H), 5.73 (d, *J* = 2.4 Hz, 1H), 3.86 (s, 3H), 3.63-3.80 (m, 2H), 1.21 (t, *J* = 7.2 Hz, 3H).

### Example 203. methyl 4-amino-1-(6-chloropyridin-3-yl)-2-oxo-7-(trifluoromethyl)-1,2-dihydro-1,8-naphthyridine-3-carboxylate

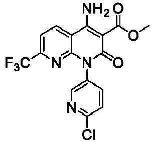

According to the preparation route of scheme 7, 2-chloro-6-(trifluoromethyl)pyridin-3-benzonitrile and 6-chloropyridin-3-amino were used as the starting material to obtain the product with reference to the preparation route of Example 165.

¹H NMR (400 MHz, CDCl₃) δ 8.28 (d, *J* = 2.8 Hz, 1H), 8.19 (d, *J* = 7.6 Hz, 1H), 7.60-8.02 (brs, 2H), 7.57 (dd, *J* = 8.0 Hz, 2.8 Hz, 1H), 7.55 (d, *J* = 8.0 Hz, 1H), 7.47 (d, *J* = 8.0 Hz, 1H), 3.93 (s, 3H).

### Example 204. methyl 4-atnino-1-(6-chloro-4-methylpyridin-3-yl)-2-oxo-7-(trifluoromethyl)-1,2-dihydro-1,8-naphthyridine-3-carboxylate

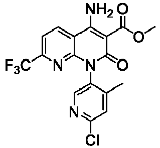

According to the preparation route of scheme 7, 2-chloro-6-(trifluoromethyl)pyridin-3-benzonitrile and 6-chloro-4-methylpyridin-3-amino were used as the starting material to obtain the product with reference to the preparation route of Example 165.

¹H NMR (400 MHz, CDCl₃) δ 8.20 *(d, J* = 8.0 Hz, 1H), 8.10 (s, 1H), 7.63-8.06 (brs, 2H), 7.54 (d, *J =* 8.0 Hz, 1H), 7.34 (s, 1H), 3.93 (s, 3H), 2.02 (s, 3H).

### Example 205. methyl 4-amino-1-(isoquinolin-5-yl)-7-methyl-2-oxo-1,2-dihydroquinoline-3-carboxylate

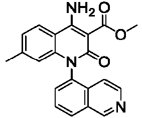

According to the preparation route of scheme 7, 2-chloro-4-methylbenzonitrile and 5-amino-isoquinoline were used as the starting material to obtain the product with reference to the preparation route of Example 165.

¹H NMR (400 MHz, DMSO-*d*₆) δ 9.45 (s, 1H), 8.54 (s, 2H), 8.40 (d, *J =* 5.6 Hz, 1H), 8.29 (d, *J =* 8.4 Hz, 1H), 8.17 *(d, J =* 8.4 Hz, 1H), 7.85 (*t, J =* 8.0 Hz, 1H), 7.72 (*d, J =* 7.2 Hz, 1H), 7.13 (d, *J =* 6.0 Hz, 1H), 7.04 (d, *J =* 7.6 Hz, 1H), 5.98 (s, 1H), 3.66 (s, 3H), 2.07 (s, 3H).

### Example 206. methyl 4-amino-1-(isoquinolin-5-yl)-7-methoxy-2-oxo-1,2-dihydroquinoline-3-carboxylate

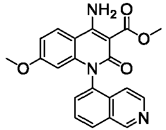

According to the preparation route of scheme 7, 4-methoxy-2-fluorobenzonitrile and 5-amino-isoquinoline were used as the starting material to obtain the product with reference to the preparation route of Example 165.

¹H NMR (400 MHz, CDCl₃) δ 9.36 (s, 1H), 8.43 (d, *J* = 6.0 Hz, 1H), 8.11 (d, *J* = 8.0 Hz, 1H), 7.76 (t, *J* = 7.6 Hz, 1H), 7.63-7.67 (m, 2H), 7.24-7.25 (m, 1H), 6.78 (dd, *J* = 9.2 Hz, 2.4 Hz, 1H), 5.75 (d, *J* = 2.4 Hz, 1H), 3.86 (s, 3H), 3.53 (s, 3H).

### Example 207. methyl 4-amino-1-(6-methylpyridin-3-yl)-2-oxo-7-(trifluoromethyl)-1,2-dihydro-1,8-naphthyridine-3-carboxylate

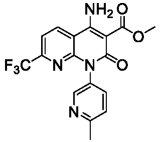

According to the preparation route of scheme 7, 2-chloro-6-(trifluoromethyl)pyridin-3-benzonitrile and 6-methylpyridin-3-amino were used as the starting material to obtain the product with reference to the preparation route of Example 165.

¹H NMR (400 MHz, d-DMSO) δ 8.94 (d, *J* = 7.6 Hz, 1H), 8.54 (s, 2H), 8.26 (d, *J* = 2.4 Hz, 1H), 7.78 (d, *J* = 8.0 Hz, 1H), 7.57 (dd, *J* = 8.4 Hz, 2.8 Hz, 1H), 7.37 (d, *J* = 8.4 Hz, 1H), 3.73 (s, 3H), 2.53 (s, 3H).

### Example 208. methyl 4-amino-1-(6-methoxypyridin-3-yl)-2-oxo-7-(trifluoromethyl)-1,2-dihydro-1,8-naphthyridine-3-carboxylate

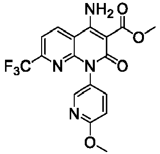

According to the preparation route of scheme 7, 2-chloro-6-(trifluoromethyl)pyridin-3-benzonitrile and 6-methoxypyridin-3-amino were used as the starting material to obtain the product with reference to the preparation route of Example 165.

¹H NMR (400 MHz, CD₃OD+CDCl₃) δ 8.56 (d, *J* = 8.0 Hz, 1H), 7.92 (d, *J* = 2.4 Hz, 1H), 7.47 (d, *J* = 8.4 Hz, 1H), 7.42 (dd, *J* = 8.4 Hz, 2.4 Hz, 1H), 6.83 (d, *J* = 8.4 Hz, 1H), 3.93 (s, 3H), 3.83 (s, 3H).

### Example 209. methyl 4-amino-7-bromo-1-(4-methylpyridin-3-yl)-2-oxo-1,2-dihydroquinoline-3 -carboxylate

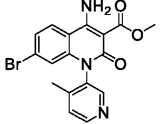

According to the preparation route of scheme 7, 2-fluoro-4-bromobenzonitrile and 4-methylpyridin-3-amine were used as the starting material to obtain the product with reference to the preparation route of Example 165.

¹H NMR (400 MHz, DMSO-*d*₆) δ 8.53-8.60 (m, 3H), 8.33 (s, 1H), 8.25 (d, *J =* 9.2 Hz, 1H), 7.50 (d, *J =* 4.8 Hz, 1H), 7.44 (dd, *J =* 8.8 Hz, 2.0 Hz, 1H), 6.36 (d, *J =* 2.0 Hz, 1H), 3.69 (s, 3H), 1.94 (s, 3H).

### Example 210. methyl 4-amino-1-(4-methylpyridin-3-yl)-2-oxo-7-(trifluoromethyl)-1,2-dihydroquinoline-3 -carboxylate

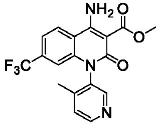

According to the preparation route of scheme 7, 2-fluoro-4-(trifluoromethyl)benzonitrile and 4-methylpyridin-3-amine were used as the starting material to obtain the product with reference to the preparation route of Example 165.

¹H NMR (400 MHz, DMSO-*d*₆) δ 8.61 (s, 2H), 8.54-8.58 (m, 2H), 8.37 (s, 1H), 7.58 (dd, *J =* 8.4 Hz, 1.2 Hz, 1H), 7.52 (d, *J =* 5.2 Hz, 1H), 6.44 (s, 1H), 3.72 (s, 3H), 1.94 (s, 3H).

### Example 211. methyl 4-amino-7-bromo-1-(6-methoxypyridin-3-yl)-2-oxo-1,2-dihydroquinoline-3 -carboxylate

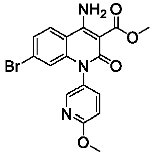

According to the preparation route of scheme 7, 2-fluoro-4-bromobenzonitrile and 6-methoxypyridin-3-amine were used as the starting material to obtain the product with reference to the preparation route of Example 165.

¹H NMR (400 MHz, DMSO-*d*₆) δ 8.43 (s, 2H), 8.17 (d, *J =* 8.8 Hz, 1H), 8.06 (d, *J =* 2.8 Hz, 1H), 7.64 (dd, *J =* 8.8 Hz, 2.8 Hz, 1H), 7.42 (dd, *J =* 8.8 Hz, 2.0 Hz, 1H), 7.00 (d, *J =* 8.8 Hz, 1H), 6.58 (d, *J =* 2.0 Hz, 1H), 3.91 (s, 3H), 3.69 (s, 3H).

### Example 212. methyl 4-amino-1-(6-methoxypyridin-3-yl)-2-oxo-7-(trifluoromethyl)-1,2-dihydroquinoline-3 -carboxylate

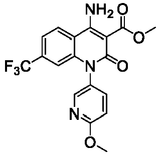

According to the preparation route of scheme 7, 2-fluoro-4-(trifluoromethyl)benzonitrile and 6-methoxypyridin-3-amine were used as the starting material to obtain the product with reference to the preparation route of Example 165.

¹H NMR (400 MHz, DMSO-*d*₆) δ 8.46 (d, *J =* 8.4 Hz, 1H), 8.44 (s, 2H), 8.10 (d, *J =* 2.8 Hz, 1H), 7.69 (dd, *J =* 8.4 Hz, 2.8 Hz, 1H), 7.57 (d, *J =* 8.4 Hz, 1H), 7.02 (d, *J =* 8.8 Hz, 1H), 6.67 (s, 1H), 3.92 (s, 3H), 3.72 (s, 3H).

### Example 213. methyl 4-atnino-1-(isoquinolin-5-yl)-7-fluoro-2-oxo-1,2-dihydroquinoline-3-carboxylate

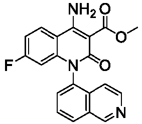

According to the preparation route of scheme 7, 2-chloro-4-fluorobenzonitrile and 5-amino-isoquinoline were used as the starting material to obtain the product with reference to the preparation route of Example 165.

¹H NMR (400 MHz, DMSO-*d*₆) δ 9.46 (s, 1H), 8.65 (s, 2H), 8.40-8.44 (m, 2H), 8.30 (d, *J =* 8.4 Hz, 1H), 7.85 (t, *J =* 7.6 Hz, 1H), 7.77 (d, *J =* 7.2 Hz, 1H), 7.19 (d, *J =* 5.6 Hz, 1H), 7.09-7.14 (m, 1H), 5.87 (dd, *J =* 10.8 Hz, 2.4 Hz, 1H), 3.68 (s, 3H).

### Example 214. methyl 4-amino-7-bromo-1-(6-methylpyridin-3-yl)-2-oxo-1,2-dihydroquinoline-3 -carboxylate

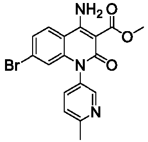

According to the preparation route of scheme 7, 2-fluoro-4-bromobenzonitrile and 6-methylpyridin-3-amine were used as the starting material to obtain the product with reference to the preparation route of Example 165.

¹H NMR (400 MHz, DMSO-*d*₆) δ 8.44 (s, 2H), 8.31 (d, *J =* 2.4 Hz, 1H), 8.18 (d, *J =* 9.2 Hz, 1H), 7.64 (dd, *J =* 8.4 Hz, 2.4 Hz, 1H), 7.46 (d, *J =* 8.0 Hz, 1H), 7.42 (dd, *J =* 8.4 Hz, 1.6 Hz, 1H), 6.49 (d, *J =* 1.6 Hz, 1H), 3.69 (s, 3H), 2.56 (s, 3H).

### Example 215. methyl 4-amino-1-(6-methylpyridin-3-yl)-2-oxo-7-(trifluoromethyl)-1,2-dihydroquinoline-3 -carboxylate

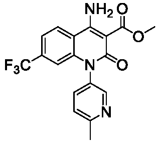

According to the preparation route of scheme 7, 2-fluoro-4-(trifluoromethyl)benzonitrile and 6-methylpyridin-3-amine were used as the starting material to obtain the product with reference to the preparation route of Example 165.

¹H NMR (400 MHz, DMSO-*d*₆) δ 8.46-8.48 (m, 3H), 8.35 (d, *J =* 2.4 Hz, 1H), 7.68 (dd, *J =* 8.0 Hz, 2.4 Hz, 1H), 7.58 (dd, *J =* 8.8 Hz, 1.6 Hz, 1H), 7.47 (d, *J =* 8.8 Hz, 1H), 6.59 (s, 1H), 3.71 (s, 3H), 2.57 (s, 3H).

### Example 216. methyl 4-amino-1-(1-chloroisoquinolin-5-yl)-7-bromo-2-oxo-1,2-dihydroquinoline-3 -carboxylate

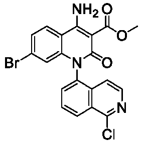

According to the preparation route of scheme 7, 2-fluoro-4-bromobenzonitrile and 1-chloro-5-amino-isoquinoline were used as the starting material to obtain the product with reference to the preparation route of Example 165.

¹H NMR (400 MHz, DMSO-*d*₆) δ 8.62 (s, 2H), 8.46 (d, *J =* 8.4 Hz, 1H), 8.25 (d, *J =* 8.8 Hz, 1H), 8.22 (d, *J =* 6.0 Hz, 1H), 7.99 (t, *J =* 7.2 Hz, 1H), 7.91 (d, *J =* 6.4 Hz, 1H), 7.43 (dd, *J =* 8.8 Hz, 1.6 Hz, 1H), 7.30 (d, *J =* 6.0 Hz, 1H), 6.32 (d, *J =* 1.6 Hz, 1H), 3.68 (s, 3H).

### Example 217. methyl 4-amino-1-(3-chloroisoquinolin-5-yl)-7-bromo-2-oxo-1,2-dihydroquinoline-3 -carboxylate

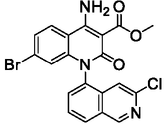

According to the preparation route of scheme 7, 2-fluoro-4-bromobenzonitrile and 3-chloro-5-amino-isoquinoline were used as the starting material to obtain the product with reference to the preparation route of Example 165.

¹H NMR (400 MHz, DMSO-*d*₆) δ 9.38 (s, 1H), 8.63 (s, 2H), 8.36 (d, *J =* 7.6 Hz, 1H), 8.24 (d, *J =* 8.4 Hz, 1H), 7.87 (d, *J =* 8.4 Hz, 1H), 7.83 (d, *J =* 6.4 Hz, 1H), 7.42-7.44 (m, 2H), 6.30 *(d, J=* 1.6 Hz, 1H), 3.68 (s, 3H).

### Example 218. methyl 4-amino-1-(3-methylisoquinolin-5-yl)-7-bromo-2-oxo-1,2-dihydroquinoline-3 -carboxylate

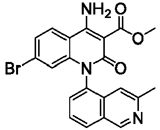

According to the preparation route of scheme 7, 2-fluoro-4-bromobenzonitrile and 3-methyl-5-amino-isoquinoline were used as the starting material to obtain the product with reference to the preparation route of Example 165.

¹H NMR (400 MHz, DMSO-*d*₆) δ 9.36 (s, 1H), 8.59 (s, 2H), 8.23-8.26 (m, 2H), 7.75 (t, *J =* 7.6 Hz, 1H), 7.69 (dd, *J =* 7.2 Hz, 1.2 Hz, 1H), 7.42 (dd, *J =* 8.4 Hz, 1.6 Hz, 1H), 7.05 (s, 1H), 6.23 (d, *J =* 2.0 Hz, 1H), 3.68 (s, 3H), 2.48 (s, 3H).

### Example 219. methyl 4-amino-7-bromo-1-(isoquinolin-7-yl)-2-oxo-1,2-dihydroquinoline-3-carboxylate

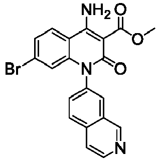

According to the preparation route of scheme 7, 2-fluoro-4-bromobenzonitrile and isoquinolin-7-amine were used as the starting material to obtain the product with reference to the preparation route of Example 165.

¹H NMR (400 MHz, DMSO-*d*₆) δ 9.35 (s, 1H), 8.60 (d, *J =* 6.0 Hz, 1H), 8.45 (s, 2H), 8.21 (d, *J =* 8.8 Hz, 1H), 8.16 (d, *J =* 8.8 Hz, 1H), 8.11 (s, 1H), 7.95 (d, *J* = 6.0 Hz, 1H), 7.64 (dd, *J =* 8.8 Hz, 2.0 Hz, 1H), 7.43 (dd, *J =* 8.8 Hz, 2.0 Hz, 1H), 6.52 (d, *J =* 2.0 Hz, 1H), 3.69 (s, 3H).

### Example 220. methyl 4-atnino-7-bromo-2-oxo-1-(quinoxalin-6-yl)-1,2-dihydroquinoline-3-carboxylate

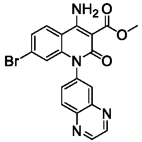

According to the preparation route of scheme 7, 2-fluoro-4-bromobenzonitrile and quinoxalin-6-amine were used as the starting material to obtain the product with reference to the preparation route of Example 165.

¹H NMR (400 MHz, DMSO-*d*₆) δ 9.05 (d, *J* = 2.0 Hz, 1H), 9.02 (d, *J* = 2.0 Hz, 1H), 8.51 (s, 2H), 8.28 (d, *J* = 8.4 Hz, 1H), 8.22 (d, *J* = 9.2 Hz, 1H), 8.10 (d, *J* = 2.0 Hz, 1H), 7.74 (dd, *J* = 8.8 Hz, 2.0 Hz, 1H), 7.44 (dd, *J* = 8.8 Hz, 2.0 Hz, 1H), 6.59 (d, *J* = 2.0 Hz, 1H), 3.70 (s, 3H).

### Example 221. methyl 4-amino-7-bromo-1-(isoquinolin-5-yl)-2-oxo-1,2-dihydro-1,8-naphthyridine-3 -carboxylate

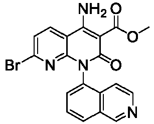

According to the preparation route of scheme 7, 2,6-dibromo-3-cyanopyridine and 5-amino-isoquinoline were used as the starting material to obtain the product with reference to the preparation route of Example 165.

¹H NMR (400 MHz, CD₃OD+CDCl₃) δ 9.18 (s, 1H), 8.23 (d, *J* = 5.6 Hz, 1H), 8.16 (d, *J* = 8.0 Hz, 1H), 8.02 (d, *J* = 8.4 Hz, 1H), 7.67 (t, *J* = 7.6 Hz, 1H), 7.53 (d, *J* = 6.4 Hz, 1H), 7.21 (d, *J* = 8.4 Hz, 1H), 7.13 *(d, J* = 6.0 Hz, 1H), 3.74 (s, 3H).

### Example 222. methyl 4-amino-7-bromo-1-(1,3-dihydro-2-benzofuran-4-yl)-2-oxo-1,2-dihydroquinoline-3 -carboxylate

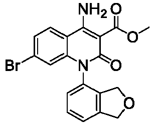

According to the preparation route of scheme 7, 2-fluoro-4-bromobenzonitrile and 1,3-dihydro-2-benzofuran-4-amine were used as the starting material to obtain the product with reference to the preparation route of Example 165.

¹H NMR (400 MHz, DMSO-*d*₆) δ 8.45 (s, 2H), 8.18 (d, *J =* 8.8 Hz, 1H), 7.41-7.51 (m, 3H), 7.14 (d, *J =* 6.8 Hz, 1H), 6.49 (d, *J =* 2.0 Hz, 1H), 5.06-5.15 (m, 2H), 4.59-4.70 (m, 2H), 3.69 (s, 3H).

### Example 223. methyl 4-amino-1-(3,4-dihydro-1H-isochroman-5-yl)-2-oxo-7-(trifluoromethyl)-1,2-dihydroquinoline-3 -carboxylate

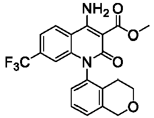

According to the preparation route of scheme 7, 2-fluoro-4-(trifluoromethyl)benzonitrile and 3,4-dihydro-*1H*-isochroman-5-amine were used as the starting material to obtain the product with reference to the preparation route of Example 165.

¹H NMR (400 MHz, DMSO-*d*₆) δ 8.48 (d, *J =* 8.0 Hz, 1H), 8.44 (s, 2H), 7.57 (d, *J =* 7.2 Hz, 1H), 7.38 (t, *J* = 7.6 Hz, 1H), 7.22 (d, *J* = 6.8 Hz, 1H), 7.10 (d, *J* = 7.6 Hz, 1H), 6.50 (s, 1H), 4.69-4.80 (m, 2H), 3.72-3.77 (m, 5H), 1.94-2.10 (m, 2H).

### Example 224. methyl 4-amino-1-(isoquinolin-7-yl)-2-oxo-7-(trifluoromethyl)-1,2-dihydroquinoline-3 -carboxylate

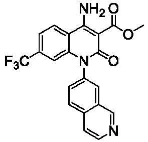

According to the preparation route of scheme 7, 2-fluoro-4-(trifluoromethyl)benzonitrile and isoquinolin-7-amine were used as the starting material to obtain the product with reference to the preparation route of Example 165.

¹H NMR (400 MHz, DMSO-*d*₆) δ 9.36 (s, 1H), 8.61 (d, *J =* 6.0 Hz, 1H), 8.50 (d, *J =* 8.8 Hz, 1H), 8.48 (s, 2H), 8.18 (d, *J* = 9.2 Hz, 1H), 8.15 (d, *J =* 1.6 Hz, 1H), 7.96 (d, *J =* 5.6 Hz, 1H), 7.68 (dd, *J=* 8.8 Hz, 2.0 Hz, 1H), 7.58 (d, *J =* 7.6 Hz, 1H), 6.61 (s, 1H), 3.72 (s, 3H).

### Example 225. methyl 4-amino-1-(6-chloro-4-methylpyridin-3-yl)-7-iodo-2-oxo-1,2-dihydroquinoline-3 -carboxylate

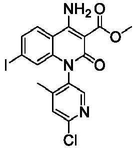

According to the preparation route of scheme 7, 2-fluoro-4-iodobenzonitrile and 6-chloro-4-methylpyridin-3-amine were used as the starting material to obtain the product with reference to the preparation route of Example 165.

¹H NMR (400 MHz, DMSO-*d*₆) δ 8.54 (s, 2H), 8.22 (s, 1H), 8.02 (d, *J =* 8.8 Hz, 1H), 7.69 (s, 1H), 7.61 (dd, *J* = 8.0 Hz, 1.6 Hz, 1H), 6.69 (d, *J =* 1.6 Hz, 1H), 3.69 (s, 3H), 1.94 (s, 3H).

### Example 226. methyl 4-amino-7-bromo-1-(isoquinolin-6-yl)-2-oxo-1,2-dihydro quinoline-3 -carboxylate

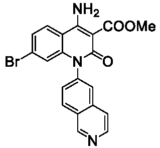

According to the preparation route of scheme 7, 4-bromo-2-fluoro-benzonitrile and 6-amino-isoquinoline were used as the starting material to obtain the product with reference to the preparation route of Example 165.

¹H NMR (400 MHz, DMSO-*d*₆) δ 9.43 (s, 1H), 8.57 (d, *J =* 5.6 Hz, 1H), 8.46 (s, 2H), 8.32 (d, *J =* 8.4 Hz, 1H), 8.20 (d, *J =* 8.8 Hz, 1H), 7.97 (s, 1H), 7.87 (d, *J =* 5.6 Hz, 1H), 7.54 (dd, *J =* 8.4 Hz, 2.0 Hz, 1H), 7.43 (dd, *J =* 8.8 Hz, 2.0 Hz, 1H), 6.49 (d, *J* = 2.0 Hz, 1H), 3.69 (s, 3H).

### Example 227. methyl 4-amino-1-(8-chloroisoquinolin-5-yl)-7-iodo-2-oxo-1,2-dihydroquinoline-3 -carboxylate

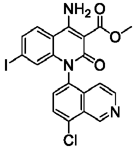

According to the preparation route of scheme 7, 2-fluoro-4-iodobenzonitrile and 8-chloroisoquinolin-5-amine were used as the starting material to obtain the product with reference to the preparation route of Example 165.

¹H NMR (400 MHz, DMSO-*d*₆) δ 9.66 (s, 1H), 8.60 (s, 2H), 8.55 (d, *J =* 5.6 Hz, 1H), 8.05 (d, *J =* 8.4 Hz, 1H), 8.01 (d, *J =* 8.0 Hz, 1H), 7.79 (d, *J =* 8.0 Hz, 1H), 7.59 (d, *J =* 8.4 Hz, 1H), 7.28 (d, *J =* 6.0 Hz, 1H), 6.57 (d, *J =* 1.6 Hz, 1H), 3.67 (s, 3H).

### Example 228. methyl 4-amino-2-oxo-1-(1,2,3,4-tetrahydroisoquinolin-5-yl)-7-(trifluoromethyl)-1,2-dihydroquinoline-3 -carboxylate

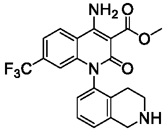

According to the preparation route of scheme 7, 2-fluoro-4-(trifluoromethyl)benzonitrile and 1,2,3,4-tetrahydroisoquinolin-5-amine were used as the starting material to obtain the product with reference to the preparation route of Example 165.

¹H NMR (400 MHz, DMSO-*d*₆) δ 9.26-9.60 (brs, 2H), 8.44-8.58 (m, 3H), 7.58 (d, *J =* 8.8 Hz, 1H), 7.46 (t, *J =* 7.6 Hz, 1H), 7.40 (d, *J =* 7.6 Hz, 1H), 7.21 (d, *J =* 7.6 Hz, 1H), 6.52 (s, 1H), 4.36 (s, 2H), 3.71 (s, 3H), 3.23 (t, *J =* 6.4 Hz, 2H), 2.58-2.64 (m, 1H), 2.28-2.38 (m, 1H).

### Example 229. methyl 4-amino-1-(1H-indol-4-yl)-2-oxo-7-(trifluoromethyl)-1,2-dihydroquinoline-3 -carboxylate

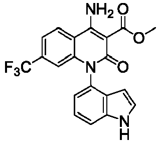

According to the preparation route of scheme 7, 2-fluoro-4-(trifluoromethyl)benzonitrile and *1H*-indol-4-amine were used as the starting material to obtain the product with reference to the preparation route of Example 165.

¹H NMR (400 MHz, DMSO-*d*₆) δ 11.36 (s, 1H), 8.46 (d, *J =* 8.4 Hz, 1H), 8.38 (s, 2H), 7.54 (d, *J =* 8.0 Hz, 1H), 7.50 (d, *J =* 8.4 Hz, 1H), 7.32 (t, *J =* 2.4 Hz, 1H), 7.24 (t, *J =* 8.0 Hz, 1H), 6.90 (d, *J* = 7.6 Hz, 1H), 6.51 (s, 1H), 5.91 (t, *J* = 2.4 Hz, 1H), 3.71 (s, 3H).

### Example 230. methyl 4-amino-2-oxo-1-(2-oxo-1,2-dihydropyridin-4-yl)-7-(trifluoromethyl)-1,2-dihydroquinoline-3 -carboxylate

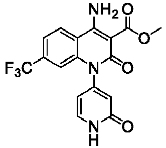

According to the preparation route of scheme 6, 4-bromo-2-hydroxypyridine and 2-amino-4-(trifluoromethyl)benzonitrile were used as the starting material to obtain the product with reference to the preparation route of Example 81.

¹H NMR (400 MHz, CD₃OD) δ 8.28 (d, *J =* 8.0 Hz, 1H), 7.61 (d, *J =* 6.8 Hz, 1H), 7.50 (d, *J* = 8.8 Hz, 1H), 7.42 (d, *J =* 8.4 Hz, 1H), 7.01(s, 1H), 6.50 (s, 1H), 3.87 (s, 3H).

### Example 231. methyl 4-amino-1-(1-methylisoquinolin-5-yl)-7-bromo-2-oxo-1,2-dihydroquinoline-3 -carboxylate

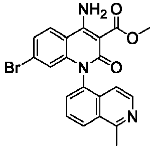

According to the preparation route of scheme 7, 2-fluoro-4-bromobenzonitrile and 1-methyl-5-amino-isoquinoline were used as the starting material to obtain the product with reference to the preparation route of Example 165.

¹H NMR (400 MHz, DMSO-*d*₆) δ 8.56 (s, 2H), 8.39 (d, *J =* 8.4 Hz, 1H), 8.23-8.26 (m, 2H), 7.84 (t, *J =* 8.0 Hz, 1H), 7.76 (t, *J =* 7.6 Hz, 1H), 7.41 (dd, *J =* 8.8 Hz, 2.0 Hz, 1H), 7.02 (d, *J =* 6.0 Hz, 1H), 6.23 (d, *J =* 2.0 Hz, 1H), 3.68 (s, 3H), 2.96 (s, 3H).

### Example 232. methyl 4-amino-1-(isoquinolin-6-yl)-2-oxo-7-(trifluoromethyl)-1,2-dihydroquinoline-3 -carboxylate

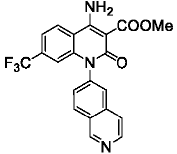

According to the preparation route of scheme 7, 2-fluoro-4-trifluoromethyl-benzonitrile and 6-amino-isoquinoline were used as the starting material to obtain the product with reference to the preparation route of Example 165.

¹H NMR (400 MHz, DMSO-*d*₆) δ 9.44 (s, 1H), 8.58 (d, *J =* 5.6 Hz, 1H), 8.44-8.51 (m, 3H), 8.34 (d, *J =* 9.2 Hz, 1H), 8.01 (s, 1H), 7.88 (d, *J =* 6.0 Hz, 1H), 7.56-7.59 (m, 2H), 6.59 (s, 1H), 3.72 (s, 3H).

### Example 233. methyl 4-atnino-7-iodo-1-(isoquinolin-6-yl)-2-oxo-1,2-dihydroquinoline-3-carboxylate

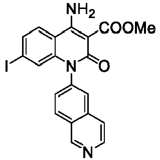

According to the preparation route of scheme 7, 2-fluoro-4-iodo-benzonitrile and 6-amino-isoquinoline were used as the starting material to obtain the product with reference to the preparation route of Example 165.

¹H NMR (400 MHz, DMSO-*d*₆) δ 9.43 (s, 1H), 8.57 (d, *J =* 5.6 Hz, 1H), 8.44 (s, 2H), 8.32 (d, *J =* 8.8 Hz, 1H), 8.01 (d, *J =* 8.8 Hz, 1H), 7.95 (s, 1H), 7.88 (d, *J =* 5.6 Hz, 1H), 7.58 (dd, *J =* 8.8 Hz, 1.2 Hz, 1H), 7.53 (dd, *J =* 8.8 Hz, 2.0 Hz, 1H), 6.68 (d, *J =* 2.0 Hz, 1H), 3.69 (s, 3H).

### Example 234. methyl 4-amino-2-oxo-1-(5,6,7,8-tetrahydroisoquinolin-5-yl)-7-(trifluoromethyl)-1,2-dihydroquinoline-3 -carboxylate

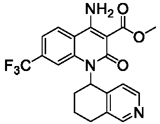

According to the preparation route of scheme 7, 2-fluoro-4-(trifluoromethyl)benzonitrile and 5,6,7,8-tetrahydroisoquinolin-5-amine were used as the starting material to obtain the product with reference to the preparation route of Example 165.

¹H NMR (400 MHz, DMSO-*d*₆) δ 8.46 (s, 2H), 8.41 (*d, J =* 8.8 Hz, 1H), 8.35 (*d, J =* 5.1 Hz, 1H), 8.30 (s, 1H), 7.22 (d, *J =* 6.8 Hz, 1H), 7.31 (d, *J =* 5.1 Hz, 1H), 6.60 (s, 1H), 3.89 (dd, *J =* 7.6, 5.3 Hz, 1H), 3.68 (s, 3H), 2.78-2.69 (m, 2H), 2.07 (m, 1H), 1.94 (m, 1H), 1.76 (m, 2H).

### Example 235. methyl 4-amino-1-(2-methyl-4-nitrophenyl)-2-oxo-7-(trifluoromethyl)-1,2-dihydroquinoline-3 -carboxylate

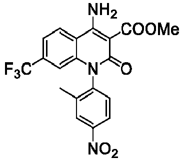

According to the preparation route of scheme 7, 2-amino-4-trifluoromethyl-benzonitrile and 1-fluoro-2-methyl-4-nitrobenzene were used as the starting material to obtain the product with reference to the preparation route of Example 165.

¹H NMR (400 MHz, DMSO-*d*₆) δ 8.53 (s, 2H), 8.35 (d, *J =* 2.8 Hz, 1H), 8.20-8.23 (m, 2H), 7.51 (d, *J* = 8.8 Hz, 1H), 7.45 (dd, *J* = 8.8 Hz, 2.0 Hz, 1H), 6.41 (d, *J =* 2.0 Hz, 1H), 3.70 (s, 3H), 2.02 (s, 3H).

### Example 236. methyl 4-amino-7-bromo-1-(2-methyl-4-nitrophenyl)-2-oxo-1,2-dihydroquinoline-3 -carboxylate

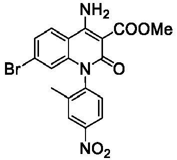

According to the preparation route of scheme 7, 2-amino-4-bromo-benzonitrile and 1-fluoro-2-methyl-4-nitrobenzene were used as the starting material to obtain the product with reference to the preparation route of Example 165.

¹H NMR (400 MHz, DMSO-*d*₆) δ 8.54 (s, 2H), 8.35 (d, *J =* 2.4 Hz, 1H), 8.20-8.23 (m, 2H), 7.51 (d, *J =* 8.8 Hz, 1H), 7.45 (dd, *J =* 8.4 Hz, 1.6 Hz, 1H), 6.41 (d, *J =* 1.6 Hz, 1H), 3.70 (s, 3H), 2.02 (s, 3H).

### Example 237. methyl 4-amino-1-(4-amino-2-methylphenyl)-2-oxo-7-(trifluoromethyl)-1,2-dihydroquinoline-3 -carboxylate

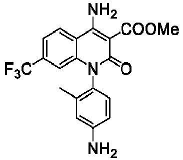

According to the preparation route of scheme 7, 2-amino-4-trifluoromethyl-benzonitrile and 1-fluoro-2-methyl-4-nitrobenzene were used as the starting material to obtain the product with reference to the preparation route of Example 165 and Example 147.

¹H NMR (400 MHz, DMSO-*d*₆) δ 8.42 (d, *J =* 8.4 Hz, 1H), 8.29 (s, 2H), 7.51 (dd, *J =* 8.8 Hz, 1.6 Hz, 1H), 6.73 (d, *J* = 8.0 Hz, 1H), 6.63 (s, 1H), 6.56 (d, *J =* 2.4 Hz, 1H), 6.51 (dd, *J* = 8.8 Hz, 2.0 Hz, 1H), 5.26 (s, 2H), 3.71 (s, 3H), 1.69 (s, 3H).

### Example 238. methyl 4-amino-1-(4-amino-2-methylphenyl)-7-bromo-2-oxo-1,2-dihydroquinoline-3 -carboxylate

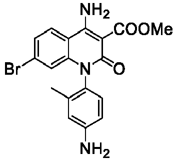

According to the preparation route of scheme 7, 2-amino-4-bromo-benzonitrile and 1-fluoro-2-methyl-4-nitrobenzene were used as the starting material to obtain the product with reference to the preparation route of Example 165 and Example 147.

¹H NMR (400 MHz, DMSO-*d*₆) δ 8.28 (s, 2H), 8.13 (d, *J=* 8.4 Hz, 1H), 7.36 (dd, *J=* 8.8 Hz, 2.0 Hz, 1H), 6.70 (d, *J =* 8.0 Hz, 1H), 6.49-6.56 (m, 3H), 5.25 (s, 2H), 3.70 (s, 3H), 1.70 (s, 3H).

### Example 239. methyl 4-amino-1-(1-aminoisoquinolin-5-yl)-7-bromo-2-oxo-1,2-dihydroquinoline-3 -carboxylate

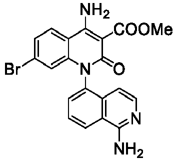

Example 216 (50 mg), potassium carbonate (45 mg) and 1 mol/L ammonia methanol solution (5 mL) were added into a sealed tube, and the mixture was heated to 80 °C and stirred overnight. The reaction solution was cooled to room temperature and concentrated under reduced pressure, and the residue was purified through column chromatography (dichloromethane/methanol =10:1) to obtain the product (12 mg).

¹H NMR (400 MHz, CD₃OD+CDCl₃) δ 8.46 (d, *J* = 8.0 Hz, 1H), 8.01 (d, *J* = 8.8 Hz, 1H), 7.88 (d, *J =* 6.0 Hz, 1H), 7.76 (t, *J =* 7.6 Hz, 1H), 7.63 (dd, *J =* 7.6 Hz, 1.2 Hz, 1H), 7.36 (dd, *J =* 8.8 Hz, 2.0 Hz, 1H), 7.73 (d, *J* = 6.0 Hz, 1H), 6.44 *(d, J=* 1.6 Hz, 1H), 4.14 (s, 3H).

### Example 240. methyl 4-atnino-1-(1-chloroisoquinolin-5-yl)-7-bromo-2-oxo-1,2-dihydro-1,8-naphthyridine-3 -carboxylate

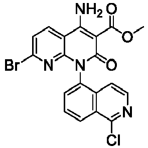

According to the preparation route of scheme 7, 2,6-dibromonicotinonitrile and 1-chloro-5-amino-isoquinoline were used as the starting material to obtain the product with reference to the preparation route of Example 165.

¹H NMR (400 MHz, DMSO-*d*₆) δ 8.67 (s, 2H), 8.63 (d, *J* = 8.4 Hz, 1H), 8.39 (d, *J* = 8.0 Hz, 1H), 8.19 (d, *J* = 6.0 Hz, 1H), 7.94 (t, *J* = 8.0 Hz, 1H), 7.83 (d, *J* = 7.6 Hz, 1H), 7.53 (d, *J* = 8.8 Hz, 1H), 7.40 (d, *J* = 5.6 Hz, 1H), 3.69 (s, 3H).

### Example 241. methyl 4-amino-1-(6-aminopyridin-3-yl)-7-bromo-2-oxo-1,2-dihydro-1,8-naphthyridine-3 -carboxylate

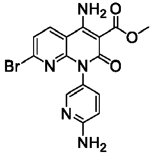

According to the preparation route of scheme 7, 2-chloro-4-(trifluoromethyl)benzonitrile and 3-amino-6-nitropyridine were used as the starting material to obtain the product with reference to the preparation route of Example 165 and 147.

¹H NMR (400 MHz, CD₃OD+CDCl₃) δ 8.20 (d, *J* = 8.4 Hz, 1H), 7.73 (d, *J* = 2.4 Hz, 1H), 7.29 (d, *J* = 8.8 Hz, 1H), 7.25 (dd, *J* = 8.4 Hz, 2.4 Hz, 1H), 6.63 (d, *J* = 9.2 Hz, 1H), 3.82 (s, 3H).

### Example 242. methyl 4-atnino-7-iodo-1-(isoquinolin-7-yl)-2-oxo-1,2-dihydroquinoline-3-carboxylate

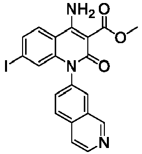

According to the preparation route of scheme 7, 2-fluoro-4-iodobenzonitrile and isoquinolin-7-amine were used as the starting material to obtain the product with reference to the preparation route of Example 165.

¹H NMR (400 MHz, DMSO-*d*₆) δ 9.36 (s, 1H), 8.61 (d, *J* = 6.0 Hz, 1H), 8.43 (s, 2H), 8.17 (d, *J* = 8.4 Hz, 1H), 8.11 (s, 1H), 8.02 (d, *J* = 8.8 Hz, 1H), 7.96 (d, *J* = 6.0 Hz, 1H), 7.63 (dd, *J* = 8.4 Hz, 1.6 Hz, 1H), 7.59 (dd, *J* = 8.4 Hz, 1.6 Hz, 1H), 6.72 (d, *J* = 1.6 Hz, 1H), 3.70 (s, 3H).

### Example 243. methyl 4-amino-1-(1-quinazolin-6-yl)-7-bromo-2-oxo-1,2-dihydroquinoline-3-carboxylate

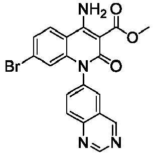

According to the preparation route of scheme 7, 2-fluoro-4-bromobenzonitrile and 5-aminoquinazoline were used as the starting material to obtain the product with reference to the preparation route of Example 165.

¹H NMR (400 MHz, DMSO-*d*₆) δ 9.65 (s, 1H), 9.40 (s, 1H), 8.52 (s, 2H), 8.19-8.26 (m, 3H), 7.91 (d, *J =* 8.4 Hz, 1H), 7.44 (d, *J* = 8.8 Hz, 1H), 6.60 (s, 1H), 3.70 (s, 3H).

### Example 244. methyl 4-atnino-7-bromo-1-(isoquinolin-7-yl)-2-oxo-1,2-dihydro-1,8-naphthyridine-3 -carboxylate

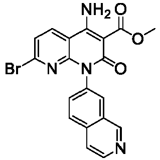

According to the preparation route of scheme 7, 2,6-dibromopyridin-3-carbonitrile and isoquinolin-7-amine were used as the starting material to obtain the product with reference to the preparation route of Example 165.

¹H NMR (400 MHz, DMSO-*d*₆) δ 9.32 (s, 1H), 8.56-8.59 (m, 2H), 8.50 (s, 2H), 8.06 (d, *J* = 8.8 Hz, 1H), 8.02 (s, 1H), 7.91 (d, *J* = 6.0 Hz, 1H), 7.61 (dd, *J=* 8.4 Hz, 2.0 Hz, 1H), 7.52 (d, *J* = 8.4 Hz, 1H), 3.72 (s, 3H).

### Example 245. methyl 4-atnino-7-bromo-2-oxo-1-(quinoxalin-5-yl)-1,2-dihydroquinoline-3-carboxylate

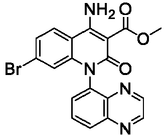

According to the preparation route of scheme 7, 2-fluoro-4-bromobenzonitrile and quinoxalin-5-amine were used as the starting material to obtain the product with reference to the preparation route of Example 165.

¹H NMR (400 MHz, DMSO-*d*₆) δ 9.00 (d, *J* = 1.6 Hz, 1H), 8.83 (d, *J* = 1.6 Hz, 1H), 8.51 (s, 2H), 8.29 (dd, *J* = 8.0 Hz, 1.2 Hz, 1H), 8.21 (d, *J* = *8.4* Hz, 1H), 8.05 (t, *J* = 7.6 Hz, 1H), 7.90 (dd, *J* = 7.2 Hz, 1.2 Hz, 1H), 7.39 (dd, *J =* 8.4 Hz, 2.0 Hz, 1H), 8.37 (d, *J* = 2.0 Hz, 1H), 3.68 (s, 3H).

### Example 246. methyl 4-atnino-1-(3-methyl-1-benzofuran-6-yl)-2-oxo-7-(trifluoromethyl)-1,2-dihydroquinoline-3 -carboxylate

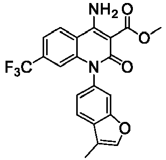

According to the preparation route of scheme 7, 2-amino-4-(trifluoromethyl)benzonitrile and 6-bromo-3-methyl-1-benzofuran were used as the starting material to obtain the product with reference to the preparation route of Example 165.

¹H NMR (400 MHz, DMSO-*d*₆) δ 8.46 (d, *J* = 8.4 Hz, 1H), 8.39 (s, 2H), 7.89 (d, *J* = 1.2 Hz, 1H), 7.78 (d, *J =* 8.0 Hz, 1H), 7.54-7.57 (m, 2H), 7.14 (dd, *J =* 8.4 Hz, 2.0 Hz, 1H), 6.59 (s, 1H), 3.72 (s, 3H), 2.27 (s, 3H).

### Example 247. methyl 4-amino-1-(4-amino-2-chlorophenyl)-7-bromo-2-oxo-1,2-dihydro quinoline-3 -carboxylate

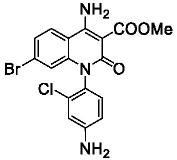

According to the preparation route of scheme 7, 2-amino-4-bromo-benzonitrile and 2-chloro-1-fluoro-4-nitrobenzene were used as the starting material to obtain the product with reference to the preparation route of Example 165 and Example 147.

¹H NMR (400 MHz, DMSO-*d*₆) δ 8.39 (s, 2H), 8.15 *(d, J=* 9.2 Hz, 1H), 7.39 (dd, *J* = 8.4 Hz, 1.2 Hz, 1H), 6.96 (d, *J* = 8.4 Hz, 1H), 6.78 (d, *J* = 2.4 Hz, 1H), 6.63 (dd, *J* = 8.8 Hz, 2.0 Hz, 1H), 6.51 (s, 1H), 5.68 (s, 2H), 3.70 (s, 3H).

### Example 248. methyl 4-amino-7-bromo-1-(6-chloro-2-methylpyridin-3-yl)-2-oxo-1,2-dihydro-1,8-naphthyridine-3-carboxylate

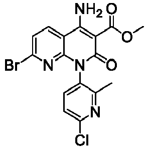

According to the preparation route of scheme 7, 2,6-dibromo-3-cyanopyridine and 3-amino-6-chloro-2-methylpyridine were used as the starting material to obtain the product with reference to the preparation route of Example 165.

¹H NMR (400 MHz, CDCl₃₊CD₃OD) δ 7.81 (d, *J* = 8.0 Hz, 1H), 7.41 (d, *J* = 8.4 Hz, 1H), 7.35 (d, *J =* 8.4 Hz, 1H), 7.30 (d, *J =* 8.4 Hz, 1H), 3.91 (s, 3H), 2.22 (s, 3H).

### Example 249. methyl 4-amino-7-bromo-1-(6-chloro-4-methylpyridin-3-yl)-2-oxo-1,2-dihydro-1,8-naphthyridine-3-carboxylate

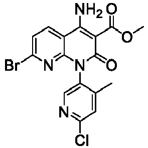

According to the preparation route of scheme 7, 2,6-dibromo-3-cyanopyridine and 3-amino-6-chloro-4-methylpyridine were used as the starting material to obtain the product with reference to the preparation route of Example 165.

¹H NMR (400 MHz, CD₃OD+CDCl₃) δ 8.10 (s, 1H), 7.81 (d, *J* = 8.0 Hz, 1H), 7.34-7.36 (m, 2H), 3.91 (s, 3H), 2.05 (s, 3H).

### Example 250. methyl 4-amino-1-(2-aminophenyl)-7-bromo-2-oxo-1,2-dihydroquinoline-3-carboxylate

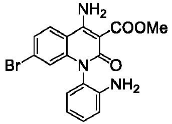

According to the preparation route of scheme 7, 2-amino-4-bromo-benzonitrile and 1-fluoro-2- toluene were used as the starting material to obtain the product with reference to the preparation route of Example 165 and Example 147.

¹H NMR (400 MHz, CD₃OD) δ 8.00 (d, *J* = 8.8 Hz, 1H), 7.37 (d, *J =* 8.8 Hz, 1H), 7.26 (t, *J* = 8.0 Hz, 1H), 6.96 (d, *J =* 8.0 Hz, 1H), 6.92 (d, *J =* 8.0 Hz, 1H), 6.28 (t, *J =* 8.0 Hz, 1H), 6.76 (s, 1H), 3.81 (s, 3H).

### Example 251. methyl 4-amino-1-(2-atninopyridin-4-yl)-2-oxo-7-(trifluoromethyl)-1,2-dihydroquinoline-3 -carboxylate

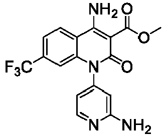

According to the preparation route of scheme 7, 2-amino-4-trifluoromethylbenzonitrile and 2-amino-4-bromopyridine were used as the starting material to obtain the product with reference to the preparation route of Example 165.

¹H NMR (400 MHz, DMSO-*d₆*) δ 8.38-8.46 (m, 3H), 8.09 (d, *J =* 5.2 Hz, 1H), 7.57 (d, *J =* 8.0 Hz, 1H), 6.74 (s, 1H), 6.39 (dd, *J =* 5.6 Hz, 1.6 Hz, 1H), 6.30 (s, 1H), 6.24 (s, 2H), 3.72 (s, 3H).

### Example 252. methyl 4-amino-1-(4-aminophenyl)-7-iodo-2-oxo-1,2-dihydro quinoline-3-carboxylate

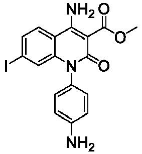

According to the preparation route of scheme 7, 2-fluoro-4-iodobenzonitrile and 4-nitroaniline were used as the starting material to obtain the product with reference to the preparation route of Example 165 and 147.

¹H NMR (400 MHz, DMSO-*d₆*) δ 8.22 (s, 2H), 7.92 (d, *J =* 8.0 Hz, 1H), 7.50 (d, *J =* 8.0 Hz, 1H), 6.83 (s, 1H), 6.78 (d, *J* = 8.4 Hz, 2H), 6.67 (d, *J* = 8.4 Hz, 2H), 5.34 (s, 2H), 3.69 (s, 3H).

### Example 253. methyl 4-amino-1-(3-amino-4-methylphenyl)-2-oxo-7-(trifluoro methyl)-1,2-dihydroquinoline-3 -carboxylate

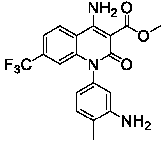

According to the preparation route of scheme 7, 2-amino-4-(trifluoromethyl)benzonitrile and 5-bromo-2-methyl aniline were used as the starting material to obtain the product with reference to the preparation route of Example 165 and Example 147.

¹H NMR (400 MHz, CD₃OD-*d₆*) δ 8.28 (d, *J* = 8.8 Hz, 1H), 7.48 (d, *J* = 8.8 Hz, 1H), 7.22 (d, *J* = 7.6 Hz, 1H), 6.92 (s, 1H), 6.57 (d, *J* = 2.4 Hz, 1H), 6.45 (dd, *J* = 8.0 Hz, 2.0 Hz, 1H), 3.83 (s, 3H), 2.25 (s, 3H).

### Example 254. methyl 4-amino-1-(5-aminopyridin-3-yl)-2-oxo-7-(trifluoromethyl)-1,2-dihydroquinoline-3 -carboxylate

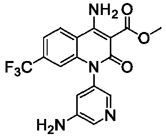

According to the preparation route of scheme 7, 3,5-diaminopyridine and 2-fluoro-4-trifluoromethylbenzonitrile were used as the starting material to obtain the product with reference to the preparation route of Example 165.

¹H NMR (400 MHz, CD₃OD) δ 8.34 (d, *J* = 8.4 Hz, 1H), 8.13 (s, 1H), 7.68 (s, 1H), 7.54 (d, *J* = 8.8 Hz, 1H), 7.02 (s, 1H), 6.84 (s, 1H), 3.84 (s, 3H).

### Example 255. methyl 4-atnino-1-(isoquinolin-4-yl)-2-oxo-7-(trifluoromethyl)-1,2-dihydro quinoline-3 -carboxylate

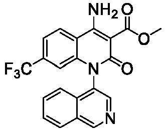

According to the preparation route of scheme 6, 4-bromoisoquinoline and 2-amino-4-(trifluoromethyl)benzonitrile were used as the starting material to obtain the product with reference to the preparation route of Example 81.

¹H NMR (400 MHz, CD₃OD) δ 9.42 (s, 1H), 8.49 (s, 1H), 8.14 (d, *J* = 8.8 Hz, 1H), 7.86 *(d, J* = 8.8 Hz, 1H), 7.65-7.68(m, 2H), 7.26-7.43 (m, 2H), 6.66 (s, 1H), 3.90 (s, 3H).

### Example 256. methyl 4-amino-1-(6-aminopyridin-2-yl)-2-oxo-7-(trifluoromethyl)-1,2-dihydroquinoline-3 -carboxylate

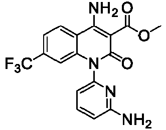

According to the preparation route of scheme 7, 2,6-diaminopyridine and 2-fluoro-4-trifluoromethylbenzonitrile were used as the starting material to obtain the product with reference to the preparation route of Example 165.

¹H NMR (400 MHz, CD₃OD) δ 7.80 (d, *J* = 8.8 Hz, 1H), 7.65 (t, *J* = 8.0 Hz, 1H), 7.38 (d, *J* = 8.4 Hz, 1H), 6.85 (s, 1H), 6.69 (d, *J* = 7.2 Hz, 1H), 6.61 (d, *J* = 8.0 Hz, 1H), 3.89 (s, 3H).

### Example 257. ethyl 4-amino-1-(benzo[d]oxazol-6-yl)-7-bromo-2-oxo-1,2-dihydroquinoline-3-carboxylate

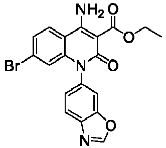

According to the preparation route of scheme 7, 2-fluoro-4-bromobenzonitrile and 6-amino-1,3-benzo[d]oxazole were used as the starting material to obtain the product with reference to the preparation route of Example 165.

¹H NMR (400 MHz, CD₃OD+CDCl₃) δ 8.27 (s, 1H), 7.91 (d, *J* = 8.4 Hz, 1H), 7.79 (d, *J* = 8.8 Hz, 1H), 7.50 (d, *J* = 1.6 Hz, 1H), 7.28 (dd, *J* = 8.8 Hz, 2.0 Hz, 1H), 7.20 (dd, *J* = 8.4 Hz, 1.6 Hz, 1H), 6.61 (d, *J* = 2.0 Hz, 1H), 4.29 (q, *J* = 7.2 Hz, 2H), 1.30 (t, *J* = 7.2 Hz, 3H).

### Example 258. methyl 4-amino-7-bromo-1-(6-methylpyridin-3-yl)-2-oxo-1,2-dihydro-1,8-naphthyridine-3 -carboxylate

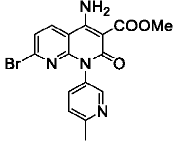

According to the preparation route of scheme 7, 2,6-dibromonicotinonitrile and 6-methylpyridin-3-amine were used as the starting material to obtain the product with reference to the preparation route of Example 165.

¹H NMR (400 MHz, DMSO-*d*₆) δ 8.58 (d, *J* = 8.4 Hz, 1H), 8.27 (d, *J* = 2.4 Hz, 1H), 7.59 (dd, *J =* 8.0 Hz, 2.4 Hz, 1H), 7.55 (d, *J =* 8.2 Hz, 1H), 7.40 (d, *J =* 8.2 Hz, 1H), 3.73 (s, 3H), 2.55 (s, 3H).

### Example 259. methyl 4-amino-1-(4-amino-2-fluorophenyl)-7-bromo-2-oxo-1,2-dihydro quinoline-3 -carboxylate

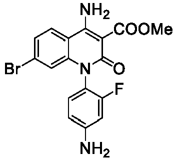

According to the preparation route of scheme 7, 2-amino-4-bromo-benzonitrile and 1,2-difluoro-4-nitrobenzene were used as the starting material to obtain the product with reference to the preparation route of Example 165 and Example 147.

¹H NMR (400 MHz, DMSO-*d*₆) δ 8.37 (s, 2H), 8.15 (d, *J* = 8.8 Hz, 1H), 7.41 (dd, *J* = 8.8 Hz, 1.6 Hz, 1H), 6.91 (t, *J =* 8.4 Hz, 1H), 6.65 (d, *J =* 2.0 Hz, 1H), 6.48-6.51 (m, 2H), 5.70 (s, 2H), 3.70 (s, 3H).

### Example 260. methyl 4-amino-1-(3-cyanophenyl)-7-bromo-2-oxo-1,2-dihydroquinoline-3-carboxylate

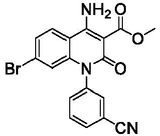

According to the preparation route of scheme 7, 2-fluoro-4-bromobenzonitrile and 3-cyanoaniline were used as the starting material to obtain the product with reference to the preparation route of Example 165.

¹H NMR (400 MHz, DMSO-*d*₆) δ 8.48 (s, 2H), 8.19 (d, *J =* 8.8 Hz, 1H), 7.99 (d, *J =* 7.6 Hz, 1H), 7.89 (s, 1H), 7.78 (t, *J =* 7.6 Hz, 1H), 7.67 (d, *J =* 8.4 Hz, 1H), 7.44 (d, *J =* 7.6 Hz, 1H), 7.48 (d, *J* = 1.6 Hz, 1H), 3.70 (s, 3H).

### Example 261. methyl 4-amino-1-(4-amino-2-methylphenyl)-2-oxo-7-(trifluoromethyl)-1,2-dihydroquinoline-3 -carboxylate

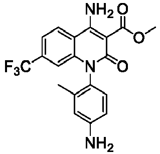

According to the preparation route of scheme 7, 2-fluoro-4-(trifluoromethyl)benzonitrile and 2-methyl-4-nitroaniline were used as the starting material to obtain the product with reference to the preparation route of Example 165 and 147.

¹H NMR (400 MHz, DMSO-*d*₆) δ 8.28 (d, *J* = 8.8 Hz, 1H), 7.48 (d, *J* = 8.8 Hz, 1H), 7.22 (d, J = 7.6 Hz, 1H), 6.92 (s, 1H), 6.57 (d, *J* = 2.4 Hz, 1H), 6.45 (dd, J = 8.0 Hz, 2.0 Hz, 1H), 3.83 (s, 3H), 2.25 (s, 3H).

### Example 262. methyl 4-amino-1-(4-acetylphenyl)-7-bromo-2-oxo-1,2-dihydro-1,8-naphthyridine-3 -carboxylate

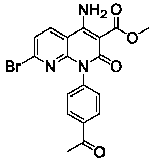

According to the preparation route of scheme 7, 2,6-dibromopyridin-3-carbonitrile and 1-(4-aminophenyl)ethan-1-one were used as the starting material to obtain the product with reference to the preparation route of Example 165.

¹H NMR (400 MHz, DMSO-*d*₆) δ 8.56 (d, *J =* 8.4 Hz, 1H), 8.48 (s, 2H), 8.05 (d, *J =* 8.4 Hz, 2H), 7.52 (d, *J =* 8.4 Hz, 1H), 7.37 (d, *J* = 8.4 Hz, 2H), 3.71 (s, 3H), 2.63 (s, 3H).

### Example 263. methyl 4-amino-1-(4-aminophenyl)-7-bromo-2-oxo-1,2-dihydro-1,8-naphthyridine-3 -carboxylate

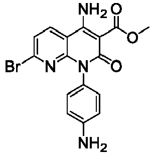

According to the preparation route of scheme 7, 2,6-dibromo-3-cyanopyridine and 4-nitroaniline were used as the starting material to obtain the product with reference to the preparation route of Example 165 and 147.

¹H NMR (400 MHz, d-DMSO) δ 8.50 (d, *J* = 8.8 Hz, 1H), 8.30 (s, 2H), 7.44 (d, *J* = 8.8 Hz, 1H), 6.74 *(d, J* = 8.8 Hz, 2H), 6.58 *(d, J* = 8.8 Hz, 2H), 5.17 (s, 2H), 3.70 (s, 3H).

### Example 264. methyl 4-amino-1-(4-aminophenyl)-7-hydroxy-2-oxo-1,2-dihydro-1,8-naphthyridine-3 -carboxylate

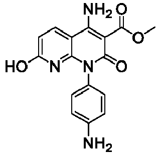

According to the preparation route of scheme 7, 2,6-dibromo-3-cyanopyridine and 4-nitroaniline were used as the starting material to obtain the product with reference to the preparation route of Example 165 and 147.

¹H NMR (400 MHz, d-DMSO) δ 9.63 (s, 1H), 9.27 (s, 1H), 8.58 (s, 1H), 8.22 (d, *J* = 8.8 Hz, 1H), 7.20-7.26 (m, 2H), 6.60 (d, *J* = 8.8 Hz, 1H), 6.55 (d, *J* = 8.8 Hz, 2H), 4.95 (s, 2H), 3.69 (s, 3H).

### Example 265. methyl 4-amino-7-bromo-1-(4-chlorophenyl)-2-oxo-1,2-dihydro-1,8-naphthyridine-3 -carboxylate

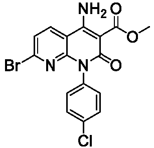

According to the preparation route of scheme 7, 2,6-dibromonicotinonitrile and 4-chloroaniline were used as the starting material to obtain the product with reference to the preparation route of Example 165.

¹H NMR (400 MHz, DMSO-*d*₆) δ 8.54 (d, *J* = 8.8 Hz, 1H), 8.46 (s, 2H), 7.49-7.54 (m, 3H), 7.24 (d, *J* = 8.4 Hz, 2H), 3.71 (s, 3H).

### Example 266. methyl 4-amino-1-(4-(1-hydroxyethyl)phenyl)-7-bromo-2-oxo-1,2-dihydro-1,8-naphthyridine-3 -carboxylate

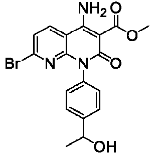

Example 262 was used as the starting material to obtain the product with reference to the preparation route of Example 122.

¹H NMR (400 MHz, DMSO-*d*₆) δ 8.56 (d, *J* = 8.4 Hz, 1H), 8.42 (s, 2H), 7.48 (d, *J* = 8.4 Hz, 1H), 7.37-7.45 (m, 2H), 7.10 (d, *J* = 8.8 Hz, 2H), 5.27 (d, *J* = 4.4 Hz, 1H), 4.75-4.81 (m, 1H), 3.70 (s, 3H), 1.37 (d, *J* = 6.0 Hz, 3H).

### Example 267. methyl 4-amino-1-(2H indazol-7-yl)-2-oxo-7-(trifluoromethyl)-1,2-dihydroquinoline-3-carboxylate

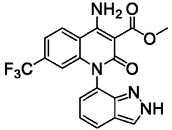

According to the preparation route of scheme 6, 2-amino-4-(trifluoromethyl)benzonitrile and 7-bromo-2H-indazole were used as the starting material to obtain the product with reference to the preparation route of Example 81.

¹H NMR (400 MHz, CD₃OD+CDCl₃) δ 8.03-8.07 (m, 2H), 7.82 (d, *J* = 8.0 Hz, 1H), 7.35 (d, *J* = 8.8 Hz, 1H), 7.18-7.24 (m, 2H), 6.65 (s, 1H), 3.77 (s, 3H).

### Example 268. methyl 4-amino-1-(benzo[d]oxazol-6-yl)-7-bromo-2-oxo-1,2-dihydroquinoline-3 -carboxylate

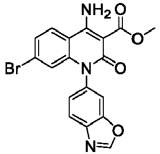

According to the preparation route of scheme 7, 2-fluoro-4-bromobenzonitrile and 6-amino-1,3-benzo[d]oxazole were used as the starting material to obtain the product with reference to the preparation route of Example 165.

¹H NMR (400 MHz, CD₃OD+CDCl₃) δ 8.21 (s, 1H), 7.86 (d, *J* = 8.8 Hz, 1H), 7.74 (d, *J* = 8.4 Hz, 1H), 7.44 (s, 1H), 7.22-7.26 (m, 1H), 7.15 (d, *J* = 8.0 Hz, 1H), 6.58 (s, 1H), 3.76 (s, 3H).

### Example 269. methyl 4-amino-1-(4-(1-hydroxyethyl)-2-methylphenyl)-2-oxo-7-(trifluoromethyl)-1,2-dihydroquinoline-3-carboxylate

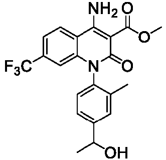

According to the preparation route of scheme 6, 1-(4-bromo-3-methylphenyl)ethan-1-one and 2-amino-4-(trifluoromethyl)benzonitrile were used as the starting material to obtain the product with reference to the preparation route of Example 81 and Example 122.

¹H NMR (400 MHz, CDCl₃) δ 8.00 (d, *J* = 8.4 Hz, 1H), 7.60-8.20 (brs, 2H), 7.48 (s, 1H), 7.37-7.43 (m, 2H), 7.10 (dd, *J* = 8.4 Hz, 5.6 Hz, 1H), 6.74 (s, 1H), 4.98 (q, *J* = 6.8 Hz, 1H), 3.89 (s, 3H), 2.04-2.10 (brs, 1H), 2.01 (s, 3H), 1.55 (d, *J* = 6.4 Hz, 3H).

### Example 270. methyl 4-amino-7-bromo-1-(isoquinolin-4-yl)-2-oxo-1,2-dihydro quinoline-3 -carboxylate

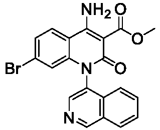

According to the preparation route of scheme 7, 2-fluoro-4-bromobenzonitrile and isoquinolin-4-amine were used as the starting material to obtain the product with reference to the preparation route of Example 165.

¹H NMR (400 MHz, DMSO-*d*₆) δ 9.10 (d, *J =* 4.8 Hz, 1H), 8.63 (s, 2H), 8.24 (d, *J =* 8.8 Hz, 1H), 8.18 (d, *J =* 8.4 Hz, 1H), 7.80-7.84 (m, 1H), 7.59 (d, *J =* 4.0 Hz, 1H), 7.53-7.57 (m, 1H), 7.41-7.45 (m, 2H), 6.32 *(d, J=* 1.6 Hz, 1H), 3.67 (s, 3H).

### Example 271. methyl 4-amino-1-(4-(1-(S)-hydroxyethyl)phenyl)-2-oxo-7-(trifluoromethyl)-1,2-dihydroquinoline-3 -carboxylate

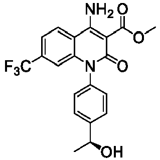

According to the preparation route of scheme 6, (*S*)-1-(4-bromophenyl)ethan-1-ol and 2-amino-4-(trifluoromethyl)benzonitrile were used as the starting material to obtain the product with reference to the preparation route of Example 81.

¹H NMR (400 MHz, CD₃OD) δ 8.02 (d, *J =* 8.4 Hz, 1H), 7.46-7.50 (m, 2H), 7.32 (d, *J =* 8.8 Hz, 1H), 7.09 (d, *J =* 8.8 Hz, 2H), 6.73 (s, 1H), 4.84 (q, *J =* 6.4 Hz, 1H), 3.77 (s, 3H), 1.43 (d,*J* = 6.0 Hz, 3H).

### Example 272. methyl 4-amino-1-(4-(1-(R)-hydroxyethyl)phenyl)-2-oxo-7-(trifluoromethyl)-1,2-dihydroquinoline-3 -carboxylate

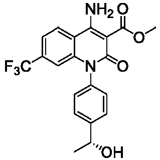

According to the preparation route of scheme 6, *(R)*-1-(4-bromophenyl)ethan-1-ol and 2-amino-4-(trifluoromethyl)benzonitrile were used as the starting material to obtain the product with reference to the preparation route of Example 81.

¹H NMR (400 MHz, CD₃OD) δ 8.10 (d, *J* = 8.8 Hz, 1H), 7.53-7.55 (m, 2H), 7.38 (d, *J* = 8.4 Hz, 1H), 7.14 (d, *J* = 8.4 Hz, 2H), 6.77 (s, 1H), 4.85-4.95 (m, 1H), 3.83 (s, 3H), 1.49 (d, *J* = 6.4 Hz, 3H).

### Example 273. methyl 4-amino-1-(4-atninophenyl)-2-oxo-7-(trifluoromethyl)-1,2-dihydroquinoline-3 -carboxylate

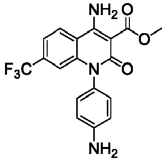

According to the preparation route of scheme 7, 2-fluoro-4-(trifluoromethyl)phenyl-1-carbonitrile and 4-nitroaniline were used as the starting material to obtain the product with reference to the preparation route of Example 165 and Example 147.

¹H NMR (400 MHz, DMSO-*d*₆) δ 8.40 (d, *J =* 8.4 Hz, 1H), 8.26 (s, 2H), 7.50 (dd, *J* = 8.4 Hz, 1.6 Hz, 1H), 6.83 (d, *J =* 8.8 Hz, 2H), 6.74 (s, 1H), 6.68 (d, *J =* 8.8 Hz, 2H), 5.37 (s, 2H), 3.71 (s, 3H).

### Example 274. methyl 4-amino-1-(4-aminophenyl)-2-oxo-7-(trifluoromethyl)-1,2-dihydro-1,8-naphthyridine-3 -carboxylate

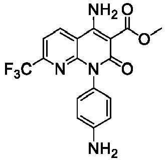

According to the preparation route of scheme 7, 2-chloro-6-(trifluoromethyl)pyridin-3-benzonitrile and 4-nitroaniline were used as the starting material to obtain the product with reference to the preparation route of Example 165 and 147.

¹H NMR (400 MHz, DMSO-*d*₆) δ 8.84 (d, *J* = 8.4 Hz, 1H), 8.31 (br, 2H), 7.68 (d, *J* = 8.4 Hz, 1H), 6.74 *(d, J* = 8.8Hz, 2H), 6.58 *(d, J* = 8.4 Hz, 2H), 5.14 (br, 2H), 3.78 (s, 3H).

### Example 275. methyl 4-amino-7-bromo-1-(4-iodophenyl)-2-oxo-1,2-dihydro-1,8-naphthyridine-3 -carboxylate

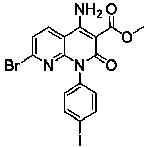

According to the preparation route of scheme 7, 2,6-dibromo-3-cyanopyridine and 4-iodoaniline were used as the starting material to obtain the product with reference to the preparation route of Example 165.

¹H NMR (400 MHz, CD₃OD) δ 8.11 (d, *J* = 8.0 Hz, 1H), 7.75 (d, *J* = 8.4 Hz, 2.0 Hz, 1H), 7.26 (d, *J* = 8.0 Hz, 1H), 6.89 (d, *J* = 8.4 Hz, 2H), 3.78 (s, 3H).

### Example 276. methyl 4-amino-1-(4-aminophenyl)-7-ethoxy-2-oxo-1,2-dihydroquinoline-3-carboxylate

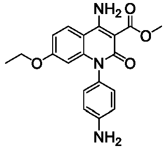

According to the preparation route of scheme 7, 4-ethoxy-2-fluorobenzonitrile and 4-nitroaniline were used as the starting material to obtain the product with reference to the preparation route of Example 165 and 147.

¹H NMR (400 MHz, DMSO-*d*₆) δ 8.26 (br, 2H), 8.08 (d, *J* = 8.8 Hz, 1H), 6.74-6.80 (m, 3H), 6.65 (d, *J* = 8.4 Hz, 2H), 5.89 (d, *J* = 2.8 Hz, 1H), 5.29 (br, 2H), 3.85-3.90 (m, 2H), 3.66 (s, 3H), 1.22 (t, *J* = 7.2 Hz, 3H).

### Example 277. methyl 4-amino-1-(4-(cyclopropyl(hydroxy)methyl)phenyl)-2-oxo-7-(trifluoromethyl)-1,2-dihydroquinoline-3 -carboxylate

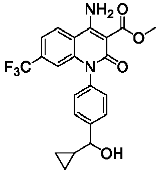

According to the preparation route of scheme 6, (4-bromophenyl)(cyclopropyl)methanol and 2-amino-4-(trifluoromethyl)benzonitrile were used as the starting material to obtain the product with reference to the preparation route of Example 81.

¹H NMR (400 MHz, CDCl₃) δ 7.81 (d, *J* = 8.4 Hz, 1H), 7.69 (d, *J* = 8.0 Hz, 1H), 7.59 (d, *J* = 8.0 Hz, 1H), 7.45-7.85 (brs, 2H), 7.40 *(d, J=* 8.4 Hz, 1H), 7.19-7.25 (m, 2H), 6.89 (s, 1H), 4.13 (d, *J* = 8.4 Hz, 1H), 3.90 (s, 3H), 1.95-2.10 (brs, 1H), 1.25-1.29 (m, 1H), 0.61-0.69 (m, 2H), 0.44-0.53 (m, 2H).

### Example 278. methyl 4-atnino-1-(4-aminophenyl)-2-oxo-7-methyl-1,2-dihydroquinoline-3-carboxylate

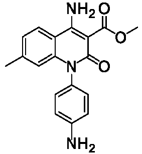

Example 147 and methylboronic acid were used as the starting material to obtain the product with reference to the preparation route of Example 24.

¹H NMR (400 MHz, CD₃OD) δ 7.95 (d, *J =* 8.4 Hz, 1H), 7.08 (d, *J* = 8.3 Hz, 1H), 6.96-6.69 (m, 4H), 6.50 (s, 1H), 3.81 (s, 3H), 2.26 (s, 3H).

### Example 279. methyl 4-atnino-7-vinyl-1-(4-aminophenyl)-2-oxo-1,2-dihydroquinoline-3-carboxylate

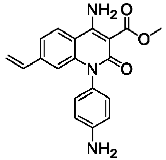

Example 147 and potassium vinyltrifluoroborate were used as the starting material to obtain the product with reference to the preparation route of Example 24.

¹H NMR (400 MHz, DMSO-d*₆*) δ 8.27 (s, 2H), 8.16 (d, *J* = 8.4 Hz, 1H), 7.37 (d, *J* = 8.4 Hz, 1H), 6.80-6.94 (m, 4H), 6.59 (dd, *J =* 17.6 Hz, 10.4 Hz, 1H), 6.44 (s, 1H), 5.75 (d, *J =* 17.6 Hz, 1H), 5.30 *(d, J* = 10.4 Hz, 1H), 3.69 (s, 3H).

### Example 280. methyl 4-amino-1-(6-amino-4-methylpyridin-3-yl)-7-bromo-2-oxo-1,2-dihydro-1,8-naphthyridine-3-carboxylate

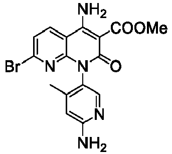

According to the preparation route of scheme 7, 2,6-dibromonicotinonitrile and *tert*-butyl (5-amino-4-methylpyridin-2-yl)carbamate were used as the starting material to obtain the product with reference to the preparation route of Example 165.

¹H NMR (400 MHz, DMSO-*d*₆) δ 8.54 (d, *J* = 8.0 Hz, 1H), 8.43 (s, 2H), 7.52 (s, 1H), 7.50 (d, *J* = 8.0 Hz, 1H), 6.37 (s, 1H), 5.93 (s, 2H), 3.71 (s, 3H), 1.72 (s, 3H).

### Example 281. methyl 4-amino-7-bromo-1-(imidazo[3,2-a]pyridin-6-yl)-2-oxo-1,2-dihydroquinoline-3 -carboxylate

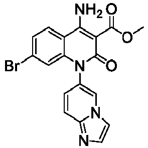

According to the preparation route of scheme 7, 2-fluoro-4-bromobenzonitrile and imidazo[3,2-a]pyridin-6-amine were used as the starting material to obtain the product with reference to the preparation route of Example 165.

¹H NMR (400 MHz, DMSO-*d*₆) δ 8.68 (s, 1H), 8.46 (s, 2H), 8.19 *(d, J* = 8.8 Hz, 1H), 7.98 (s, 1H), 7.71 (d, *J* = 9.2 Hz, 1H), 7.67 (s, 1H), 7.44 (dd, *J* = 9.2 Hz, 2.4 Hz, 1H), 7.10 (dd, *J =* 9.2 Hz, 2.0 Hz, 1H), 6.83 (d, *J* = 2.0 Hz, 1H), 3.70 (s, 3H).

### Example 282. methyl 4-amino-1-(4-amino-2-methylphenyl)-2-oxo-7-(trifluoromethyl)-1,2-dihydro-1,8-naphthyridine-3-carboxylate

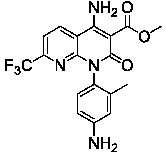

According to the preparation route of scheme 7, 2-chloro-6-(trifluoromethyl)pyridin-3-carbonitrile and 2-methyl-4-nitroaniline were used as the starting material to obtain the product with reference to the preparation route of Example 165 and Example 147.

¹H NMR (400 MHz, DMSO-*d*₆) δ 8.75 *(d, J=* 8.0 Hz, 1H), 7.62 *(d, J=* 8.0 Hz, 1H), 6.79 (d, *J* = 8.0 Hz, 1H), 6.72 (d, *J* = 2.8 Hz, 1H), 6.67 (dd, *J* = 8.0 Hz, 2.8 Hz, 1H), 3.84 (s, 3H), 1.83 (s, 3H).

### Example 283. methyl 4-atnino-1-(isoquinolin-7-yl)-2-oxo-7-(trifluoromethyl)-1,2-dihydro-1,8-naphthyridine-3 -carboxylate

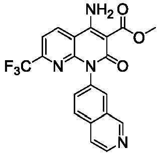

According to the preparation route of scheme 7, 2-chloro-6-(trifluoromethyl)pyridin-3-carbonitrile and 7-amino-isoquinoline were used as the starting material to obtain the product with reference to the preparation route of Example 165.

¹H NMR (400 MHz, DMSO-*d*₆) δ 9.31 (s, 1H), 8.96 (d, *J* = 8.4 Hz, 1H), 8.56 (d, *J* = 6.0 Hz, 1H), 8.54 (s, 2H), 8.06 (d, *J* = 8.4 Hz, 1H), 8.03 (s, 1H), 7.91 (d, *J* = 6.0 Hz, 1H), 7.78 (d, *J* = 8.0 Hz, 1H), 7.63 (dd, *J* = 8.4 Hz, 1.2 Hz, 1H), 3.74 (s, 3H).

### Example 284. methyl 4-amino-1-(3-aminophenyl)-7-bromo-2-oxo-1,2-dihydroquinoline-3-carboxylate

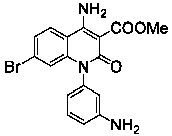

According to the preparation route of scheme 7, 4-bromo-2-fluorobenzonitrile and 3-nitroaniline were used as the starting material to obtain the product with reference to the preparation route of Example 165 and Example 147.

¹H NMR (400 MHz, DMSO-*d*₆) δ 8.29 (s, 2H), 8.13 (d, *J* = 8.8 Hz, 1H), 7.37 (dd, *J* = 8.8 Hz, 2.0Hz, 1H), 7.18 (t, *J* = 8.0 Hz, 1H), 6.66 (d, *J* = 8.0 Hz, 1H), 6.60 (d, *J* = 2.0 Hz, 1H), 6.32 (t, *J* = 2.0 Hz, 1H), 6.29 (d, *J* = 8.0 Hz, 1H), 5.34 (s, 2H), 3.70 (s, 3H).

### Example 285. methyl 4-amino-7-bromo-2-oxo-1-(o-tolyl)-1,2-dihydro-1,8-naphthyridine-3-carboxylate

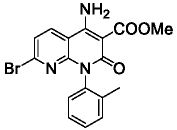

According to the preparation route of scheme 7, 2,6-dibromonicotinonitrile and o-toluidine were used as the starting material to obtain the product with reference to the preparation route of Example 165.

¹H NMR (400 MHz, DMSO-*d*₆) δ 8.56 (d, *J =* 8.4 Hz, 1H), 8.46 (s, 2H), 7.50 (d, *J =* 8.4 Hz, 1H), 7.25-7.35 (m, 3H), 7.08 (d, *J =* 6.8 Hz, 1H), 3.71 (s, 3H), 1.87 (s, 3H).

### Example 286. methyl 4-amino-1-(6-amino-4-methylpyridin-3-yl)-2-oxo-7-(trifluoromethyl)-1,2-dihydro-1,8-naphthyridine-3 -carboxylate

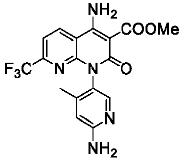

According to the preparation route of scheme 7, 2-chloro-6-(trifluoromethyl)nicotinonitrile and *tert*-butyl (5-amino-4-methylpyridin-2-yl)carbamate were used as the starting material to obtain the product with reference to the preparation route of Example 165.

¹H NMR (400 MHz, DMSO-*d*₆) δ 8.91 (d, *J =* 8.0 Hz, 1H), 8.46 (s, 2H), 7.75 (d, *J =* 8.0 Hz, 1H), 7.53 (s, 1H), 6.37 (s, 1H), 5.92 (s, 2H), 3.74 (s, 3H), 1.70 (s, 3H).

### Example 287. methyl 4-amino-7-bromo-1-(4-chloro-2-methylphenyl)-2-oxo-1,2-dihydro-1,8-naphthyridine-3 -carboxylate

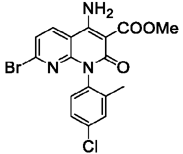

According to the preparation route of scheme 7, 2,6-dibromonicotinonitrile and 4-chloro-2-methylaniline were used as the starting material to obtain the product with reference to the preparation route of Example 165.

¹H NMR (400 MHz, DMSO-*d*₆) δ 8.57 (d, *J* = 8.8 Hz, 1H), 8.50 (s, 2H), 7.52 (d, *J* = 8.4 Hz, 1H), 7.45 (d, *J* = 2.4 Hz, 1H), 7.34 (dd, *J* = 8.8 Hz, 2.4 Hz, 1H), 7.14 (d, *J* = 8.4 Hz, 1H), 3.71 (s, 3H), 1.87 (s, 3H).

### Example 288. methyl 4-amino-7-iodo-1-(6-methylpyridin-3-yl)-2-oxo-1,2-dihydroquinoline-3-carboxylate

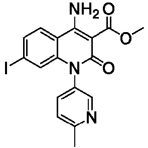

According to the preparation route of scheme 7, 2-fluoro-4-iodobenzonitrile and 6-methylpyridin-3-amine were used as the starting material to obtain the product with reference to the preparation route of Example 165.

¹H NMR (400 MHz, DMSO-*d*₆) δ 8.43 (s, 2H), 8.30 (d, *J =* 2.4 Hz, 1H), 7.99 (d, *J =* 8.8 Hz, 1H), 6.64 (dd, *J=* 8.8 Hz, 2.4 Hz, 1H), 7.58 (d, *J =* 8.0 Hz, 1H), 7.46 (d, *J =* 8.0 Hz, 1H), 6.69 (s, 1H), 3.69 (s, 3H), 2.57 (s, 3H).

### Example 289. methyl 4-amino-1-(8-chloroisoquinolin-5-yl)-7-bromo-2-oxo-1,2-dihydro-1,8-naphthyridine-3 -carboxylate

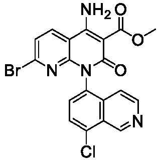

According to the preparation route of scheme 7, 2,6-dibromo-3-cyanopyridine and 8-chloroisoquinolin-5-amine were used as the starting material to obtain the product with reference to the preparation route of Example 165.

¹H NMR (400 MHz, DMSO-*d*₆) δ 9.62 (s, 1H), 8.65-8.71 (brs, 2H), 8.53 (d, *J =* 8.4 Hz, 1H), 8.53 (d, *J =* 6.4 Hz, 1H), 7.97 (d, *J =* 7.6 Hz, 1H), 7.73 (d, *J =* 7.6 Hz, 1H), 7.53 (d, *J =* 8.4 Hz, 1H), 7.40 (d, *J =* 6.0 Hz, 1H), 3.69 (s, 3H).

### Example 290. methyl 4-amino-1-(1-methylisoquinolin-5-yl)-7-bromo-2-oxo-1,2-dihydro-1,8-naphthyridine-3-carboxylate

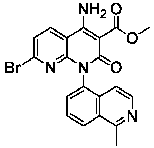

According to the preparation route of scheme 7, 2,6-dibromo-3-cyanopyridine and 1-methylisoquinolin-5-amine were used as the starting material to obtain the product with reference to the preparation route of Example 165.

¹H NMR (400 MHz, DMSO-*d*₆) δ 8.56-8.66 (m, 3H), 8.30 (d, *J =* 8.4 Hz, 1H), 8.21 (d, *J =* 6.4 Hz, 1H), 7.77 (t, *J* = 7.6 Hz, 1H), 7.66 (d, *J* = 6.8 Hz, 1H), 7.51 (d, *J* = 8.0 Hz, 1H), 7.09 (d, *J* = 6.0 Hz, 1H), 3.69 (s, 3H), 2.95 (s, 3H).

### Example 291. methyl 4-amino-1-(6-(1-hydroxyethyl)pyridin-3-yl)-2-oxo-7-bromo-1,2-dihydroquinoline-3 -carboxylate

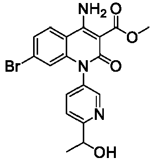

According to the preparation route of scheme 7, 1-(5-aminopyridin-2-yl)ethan-1-one and 4-bromo-2-fluorobenzonitrile were used as the starting material to obtain the product with reference to the preparation route of Example 165 and Example 122.

¹H NMR (400 MHz, DMSO-*d*₆) δ 8.47 (s, 2H), 8.36 (s, 1H), 8.19 (d, *J* = 8.8 Hz, 1H), 7.79-7.67 (m, 2H), 7.47-7.40 (m, 1H), 6.47 (s, 1H), 5.51 (t, *J =* 5.4 Hz, 1H), 4.82 (d, *J =* 6.1 Hz, 1H), 3.70 (d, *J* = 0.7 Hz, 3H), 1.43 (dd, *J* = 9.3, 6.6 Hz, 3H).

### Example 292. methyl 4-amino-7-bromo-1-(2-methylpyridin-3-yl)-2-oxo-1,2-dihydro-1,8-naphthyridine-3 -carboxylate

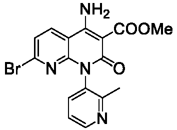

According to the preparation route of scheme 7, 2,6-dibromonicotinonitrile and 2-methylpyridin-3-amine were used as the starting material to obtain the product with reference to the preparation route of Example 165.

¹H NMR (400 MHz, DMSO-*d*₆) δ 8.61 (d, *J* = 8.4 Hz, 1H), 8.58 (s, 2H), 8.48 (dd, *J* = 5.2 Hz, 1.6 Hz, 1H), 7.58 (dd, *J* = 8.0 Hz, 2.4 Hz, 1H), 7.54 (d, *J* = 8.4 Hz, 1H), 7.35 (dd, *J* = 8.0 Hz, 5.2 Hz, 1H), 3.71 (s, 3H), 2.08 (s, 3H).

### Example 293. methyl 4-amino-7-bromo-1-(2-methylpyridin-3-yl)-2-oxo-1,2-dihydroquinoline-3-carboxylate

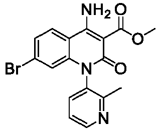

According to the preparation route of scheme 7, 4-bromo-2-fluorobenzonitrile and 2-methylpyridin-3-amine were used as the starting material to obtain the product with reference to the preparation route of Example 165.

¹H NMR (400 MHz, CD₃OD) δ 8.61 *(d, J=* 4.8 Hz, 1H), 8.08 *(d, J=* 8.4 Hz, 1H), 7.71 (d, J = 8.4 Hz, 1H), 7.53 (dd, *J* = 8.4 Hz, 4.8 Hz, 1H), 7.46 (dd, *J=* 8.4 Hz, 2.0 Hz, 1H), 6.56 (d, *J* = 2.0 Hz, 1H), 3.82 (s, 3H), 2.23 (s, 3H).

### Example 294. methyl 4-amino-1-(2,6-dimethylpyridin-3-yl)-2-oxo-7-iodo-1,2-dihydroquinoline-3 -carboxylate

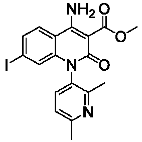

According to the preparation route of scheme 7, 2-fluoro-4-iodobenzonitrile and 2,6-dimethylpyridin-3-amine were used as the starting material to obtain the product with reference to the preparation route of Example 165.

¹H NMR (400 MHz, DMSO-*d*₆) δ 8.48 (s, 2H), 8.04 (d, *J* = 8.4 Hz, 1H), 7.59 (dd, *J* = 8.4 Hz, 1.6 Hz, 1H), 7.53 (d, *J =* 8.4 Hz, 1H), 7.29 (d, *J =* 8.4 Hz, 1H), 6.61 (d, *J =* 1.6 Hz, 1H), 3.70 (s, 3H), 2.53 (s, 3H), 2.04 (s, 3H).

### Example 295. methyl 4-amino-1-(4-amino-2-chlorophenyl)-2-oxo-7-bromo-1,2- dihydro-1,8-naphthyridine-3 -carboxylate

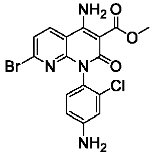

According to the preparation route of scheme 7, 2,6-dibromopyridin-3-carbonitrile and 2-chloro-4-nitroaniline were used as the starting material to obtain the product with reference to the preparation route of Example 165 and Example 147.

¹H NMR (400 MHz, DMSO-*d*₆) δ 8.53 (d, *J* = 8.8 Hz, 1H), 8.44 (s, 2H), 7.49 (d, *J* = 8.8 Hz, 1H), 6.89 (d, *J* = 8.4 Hz, 1H), 6.70 (d, *J* = 2.4 Hz, 1H), 6.55 (dd, *J* = 8.8 Hz, 2.4 Hz, 1H), 5.51 (s, 2H), 3.71 (s, 3H).

### Example 296. methyl 4-amino-7-bromo-1-(6-chloro-4-methylpyridin-3-yl)-2-oxo-1,2-dihydro-1,8-naphthyridine-3-carboxylate

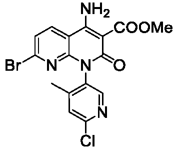

According to the preparation route of scheme 7, 2,6-dibromonicotinonitrile and 6-chloro-4-methylpyridin-3-amine were used as the starting material to obtain the product with reference to the preparation route of Example 165.

¹H NMR (400 MHz, DMSO-*d*₆) δ 8.63 (s, 2H), 8.60 (d, *J* = 8.4 Hz, 1H), 8.18 (s, 1H), 7.62 (s, 1H), 7.57 (d, *J =* 8.4 Hz, 1H), 3.72 (s, 3H), 1.95 (s, 3H).

### Example 297. methyl 4-amino-7-bromo-1-(6-chloropyridin-3-yl)-2-oxo-1,2-dihydro-1,8-naphthyridine-3 -carboxylate

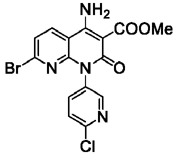

According to the preparation route of scheme 7, 2,6-dibromonicotinonitrile and 6-chloropyridin-3-amine were used as the starting material to obtain the product with reference to the preparation route of Example 165.

¹H NMR (400 MHz, CD₃OD+CDCl₃) δ 8.54-8.60 (m, 3H), 8.32 (d *J* = 2.0 Hz, 1H), 7.83 (dd, *J =* 8.8 Hz, 2.8 Hz, 1H), 7.68 (d, *J =* 8.8 Hz, 1H), 7.55 (d, *J =* 8.4 Hz, 1H), 3.72 (s, 3H).

### Example 298. methyl 4-amino-1-(4-(1-hydroxyethyl)phenyl)-2-oxo-7-iodo-1,2-dihydroquinoline-3 -carboxylate

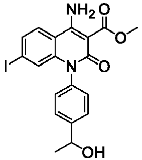

According to the preparation route of scheme 7, 1-(4-aminophenyl)ethan-1-ol and 4-iodo-2-fluorobenzonitrile were used as the starting material to obtain the product with reference to the preparation route of Example 165.

¹H NMR (400 MHz, CD₃OD) δ 7.53 (d, *J* = 8.4 Hz, 1H), 7.40-7.48 (m, 3H), 7.05 (dd, *J* = 6.0 Hz, 2.8Hz, 2H), 6.82 (s, 1H), 4.83 (q, *J* = 6.4 Hz, 1H), 3.75 (s, 3H), 1.42 (d, *J* = 6.8 Hz, 3H).

### Example 299. methyl 4-amino-1-(4-(2,2,2-trifluoro-1-hydroxyethyl)phenyl)-2-oxo-7-(trifluoromethyl)-1,2-dihydroquinoline-3 -carboxylate

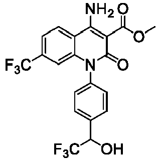

According to the preparation route of scheme 6, 1-(4-bromophenyl)-2,2,2-trifluoroethanol and 2-amino-4-(trifluoromethyl)benzonitrile were used as the starting material to obtain the product with reference to the preparation route of Example 81.

¹H NMR (400 MHz, CD₃OD) δ 8.68 (d, *J =* 7.6 Hz, 1H), 7.78-7.82 (m, 2H), 7.64 (d, *J =* 8.4 Hz, 1H), 7.34 (d, *J* = 8.0 Hz, 2H), 6.94 (s, 1H), 5.11 (d, *J* = 7.6 Hz, 1H), 3.92 (s, 3H).

### Example 300. methyl 4-amino-1-(4-atninophenyl)-7-ethynyl-2-oxo-1,2-dihydroquinoline-3-carboxylate

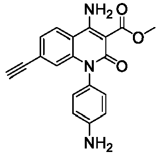

Example 147 and ethynyltrimethylsilane were used as the starting material to obtain the product with reference to the preparation route of Example 65.

¹H NMR (400 MHz, DMSO-*d*₆) δ 8.22 (s, 2H), 8.18 (d, *J =* 8.4 Hz, 1H), 7.23 (d, *J =* 8.4 Hz, 1H), 6.80 (d, *J =* 8.4 Hz, 2H), 6.69 (d, *J =* 8.4 Hz, 2H), 6.52 (s, 1H), 5.20-5.63 (brs, 2H), 4.33 (s, 1H), 3.69 (s, 3H).

### Example 301. methyl 4-amino-1-(imidazo[3,2-a]pyridin-6-yl)-7-iodo-2-oxo-1,2-dihydroquinoline-3 -carboxylate

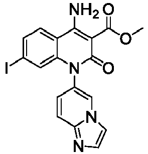

According to the preparation route of scheme 7, 2-fluoro-4-iodobenzonitrile and imidazo[3,2-a]pyridin-6-amine were used as the starting material to obtain the product with reference to the preparation route of Example 165.

¹H NMR (400 MHz, DMSO-*d*₆) δ 8.67 (d, *J* = 2.0 Hz, 1H), 8.44 (s, 2H), 7.99-8.01 (m, 2H), 7.72 (d, *J* = 9.2 Hz, 1H), 7.68 (s, 1H), 7.60 (dd, *J =* 9.2 Hz, 2.0 Hz, 1H), 7.10 (dd, *J =* 9.2 Hz, 2.0 Hz, 1H), 6.98 (d, *J =* 2.0 Hz, 1H), 3.70 (s, 3H).

### Example 302. methyl 4-amino-1-(imidazo[3,2-a]pyridin-6-yl)-2-oxo-7-(trifluoromethyl)-1,2-dihydroquinoline-3 -carboxylate

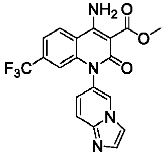

According to the preparation route of scheme 7, 2-fluoro-4-(trifluoromethyl)benzonitrile and imidazo[3,2-a]pyridin-6-amine were used as the starting material to obtain the product with reference to the preparation route of Example 165.

¹H NMR (400 MHz, DMSO-*d*₆) δ 8.73 (s, 1H), 8.47-8.50 (m, 3H), 8.00 (s, 1H), 7.73 *(d, J=* 9.6 Hz, 1H), 7.69 (s, 1H), 7.60 (dd, *J* = 9.2 Hz, 1.2 Hz, 1H), 7.14 (dd, *J* = 9.2 Hz, 2.0 Hz, 1H), 6.89 (s, 1H), 3.72 (s, 3H).

### Example 303. methyl 4-amino-1-(4-(1-hydroxyethyl)phenyl)-2-oxo-7-(trifluoro methyl)-1,2-dihydro-1,8-naphthyridine-3-carboxylate

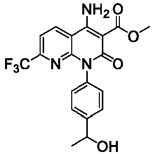

According to the preparation route of scheme 7, 2-chloro-6-(trifluoromethyl)pyridin-3-carbonitrile and 1-(4-aminophenyl)ethan-1-one were used as the starting material to obtain the product with reference to the preparation route of Example 165 and Example 122.

¹H NMR (400 MHz, DMSO-*d*₆) δ 8.90 (d, *J* = 8.0 Hz, 1H), 8.42 (s, 2H), 7.73 (d, *J* = 8.0 Hz, 1H), 7.41 *(d, J=* 8.0 Hz, 2H), 7.11 *(d, J=* 8.0 Hz, 2H), 5.10-5.44 (brs, 1H), 4.79 *(q, J=* 6.4 Hz, 1H), 3.73 (s, 3H), 1.36 (d, *J* = 6.4 Hz, 3H).

### Example 304. methyl 4-amino-1-(4-amino-2-methylphenyl)-7-bromo-2-oxo-1,2-dihydro-1,8-naphthyridine-3 -carboxylate

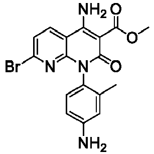

According to the preparation route of scheme 7, 2-chloro-6-bromopyridin-3-carbonitrile and 2-methyl-4-nitroaniline were used as the starting material to obtain the product with reference to the preparation route of Example 165 and Example 147.

¹H NMR (400 MHz, DMSO-*d*₆) δ 8.51 (s, 1H), 8.47 (d, *J* = 8.0 Hz, 1H), 8.44 (s, 2H), 7.94 (d, *J* = 8.0 Hz, 2H), 7.82 (s, 1H), 7.57 (d, *J* = 8.0 Hz, 1H), 7.42 (d, *J* = 8.0 Hz, 2H), 6.64 (s, 1H), 3.72 (s, 3H).

### Example 305. methyl 4-amino-1-(6-amino-2-methylpyridin-3-yl)-2-oxo-7-(trifluoromethyl)-1,2-dihydroquinoline-3 -carboxylate

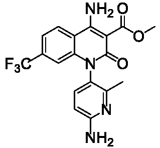

According to the route of Scheme 6, compound 2-amino-4-(trifluoromethyl)benzonitrile and tert-butyl N (5-bromo-6-methylpyridin-2-yl)carbamate were used as the starting material to obtain the product with reference to the preparation route of Example 81.

¹H NMR (400 MHz, CD₃OD+CDCl₃) δ 8.16 *(d, J=* 8.5 Hz, 1H), 7.45-7.40 (m, 1H), 7.17 (d, *J* = 8.6 Hz, 1H), 6.80 (s, 1H), 6.55 (d, *J* = 8.6 Hz, 1H), 3.83 (s, 3H), 1.99 (s, 3H).

### Example 306. methyl 4-amino-1-(4-aminophenyl)-7-cyclopropyl-2-oxo-1,2-dihydroquinoline-3 -carboxylate

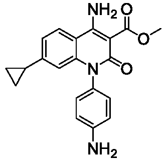

Example 147 and cyclopropylboronic acid were used as the starting material to obtain the product with reference to the preparation route of Example 66.

¹H NMR (400 MHz, DMSO-*d*₆) δ 8.23 (s, 2H), 8.03 (d, *J =* 8.8 Hz, 1H), 6.76-6.81 (m, 3H), 6.70 (d, *J =* 8.8 Hz, 2H), 6.24 (d, *J =* 1.2 Hz, 1H), 5.14-5.68 (brs, 2H), 3.67 (s, 3H), 1.73-1.78 (m, 1H), 0.88-0.93 (m, 2H), 0.53-0.57 (m, 2H).

### Example 307. methyl 4-amino-1-(6-(1-hydroxyethyl)pyridin-3-yl)-2-oxo-7-iodo-1,2-dihydroquinoline-3 -carboxylate

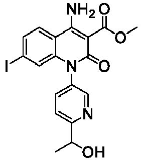

According to the preparation route of scheme 7, 1-(5-aminopyridin-2-yl)ethan-1-one and 4-iodo-2-fluoro benzonitrile were used as the starting material to obtain the product with reference to the preparation route of Example 165 and Example 122.

¹H NMR (400 MHz, CD₃OD) δ 8.26 (d, *J* = 2.4 Hz, 1H), 7.54-7.61 (m, 3H), 7.46 (dd, *J* = 8.4 Hz, 1.6 Hz, 1H), 6.79 (d, *J =* 6.0 Hz, 1H), 4.90 (q, *J =* 6.4 Hz, 1H), 3.76 (s, 3H), 1.48 (d, *J =* 6.4 Hz, 3H).

### Example 308. methyl 4-amino-1-(6-amino-2-methylpyridin-3-yl)-2-oxo-7-(trifluoromethyl)-1,2-dihydro-1,8-naphthyridine-3 -carboxylate

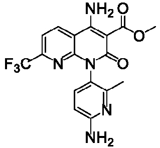

According to the route of scheme 7, compound 2-chloro-6-(trifluoromethyl)nicotinonitrile and tert-butyl A-(5-amino-6-methylpyridin-2-yl)carbamate were used as the starting material to obtain the product with reference to the preparation route of Example 165.

¹H NMR (400 MHz, CD₃OD+CDCl₃) δ 8.73 *(d, J=* 8.2 Hz, 1H), 7.56 *(d, J=* 8.3 Hz, 1H), 7.21 (dd, *J* = 8.7, 1.3 Hz, 1H), 6.57 (d, *J* = 8.7 Hz, 1H), 3.85 (d, *J* = 1.4 Hz, 3H), 1.99 (s, 3H), 1.93 (s, 2H).

### Example 309. methyl 4-amino-1-(imidazo[3,2-a]pyridin-6-yl)-2-oxo-7-(trifluoromethyl)-1,2-dihydro-1,8-naphthyridine-3 -carboxylate

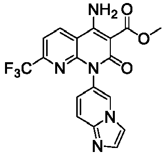

According to the preparation route of scheme 7, 2-chloro-6-(trifluoromethyl)pyridin-3-carbonitrile and imidazo[3,2-a]pyridin-6-amine were used as the starting material to obtain the product with reference to the preparation route of Example 165.

¹H NMR (400 MHz, DMSO-*d*₆) δ 8.97 (d, *J* = 8.4 Hz, 1H), 8.61 (s, 1H), 8.57 (s, 2H), 7.99 (s, 1H), 7.80 (d, *J* = 8.4 Hz, 1H), 7.60-7.68 (m, 2H), 7.10-7.16 (m, 1H), 3.74 (s, 3H).

### Example 310. methyl-d₃ 4-amino-1-(4-(1-hydroxyethyl)phenyl)-2-oxo-7-(trifluoromethyl)-1,2-dihydro-1,8-naphthyridine-3 -carboxylate

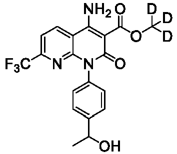

According to the preparation route of scheme 7, 2-chloro-6-(trifluoromethyl)pyridin-3-carbonitrile and 1-(4-aminophenyl)ethan-1-one were used as the starting material to obtain the product with reference to the preparation route of Example 165 and Example 122.

¹H NMR (400 MHz, DMSO-*d*₆) δ 8.95 *(d, J=* 8.0 Hz, 1H), 8.47 (s, 2H), 7.74 *(d, J=* 8.0 Hz, 1H), 7.41 *(d, J=* 8.0 Hz, 2H), 7.12 *(d, J=* 8.0 Hz, 2H), 5.27 (s, 1H), 4.78 *(q, J=* 6.4 Hz, 1H), 1.28 (d, *J* = 6.4 Hz, 3H).

### Example 311. methyl-d3 4-amino-7-bromo-1-(4-(1-hydroxyethyl)phenyl)-2-oxo-1,2-dihydroquinoline-3 -carboxylate

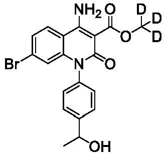

The product obtained in Example 200 (20 mg) was dissolved in methanol (3 mL), an aqueous solution of 1 mol/L sodium hydroxide(1 mL) was added, the mixture was stirred at room temperature for 2 hours, then 1 mol/L hydrochloric acid was used to adjust the pH to 5. The mixture was extracted with ethyl acetate, the extract was dried over anhydrous sodium sulphate and filtered, and the filtrate was concentrated under reduced pressure. The residue was dissolved in anhydrous *N*,*N*-dimethylformamide (2 mL) and methanol-*d₄* (3 mL), 2-(7-azabenzotriazol-1-yl)-*N*,*N*,*N*',*N*'-tetramethyluronium hexafluorophosphate (4 mg) and diisopropylethylamine (3 mg) were added, and the mixture was stirred at room temperature overnight. Water was added to quench the reaction, the mixture was extracted with ethyl acetate, the extract was washed with brine, dried over anhydrous sodium sulphate and filtered, the filtrate was concentrated under reduced pressure, and the residue was purified through silica gel Thin-Layer Chromatography ( (15:1 dichloromethane/methanol) to obtain the product (10 mg).

¹H NMR (400 MHz, DMSO-*d*₆) δ 8.36 (br, 2H), 8.15 (d, *J* = 8.8 Hz, 1H), 7.49-7.56 (m, 2H), 7.38-7.49 (m, 1H), 7.15-7.18 (m, 2H), 6.47(d, J=2.0Hz, 1H), 5.31 (d, *J* = 4.4 Hz, 1H), 4.81 (t, *J*=5.2Hz,1H), 5.73 (d, *J* = 6.8 Hz, 3H).

### Example 312. methyl 4-amino-1-(6-hydroxypyridin-3-yl)-2-oxo-7-(trifluoromethyl)-1,2-dihydro-1,8-naphthyridine-3-carboxylate

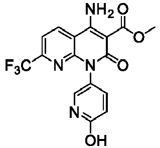

While being cooled with an ice-batch, methyl 4-amino-1-(6-methoxypyridin-3-yl)-2-oxo-7-(trifluoromethyl)-1,2-dihydro-1,8-naphthyridine-3-carboxylate (27 mg) was dissolved in dichloromethane (10 mL), 1 M boron tribromide dichloromethane solution (0.34 mL) was added, and the mixture was stirred at room temperature overnight. An aqueous solution of saturated sodium bicarbonate (10 mL) was added to quench the reaction, the mixture was extracted with dichloromethane, the extract was concentrated under reduced pressure, and the residue was purified through column chromatography (dichloromethane/methanol = 5:1) to obtain the product (10 mg).

¹H NMR (400 MHz, CD₃OD) δ 8.81 (d, *J* = 8.0 Hz, 1H), 7.99 (d, *J* = 6.8 Hz, 1H), 7.66 (d, *J*=8.4 Hz, 1H), 7.48-7.51 (m, 1H), 6.89-6.93 (m, 1H), 3.96 (s, 3H).

### Example 313. methyl 4-amino-1-(4-amino-3-methylphenyl)-7-bromo-2-oxo-1,2-dihydroquinoline-3 -carboxylate

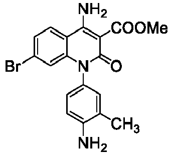

According to the preparation route of scheme 7, 2-amino-4-bromobenzonitrile and 4-fluoro-2-methyl-1-nitrobenzene were used as the starting material to obtain the product with reference to the preparation route of Example 165 and Example 147.

¹H NMR (400 MHz, DMSO-*d*₆) δ 8.23 (s, 2H), 8.11 *(d, J* = 8.8 Hz, 1H), 7.35 (dd, *J* = 8.8 Hz, 2.0 Hz, 1H), 6.67-6.73 (m, 3H), 6.61 (d, *J* = 1.6 Hz, 1H), 5.11 (s, 2H), 3.69 (s, 3H), 2.07 (s, 3H).

### Example 314. methyl 4-atnino-1-(4-amino-3-methylphenyl)-2-oxo-7-(trifluoromethyl)-1,2-dihydroquinoline-3 -carboxylate

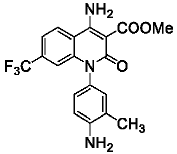

According to the preparation route of scheme 7, 2-amino-4-(trifluoromethyl)benzonitrile and 4-fluoro-2-methyl-1-nitrobenzene were used as the starting material to obtain the product with reference to the preparation route of Example 165 and Example 147.

¹H NMR (400 MHz, CD₃OD) δ 8.40 (d, *J =* 8.0 Hz, 1H), 8.24 (s, 2H), 7.50 (d, *J =* 7.2 Hz, 1H), 6.72-6.75 (m, 4H), 5.13 (s, 2H), 3.71 (s, 3H), 2.06 (s, 3H).

### Example 315. methyl 4-atnino-1-(8-chloroisoquinolin-5-yl)-2-oxo-7-(trifluoromethyl)-1,2-dihydro-1,8-naphthyridine-3-carboxylate

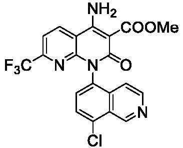

According to the preparation route of scheme 7, 2-chloro-6-(trifluoromethyl)nicotinonitrile and 8-chloroisoquinolin-5-amine were used as the starting material to obtain the product with reference to the preparation route of Example 165.

¹H NMR (400 MHz, DMSO-*d*₆) δ 9.62 (s, 1H), 8.99 (d, *J* = 8.0 Hz, 1H), 8.72 (s, 2H), 8.51 (d, *J =* 6.0 Hz, 1H), 7.96 (d, *J =* 7.6 Hz, 1H), 7.78 (d, *J* = 8.4 Hz, 1H), 7.73 (d, *J* = 7.6 Hz, 1H), 7.41 (d, *J* = 6.0 Hz, 1H), 3.71 (s, 3H).

### Example 316. methyl 4-amino-1-(6-methylpyridin-3-yl)-2-oxo-7-methyl-1,2-dihydroquinoline-3 -carboxylate

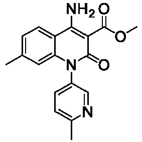

According to the preparation route of scheme 7, 2-bromo-4-methylbenzonitrile and 6-methylpyridin-3-amine were used as the starting material to obtain the product with reference to the preparation route of Example 165.

¹H NMR (400 MHz, DMSO-*d*₆) δ 8.41 (s, 2H), 8.26 (d, *J =* 2.4 Hz, 1H), 8.11 (d, *J =* 8.4 Hz, 1H), 7.59 (dd, *J* = 8.0 Hz, 2.4 Hz, 1H), 7.44 (d, *J* = 8.4 Hz, 1H), 7.05 (d, *J* = 8.0 Hz, 1H), 6.21 (s, 1H), 3.68 (s, 3H), 2.56 (s, 3H), 2.21 (s, 3H).

### Example 317. methyl 4-atnino-1-(4-amino-3-methylphenyl)-2-oxo-7-(trifluoromethyl)-1,2-dihydro-1,8-naphthyridine-3-carboxylate

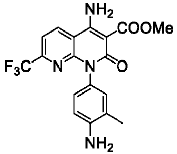

According to the preparation route of scheme 7, 2-chloro-6-(trifluoromethyl)nicotinonitrile and 3-methyl-4-nitroaniline were used as the starting material to obtain the product with reference to the preparation route of Example 165 and Example 147.

¹H NMR (400 MHz, DMSO-*d*₆) δ 8.84 (d, *J* = 8.4 Hz, 1H), 8.30 (s, 2H), 7.69 (d, *J* = 8.4 Hz, 1H), 6.67 (s, 1H), 6.60-6.65 (m, 2H), 4.93 (s, 2H), 3.72 (s, 3H), 2.03 (s, 3H).

### Example 318. methyl 4-atnino-1-(1-chloroisoquinolin-5-yl)-2-oxo-7-(trifluoromethyl)-1,2-dihydro-1,8-naphthyridine-3-carboxylate

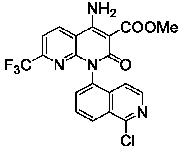

According to the preparation route of scheme 7, 2-chloro-6-(trifluoromethyl)nicotinonitrile and 1-chloroisoquinolin-5-amine were used as the starting material to obtain the product with reference to the preparation route of Example 165.

¹H NMR (400 MHz, DMSO-*d*₆) δ 8.98 (d, *J* = 8.0 Hz, 1H), 8.71 (s, 2H), 8.39 (d, *J* = 8.0 Hz, 1H), 8.16 (d, *J* = 5.6 Hz, 1H), 7.93 (t, *J* = 8.0 Hz, 1H), 7.83 (d, *J* = 7.2 Hz, 1H), 7.77 (d, *J* = 8.0 Hz, 1H), 7.40 (d, *J* = 5.6 Hz, 1H), 3.72 (s, 3H).

### Example 319. methyl 4-amino-1-(1-methyfisoquinolin-5-yl)-2-oxo-7-(trifluoromethyl)-1,2-dihydro-1,8-naphthyridine-3-carboxylate

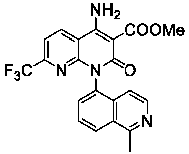

According to the preparation route of scheme 7, 2-chloro-6-(trifluoromethyl)nicotinonitrile and 1-methylisoquinolin-5-amine were used as the starting material to obtain the product with reference to the preparation route of Example 165.

¹H NMR (400 MHz, DMSO-*d*₆) δ 8.97 (d, *J* = 8.0 Hz, 1H), 8.64 (s, 2H), 8.31 (d, *J* = 8.4 Hz, 1H), 8.19 (d, *J* = 6.0 Hz, 1H), 7.77 (t, *J* = 8.0 Hz, 1H), 7.75 (d, *J* = 8.4 Hz, 1H), 7.66 (d, *J* = 7.2 Hz, 1H), 7.10 (d, *J* = 6.0 Hz, 1H), 3.71 (s, 3H), 2.95 (s, 3H).

### Example 320. methyl 4-amino-1-(4-aminophenyl)-2-oxo-7-(trifluoromethoxy)-1,2-dihydroquinoline-3 -carboxylate

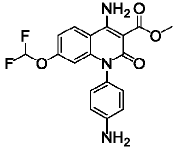

### Step A: 4-difluoromethoxy-2-fluorobenzonitrile

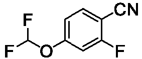

2-Fluoro-4-hydroxybenzonitrile (1.37 g) and sodium chlorodifluoroacetate (4.56 g) were dissolved in *N*,*N*-dimethylformamide (45 ml) and water (5 ml), cesium carbonate (4.89 g) was added, the mixture was reacted at 100 °C for 16 hours and cooled to room temperature, water (30 mL) was added, the mixture was extracted with ethyl acetate, the extract was dried over anhydrous sodium sulphate and filtered, the filtrate was concentrated under reduced pressure, and the residue was separated through column chromatography (5:1 petroleum ether/ethyl acetate ) to obtain a white solid (0.89 g).

¹H NMR (400 MHz, CDCl₃) δ 7.74-7.49 (m, 1H), 7.13-6.93 (m, 2H), 6.86-6.30 (m, 1H).

### Step B: methyl 4-amino-1-(4-aminophenyl)-2-oxo-7-(trifluoromethoxy)-1,2-dihydroquinoline-3 -carboxylate

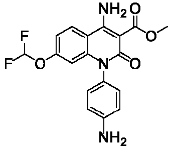

According to the route of scheme 7, compound 4-difluoromethoxy-2-fluorobenzonitrile and 4-nitroaniline were used as the starting material to obtain the product with reference to the preparation route of Example 165 (15 mg).

¹H NMR (400 MHz, CD₃OD+CDCl₃) δ 8.34 (s, 1H), 7.94 (d, *J* = 9.0 Hz, 1H), 6.93-6.84 (m, 3H), 6.84-6.74 (m, 2H), 6.71-6.24 (m, 2H), 3.80 (d, *J* = 0.9 Hz, 3H).

### Example 321. methyl 4-amino-7-bromo-1-(5-methylpyridin-2-yl)-2-oxo-1,2-dihydroquinoline-3 -carboxylate

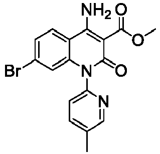

According to the preparation route of scheme 7, 4-bromo-2-fluorobenzonitrile and 5-methylpyridin-2-amine were used as the starting material to obtain the product with reference to the preparation route of Example 165.

¹H NMR (400 MHz, CD₃OD) δ 8.53 (d, *J* = 1.6 Hz, 1H), 8.03 (d, *J* = 8.8 Hz, 1H), 7.95 (dd, *J* = 7.6 Hz, 1.6 Hz, 1H), 7.41 (dd, *J* = 8.8 Hz, 2.0 Hz, 1H), 7.37 (d, *J* = 8.4 Hz, 1H), 6.51 (d, *J* = 2.0 Hz, 1H), 3.81 (s, 3H), 2.50 (s, 3H).

### Example 322. methyl 4-atnino-7-chloro-1-(4-aminophenyl)-2-oxo-1,2-dihydroquinoline-3-carboxylate

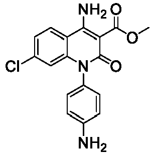

According to the preparation route of scheme 7, 2-amino-4-chlorobenzonitrile and 4-fluoronitrobenzene were used as the starting material to obtain the product with reference to the preparation route of Example 165.

¹H NMR (400 MHz, DMSO-*d*₆) δ 8.25 (s, 2H), 8.19 *(d, J=* 8.8 Hz, 1H), 7.23 (dd, *J* = 8.8 Hz, 2.0 Hz, 1H), 6.80 (d, *J =* 8.8 Hz, 2H), 6.67 (d, *J* = 8.8 Hz, 2H), 6.45 (d, *J* = 2.0 Hz, 1H), 5.34 (s, 2H), 3.69 (s, 3H).

### Example 323. methyl 4-amino-1-(4-(1-hydroxypropyl)phenyl)-2-oxo-7-(trifluoromethyl)-1,2-dihydroquinoline-3 -carboxylate

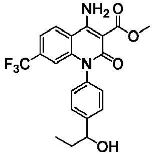

According to the preparation route of scheme 7, 2-fluoro-4-(trifluoromethyl)benzonitrile and 1-(4-aminophenyl)propan-1-one were used as the starting material to obtain the product with reference to the preparation route of Example 165 and 122.

¹H NMR (400 MHz, DMSO-*d*₆) δ 8.44 (d, *J =* 8.4 Hz, 1H), 8.38 (s, 2H), 7.48-7.55 (m, 3H), 7.20 (d, *J =* 8.0 Hz, 2H), 6.58 (s, 1H), 5.29 (d, *J =* 3.6 Hz, 1H), 4.56 (q, *J =* 4.8 Hz, 1H), 3.72 (s, 3H), 1.62-1.70 (m, 2H), 0.83 (t, *J =* 7.2 Hz, 3H).

### Example 324. methyl 4-amino-1-(4-(1-hydroxypropyl)phenyl)-2-oxo-7-bromo-1,2-dihydroquinoline-3 -carboxylate

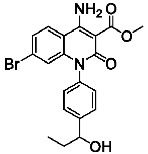

According to the preparation route of scheme 7, 4-bromo-2-fluorobenzonitrile and 1-(4-aminophenyl)propan-1-one were used as the starting material to obtain the product with reference to the preparation route of Example 165 and 122.

¹H NMR (400 MHz, DMSO-*d*₆) δ 8.36 (s, 2H), 8.15 *(d, J=* 8.8 Hz, 1H), 7.52 *(d, J=* 7.2 Hz, 1H), 7.47 *(d, J=* 7.2 Hz, 1H), 7.39 (dd, *J* = 8.4 Hz, 2.0 Hz, 1H), 7.14-7.19 (m, 2H), 6.46 (d, *J* = 1.6 Hz, 1H), 5.28 (d, *J* = 4.8 Hz, 1H), 4.55 (q, *J* = 4.0 Hz, 1H), 3.69 (s, 3H), 1.63-1.70 (m, 2H), 0.86 (t, *J* = 7.2 Hz, 3H).

### Example 325. methyl 4-atnino-1-(4-amino-2-chlorophenyl)-2-oxo-7-trifluoromethyl-1,2-dihydro-1,8-naphthyridine-3-carboxylate

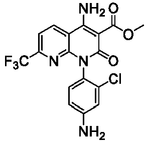

According to the preparation route of scheme 7, 2-chloro-6-(trifluoromethyl)pyridin-3-carbonitrile and 2-chloro-4-nitroaniline were used as the starting material to obtain the product with reference to the preparation route of Example 165 and Example 147.

¹H NMR (400 MHz, DMSO-*d*₆) δ 8.90 (d, *J =* 8.0 Hz, 1H), 8.46 (s, 2H), 7.74 (d, *J =* 8.0 Hz, 1H), 6.90 (d, *J* = 8.8 Hz, 1H), 6.70 (d, *J* = 2.0 Hz, 1H), 6.55 (dd, *J* = 8.8 Hz, 2.0 Hz, 1H), 5.49 (s, 2H), 3.73 (s, 3H).

### Example 326. methyl 4-amino-1-(6-methylpyridin-3-yl)-2-oxo-7-methoxy-1,2-dihydroquinoline-3 -carboxylate

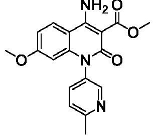

According to the preparation route of scheme 7, 2-fluoro-4-methoxybenzonitrile and 6-methylpyridin-3-amine were used as the starting material to obtain the product with reference to the preparation route of Example 165.

¹H NMR (400 MHz, DMSO-*d*₆) δ 8.45 (s, 2H), 8.27 (d, *J =* 2.4 Hz, 1H), 8.18 (d, *J* = 9.2 Hz, 1H), 7.60 (dd, *J* = 8.4 Hz, 2.4 Hz, 1H), 7.44 (d, *J* = 8.4 Hz, 1H), 6.87 (dd, *J* = 9.2 Hz, 2.4 Hz, 1H), 5.75 (d, *J* = 2.4 Hz, 1H), 3.67 (s, 3H), 3.63 (s, 3H), 2.56 (s, 3H).

### Example 327. methyl 4-amino-1-(4-aminophenyl)-2-oxo-7-(difluoromethyl)-1,2-dihydro quinoline-3 -carboxylate

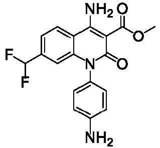

### Step A: 4-difluoromethyl-2-fluorobenzonitrile

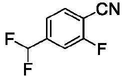

2-Fluoro-4-formylbenzonitrile (1.49 g) was dissolved in dichloromethane (30 ml), diethylaminosulfur trifluoride (3.1 g) was added, and the mixture was reacted at room temperature until the starting material disappeared. Saturated sodium bicarbonate solution was added, the mixture was stirred for 1 hour, extracted with dichloromethane and saturated sodium bicarbonate solution, the organic phase was dried over anhydrous sodium sulphate and rotavapped to dryness, and the residue was separated through column chromatography (5: 1 petroleum ether/ethyl acetate ) to obtain the target product (0.98 g).

¹H NMR (400 MHz, CDCl₃) δ 7.89-7.60 (m, 1H), 7.41 (t, *J =* 9.4 Hz, 2H), 6.98-6.36 (m, 1H).

### Step B: methyl 4-amino-1-(4-aminophenyl)-2-oxo-7-(difluoromethyl)-1,2-dihydroquinoline-3-carboxylate

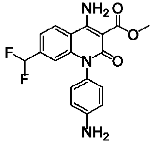

According to the route of scheme 7, compound 4-difluoromethyl-2-fluorobenzonitrile and 4-nitroaniline were used as the starting material to obtain the product with reference to the preparation route of Example 165 (13 mg).

¹H NMR (400 MHz, CD₃OD+CDCl₃) δ 8.10 *(d, J=* 8.5 Hz, 1H), 7.31 (d, *J* = 8.5 Hz, 1H), 6.98-6.87 (m, 2H), 6.87-6.80 (m, 2H), 6.78 (s, 1H), 6.73-6.37 (m, 1H), 3.82 (d, *J* = 1.0 Hz, 3H).

### Example 328. methyl 4-amino-1-(4-(1-hydroxyethyl)phenyl)-2-oxo-7-cyclopropyl-1,2-dihydroquinoline-3 -carboxylate

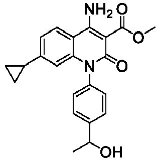

Under nitrogen protection, compound methyl amino-1-(4-(1-hydroxy ethyl)phenyl)-2-oxo-7-bromo-1,2-dihydroquinoline-3-carboxylate obtained in Example 200 (80 mg), cyclopropylboronic acid (33mg), [1,1'-bis(diphenylphosphino)ferrocene]dichloropalladium(II) (14 mg) and cesium fluoride (144 mg) were added to a mixed solvent of 1,4-dioxane (10 mL) and water (1 mL), the mixture was heated and refluxed overnight, cooled to room temperature, filtered through celite, and washed with ethyl acetate, the filtrate was evaporated to dryness, and the residue was purified through silica gel Thin-Layer Chromatography (15:1 dichloromethane/methanol ) to obtain the product (18 mg).

¹H NMR (400 MHz, CD₃OD) δ 7.97 (d, *J =* 8.4 Hz, 1H), 7.58-7.63 (m, 2H), 7.19 (d, *J =* 8.4 Hz, 2H), 6.91 (d, *J =* 8.4 Hz, 1H), 6.26 (s, 1H), 4.95 (q, *J =* 6.4 Hz, 1H), 3.81 (s, 3H), 1.60-1.76 (m, 1H), 1.51 (d, *J=* 6.4 Hz, 3H), 0.94-0.97 (m, 2H), 0.55-0.57 (m, 2H).

### Example 329. methyl 4-amino-1-(4-(1-hydroxyethyl)phenyl)-2-oxo-7-methyl-1,2-dihydroquinoline-3 -carboxylate

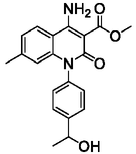

Example 200 and trimethylboroxine were used as the main starting material to obtain the product with reference to the preparation method of Example 328.

¹H NMR (400 MHz, CD₃OD) δ 7.98 (d, *J =* 8.4 Hz, 1H), 7.57-7.62 (m, 2H), 7.19 (d, *J =* 8.0 Hz, 2H), 7.09 (d, *J* = 8.8 Hz, 1H), 6.37 (s, 1H), 4.93 (q, *J* = 6.0 Hz, 1H), 3.81 (s, 3H), 2.24 (s, 3H), 1.51 (d, *J* = 6.0 Hz, 3H).

### Example 330. ethyl 4-amino-1-(4-(1-(R)-hydroxyethyl)phenyl)-2-oxo-7-(trifluoromethyl)-1,2-dihydroquinoline-3 -carboxylate

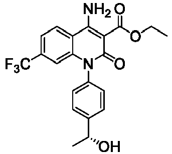

The product methyl 4-amino-1-(4-(1-(*R*)-hydroxyethyl) phenyl)-2-oxo-7-(trifluoromethyl)-1,2-dihydroquinoline-3-carboxylate obtained in Example 272 (15 mg) was dissolved in ethanol (10 mL), concentrated hydrochloric acid (1 mL) was added, and the reaction solution was heated to 70 °C and stirred for 2 hours, then cooled to room temperature. An aqueous solution of saturated NaHCO₃ (10 mL) was added, the mixture was extracted with ethyl acetate, the organic phase was washed with brine, dried over anhydrous sodium sulphate, and filtered, and the solution was evaporated to dryness to obtain the product (13 mg).

¹H NMR (400 MHz, CDCl₃) δ 7.80 (d, *J =* 8.4 Hz, 1H), 7.50-7.80 (brs, 2H), 7.52-7.58 (m, 2H), 7.40 *(d, J* = 8.8 Hz, 1H), 7.21-7.25 (m, 2H), 6.85 (s, 1H), 5.03 (q, *J* = 6.0 Hz, 1H), 4.38 (q,*J* = 7.2 Hz, 2H), 1.98-2.15 (brs, 1H), 1.45 (d, *J* = 6.4 Hz, 3H), 1.38 (t, *J=* 7.2 Hz, 3H).

### Example 331. methyl-d₃ 4-amino-1-(4-(1-hydroxyethyl)phenyl)-2-oxo-7-(trifluoromethyl)-1,2-dihydroquinolin-3-carboxylate

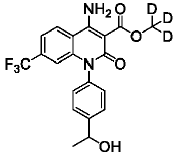

Example 122 was used as the starting material to obtain the product with reference to the preparation route of Example 311.

¹H NMR (400 MHz, CD₃OD) δ 8.32 (d, *J =* 8.8 Hz, 1H), 7.60-7.68 (m, 2H), 7.50 (d, *J =* 8.0 Hz, 1H), 7.23-7.26 (m, 2H), 6.79 (s, 1H), 4.95 (q, *J* = 6.0 Hz, 1H), 1.51 (d, *J* = 6.4 Hz, 3H).

### Example 332. methyl 4-amino-7-bromo-1-(6-(1-hydroxyethyl)pyridin-3-yl)-2-oxo-1,2-dihydro-1,8-naphthyridine-3-carboxylate

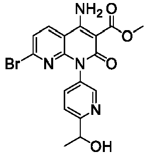

According to the preparation route of scheme 7, 2,6-dibromonicotinonitrile and 1-(5-aminopyridin-2-yl)ethan-1-one were used as the starting material to obtain the product with reference to the preparation route of Example 120 and 122.

¹H NMR (400 MHz, DMSO-*d*₆) δ 8.54 (d, *J =* 8.4 Hz, 1H), 8.50 (s, 2H), 8.28 (d, *J =* 2.0 Hz, 1H), 7.66 (dd, J = 8.4 Hz, 2.0 Hz, 1H), 7.61 (d, *J* = 8.4 Hz, 1H), 7.52 (d, *J* = 8.4 Hz, 1H), 5.50 (s, 1H), 4.76-4.82 (m, 1H), 3.71 (s, 3H), 1.41 (d, *J* = 6.0 Hz, 3H).

### Example 333. methyl-d₃ 4-amino-1-(4-amino-2-methylphenyl)-7-bromo-2-oxo-1,2-dihydroquinolin-3 -carboxylate

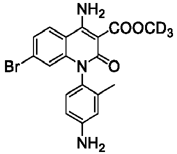

Example 238 was used as the starting material to obtain the product with reference to the preparation route of Example 189.

¹H NMR (400 MHz, CD₃OD+ CDCl₃) δ 7.83 (d, *J =* 8.8 Hz, 1H), 7.28 (d, *J =* 8.8 Hz, 1H), 6.79 (d, *J=* 8.4 Hz, 1H), 6.61-6.74 (m, 3H), 1.83 (s, 3H).

### Example 334. methyl 4-amino-1-(4-(1-hydroxyethyl)naphthalen-1-yl)-2-oxo-7-(trifluoromethyl)-1,2-dihydroquinoline-3 -carboxylate

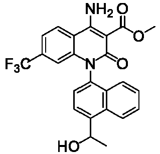

According to the preparation route of scheme 6, 1-(4-bromonaphthalen-1-yl)ethan-1-one and 2-amino-4-(trifluoromethyl)benzonitrile were used as the starting material to obtain the product with reference to the preparation route of Example 81 and Example 122.

¹H NMR (400 MHz, CDCl₃+CD₃OD) δ 8.30 (d, *J =* 8.8 Hz, 1H), 8.27 (d, *J =* 8.4 Hz, 1H), 7.84 (d, *J =* 7.6 Hz, 1H), 7.33-7.46 (m, 5H), 6.60 (s, 1H), 5.68 (q, *J =* 6.8 Hz, 1H), 3.83 (s, 3H), 1.69 (d, *J* = 6.8 Hz, 3H).

### Example 335. methyl 4-amino-7-(difluoromethoxy)-1-(4-(1-hydroxyethyl)phenyl)-2-oxo-1,2-dihydroquinoline-3 -carboxylate

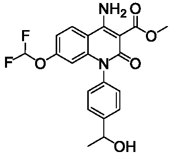

According to the route of scheme 7, compound 4-difluoromethoxy-2-fluorobenzonitrile and 1-(4-aminophenyl)ethan-1-one were used as the starting material to obtain the product with reference to the preparation route of Example 165.

¹H NMR (400 MHz, CD₃OD) δ 8.15 (dd, J= 9.0, 0.8 Hz, 1H), 7.66-7.53 (m, 2H), 7.21 (d,*J* = 8.5 Hz, 2H), 7.03 (dd, *J* = 9.1, 2.4 Hz, 1H), 6.99-6.54 (m, 1H), 6.21 (d, *J* = 2.4 Hz, 1H), 4.94 (q, *J* = 6.5 Hz, 1H), 3.81 (d, *J* = 0.8 Hz, 3H), 1.50 (dd, *J* = 6.5, 0.8 Hz, 3H).

### Example 336. methyl 4-amino-7-(difluoromethyl)-1-(4-(1-hydroxyethyl)phenyl)-2-oxo-1,2-dihydroquinoline-3 -carboxylate

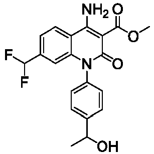

According to the route of scheme 7, compound 4-difluoromethoxy-2-fluorobenzonitrile and 1-(4-aminophenyl)ethan-1-one were used as the starting material to obtain the product with reference to the preparation route of Example 165.

¹H NMR (400 MHz, CD₃OD) δ 8.23 *(d, J=* 8.5 Hz, 1H), 7.68-7.55 (m, 2H), 7.39 *(d, J=* 8.5 Hz, 1H), 7.32-7.10 (m, 2H), 6.85-6.39 (m, 2H), 4.95 (q, *J=* 6.5 Hz, 1H), 3.82 *(d, J* = 0.8 Hz, 3H), 1.57-1.40 (m, 3H).

### Example 337. methyl 4-amino-1-(4-(1-hydroxypropyl)phenyl)-2-oxo-7-(trifluoromethyl)-1,2-dihydro-1,8-naphthyridine-3-carboxylate

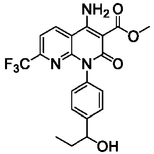

According to the preparation route of scheme 7, 2-chloro-6-(trifluoromethyl)pyridin-3-carbonitrile and 1-(4-aminophenyl)propan-1-one were used as the starting material to obtain the product with reference to the preparation route of Example 165 and 122.

¹H NMR (400 MHz, DMSO-*d*₆) δ 8.89 (d, *J=* 8.4 Hz, 1H), 8.42 (s, 2H), 7.73 (d, *J=* 8.4 Hz, 1H), 7.38 (d, *J* = 8.0 Hz, 2H), 7.10 (d, *J* = 8.0 Hz, 2H), 5.23 (d, *J* = 4.4 Hz, 1H), 4.51 (q, *J* = 4.4 Hz, 1H), 3.73 (s, 3H), 1.60-1.68 (m, 2H), 0.83 (t, *J* = 7.6 Hz, 3H).

### Example 338. methyl 4-amino-1-(4-aminophenyl)-7-bromo-8-fluoro-2-oxo-1,2-dihydroquinoline-3 -carboxylate

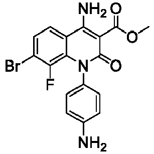

According to the preparation route of scheme 7, 4-bromo-2,3-difluorobenzonitrile and 4-fluoronitrobenzene were used as the starting material to obtain the product with reference to the preparation route of Example 165.

¹H NMR (400 MHz, CD₃OD) δ 7.82 (d, *J* = 8.8 Hz, 1H), 7.46 (dd, *J* = 8.8 Hz, 6.0 Hz, 1H), 6.93 *(d, J* = 8.8 Hz, 2H), 6.78 *(d, J=* 8.8 Hz, 2H), 3.81 (s, 3H).

### Example 339. methyl 4-amino-1-(4-aminophenyl)-7-bromo-5-fluoro-2-oxo-1,2-dihydroquinoline-3 -carboxylate

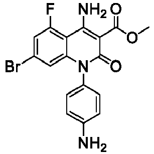

According to the preparation route of scheme 7, 4-bromo-2,6-difluorobenzonitrile and 4-aminonitrobenzene were used as the starting material to obtain the product with reference to the preparation route of Example 165.

¹H NMR (400 MHz, DMSO-*d*₆) δ 8.09 (s, 2H), 7.36 *(d, J=* 11.6 Hz, 1H), 6.80 *(d, J=* 8.8 Hz, 2H), 6.67 (d, *J =* 8.8 Hz, 2H), 6.45 (s, 1H), 5.37 (s, 2H), 3.69 (s, 3H).

### Example 340. methyl 4-amino-7-bromo-1-(4-(1-(R)-hydroxyethyl)phenyl)-2-oxo-1,2-dihydroquinoline-3 -carboxylate

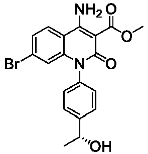

According to the preparation route of scheme 8:

### Step A: 4-bromo-2-((4-(1-(R)-((tert-butyldimethylsilyl)oxy)ethyl)phenyl)amino)benzonitrile

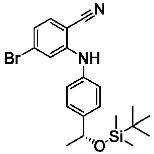

Under nitrogen protection, 4-(1-(*R*)-*(*(*tert*-butyldimethylsilyl)oxy)ethyl)aniline (504 mg) was dissolved in tetrahydrofuran (30 mL), the mixture was cooled to -78 °C, a solution of lithium bis(trimethylsilyl)amide in tetrahydrofuran (1 M, 6.0 mL) was added dropwise, the mixture was stirred at -78 °C for 0.5 hour, then a solution of 4-bromo-2-fluorobenzonitrile (400 mg) in tetrahydrofuran (10 mL) was added dropwise, the mixture was stirred at -78 °C for 1 hour, slowly warmed to room temperature and stirred overnight. An aqueous solution of saturated ammonium chloride was added to quench the reaction, the mixture was extracted with ethyl acetate, the extract was concentrated under reduced pressure, and the residue was purified through column chromatography (petroleum ether/ethyl acetate = 30: 1) to obtain the product (570 mg).

### Step B: methyl 3-((5-bromo-2-cyanophenyl)(4-(1-(R)-((tert-butyldimethylsilyl)oxy)ethyl) phenyl)amino)-3 -oxopropanate

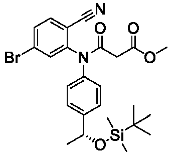

4-Bromo-2-((4-(1-(*R)*-*(*(*tert*-butyldimethylsilyl)oxy)ethyl)phenyl)amino)benzonitrile (430 mg) was dissolved in dichloromethane (20 mL), pyridine (158 mg), *N*,*N*-dimethylpyridine (12 mg), and 3-chloro-3-oxopropanatemethyl (196 mg) were added, the mixture was stirred at room temperature overnight, washed with an aqueous solution of saturated sodium bicarbonate, extracted with dichloromethane, and concentrated under reduced pressure, and the residue was purified through column chromatography (petroleum ether/ethyl acetate = 5: 1) to obtain the product (320 mg).

### Step C: methyl 4-amino-7-bromo-1-(4-(1-(R)-((tert-butyldimethylsilyl)oxy)ethyl)phenyl)-2-oxo-1,2-dihydroquinoline-3-carboxylate

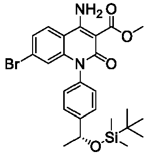

3-((5-Bromo-2-cyanophenyl)(4-(1-(*R)*-*(*(*tert*-butyldimethylsilyl)oxy)ethyl)phenyl)amino)-3-o xopropanatemethyl (260 mg) was dissolved in methanol (5 mL), a sodium methoxide solution (5.4 M, 0.27 mL) was added, and the mixture was stirred at 90 °C in a sealed tube for 2 hours. Water was added, the mixture was extracted with ethyl acetate, the extract was concentrated under reduced pressure, and the residue was purified through column chromatography (dichloromethane/ methanol = 30:1) to obtain the product (200 mg).

### Step D: methyl 4-amino-7-bromo-1-(4-(1-(R)-hydroxyethyl)phenyl)-2-oxo-1,2-dihydroquinoline-3 -carboxylate

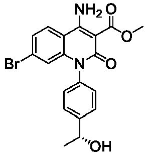

Methyl 4-amino-7-bromo-1-(4-(1-(*R)*-*(*(*tert*-butyldimethylsilyl)oxy)ethyl)phenyl)-2-oxo-1,2-dihydroquinoline-3-carboxylate (200 mg) was dissolved in ethanol (10 mL), concentrated hydrochloric acid (0.2 mL) was added, the mixture was stirred at room temperature for 1 hour, an aqueous solution of 1 mol/L sodium hydroxide was added to adjust pH to 9, the mixture was extracted with ethyl acetate, the extract was concentrated under reduced pressure, and the residue was purified through column chromatography (dichloromethane/methanol = 20:1) to obtain the product (106 mg).

¹H NMR (400 MHz, CDCl₃) δ 7.86 (d, *J* = 9.6 Hz, 1H), 7.54 (d, *J* = 7.6 Hz, 2H), 7.28-7.31 (m, 1H), 7.14 (d, *J* = 8.4 Hz, 2H), 6.78 (d, *J* = 1.6 Hz, 1H), 4.90 (d, *J* = 6.4 Hz, 1H), 3.81 (s, 3H), 1.49 (d, *J* = 6.4 Hz, 3H).

### Example 341. methyl (R)-5-amino-8-(4-(1-hydroxyethyl)phenyl)-2-methyl-7-oxo-7,8-dihydropyrido [2,3 -d]pyrimidine-6-carboxylate

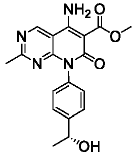

According to the preparation route of scheme 6, 2-methyl-4-aminopyrimidine-5-carbonitrile and *(R)*-(1-(4-bromophenyl)ethoxy)(*tert*-butyl)dimethylsilane were used as the starting material to obtain the product with reference to the preparation route of Example 81 and 340.

¹H NMR (400 MHz, DMSO-*d*₆) δ 9.38 (s, 1H), 8.53 (s, 2H), 7.41 (d, *J* = 7.7 Hz, 2H), 7.09 (d, *J*= 8.3 Hz, 2H), 5.25 (d, *J* = 4.3 Hz, 1H), 4.83-4.72 (m, 1H), 3.70 (d, *J=* 0.9 Hz, 3H), 2.38 (d, *J* = 0.9 Hz, 3H), 1.37 (d, *J=* 6.4 Hz, 3H).

### Example 342. methyl 4-amino-1-(5-methylpyridin-2-yl)-2-oxo-7-(trifluoromethyl)-1,2-dihydro-1,8-naphthyridine-3-carboxylate

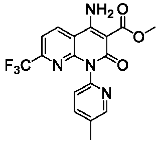

According to the preparation route of scheme 7, 2-chloro-6-(trifluoromethyl)nicotinonitrile and 5-methylpyridin-2-amine were used as the starting material to obtain the product with reference to the preparation route of Example 165.

¹H NMR (400 MHz, CD₃OD) δ 8.78 (d, *J* = 8.0 Hz, 1H), 8.44 (d, *J* = 2.4 Hz, 1H), 7.88 (dd, *J* = 8.0 Hz, 2.4 Hz, 1H), 7.65 (d, *J =* 8.0 Hz, 1H), 7.33 (d, *J =* 8.0 Hz, 1H), 3.84 (s, 3H), 2.47 (s, 3H).

### Example 343. methyl 4-amino-(1-(4-(1R)-1-hydroxyethyl)phenyl)-7-methyl-2-oxo-pyrido [2,3 -b]pyridin-3 -carboxylate

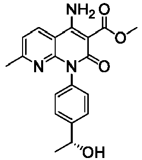

According to the preparation route of scheme 6 and 8, 2-chloro-3-cyano-6-methylpyridine and 4-(1-(*R)*-*(*(*tert*-butyldimethylsilyl)oxy)ethyl)aniline were used as the starting material to obtain the product with reference to the preparation route of Example 81 and step D of Example 340.

¹H NMR(400 MHz, DMSO-*d*₆) δ 8.50 (d, *J =* 8.0 Hz, 1H), 8.34 (s, 2H), 7.39 (d, *J =* 6.8 Hz, 2H), 7.12 (d, *J* = 8.0 Hz, 1H), 7.05 (d, *J* = 8.4 Hz, 2H), 5.23 (d, *J* = 4.4 Hz, 1H), 4.75-4.81 (m, 1H), 3.69 (s, 3H), 2.27 (s, 3H), 1.37 (d, *J* = 6.0 Hz, 3H).

### Example 344. methyl 4-amino-1-(4-aminophenyl)-7-(difluoromethyl)-2-oxo-1,2-dihydro-1,8-naphthyridine-3 -carboxylate

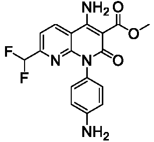

According to the route of Scheme 6, compound 2-chloro-6-(difluoromethyl)nicotinonitrile and 4-nitroaniline were used as the starting material to obtain the product with reference to the preparation route of Example 81.

¹H NMR (400 MHz, CDCl₃+CD₃OD) δ 8.52 *(d, J=* 8.8 Hz, 1H), 7.43 (d, *J* = 8.8 Hz, 1H), 6.89 (d, *J* = 8.4 Hz, 2H), 6.77 (d, *J* = 8.4 Hz, 2H), 6.29 (t, *J* = 55.6 Hz, 1H), 3.82 (s, 3H).

### Example 345. methyl 4-amino-7-(cyclopropylamino)-1-(4-(1-hydroxyethyl)phenyl)-2-oxo-1,2-dihydroquinoline-3-carboxylate

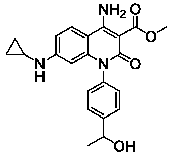

According to the preparation route of scheme 7, 4-(cyclopropylamino)-2-fluorobenzonitrile and 1-(4-aminophenyl)ethan-1-one were used as the starting material to obtain the product with reference to the preparation route of Example 120 and 122.

¹H NMR(400 MHz, DMSO-*d*₆) δ 8.26 (s, 2H), 7.88 (d, *J =* 9.2 Hz, 1H), 7.47 (d, *J =* 8.0 Hz, 2H), 7.07 (d, *J* = 8.0 Hz, 2H), 6.84 (s, 1H), 6.52 (dd, *J* = 9.2 Hz, 2.0 Hz, 1H), 5.61 (d, *J* = 2.0 Hz, 1H), 5.25 (d, *J* = 4.4 Hz, 1H), 4.76-4.82 (m, 1H), 3.63 (s, 3H), 2.06-2.13 (m, 1H), 1.36 (d, *J* = 6.4 Hz, 3H), 0.42-0.48 (m, 2H), 0.21-0.28 (m, 2H).

### Example 346. methyl 4-amino-1-(4-(1-hydroxypropyl-2-yl)phenyl)-2-oxo-7-(trifluoromethyl)-1,2-dihydroquinoline-3 -carboxylate

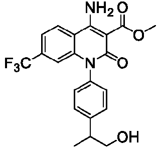

According to the preparation route of scheme 6, 2-amino-4-trifluoromethylbenzonitrile and 2-(4-bromophenyl)propan-1-ol were used as the starting material to obtain the product with reference to the preparation route of Example 272.

¹H NMR(400 MHz, DMSO-*d*₆) δ 8.45 (d, *J* = 8.8 Hz, 1H), 8.37 (s, 2H), 7.54 (dd, *J* = 8.8 Hz, 1.6 Hz, 1H), 7.43 (d, *J* = 8.4 Hz, 2H), 7.17 (d, *J* = 8.4 Hz, 2H), 6.60 (s, 1H), 4.72 (t, *J* = 4.8 Hz, 1H), 3.71 (s, 3H), 3.55-3.60 (m, 1H), 3.46-3.52 (m, 1H), 2.87-2.96 (m, 1H), 1.24 (d, *J* = 7.2 Hz, 3H).

### Example 347. methyl 8-amino-1-(4-(1-hydroxyethyl)phenyl)-2-oxo-7-(1,2,3-triazol-1-yl) pyrido [2,3 -b]pyridin-3 -carboxylate

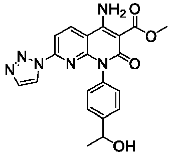

2-Bromo-7-(1,2,3-triazol-1-yl)nicotinonitrile and 1-(4-aminophenyl)ethan-1-one were used as the starting material to obtain the product with reference to the preparation route of Example 120 and Example 122.

¹H NMR(400 MHz, CDCl₃+CD₃OD) δ 8.69 (d, *J* = 8.8 Hz, 1H), 7.99 (d, *J* = 8.8 Hz, 1H), 7.82 (s, 1H), 7.65 (s, 1H), 7.51 (d, J = 8.4 Hz, 2H), 7.17 (d, *J* = 8.4 Hz, 2H), 4.93 *(q, J=* 6.8 Hz, 1H), 3.84 (s, 3H), 1.50 (d, *J* = 6.8 Hz, 3H).

### Example 348. methyl 4-amino-(1-(4-(1R)-1-hydroxyethyl)phenyl)-7-(trifluoromethyl)-2-oxo-pyrido[2,3-b]pyridin-3-carboxylate

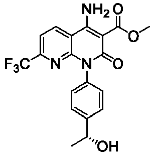

According to the preparation route of scheme 6 and 8, 2-chloro-3-cyano-6-(trifluoromethyl) pyridine and 4-(1-(*R)*-*(*(*tert*-butyldimethylsilyl)oxy)ethyl)aniline were used as the starting material to obtain the product with reference to the preparation route of Example 81 and step D of Example 340.

¹H NMR(400 MHz, DMSO-*d*₆) δ 8.90 (d, *J =* 8.0 Hz, 1H), 8.42 (s, 2H), 7.74 (d, *J =* 8.0 Hz, 1H), 7.41 (d, *J* = 6.8 Hz, 2H), 7.11 (d, *J* = 8.4 Hz, 2H), 5.26 *(d, J* = 4.4 Hz, 1H), 4.75-4.82 (m, 1H), 3.72 (s, 3H), 1.36 (d, *J* = 6.8 Hz, 3H).

### Example 349. methyl 4-amino-1-(6-(1-hydroxyethyl)pyridin-3-yl)-2-oxo-7-(trifluoromethyl)-1,2-dihydro-1,8-naphthyridine-3-carboxylate

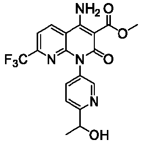

According to the preparation route of scheme 7, 2-chloro-6-(trifluoromethyl)nicotinonitrile and 1-(5-aminopyridin-2-yl)ethan-1-one were used as the starting material to obtain the product with reference to the preparation route of Example 120 and 122.

¹H NMR(400 MHz, CDCl₃) δ 8.41 (d, *J =* 2.4 Hz, 1H), 8.21 (d, *J =* 8.4 Hz, 1H), 7.66-7.96 (brs, 2H), 7.61 (dd, *J =* 8.4 Hz, 2.4 Hz, 1H), 7.53 (d, *J =* 8.0 Hz, 1H), 7.44 (d, *J =* 8.4 Hz, 1H), 5.00 (q, *J* = 6.4 Hz, 1H), 3.98-4.20 (brs, 1H), 3.93 (s, 3H), 1.56 (d, *J =* 6.4 Hz, 3H).

### Example 350. methyl 4-amino-7-(cyclopropylamino)-1-(4-(1-hydroxyethyl)phenyl)-2-oxo-1,2-dihydro-1,8-naphthyridine-3 -carboxylate

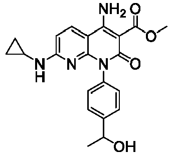

According to the preparation route of scheme 7, 2-chloro-6-(cyclopropylamino) nicotinonitrile and 1-(4-aminophenyl)ethan-1-one were used as the starting material to obtain the product with reference to the preparation route of Example 165.

¹H NMR(400 MHz, DMSO-*d*₆) δ 8.04-8.44 (m, 3H), 7.25-7.52 (m, 3H), 7.01 (d, *J =* 8.0 Hz, 2H), 6.20-6.56 (m, 1H), 5.19 (d, *J =* 4.8 Hz, 1H), 4.71-4.77 (m, 1H), 3.63 (s, 3H), 2.11-2.32 (m, 1H), 1.33 (d, *J* = 6.4 Hz, 3H), 0.08-0.66 (m, 4H).

### Example 351. methyl-d₃ (R)-4-amino-7-bromo-1-(4-(1-hydroxyethyl)phenyl)-2-oxo-1,2-dihydroquinolin-3-carboxylate

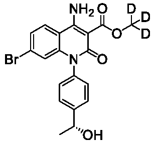

Methyl *(R)*-4-amino-7-bromo-1-(4-(1-hydroxyethyl)phenyl)-2-oxo-1,2-dihydroquinoline-3-carboxylate was used as the starting material to obtain the product with reference to the preparation route of Example 331.

¹H NMR (400 MHz, DMSO-*d*₆) δ 8.36 (br, 2H), 8.15 (d, *J* = 8.8 Hz, 1H), 7.49-7.56 (m, 2H), 7.38-7.49 (m, 1H), 7.15-7.18 (m, 2H), 6.47(d, J=2.0Hz, 1H), 5.31 (d, *J* = 4.4 Hz, 1H), 4.81 (t, *J*=5.2Hz,1H), 5.73 (d, *J =* 6.8 Hz, 3H).

### Example 352. methyl 4-amino-7-bromo-1-(6-(1-hydroxyethyl)pyridin-3-yl)-2-oxo-1,2-dihydroquinoline-3 -carboxylate

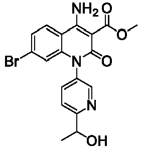

According to the preparation route of Example 120 and 122, 4-bromo-2-fluorobenzonitrile and 1-(5-aminopyridin-2-yl)ethan-1-one were used as the starting material to obtain the product.

¹H NMR(400 MHz, DMSO-*d*₆) δ 8.47 (s, 2H), 8.36 (s, 1H), 8.19 (d, *J* = 8.8 Hz, 1H), 7.74-7.78 (m, 1H), 7.68-7.72 (m, 1H), 7.44 (dd, *J =* 8.8 Hz, 2.0 Hz, 1H), 6.47 (s, 1H), 5.50-5.52 (m, 1H), 4.78-4.85 (m, 1H), 3.70 (s, 3H), 1.41-1.45 (m, 3H).

### Example 353. methyl 4-amino-(1-(4-(1R)-1-hydroxyethyl)phenyl)-7-cyclopropyl-2-oxo-1,2-dihydroquinoline-3 -carboxylate

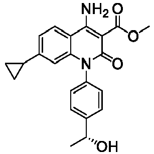

Under nitrogen protection, Example 340 (25 mg), cyclopropylboronic acid (20 mg), palladium (II) acetate (3 mg), tricyclohexyl phosphine (20 mg) and potassium phosphate (60 mg) were added to toluene (10 mL) and water (1 mL), and the mixture was heated to 100 °C and stirred for 12 hours. The reaction solution was cooled to room temperature and filtered, to the filtrate was added water (20 mL), the mixture was extracted with dichloromethane (20 mL*3), the extract was dried over anhydrous sodium sulphate and concentrated under reduced pressure, and the residue was purified through column chromatography (dichloromethane/methanol = 30:1) to obtain the product (16 mg).

¹H NMR(400 MHz, DMSO-*d*₆) δ 8.32 (s, 2H), 8.07 (d, *J* = 8.8 Hz, 1H), 7.47-7.54 (m, 2H), 7.11-7.13 (m, 2H), 6.80 (d, *J* = 8.8 Hz, 1H), 6.13 (s, 1H), 5.29 (d, *J* = 4.4 Hz, 1H), 4.78-4.85 (m, 1H), 3.67 (s, 3H), 1.68-1.74 (m, 1H), 1.38 (d, *J* = 6.8 Hz, 3H), 0.86-0.92 (m, 2H), 0.52-0.57 (m, 2H).

### Example 354. methyl (R)-4-amino-7-(difluoromethoxy)-1-(4-(1-hydroxyethyl)phenyl)-2-oxo-1,2-dihydroquinoline-3-carboxylate

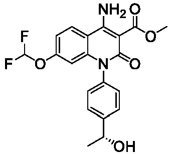

According to the preparation route of scheme 8, compound 4-difluoromethoxy-2-fluorobenzonitrile and *(R)*-4-(1-((*tert*-butyldimethylsilyl)oxy)ethyl)aniline were used as the starting material to obtain the product with reference to the preparation route of Example 340.

¹H NMR (400 MHz, DMSO-*d*₆) δ 8.38 (s, 2H), 8.29 *(d, J* = 8.8 Hz, 1H), 7.48-7.55 (m, 2H), 7.21 *(t, J =* 73.2 Hz, 1H), 7.14-7.16 (m, 2H), 7.04 (dd, *J* = 8.8 Hz, 2.4 Hz, 1H), 6.00 (d, *J* = 2.4 Hz, 1H), 5.29 (d, *J* = 4.0 Hz, 1H), 4.78-4.84 (m, 1H), 3.69 (s, 3H), 1.37 (d, *J* = 6.4 Hz, 3H).

### Example 355. methyl 4-amino-1-(4-(1-hydroxyethyl)phenyl)-7-(1H-imidazol-1-yl)-2-oxo-1,2-dihydro-1,8-naphthyridine-3 -carboxylate

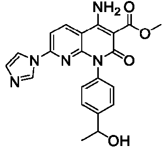

According to the preparation route of scheme 7, 2-chloro-6-(*1H*-imidazol-1-yl)nicotinonitrile and 1-(4-aminophenyl)ethan-1-one were used as the starting material to obtain the product with reference to the preparation route of Example 120 and 122.

¹H NMR(400 MHz, DMSO-*d*₆) δ 8.80 (d, *J* = 8.8 Hz, 1H), 8.43 (s, 2H), 8.10 (s, 1H), 7.68 (d, *J* = 8.8 Hz, 1H), 7.53 (s, 1H), 7.46 (d, *J* = 7.6 Hz, 2H), 7.15 (d, *J* = 8.4 Hz, 2H), 7.02 (s, 1H), 5.28 (d, *J* = 4.8 Hz, 1H), 4.79-4.85 (m, 1H), 3.72 (s, 3H), 1.38 (d, *J* = 6.4 Hz, 3H).

### Example 356. methyl 8-amino-3-cyclopropyl-5-(4-(1-hydroxyethyl)phenyl)-6-oxo-pyrido[3,2-b]pyrazine-3-carboxylate

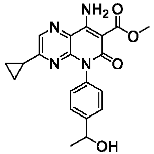

According to the preparation route of scheme 6, 3,5-dichloropyrazine-2-carbonitrile and 1-(4-aminophenyl)ethan-1-one were used as the starting material to obtain the product with reference to the preparation route of Example 81, Example 122 and Example 353.

¹H NMR(400 MHz, CDCl₃+CD₃OD) δ 8.27 (s, 1H), 7.42 (d, *J* = 7.6 Hz, 2H), 7.06 (d, *J* = 8.8 Hz, 2H), 4.87 (d, *J =* 6.8 Hz, 1H), 3.83 (s, 3H), 1.95-2.03 (m, 1H), 1.45 (d, *J =* 6.4 Hz, 3H), 0.90-0.95 (m, 2H), 0.66-0.70 (m, 2H).

### Example 357. methyl (R)-4-amino-7-(difluoromethyl)-1-(4-(1-hydroxyethyl)phenyl)-2-oxo-1,2-dihydroquinoline-3 -carboxylate

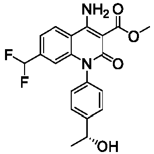

According to the preparation route of scheme 8, compound 4-difluoromethyl-2-fluorobenzonitrile and *(R)*-4-(1-((*tert*-butyldimethylsilyl)oxy)ethyl)aniline were used as the starting material to obtain the product with reference to the preparation route of Example 340.

¹H NMR (400 MHz, DMSO-*d*₆) δ 8.30-8.42 (m, 3H), 7.49-7.56 (m, 2H), 7.36 *(d, J=* 8.0 Hz, 1H), 7.15-7.18 (m, 2H), 6.97 (t, *J* = 56.0 Hz, 1H), 6.57 (s, 1H), 5.32 (d, *J =* 4.4 Hz, 1H), 4.79-4.85 (m, 1H), 3.70 (s, 3H), 1.38 (d, *J* = 6.4 Hz, 3H).

### Example 358. methyl 4-amino-7-(1,1-difluoroethyl)-1-(4-((1R)-1-hydroxyethyl)phenyl)-2-oxo-1,2-dihydroquinoline-3-carboxylate

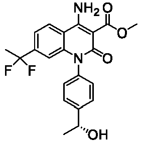

According to the preparation route of scheme 8, 4-(1,1-difluoroethyl)-2-fluorobenzonitrile and (*R*)4-(1-((*tert*-butyldimethylsilyl)oxy)ethyl)aniline were used as the starting material to obtain the product with reference to the preparation route of Example 340.

¹H NMR(400 MHz, CDCl₃+CD₃OD) δ 8.01 (d, *J* = 8.8 Hz, 1H), 7.51-7.55 (m, 2H), 7.27 (dd, *J* = 8.8 Hz, 1.6 Hz, 1H), 7.14 (d, *J* = 8.4 Hz, 2H), 6.66 (s, 1H), 4.90 (q, *J* = 6.4 Hz, 1H), 3.82 (s, 3H), 1.73 (t, *J* = 18.4 Hz, 3H), 1.48 (d, *J* = 6.4 Hz, 3H).

### Example 359. methyl 4-amino-1-(4-(1-hydroxyethyl)phenyl)-2-oxo-7-(1H-1,2,4-triazol-1-yl)-1,2-dihydro-1,8-naphthyridine-3-carboxylate

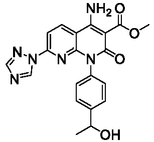

2-bromo-7-(*1H*-1,2,4-triazol-1-yl)nicotinonitrile and 1-(4-aminophenyl)ethan-1-one were used as the starting material to obtain the product with reference to the preparation route of Example 120 and Example 122.

¹H NMR (400 MHz, DMSO-*d*₆) δ 8.88 (d, *J* = 8.8 Hz, 1H), 8.46 (s, 2H), 8.30 (s, 1H), 8.27 (s, 1H), 7.65 *(d, J=* 8.8 Hz, 1H), 7.47 *(d, J=* 7.2 Hz, 2H), 7.18 *(d, J=* 8.4 Hz, 2H), 5.00-5.48 (brs, 1H), 4.82 (q, *J* = 6.4 Hz, 1H), 3.73 (s, 3H), 1.38 (d, *J* = 6.4 Hz, 3H).

### Example 360. methyl 4-amino-7-cyclopropyl-1-(4-(1-hydroxyethyl)phenyl)-2-oxo-1,2-dihydro-1,8-naphthyridine-3-carboxylate

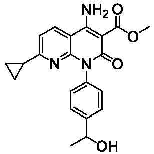

According to the preparation route of scheme 7, 2-chloro-6-cyclopropylnicotinonitrile and 1-(4-aminophenyl)ethan-1-one were used as the starting material to obtain the product with reference to the preparation route of Example 165.

¹H NMR(400 MHz, CDCl₃) δ 7.80 (d, *J* = 8.0 Hz, 1H), 7.46 (d, *J* = 8.4 Hz, 2H), 7.13 (d, *J* = 8.4 Hz, 2H), 7.02 (d, *J* = 8.0 Hz, 1H), 4.98 (q, *J* = 6.8 Hz, 1H), 3.88 (s, 3H), 1.86-1.92 (m, 1H), 1.54 (d, *J* = 6.4 Hz, 3H), 0.81-0.86 (m, 2H), 0.64-0.68 (m, 2H).

### Example 361. methyl 4-amino-7-(trifluoromethyl)-1-(4-aminophenyl)-2-oxo-pyrido[3,2-b] pyridin-3 -carboxylate

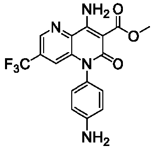

According to the preparation route of scheme 7, 3-chloro-5-(trifluoromethyl)pyridin-2-carbonitrile and 4-nitroaniline were used as the starting material to obtain the product with reference to the preparation route of Example 165.

¹H NMR(400 MHz, DMSO-*d*₆) δ 8.77 (s, 1H), 8.21 (s, 2H), 7.05 (s, 1H), 6.88 (d, *J* = 8.4 Hz, 2H), 6.68 *(d, J=* 8.4 Hz, 2H), 5.41 (s, 2H), 3.73 (s, 3H).

### Example 362. 4-amino-7-(trifluoromethyl)-1-(4-aminophenyl)-2-oxo-pyrido[3,2-b]pyridin-3-carboxamide

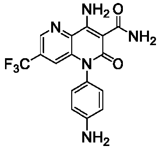

According to the preparation route of scheme 7, 3-chloro-5-(trifluoromethyl)pyridin-2-carbonitrile and 4-nitroaniline were used as the starting material to obtain the product with reference to the preparation route of Example 165.

¹H NMR(400 MHz, DMSO-*d*₆) δ 10.33 (s, 1H), 9.32 (s, 1H), 8.83 (s, 1H), 8.33 (s, 1H), 7.42 (s, 1H), 7.08 (s, 1H), 6.93 (d, *J* = 8.0 Hz, 2H), 6.69 (d, *J* = 8.0 Hz, 2H), 5.45 (s, 2H).

### Example 363. methyl 8-amino-5-(4-(1-hydroxyethyl)phenyl)-3-trifluoromethyl-6-oxo-pyrido [3,2-b]pyrazine-3-carboxylate

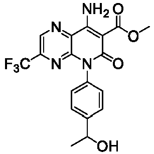

According to the preparation route of scheme 6, 3-chloro-5-trifluoromethylpyrazine-2-carbonitrile and 1-(4-aminophenyl)ethan-1-one were used as the starting material to obtain the product with reference to the preparation route of Example 81 and Example 122.

¹H NMR(400 MHz, CDCl₃+CD₃OD) δ 8.70 (s, 1H), 7.47-7.50 (m, 2H), 7.11 (d, *J* = 8.4 Hz, 2H), 4.88 (q, *J* = 6.8 Hz, 1H), 3.85 (s, 3H), 1.46 (d, *J* = 6.8 Hz, 3H).

### Example 364. methyl 4-amino-7-bromo-8-fluoro-1-(4-(1-hydroxyethyl)phenyl)-2-oxo-1,2-dihydroquinoline-3 -carboxylate

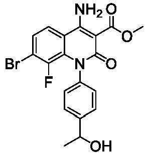

2-Amino-4-bromo-3-fluorobenzonitrile and 1-(4-iodophenyl)ethan-1-one were used as the starting material to obtain the product with reference to the preparation route of Example 120 and 122.

¹H NMR (400 MHz, CD₃OD) δ 7.84-7.88 (m, 1H), 7.66 (d, *J =* 8.4 Hz, 2H), 7.58 (d, *J =* 8.0 Hz, 1H), 7.43 *(d, J=* 8.4 Hz, 2H), 4.43 *(q, J=* 6.8 Hz, 1H), 3.82 (s, 3H), 1.62 *(d, J=* 6.4 Hz, 3H).

### Example 365. methyl (R)-4-amino-1-(4-(1-hydroxyethyl)phenyl)-7-methoxy-2-oxo-1,2-dihydroquinoline-3 -carboxylate

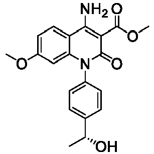

According to the preparation route of scheme 8, compound 2-fluoro-4-methoxybenzonitrile and *(R)*-4-(1-((*tert*-butyldimethylsilyl)oxy)ethyl)aniline were used as the starting material to obtain the product with reference to the preparation route of Example 340.

¹H NMR(400 MHz, DMSO-*d*₆) δ 8.37 (s, 2H), 8.16 (d, *J =* 8.8 Hz, 1H), 7.46-7.54 (m, 2H), 7.11-7.13 (m, 2H), 6.85 (dd, *J* = 8.8 Hz, 2.0 Hz, 1H), 5.75 (d, *J=* 2.0 Hz, 1H), 5.28 (d, *J* = 4.4 Hz, 1H), 4.77-4.84 (m, 1H), 3.67 (s, 3H), 3.62 (s, 3H), 1.37 (d, *J* = 6.4 Hz, 3H).

### Example 366. methyl 4-amino-7-(difluoromethyl)-1-(4-(1-hydroxyethyl)phenyl)-2-oxo-1,2-dihydro-1,8-naphthyridine-3-carboxylate

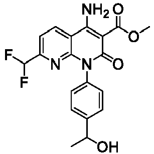

Compound 2-chloro-6-(difluoromethyl)nicotinonitrile and 1-(4-atninophenyl)ethan-1-one were used as the starting material to obtain the product with reference to the preparation route of Example 120 and 122.

¹H NMR (400 MHz, CD₃OD) δ 8.70 (d, *J* = 8.0 Hz, 1H), 7.47-7.54 (m, 3H), 7.17 (d, *J* = 8.4 Hz, 2H), 6.36 (t, *J* = 54.8 Hz, 1H), 4.91 (q, *J* = 6.4 Hz, 1H), 3.83 (s, 3H), 1.49 (d, *J=* 6.4 Hz, 3H).

### Example 367. 3-acetyl-4-amino-1-(4-((1R)-1-hydroxyethyl)phenyl)-7-(difluoromethyl)-2-oxo-1,2-dihydroquinolin-2-one

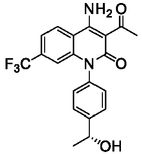

According to the preparation route of scheme 6 and 8, 2-cyano-5-(trifluoromethyl)aniline and *(R)*-(1-(4-bromophenyl)ethoxy)(*tert*-butyl)dimethylsilane were used as the starting material, with dimethyl malonate replaced by ethyl acetoacetate for the reaction, to obtain the product with reference to the preparation route of Example 81 and step D of Example 340.

¹H NMR (400 MHz, CD₃OD) δ 8.30 (d, *J* = 8.5 Hz, 1H), 7.66 (dd, *J* = 7.5, 2.9 Hz, 1H), 7.63-7.58 (m, 1H), 7.47-7.42 (m, 1H), 7.27-7.19 (m, 2H), 6.75 (s, 1H), 4.95 (q, *J* = 6.4 Hz, 1H), 2.63 (s, 3H), 1.51 (d, *J* = 6.5 Hz, 3H).

### Example 368. 4-amino-3-(1-hydroxyethyl)-1-(4-((1R)-1-hydroxyethyl)phenyl)-7-(difluoromethyl)-2-oxo-1,2-dihydroquinolin-2-one

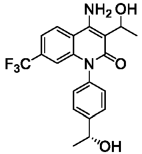

The product 3-acetyl-4-amino-1-(4-(*(1R)*-1-hydroxyethyl)phenyl)-7-(difluoromethyl)-2-oxo-1,2-dihydroquinolin-2-one obtained in Example 367 (50 mg) was dissolved in methanol (10 mL), the mixture was cooled to 0 °C, sodium boronhydride (10 mg) was added, and the mixture was stirred at room temperature for 6 hours. Water (10 mL) was added to quench the reaction, the mixture was extracted with dichloromethane, the extract was washed with brine, dried over anhydrous sodium sulphate, and filtered, and the filtrate was concentrated under reduced pressure to obtain the product (43 mg).

¹H NMR (400 MHz, CDCl₃) δ 7.68 (d, *J =* 8.5 Hz, 1H), 7.65-7.61 (m, 1H), 7.59 -7.54 (m, 1H), 7.44-7.40 (m, 1H), 7.23 (d, *J =* 8.0 Hz, 2H), 6.92 (s, 1H), 5.84 (s, 2H), 5.21 (q, *J =* 6.6 Hz, 1H), 5.08-5.00 (m, 1H), 1.56 (d, *J =* 6.5 Hz, 3H), 1.51 (d, *J =* 6.7 Hz, 3H).

### Example 369. methyl 4-atnino-1-(4-amino-2-methylphenyl)-7-iodo-2-oxo-1,2-dihydroquinoline-3 -carboxylate

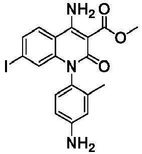

According to the preparation route of scheme 7, 2-fluoro-4-iodobenzonitrile and 2-methyl-4-nitroaniline were used as the starting material to obtain the product with reference to the preparation route of Example 165.

¹H NMR (400 MHz, CD₃OD) δ 7.82 (d, *J =* 8.8 Hz, 1H), 7.56 (dd, *J =* 8.8 Hz, 1.6 Hz, 1H), 6.94 (d, *J =* 1.6 Hz, 1H), 6.80 (d, *J=* 8.4 Hz, 1H), 6.77 (d, *J* = 2.8 Hz, 1H), 6.71 (dd, *J =* 8.4 Hz, 2.8 Hz, 1H), 3.81 (s, 3H), 1.84 (s, 3H).

### Example 370. methyl 4-amino-1-(6-aminopyridin-3-yl)-7-iodo-2-oxo-1,2-dihydroquinoline-3-carboxylate

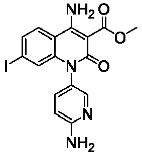

According to the preparation route of scheme 7, 2-fluoro-4-iodobenzonitrile and 6-nitropyridin-3-amine were used as the starting material to obtain the product with reference to the preparation route of Example 165.

¹H NMR (400 MHz, DMSO-*d*₆) δ 8.33 (s, 2H), 7.95 (d, *J =* 8.4 Hz, 1H), 7.69 (d, *J* = 2.4 Hz, 1H), 7.54 (d, *J* = 8.4 Hz, 1H), 7.22 (dd, *J =* 8.8 Hz, 2.4 Hz, 1H), 6.87 (s, 1H), 6.57 (d, *J* = 8.8 Hz, 1H), 6.27 (s, 2H), 3.69 (s, 3H).

### Example 371. methyl-d₃ 4-amino-1-(4-(1-hydroxyethyl-2-d)phenyl)-2-oxo-7-(trifluoromethyl)-1,2-dihydro-1,8-naphthyridine-3-carboxylate

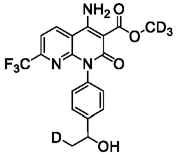

2-Chloro-6-(trifluoromethyl)pyridin-3-carbonitrile and 1-(4-aminophenyl)ethan-1-one were used as the starting material to obtain the product with reference to the preparation route of Example 331.

¹H NMR (400 MHz, CD₃OD) δ 8.75 (d, *J* = 8.4 Hz, 1H), 7.63 (d, *J* = 8.4 Hz, 1H), 7.45-7.58 (m, 2H), 7.18 (d, *J* = 8.4 Hz, 2H), 4.90-4.93 (m, 1H), 1.43-1.50 (m, 2H).

### Example 372. methyl 4-amino-7-(difluoromethoxy)-1-(4-(1-(R)-hydroxyethoxy)phenyl)-2-oxo-1,2-dihydro-1,8-naphthyridine-3-carboxylate

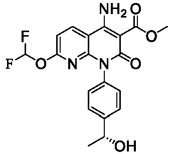

Methyl 2-chloro-6-(difluoromethoxy)nicotinate and *(R)*-4-(1-((*tert*-butyldimethylsilyl)oxy) ethyl)aniline were used as the starting material to obtain the product with reference to the preparation route of Example 400.

¹H NMR (400 MHz, CD₃OD+CDCl₃) δ 8.28 (d, *J* = 8.4 Hz, 1H), 7.36-7.46 (m, 2H), 7.06 (d, *J* = 7.6 Hz, 2H), 6.69 (t, *J* = 72.4 Hz, 1H), 6.64 (d, *J* = 8.4 Hz, 1H), 4.86 (q, *J* = 6.8 Hz, 1H), 3.80 (s, 3H), 1.43 (d, *J =* 6.8 Hz, 3H).

### Example 373. methyl 4-amino-1-(4-aminophenyl)-7-cyclopropyl-2-oxo-1,2-dihydro-1,8-naphthyridine-3 -carboxylate

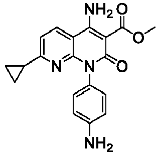

According to the preparation route of scheme 7, 2-chloro-6-cyclopropylnicotinonitrile and 4-nitroaniline were used as the starting material to obtain the product with reference to the preparation route of Example 165 and Example 147.

¹H NMR (400 MHz, DMSO-*d*₆) δ 8.42 (d, *J* = 8.2 Hz, 1H), 8.29 (s, 2H), 7.15 (d, *J* = 8.2 Hz, 1H), 6.69 (d, *J* = 8.4 Hz, 2H), 6.58 (d, *J* = 8.4 Hz, 2H), 5.13 (s, 2H), 3.70 (s, 3H).

### Example 374. methyl-d₃ 4-amino-1-(4-(1-(R)-hydroxyethyl)phenyl)-2-oxo-7-(trifluoromethyl)-1,2-dihydroquinoline-3 -carboxylate

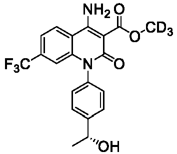

Methyl 4-amino-1-(4-(1-(*R)*-hydroxyethyl)phenyl)-2-oxo-7-(trifluoromethyl)-1,2-dihydroquinoline-3-carboxylate obtained in Example 272 was used as the starting material to obtain the product with reference to the preparation route of Example 311.

¹H NMR (400 MHz, DMSO-*d*₆) δ 8.45 (d, *J* = 8.5 Hz, 1H), 8.38 (s, 2H), 7.60-7.53 (m, 2H), 7.53-7.47 (m, 1H), 7.21 (td, *J* = 5.8, 2.8 Hz, 2H), 6.59 (s, 1H), 5.33 (d, *J* = 4.3 Hz, 1H), 4.82 (dd, *J* = 6.5, 4.8 Hz, 1H), 1.37 (d, *J* = 6.4 Hz, 3H).

### Example 375. methyl 4-amino-7-trifluoromethyl-1-(4-(1-hydroxyethyl) phenyl)-2-oxopyrido [3,2-b]pyridin-3 -carboxylate

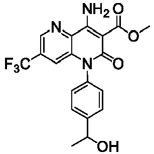

3-Chloro-5-(trifluoromethyl)pyridin-2-carbonitrile and 1-(4-aminophenyl)ethan-1-one were used as the starting material to obtain the product with reference to the preparation route of Example 120 and 122.

¹H NMR (400 MHz, CDCl₃) δ 8.82 (d, *J* = 2.0 Hz, 1H), 8.18 (d, *J* = 2.0 Hz, 1H), 7.59 (d, *J=* 8.8 Hz, 2H), 7.32 (s, 1H), 7.25 (d, *J* = 8.8 Hz, 2H), 3.78 (s, 3H), 3.56 (q, *J* = 6.8 Hz, 1H), 1.32 (d, *J* = 6.8 Hz, 3H).

### Example 376. methyl-d₃ 4-amino-7-(difluoromethoxy)-1-(4-(1-(R)-hydroxyethyl)phenyl)-2-oxo-1,2-dihydroquinoline-3-carboxylate

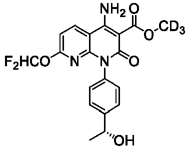

Methyl *(R)*-4-amino-7-(difluoromethoxy)-1-(4-(1-hydroxyethyl)phenyl)-2-oxo-1,2-dihydroquinoline-3-carboxylate obtained in Example 354 was used as the starting material to obtain the product with reference to the preparation route of Example 311.

¹H NMR (400 MHz, CD₃OD) δ 8.16 (d, *J =* 9.0 Hz, 1H), 7.67-7.50 (m, 2H), 7.21 (d, *J =* 8.4 Hz, 2H), 7.04 (dd, *J =* 9.0, 2.3 Hz, 1H), 7.00-6.56 (m, 1H), 6.21 (d, *J=* 2.4 Hz, 1H), 4.95 (t, *J =* 6.5 Hz, 1H), 1.50 (d, *J* = 6.5 Hz, 3H).

### Example 377. methyl 4-amino-7-cyclopropyl-1-(6-methylpyridin-3-yl)-2-oxopyrido[2,3-b] pyridin-3 -carboxylate

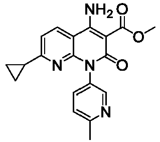

According to the preparation route of scheme 7, 2-bromo-6-cyclopropylnicotinonitrile and 6-methylpyridin-3-amine were used as the starting material to obtain the product with reference to the preparation route of Example 165.

¹H NMR (400 MHz, DMSO-*d*₆) δ 8.38-8.53 (m, 3H), 8.16 (d, *J* = 2.4 Hz, 1H), 7.48 (dd, *J* = 8.4 Hz, 2.4 Hz, 1H), 7.33 (d, *J* = 8.4 Hz, 1H), 7.20 (d, *J* = 8.0 Hz, 1H), 3.69 (s, 3H), 2.52 (s, 3H), 1.94-2.02 (m, 1H), 0.79-0.86 (m, 2H), 0.47-0.52 (m, 2H).

### Example 378. methyl 4-amino-1-(4-((1R)-1-hydroxyethyl)phenyl)-2-oxo-7-(trifluoro methoxy)-1,2-dihydroquinoline-3 -carboxylate

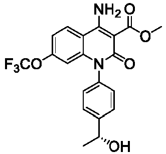

According to the preparation route of scheme 6, 2-amino-4-(trifluoromethoxy)benzonitrile and *(R)*-(1-(4-bromophenyl)ethoxy)(*tert*-butyl)dimethylsilane were used as the starting material to obtain the product with reference to the preparation route of Example 81 and 340.

¹H NMR (400 MHz, CDCl₃+CD₃OD) δ 8.17 (d, *J* = 9.2 Hz, 1H), 7.56-7.62 (m, 2H), 7.17-7.22 (m, 2H), 7.09 (d, *J =* 9.2 Hz, 1H), 6.34 (s, 1H), 4.94 (q, *J =* 6.8 Hz, 1H), 3.82 (s, 3H), 1.50 (d, *J* = 6.8 Hz, 3H).

### Example 379. methyl 4-amino-1-(4-aminophenyl)-2-oxo-7-(trifluoromethoxy)-1,2-dihydroquinoline-3 -carboxylate

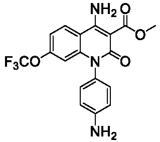

According to the preparation route of scheme 7, 4-(trifluoromethoxy)-2-aminobenzonitrile and 1-fluoro-4-nitrobenzene were used as the starting material to obtain the product with reference to the preparation route of Example 165 and Example 147.

¹H NMR (400 MHz, DMSO-*d*₆) δ 8.31 (d, *J* = 8.8 Hz, 1H), 8.29 (s, 2H), 7.17 (d, *J* = 8.8 Hz, 1H), 6.80 (d, *J* = 8.8 Hz, 2H), 6.67 (d, J = 8.8 Hz, 2H), 6.33 (s, 1H), 5.35 (s, 2H), 3.70 (s, 3H).

### Example 380. methyl 5-amino-2-(methylthio)-8-(6-methylpyridin-3-yl)-7-oxopyrido[2,3-d] pyrimidine-6-carboxylate

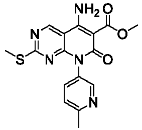

According to the preparation route of scheme 7, 4-chloro-2-methylthiopyrimidine-5-carbonitrile and 6-methylpyridin-3-amine were used as the starting material to obtain the product with reference to the preparation route of Example 165.

¹H NMR (400 MHz, DMSO-*d*₆) δ 9.29 (s, 1H), 8.64 (s, 2H), 8.27 (d, *J =* 2.4 Hz, 1H), 7.59 (dd, *J* = 8.0 Hz, 2.4 Hz, 1H), 7.37 (d, *J* = 8.0 Hz, 1H), 3.70 (s, 3H), 2.52 (s, 3H), 2.15 (s, 3H).

### Example 381. methyl 4-amino-1-(4-chlorophenyl)-2-oxo-7-((2,2,2-trifluoroethyl)amino)-1,2-dihydro-1,8-naphthyridine-3 -carboxylate

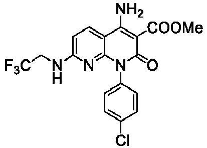

According to the preparation route of scheme 7, 2-chloro-6-(2,2,2-trifluoroethyl) aminonicotinonitrile and 4-chloroaniline were used as the starting material to obtain the product with reference to the preparation route of Example 165.

¹H NMR (400 MHz, DMSO-*d*₆) δ 8.39 (s, 2H), 8.25 (d, *J* = 8.9 Hz, 1H), 7.99 (s, 1H), 7.49 (d, *J* = 8.6 Hz, 2H), 7.16 (d, *J* = 8.6 Hz, 2H), 6.45 (d, *J* = 8.7 Hz, 1H), 3.68 (s, 3H).

### Example 382. methyl 4-amino-1-(6-methylpyridin-3-yl)-2-oxo-7-((2,2,2-trifluoroethyl) amino)-1,2-dihydro-1,8-naphthyridine-3-carboxylate

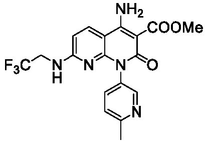

According to the preparation route of scheme 7, 2-chloro-6-(2,2,2-trifluoroethyl) aminonicotinonitrile and 6-methylpyridin-3-amine were used as the starting material to obtain the product with reference to the preparation route of Example 165.

¹H NMR (400 MHz, CDCl₃) δ 8.35 (d, *J* = 2.5 Hz, 1H), 7.72 (d, *J =* 8.7 Hz, 1H), 7.47 (dd, *J* = 8.1, 2.5 Hz, 1H), 7.28 (s, 1H), 6.35 (d, *J =* 8.7 Hz, 1H), 5.11 (s, 1H), 3.88 (s, 3H), 3.81 (s, 1H), 3.63 (s, 1H), 2.62 (s, 3H).

### Example 383. methyl 5-amino-2-methyl-8-(4-aminophenyl)-7-oxopyrido[2,3-d]pyrimidine-6-carboxylate

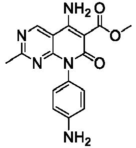

According to the preparation route of scheme 7, 2-methyl-4-aminopyrimidine-5-carbonitrile and 1-fluoro-4-nitrobenzene were used as the starting material to obtain the product with reference to the preparation route of Example 165 and Example 147.

¹H NMR (400 MHz, DMSO-*d₆*) δ 9.34 (s, 1H), 8.44 (s, 2H), 6.73 (d, *J* = 8.4 Hz, 2H), 6.58 (d, *J* = 8.4 Hz, 2H), 5.18 (s, 2H), 3.70 (s, 3H), 2.41 (s, 3H).

### Example 384. methyl 4-atnino-1-(1-chloroisoquinolin-5-yl)-7-(difluoromethoxy)-2-oxo-1,2-dihydroquinoline-3-carboxylate

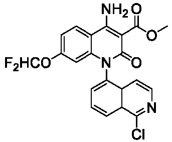

According to the preparation route of scheme 8, 4-difluoromethoxy-2-fluorobenzonitrile and 1-chloroisoquinolin-5-amine were used as the starting material to obtain the product with reference to the preparation route of Example 340.

¹H NMR (400 MHz, CD₃OD) δ 8.59 (dd, *J =* 8.5, 0.9 Hz, 1H), 8.25 (d, *J =* 9.1 Hz, 1H), 8.19 (dd, *J =* 5.9, 0.7 Hz, 1H), 8.05-7.92 (m, 1H), 7.86 (dt, *J =* 7.4, 0.9 Hz, 1H), 7.29 (dt, *J =* 5.8, 0.8 Hz, 1H), 7.07 (ddd, *J =* 9.1, 2.4, 0.7 Hz, 1H), 6.98-6.51 (m, 1H), 5.98 (d, *J =* 2.4 Hz, 1H), 3.80 (d, *J =* 0.7 Hz, 3H).

### Example 385. methyl 4-atnino-1-(1-chloroisoquinolin-5-yl)-7-hydroxy-2-oxo-1,2-dihydroquinoline-3 -carboxylate

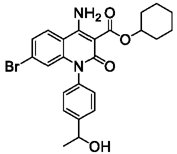

According to the preparation route of scheme 8, 4-hydroxy-2-fluorobenzonitrile and 1-chloroisoquinolin-5-amine were used as the starting material to obtain the product with reference to the preparation route of Example 340.

¹H NMR (400 MHz, CD₃OD) δ 8.56 (dt, *J =* 8.7, 1.1 Hz, 1H), 8.17 (d, *J =* 5.9 Hz, 1H), 8.07 - 7.90 (m, 2H), 7.83 (dd, *J =* 7.4, 1.1 Hz, 1H), 7.28 (dd, *J =* 5.9, 0.9 Hz, 1H), 6.71 (dd, *J =* 9.0, 2.3 Hz, 1H), 5.64 (d, *J =* 2.3 Hz, 1H), 3.78 (s, 3H).

### Example 386. cyclohexyl 4-amino-7-bromo-1-(4-(1-hydroxyethyl)phenyl)-2-oxo-1,2-dihydroquinolin-3 -carboxylate

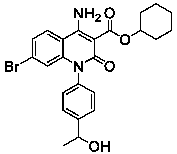

Example 200 (92 mg) was dissolved in tetrahydrofuran (5 mL), a solution of lithium hydroxide (28 mg) in water (5 mL) was added, and the mixture was heated to 50 °C and stirred overnight. The reaction solution was cooled to room temperature and concentrated under reduced pressure to remove the tetrahydrofuran, to the aqueous phase was added water (30 mL), the mixture was washed with dichloromethane (30 mL*3), to the aqueous phase was added 3 mol/L hydrochloric acid (10 mL), a white solid precipitated, and the mixture was filtered and washed with water to obtain a white solid (80 mg). The white solid (80 mg) was dissolved in *N*,*N*-dimethylformamide (5 mL), 2-(7-aza-*1H*-benzotriazole-1-yl)-1,1,3,3-tetratnethyluronium hexafluorophosphate (114 mg) and *N*,*N-*diisopropylethylamine (78 mg) were added, and the mixture was heated to 50 °C and reacted overnight. The reaction solution was cooled to room temperature, water (30 mL) was added, the mixture was extracted with ethyl acetate (30 mL*3), the extracts were combined, dried over anhydrous sodium sulphate, and concentrated under reduced pressure, and the residue was purified through column chromatography (dichloromethane/methanol = 20:1) to obtain the product (60 mg).

¹H NMR (400 MHz, CDCl₃) δ 7.44-7.61 (m, 5H), 7.25-7.28 (m, 1H), 7.19-7.24 (m, 2H), 6.71 (s, 1H), 4.92-5.02 (m, 2H), 1.94-2.05 (m, 3H), 1.72-1.82 (m, 2H), 1.51-1.64 (m, 6H), 1.17-1.36 (m, 2H).

### Example 387. methyl 8-amino-5-(4-((1R)-1-hydroxyethyl)phenyl)-3-isopropoxy-6-oxo-5,6-dihydropyrido [2,3-b]pyrazine-7-carboxylate

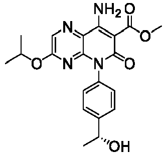

3,5-Dichloropyrazine-2-carbonitrile (522 mg) was dissolved in *N*,*N*-dimethylformamide (10 mL), isopropanol (180 mg) and sodium hydride (144 mg, 60% scattered in mineral oil) were added, and the mixture was stirred at room temperature for 24 hours. Water (10 mL) was added to quench the reaction, the mixture was extracted with dichloromethane (30 mL*3), the extract was dried over anhydrous sodium sulphate and concentrated under reduced pressure, and the residue was purified through column chromatography (petroleum ether/ethyl acetate = 10:1) to obtain a colorless liquid 3-chloro-5-isopropoxypyrizine-2-carbonitrile (121 mg).

According to the preparation route of scheme 6, 3-chloro-5-isopropoxypyrazine-2-carbonitrile and 4-(1-(*R*)-((*tert*-butyldimethylsilyl)oxy)ethyl)aniline were used as the starting material to obtain the product with reference to the preparation route of Example 81 and 340.

¹H NMR (400 MHz, CDCl₃) δ 8.78-9.18 (brs, 1H), 7.87 (s, 1H), 7.46 (d, *J =* 8.4 Hz, 2H), 7.18-7.30 (brs, 1H), 7.17 (d, *J* = 8.4 Hz, 2H), 4.96 (q, *J* = 6.8 Hz, 1H), 4.61-4.70 (m, 1H), 3.88 (s, 3H), 2.06-2.36 (brs, 1H), 1.49 (d, *J* = 6.8 Hz, 3H), 1.12 (d, *J* = 6.4 Hz, 6H).

### Example 388. methyl 4-amino-1-(4-chlorophenyl)-7-isopropoxy-2-oxo-1,2-dihydro-1,8-naphthyridine-3 -carboxylate

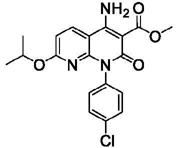

According to the preparation route of scheme 7, 2-chloro-6-isopropoxynicotinonitrile and 4-chloroaniline were used as the starting material to obtain the product with reference to the preparation route of Example 165.

¹H NMR (400 MHz, CDCl₃) δ 7.97 (d, *J* = 8.8 Hz, 1H), 7.38 (d, *J =* 8.5 Hz, 2H), 7.10 (d, *J* = 8.5 Hz, 2H), 6.45 (d, *J* = 8.7 Hz, 1H), 4.56 (m, 1H), 3.81 (s, 3H), 1.04 (d, *J* = 6.2 Hz, 6H).

### Example 389. methyl 4-amino-1-(6-atnino-2-methylpyridin-3-yl)-7-iodo-2-oxo-1,2-dihydroquinoline-3 -carboxylate

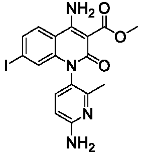

According to the preparation route of scheme 7, 2-fluoro-4-iodobenzonitrile and 2-methyl-6-nitropyridin-3-amine were used as the starting material to obtain the product with reference to the preparation route of Example 165.

¹H NMR (400 MHz, CD₃OD) δ 7.85 (d, *J =* 8.4 Hz, 1H), 7.61 (dd, *J =* 8.4 Hz, 2.0 Hz, 1H), 7.21 (d, *J =* 8.4 Hz, 1H), 6.98 (d, *J =* 2.0 Hz, 1H), 6.60 (d, *J =* 8.4 Hz, 1H), 3.82 (s, 3H), 1.97 (s, 3H).

### Example 390. methyl 4-amino-1-(6-atnino-4-methylpyridin-3-yl)-7-iodo-2-oxo-1,2-dihydroquinoline-3 -carboxylate

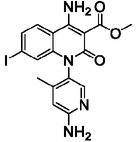

According to the preparation route of scheme 7, 2-fluoro-4-iodobenzonitrile and 4-methyl-6-nitropyridin-3-amine were used as the starting material to obtain the product with reference to the preparation route of Example 165.

¹H NMR (400 MHz, CD₃OD) δ 7.86 (d, *J =* 8.4 Hz, 1H), 7.63 (s, 1H), 7.62 (dd, *J =* 8.4 Hz, 1.6 Hz, 1H), 7.00 (d, *J* = 1.6 Hz, 1H), 6.66 (s, 1H), 3.82 (s, 3H), 1.88 (s, 3H).

### Example 391. methyl-d₃ 4-amino-1-(4-aminophenyl)-2-oxo-7-(trifluoromethoxy)-1,2-dihydroquinoline-3 -carboxylate

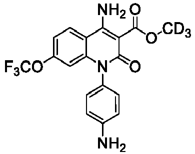

Methyl 4-amino-1-(4-aminophenyl)-2-oxo-7-(trifluoromethoxy)-1,2-dihydroquinoline-3-carboxylate obtained in Example 379 was used as the starting material to obtain the product with reference to the preparation route of Example 331.

¹H NMR (400 MHz, DMSO-*d₆*) δ 8.31 (d, J = 8.8 Hz, 1H), 8.29 (s, 2H), 7.17 (d, J = 8.8 Hz, 1H), 6.80 (d, J = 8.8 Hz, 2H), 6.67 (d, J = 8.8 Hz, 2H), 6.32 (s, 1H), 5.36 (s, 2H).

### Example 392. 3-(acetyl-d₃)-4-amino-1-(4-((1R)-1-hydroxyethyl)phenyl)-7-(difluoromethyl)-2-oxo-1,2-dihydroquinolin-2-one

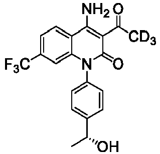

3-Acetyl-4-amino-1-(4-(*(1R)*-1-hydroxyethyl)phenyl)-7-(difluoromethyl)-2-oxo-1,2-dihydroq uinolin-2-one obtained in Example 367 (20 mg) was dissolved in 1,4-dioxane (10 mL), heavy water (5 mL) and sodium hydroxide (1 mg) were added, the mixture was stirred at room temperature for 12 hours and extracted 3 times with ethyl acetate (20 mL), and the extract was dried over anhydrous sodium sulphate and concentrated under reduced pressure to obtain the product as a white solid (15 mg).

¹H NMR (400 MHz, DMSO-*d₆*) δ 8.52 (d, *J* = 8.5 Hz, 1H), 7.62-7.54 (m, 2H), 7.54-7.48 (m, 1H), 7.24 (td, *J* = 6.7, 3.4 Hz, 2H), 6.62-6.57 (m, 1H), 5.32 (d, *J* = 4.3 Hz, 1H), 4.83 (m, 1H), 1.38 (d, *J* = 6.4 Hz, 3H).

### Example 393. methyl-d₃ 4-amino-1-(4-((1R)-1-hydroxyethyl)phenyl)-2-oxo-7-(trifluoromethoxy)-1,2-dihydroquinoline-3 -carboxylate

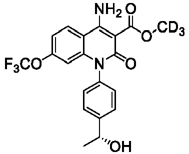

Methyl 4-amino-1-(4-(*(1R)*-1-hydroxyethyl)phenyl)-2-oxo-7-(trifluoromethoxy)-1,2-dihydroquinoline-3-carboxylate obtained in Example 378 was used as the starting material to obtain the product with reference to the preparation route of Example 331.

¹H NMR (400 MHz, CDCl₃) δ 7.52-7.80 (m, 5H), 7.19-7.23 (m, 2H), 7.04 (d, *J* = 8.4 Hz, 1H), 6.43 (d, *J* = 2.4 Hz, 1H), 4.98-5.05 (m, 1H), 1.99 (d, *J* = 2.8 Hz, 1H), 1.56 (d, *J* = 6.4 Hz, 3H).

### Example 394. methyl 4-amino-1-(6-aminopyridin-3-yl)-2-oxo-7-(trifluoromethoxy)-1,2-dihydroquinoline-3 -carboxylate

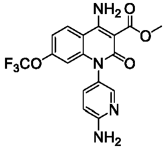

According to the preparation route of scheme 6, 4-(trifluoromethoxy)-2-bromobenzonitrile and 6-nitropyridin-3-amine were used as the starting material to obtain the product with reference to the preparation route of Example 378 and Example 147.

¹H NMR (400 MHz, DMSO-*d₆*) δ 8.39 (s, 2H), 8.35 (d, *J* = 9.2 Hz, 1H), 7.72 (d, *J* = 2.8 Hz, 1H), 7.25 (dd, *J* = 9.2 Hz, 2.8 Hz, 1H), 7.21 (d, *J* = 9.2 Hz, 1H), 6.57 (d, *J* = 8.4 Hz, 1H), 6.40 (s, 1H), 6.29 (s, 2H), 3.70 (s, 3H).

### Example 395. methyl 4-amino-1-(4-(1-d-1-hydroxyethyl)phenyl)-2-oxo-7-(trifluoromethyl)-1,2-dihydroquinoline-3-carboxylate

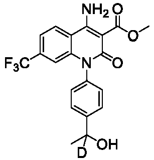

Methyl 4-amino-1-(4-acetylphenyl)-2-oxo-7-(trifluoromethyl)-1,2-dihydroquinoline-3-carboxylate obtained in Example 120 (20 mg) was dissolved in methanol-*d₄* (5 mL), sodium borodeuteride (10 mg) was added, and the mixture was reacted at room temperature for 5 hours. Water (20 mL) was added, the mixture was extracted 3 times with ethyl acetate (20 mL), and the extract was dried over anhydrous sodium sulphate and concentrated under reduced pressure to obtain the product as a white solid (16 mg).

¹H NMR (400 MHz, DMSO-*d₆*) δ 8.44 (d, *J =* 8.5 Hz, 1H), 8.38 (s, 2H), 7.61-7.48 (m, 3H), 7.20 (m, 2H), 6.59 (d, *J* = 1.6 Hz, 1H), 5.33 (d, *J* = 0.8 Hz, 1H), 3.71 (s, 3H), 1.36 (s, 3H).

### Example 396. methyl 4-amino-7-(difluoromethoxy)-1-(6-methylpyridin-3-yl)-2-oxo-1,2-dihydroquinoline-3 -carboxylate

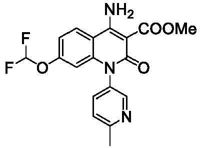

According to the preparation route of scheme 7, 4-(difluoromethoxy)-2-fluorobenzonitrile and 6-methylpyridin-3-amine were used as the starting material to obtain the product with reference to the preparation route of Example 165.

¹H NMR (400 MHz, CDCl₃) δ 8.39 (d, *J =* 2.5 Hz, 1H), 7.68 (d, *J =* 9.0 Hz, 1H), 7.51 (dd, *J* = 8.3, 2.6 Hz, 1H), 7.36 (d, *J* = 8.2 Hz, 1H), 7.01 - 6.94 (m, 1H), 6.66-6.26 (m, 2H), 3.90 (s, 3H), 2.67 (s, 3H).

### Example 397. methyl 4-amino-1-(6-atninopyridin-3-yl)-7-(difluoromethoxy)-2-oxo-1,2-dihydroquinoline-3 -carboxylate

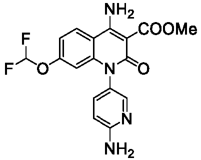

According to the preparation route of scheme 7, 4-(difluoromethoxy)-2-fluorobenzonitrile and 6-nitropyridin-3-amine were used as the starting material to obtain the product with reference to the preparation route of Example 165 and Example 147.

¹H NMR (400 MHz, DMSO-*d₆*) δ 8.39 (s, 2H), 8.29 (d, *J =* 9.0 Hz, 1H), 7.71 (d, *J* = 2.6 Hz, 1H), 7.23 (dd, *J* = 8.7, 2.6 Hz, 1H), 7.05 (dd, *J* = 8.9, 2.5 Hz, 1H), 6.59 (d, *J* = 8.7 Hz, 1H), 6.27 (s, 2H), 6.24 (d, *J* = 2.4 Hz, 1H), 3.71 (s, 3H).

### Example 398. methyl 4-amino-1-(6-aminopyridin-3-yl)-7-cyclopropyl-2-oxo-1,2-dihydroquinoline-3 -carboxylate

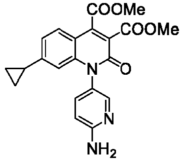

According to the preparation route of scheme 7, 2-fluoro-4-iodobenzonitrile and 6-nitropyridin-3-amine were used as the starting material to obtain the product with reference to the preparation route of Example 165 and Example 147.

¹H NMR (400 MHz, CD₃OD) δ 7.97 (d, *J =* 8.4 Hz, 1H), 7.78 (s, 1H), 7.40 (dd, *J =* 8.8 Hz, 2.4 Hz, 1H), 6.92 (dd, *J =* 8.4 Hz, 1.6 Hz, 1H), 6.83 (d, *J* = 8.8 Hz, 1H), 6.46 (d, *J* = 1.6 Hz, 1H), 3.81 (s, 3H), 1.83-1.90 (m, 1H), 0.98-1.03 (m, 2H), 0.62-0.66 (m, 2H).

### Example 399. methyl 4-amino-7-trifluoromethyl-1-(6-methylpyridin-3-yl)-2-oxopyrido [2,3-b]pyridin-3-carboxylate

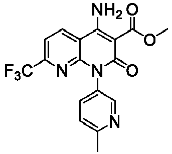

According to the preparation route of scheme 7, 2-chloro-4-(trifluoromethyl)benzonitrile and 6-methylpyridin-3-amine were used as the starting material to obtain the product with reference to the preparation route of Example 165.

¹H NMR (400 MHz, DMSO-*d₆*) δ 8.93 (d, *J =* 8.4 Hz, 1H), 8.53 (s, 2H), 8.26 (d, *J =* 2.4 Hz, 1H), 7.78 (d, *J =* 8.4 Hz, 1H), 7.57 (dd, *J =* 8.4 Hz, 2.4 Hz, 1H), 7.36 (d, *J =* 8.4 Hz, 1H), 3.73 (s, 3H), 2.53 (s, 3H).

### Example 400. methyl 4-amino-1-(4-aminophenyl)-7-(difluoromethoxy)-2-oxo-1,2-dihydro-1,8-naphthyridine-3-carboxylate

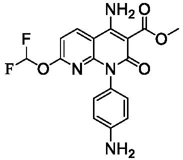

### Step A: methyl 2-chloro-6-(difluoromethoxy)nicotinate

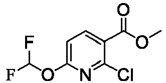

Under nitrogen protection, to acetonitrile (100 mL) were added methyl 2-chloro-6-hydroxynicotinate (3.0 g) and sodium chlorodifluoroacetate (3.2 g), and the mixture was heated to 80 °C and reacted for 24 hours. Sodium chlorodifluoroacetate (3.2 g) was added, the mixture was reacted at 80 °C for 24 hours, cooled to room temperature and filtered, the filtrate was concentrated under reduced pressure, and the residue was purified through column chromatography (petroleum ether/ethyl acetate = 10:1) to obtain the target compound (1.1 g).

### Step B: methyl 2-((4-(((tert-butoxycarbonyl)amino)phenyl)amino)-6-(difluoromethoxy) nicotinate

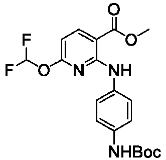

Under nitrogen protection, to toluene (20 mL) were added methyl 2-chloro-6-(difluoromethoxy)nicotinate (238 mg), *tert*-butyl (4-aminophenyl)carbamate (416 mg), tris(dibenzylideneacetone) dipalladium(0) (91 mg), 4,5-bis(diphenylphosphino)-9,9-dimethylxanthene (115 mg) and cesium carbonate (656 mg), the mixture was heated to 100 °C and reacted for 6 hours, cooled to room temperature, and filtered through celite, the filtrate was concentrated under reduced pressure, and the residue was purified through column chromatography (petroleum ether/ethyl acetate = 10:1) to obtain the target compound (350 mg).

### Step C: 2-((4-(((tert-butoxycarbonyl)amino)phenyl)atnino)-6-(difluoromethoxy)nicotinic acid

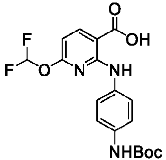

Methyl 2-((4-(((*tert*-butoxycarbonyl)amino)phenyl)amino)-6-(difluoromethoxy)nicotinate (350 mg) was dissolved in a mixed solvent of tetrahydrofuran (10 mL) and water (10 mL), lithium hydroxide monohydrate compound (108 mg) was added, and the mixture was heated to 50 °C and stirred for 6 hours and concentrated under reduced pressure. 1 N hydrochloric acid was used to adjust pH to 6, and a solid precipitated, which was collected by filtration and dried to obtain the product (300 mg).

¹H NMR (400 MHz, CDCl₃+CD₃OD) δ7.85 (d, *J* = 8.8 Hz, 1H), 7.49-7.53 (m, 2H), 7.42-7.46 (m, 1H), 7.15-7.17 (m, 2H), 6.77 (dd, *J* = 8.8 Hz, 2.4 Hz, 1H), 5.96 (d, *J* = 2.4 Hz, 1H), 4.16 (q, *J* = 8.0 Hz, 2H), 3.79 (s, 3H).

### Step D: methyl 1-(4-(((tert-butoxycarbonyl)amino)phenyl)-7-(difluoromethoxy)-4-hydroxy-2-oxo-1,2-dihydro-1,8-naphthyridine-3 -carboxylate

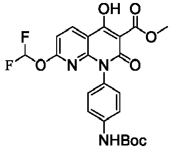

2-((4-(((*Tert*-butoxycarbonyl)amino)phenyl)amino)-6-(difluoromethoxy)nicotinic acid (300 mg) was dissolved in dichloromethane (20 mL), 1-hydroxybenzotriazole (344 mg) and 1-ethyl-(3-dimethylaminopropyl) carbodiimide hydrochloride (245 mg) were added, and the mixture was stirred at room temperature for 3 hours. Water (20 mL) was added, the mixture was extracted, the extract was washed with brine, dried over anhydrous sodium sulphate and filtered, the filtrate was collected and concentrated under reduced pressure to obtain an intermediate. Dimethyl malonate was dissolved in dry tetrahydrofuran (10 mL), sodium hydride (170 mg) was added, the mixture was stirred at room temperature for 10 minutes, to this system was added a solution of the above intermediate in tetrahydrofuran, and the mixture was stirred at room temperature overnight. To this system was added an aqueous solution of saturated ammonium chloride, the mixture was extracted with ethyl acetate, the extract was washed with brine and concentrated under reduced pressure, and the residue was purified through column chromatography (dichloromethane/methanol = 15:1) to obtain the target compound (220 mg).

### Step E: methyl 4-amino-1-(4-(((tert-butoxycarbonyl)amino)phenyl)-7-(difluoromethoxy)-2-oxo-1,2-dihydro-1,8-naphthyridine-3-carboxylate

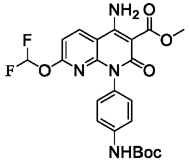

Methyl 1-(4-(((*tert*-butoxycarbonyl)amino)phenyl)-7-(difluoromethoxy)-4-hydroxy-2-oxo-1,2-dihydro-1,8-naphthyridine-3-carboxylate (220 mg) was dissolved in dichloromethane (15 mL), pyridine (74 mg) and trifluoromethanesulfonic anhydride (198 mg) were added, the mixture was reacted at room temperature for 2 hours and concentrated under reduced pressure, 7 N solution of ammonia in methanol (2 mL) was added, and the mixture was stirred at 40 °C in a sealed glass tube overnight. The mixture was concentrated under reduced pressure, and the residue was purified through column chromatography (petroleum ether/ethyl acetate = 12:1) to obtain the target compound (60 mg).

### Step F: methyl 4-amino-1-(4-aminophenyl)-7-(difluoromethoxy)-2-oxo-1,2-dihydro-1,8-naphthyridine-3 -carboxylate

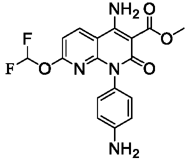

Methyl 4-amino-1-(4-(((*tert*-butoxycarbonyl)amino)phenyl)-7-(difluoromethoxy)-2-oxo-1,2-dihydro-1,8-naphthyridine-3-carboxylate (60 mg) was dissolved in methanol (2 mL), 4 N hydrogen chloride ethyl acetate solution (2 mL) was added, the reaction solution was reacted at room temperature for 8 hours and concentrated under reduced pressure, 7 N solution of ammonia in methanol (1 mL) was added to adjust pH to 8, the reaction solution was concentrated under reduced pressure, and the residue was purified through column chromatography (dichloromethane/methanol = 8:1) to obtain the target compound (15 mg).

¹H NMR (400 MHz, CD₃OD+CDCl₃) δ 8.23 (d, *J* = 8.8 Hz, 1H), 6.87 (d, *J* = 8.8 Hz, 2H), 6.84 (t, *J* = 72.4 Hz, 1H), 6.73 (d, *J* = 8.8 Hz, 2H), 6.61 (d, *J* = 8.8 Hz, 1H), 3.80 (s, 3H).

### Example 401. methyl-d₃ 4-amino-1-(4-aminophenyl)-2-oxo-7-(trifluoromethoxy)-1,2-dihydroquinoline-3 -carboxylate

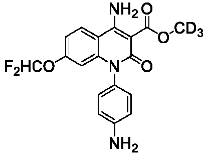

Methyl 4-amino-1-(4-aminophenyl)-2-oxo-7-(trifluoromethoxy)-1,2-dihydroquinoline-3-carboxylate obtained in Example 320 was used as the staring material to obtain the product with reference to the preparation route of Example 311.

¹H NMR (400 MHz, CD₃OD) δ 8.08 (d, *J* = 8.9 Hz, 1H), 6.97 (d, *J =* 8.6 Hz, 1H), 6.93-6.45 (m, 5H), 6.35 (d, *J* = 2.4 Hz, 1H).

### Example 402. methyl-d₃ 4-amino-1-(4-(1-d-1-hydroxyethyl)phenyl)-2-oxo-7-(trifluoromethyl)-1,2-dihydroquinoline-3-carboxylate

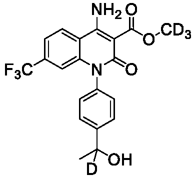

Methyl 4-amino-1-(4-(1-*d-1-*hydroxyethyl)phenyl)-2-oxo-7-(trifluoromethyl)-1,2-dihydroquinoline-3-carboxylate obtained in Example 395 was used as the starting material to obtain the product with reference to the preparation route of Example 311.

¹H NMR (400 MHz, DMSO-*d₆*) δ 8.44 (d, *J =* 8.5 Hz, 1H), 8.37 (s, 2H), 7.57-7.47 (m, 3H), 7.20 (td, *J* = 5.8, 2.8 Hz, 2H), 6.62-6.57 (m, 1H), 5.32 (s, 1H), 1.36 (s, 3H).

### Example 403. methyl 4-amino-1-(1-chloroisoquinolin-5-yl)-2-oxo-7-(trifluoromethoxy)-1,2-dihydroquinoline-3 -carboxylate

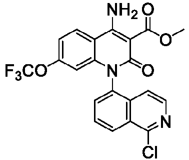

According to the preparation route of scheme 6, 2-bromo-4-(trifluoromethoxy)benzonitrile and 1-chloroisoquinolin-5-amine were used as the starting material to obtain the product with reference to the preparation route of Example 81.

¹H NMR (400 MHz, DMSO-*d₆*) δ 8.67 (s, 2H), 8.44-8.48 (m, 2H), 8.22 (d, *J =* 6.0 Hz, 1H), 7.99 (t, *J* = 8.0 Hz, 1H), 7.93 (d, *J* = 7.2 Hz, 1H), 7.31 (d, *J* = 5.6 Hz, 1H), 7.25 (d, *J* = 9.2 Hz, 1H), 6.08 (d, *J* = 2.0 Hz, 1H), 3.69 (s, 3H).

### Example 404. methyl-d₃ 4-amino-1-(6-atninopyridin-3-yl)-2-oxo-7-(trifluoromethoxy)-1,2-dihydroquinoline-3-carboxylate

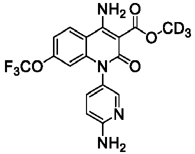

Methyl 4-amino-1-(6-aminopyridin-3 -yl)-2-oxo-7-(trifluoromethoxy)-1,2-dihydroquinoline-3-carboxylate obtained in Example 394 was used as the starting material to obtain the product with reference to the preparation route of Example 331.

¹H NMR (400 MHz, DMSO-*d₆*) δ 8.38 (s, 2H), 8.34 (d, *J* = 9.2 Hz, 1H), 7.71 (d, *J* = 2.4 Hz, 1H), 7.18-7.25 (m, 2H), 6.56 (d, *J* = 8.4 Hz, 1H), 6.39 (d, *J* = 1.6 Hz, 1H), 6.25 (s, 2H).

### Example 405. methyl 4-amino-1-(4-chlorophenyl)-7-(dimethylamino)-2-oxo-1,2-dihydro-1,8-naphthyridine-3 -carboxylate

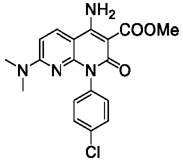

According to the preparation route of scheme 7, 2-chloro-6-dimethylaminonicotinonitrile and 4-chloroaniline were used as the starting material to obtain the product with reference to the preparation route of Example 165.

¹H NMR (400 MHz, DMSO-*d₆*) δ 8.38 (s, 2H), 8.26 (d, *J =* 9.1 Hz, 1H), 7.49 (d, *J* = 8.5 Hz, 2H), 7.18 (d, *J* = 8.6 Hz, 2H), 6.56 (d, *J* = 9.1 Hz, 1H), 3.67 (s, 3H), 2.82 (s, 6H).

### Example 406. methyl 4-atnino-1-(1-methoxyisoquinolin-5-yl)-2-oxo-7-(trifluoromethoxy)-1,2-dihydroquinoline-3 -carboxylate

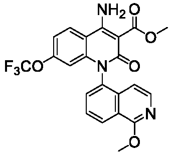

According to the preparation route of scheme 6, 2-bromo-4-(trifluoromethoxy)benzonitrile and 1-methoxyisoquinolin-5-amine were used as the starting material to obtain the product with reference to the preparation route of Example 81.

¹H NMR (400 MHz, DMSO-*d₆*) δ 8.62 (s, 2H), 8.44 (d, *J* = 9.2 Hz, 1H), 8.35 (dd, *J* = 8.0 Hz, 1.2 Hz, 1H), 7.92 (d, J= 6.4 Hz, 1H), 7.75-7.81 (m, 2H), 7.23 (d, *J* = 8.8 Hz, 1H), 6.74 (d, *J* = 6.4 Hz, 1H), 5.99 (d, *J* = 1.2 Hz, 1H), 4.09 (s, 3H), 3.70 (s, 3H).

### Example 407. methyl 4-amino-7-(difluoromethoxy)-1-(6-methylpyridin-3-yl)-2-oxo-1,2-dihydro-1,8-naphthyridine-3-carboxylate

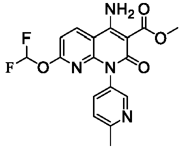

Methyl 2-chloro-6-(difluoromethoxy)nicotinate and 6-methylpyridin-3-amine were used as the starting material to obtain the product with reference to the preparation route of Example 400.

¹H NMR (400 MHz, CD₃OD+CDCl₃) δ 8.40 (d, *J* = 8.8 Hz, 1H), 8.23 (d, *J* = 2.0 Hz, 1H), 7.49 (dd, *J* = 8.4 Hz, 2.0 Hz, 1H), 7.31 (d, *J* = 8.4 Hz, 1H), 6.74 (t, *J* = 72.0 Hz, 1H), 6.71 (d, *J =* 8.8 Hz, 1H), 3.82 (s, 3H), 2.59 (s, 3H).

### Example 408. methyl-d₃ 4-amino-7-(difluoromethyl)-1-(4-(1-hydroxyethyl)phenyl)-2-oxo-1,2- dihydro-1,8-naphthyridine-carboxylate

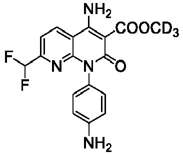

Methyl 4-amino-1-(4-aminophenyl)-7-(difluoromethyl)-2-oxo-1,2-dihydro-1,8-naphthyridine-3-carboxylate obtained in Example 344 was used as the starting material to obtain the product with reference to the preparation route of Example 311.

¹H NMR(400 MHz, CD₃OD) δ 8.67 (d, *J =* 8.3 Hz, 1H), 7.52 (d, *J =* 8.3 Hz, 1H), 6.92-6.85 (m, 2H), 6.85-6.74 (m, 2H), 6.57-6.23 (m, 1H).

### Example 409. methyl 4-amino-7-cyclopropyl-1-(1-cyclopropylisoquinolin-5-yl)-2-oxopyrido [2,3 -b]pyridin-3 -carboxylate

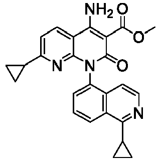

According to the preparation route of scheme 7, 1-chloroisoquinolin-5-amine and 2,6-dichloropyridin-3-carbonitrile were used as the starting material to obtain the product with reference to the preparation route of Example 165 and Example 147.

¹H NMR (400 MHz, CD₃OD) δ 8.65 (d, *J=* 8.4 Hz, 1H), 8.37 (d, *J=* 8.8 Hz, 1H), 8.11 (d, *J* = 6.0 Hz, 1H), 7.78-7.82 (m, 1H), 7.59 (d, *J =* 7.2 Hz, 1H), 7.18 (d, *J =* 8.0 Hz, 1H), 6.97 (d, *J* = 6.0 Hz, 1H), 3.80 (s, 3H), 2.91-2.98 (m, 1H), 1.13-1.36 (m, 4H), 0.84-092 (m, 1H), 0.64-0.72 (m, 1H), 0.54-0.61 (m, 1H), 0.33-0.39 (m, 1H), 0.1-0.6(m, 1H).

### Example 410. methyl 4-amino-1-(4-aminophenyl)-7-bromo-6-fluoro-2-oxo-1,2-dihydroquinoline-3-carboxylate

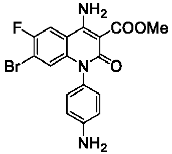

According to the preparation route of scheme 7, 2-amino-4-bromo-5-fluorobenzonitrile and 1-fluoro-4-nitrobenzene were used as the starting material to obtain the product with reference to the preparation route of Example 165.

¹H NMR (400 MHz, CD₃OD) δ 8.01 (dd, *J* = 9.6 Hz, 1.2 Hz, 1H), 6.86-6.99 (m, 5H), 3.81 (s, 3H).

### Example 411. methyl 4-atnino-1-(1-chloroisoquinolin-5-yl)-7-(difluoromethyl)-2-oxo-1,2-dihydroquinoline-3 -carboxylate

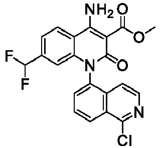

According to the preparation route of scheme 8, compound 4-difluoromethyl-2-fluorobenzonitrile and 1-chloroisoquinolin-5-amine were used as the starting material to obtain the product with reference to the preparation route of Example 340.

¹H NMR (400 MHz, CDCl₃) δ 8.52 (dd, *J =* 8.5, 1.0 Hz, 1H), 8.16 (dd, *J =* 5.8, 0.8 Hz, 1H), 7.90-7.80 (m, 2H), 7.74 (dt, *J* = 7.4, 1.0 Hz, 1H), 7.28 *(d, J=* 8.4 Hz, 1H), 7.16 (dt, *J* = 5.9, 0.9 Hz, 1H), 6.56-6.23 (m, 2H), 3.89 (d, *J* = 0.9 Hz, 3H).

### Example 412. methyl-d₃ 4-amino-1-(4-aminophenyl)-7-(trifluoromethyl)-2-oxo-1,2-dihydroquinoline-3 -carboxylate

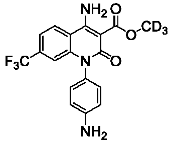

Methyl 4-amino-1-(4-aminophenyl)-2-oxo-7-(trifluoromethyl)-1,2-dihydroquinoline-3-carboxylate obtained in Example 273 was used as the starting material to obtain the product with reference to the preparation route of Example 311.

¹H NMR (400 MHz, DMSO-*d₆*) δ 8.40 (d, *J* = 8.5 Hz, 1H), 8.26 (s, 2H), 7.49 (s, 1H), 6.83 (d, *J =* 8.5 Hz, 2H), 6.74 (s, 1H), 6.68 (d, *J* = 8.5 Hz, 2H), 5.37 (s, 2H).

### Example 413. methyl 4-amino-1-(1-cyclopropylisoquinolin-5-yl)-2-oxo-7-(trifluoro methoxy)-1,2-dihydroquinoline-3 -carboxylate

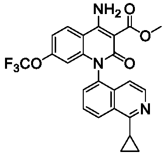

Under nitrogen protection, to a mixed solvent of toluene (5 mL) and water (1.5 mL) were added methyl 4-amino-1-(1-chloroisoquinolin-5-yl)-2-oxo-7-(trifluoromethoxy)-1,2-dihydroquinoline-3-carboxylate obtained in Example 403 (93 mg), cyclopropylboronic acid (103 mg), palladium (II) acetate (5 mg), tricyclohexyl phosphine (11 mg) and potassium phosphate (148 mg), the reaction solution was heated to 100 °C and reacted for 12 hours, cooled to room temperature and filtered, water (30 mL) was added, the mixture was extracted with ethyl acetate (30 mL*3), the extract was dried over anhydrous sodium sulphate and concentrated under reduced pressure, and the residue was purified through column chromatography (dichloromethane methanol = 10:1) to obtain the product.

¹H NMR (400 MHz, CDCl₃) δ 8.60 (d, *J =* 8.7 Hz, 1H), 8.26 (d, *J =* 5.7 Hz, 1H), 7.87-7.69 (m, 2H), 7.63 (dd, *J* = 7.3, 1.0 Hz, 1H), 7.09-6.85 (m, 2H), 6.15 (d, *J* = 2.3 Hz, 1H), 3.88 (s, 3H), 2.95-2.71 (m, 1H), 1.63-1.04 (m, 4H).

### Example 414. methyl 4-atnino-1-(1-hydroxyisoquinolin-5-yl)-2-oxo-7-(trifluoromethoxy)-1,2-dihydroquinoline-3 -carboxylate

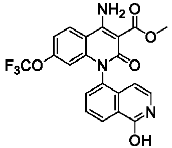

To dichloromethane (5 mL) was added methyl 4-atnino-1-(1-methoxyisoquinolin-5-yl)-2-oxo-7-(trifluoromethoxy)-1,2-dihydroquinoline-3-carboxylate obtained in Example 406 (30 mg), boron tribromide (0.3 mL, 1 M dichloromethane solution ) was added dropwise, and the mixture was reacted at room temperature for 18 hours. Water (20 mL) was added, the mixture was extracted with ethyl acetate (20 mL*3), the extract was dried over anhydrous sodium sulphate and concentrated under reduced pressure, and the residue was purified through column chromatography (dichloromethane methanol = 10:1) to obtain the product.

¹H NMR (400 MHz, DMSO-*d₆*) δ 10.19 (s, 1H), 9.21 (s, 1H), 8.60 (d, *J =* 9.1 Hz, 1H), 8.40 (d, *J =* 8.3 Hz, 1H), 7.98-7.89 (m, 2H), 7.84 (d, *J* = 7.9 Hz, 1H), 7.47-7.39 (m, 1H), 6.88 (dd, *J* = 6.1, 0.9 Hz, 1H), 6.19 (d, *J* = 2.3 Hz, 1H), 4.09 (s, 3H).

### Example 415. methyl-d₃ 4-amino-1-(1-cyclopropylisoquinolin-5-yl)-2-oxo-7-(trifluoromethoxy)-1,2-dihydroquinoline-3 -carboxylate

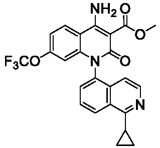

Methyl 4-amino-1-(1-cyclopropylisoquinolin-5-yl)-2-oxo-7-(trifluoromethoxy)-1,2-dihydroquinoline-3-carboxylate obtained in Example 413 was used as the starting material to obtain the product with reference to the preparation route of Example 331.

¹H NMR (400 MHz, CDCl₃) δ 8.60 (d, *J* = 8.5 Hz, 1H), 8.26 (d, *J =* 5.9 Hz, 1H), 7.87-7.70 (m, 2H), 7.62 (dd, *J* = 7.3, 1.1 Hz, 1H), 7.09-6.85 (m, 2H), 6.16 (d, *J* = 2.3 Hz, 1H), 2.88-2.74 (m, 1H), 1.39-1.18 (m, 4H).

### Example 416. methyl 4-amino-1-(6-atninopyridin-3-yl)-7-(difluoromethoxy)-2-oxo-1,2-dihydro-1,8-naphthyridine-3-carboxylate

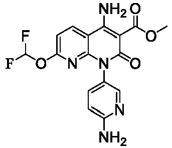

Methyl 2-chloro-6-(difluoromethoxy)nicotinate and tert-butyl (5-aminopyridin-2-yl) carbamate were used as the starting material to obtain the product with reference to the preparation route of Example 400.

¹H NMR (400 MHz, CD₃OD+CDCl₃) δ 8.34 (d, *J* = 8.8 Hz, 1H), 7.63 (d, *J* = 2.0 Hz, 1H), 7.24-7.29 (m, 1H), 6.82 (t, *J* = 72.0 Hz, 1H), 6.62-6.66 (m, 2H), 3.73 (s, 3H).

### Example 417. methyl 4-amino-7-trifluoromethyl-1-(2-chlorophenyl)-2-oxopyrido[2,3-b] pyridin-3 -carboxylate

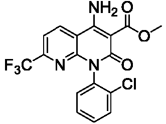

According to the preparation route of scheme 7, 2-chloro-4-(trifluoromethyl)benzonitrile and 2-chloroaniline were used as the starting material to obtain the product with reference to the preparation route of Example 165.

¹H NMR (400 MHz, DMSO-*d₆*) δ 8.74 (d, *J =* 7.6 Hz, 1H), 7.70 (dd, *J =* 7.6 Hz, 1.6 Hz, 1H), 7.66 (d, *J* = 7.2 Hz, 1H), 7.52-7.62 (m, 5H), 3.68 (s, 3H).

### Example 418. methyl 4-amino-7-trifluoromethyl-1-(4-((difluoromethyl)oxy)phenyl)-2-oxopyrido [2,3 -b]pyridin-3 -carboxylate

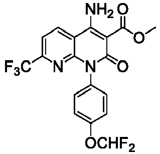

According to the preparation route of scheme 7, 2-chloro-4-(trifluoromethyl)benzonitrile and 4-difluoromethoxyaniline were used as the starting material to obtain the product with reference to the preparation route of Example 165.

¹H NMR (400 MHz, DMSO-*d₆*) δ 8.70 (d, *J =* 8.0 Hz, 1H), 7.55 (s, 2H), 7.48-7.52 (m, 3H), 7.40 (t, *J* = 73.6 Hz, 1H), 7.33 (d, *J* = 9.2 Hz, 2H), 3.68 (s, 3H).

### Example 419. methyl 4-amino-1-(1-chloroisoquinolin-5-yl)-7-cyclopropyl-2-oxopyrido [2,3-b]pyridin-3-carboxylate

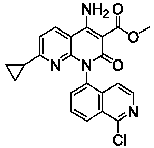

According to the preparation route of scheme 7, 1-chloroisoquinolin-5-amine and 2,6-dichloropyridin-3-carbonitrile were used as the starting material to obtain the product with reference to the preparation route of Example 165 and Example 147.

¹H NMR (400 MHz, CD₃OD) δ 8.48 (d, *J* = 8.8 Hz, 1H), 8.38 (d, *J* = 8.0 Hz, 1H), 8.11 (d, *J* = 6.0 Hz, 1H), 7.88-7.92 (m, 1H), 7.71 (d, *J =* 7.6 Hz, 1H), 7.23 (d, *J =* 6.0 Hz, 1H), 7.19 (d, *J* = 8.4 Hz, 1H), 3.80 (s, 3H), 1.84-1.90 (m, 1H), 0.67-0.74 (m, 1H), 0.58-0.65 (m, 1H), 0.31-0.37 (m, 1H), -0.08-0.02 (m, 1H).

### Example 420. methyl 4-atnino-1-(6-aminopyridin-3-yl)-2-oxo-7-(trifluoromethyl)pyrido [2,3-b]pyridin-3-carboxylate

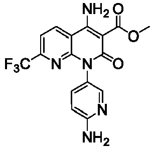

According to the preparation route of scheme 7, 2-chloro-6-(trifluoromethyl)pyridin-3-carbonitrile and 6-nitropyridin-3-amine were used as the starting material to obtain the product with reference to the preparation route of Example 165.

¹H NMR (400 MHz, DMSO-*d₆*) δ 8.89 (d, *J* = 8.4 Hz, 1H), 8.41 (s, 2H), 7.74 (d, *J* = 8.0 Hz, 1H), 7.66 (d, *J* = 2.4 Hz, 1H), 7.18 (dd, *J* = 8.8 Hz, 2.4 Hz, 1H), 6.49 (d, *J* = 8.8 Hz, 1H), 6.04 (s, 2H), 3.73 (s, 3H).

### Example 421. methyl-d₃ 4-atnino-1-(1-chloroisoquinolin-5-yl)-7-(difluoromethoxy)-2-oxo-1,2- dihydroquinoline-3-carboxylate

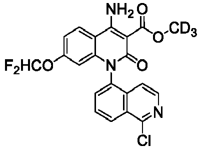

Compound methyl 4-atnino-1-(1-chloroisoquinolin-5-yl)-7-(difluoromethoxy)-2-oxo-1,2-dihydroquinoline-3-carboxylate of Example 384 was used as the starting material to obtain the product with reference to the preparation route of Example 331.

¹H NMR (400 MHz, CDCl₃) δ 8.51 (d, *J* = 8.5 Hz, 1H), 8.16 (d, *J* = 5.8 Hz, 1H), 7.85 (dd, *J* = 8.6, 7.3 Hz, 1H), 7.75-7.67 (m, 2H), 7.18 (d, *J =* 5.8 Hz, 1H), 6.89 (dd, *J =* 9.0, 2.4 Hz, 1H), 6.57-6.17 (m, 1H), 5.99 (d, *J* = 2.3 Hz, 1H).

### Example 422. methyl-d₃ 4-amino-7-(difluoromethoxy)-1-(4-(1-(R)-hydroxyethoxy)phenyl)-2-oxo-1,2-dihydro-1,8-naphthyridine-3 -carboxylate

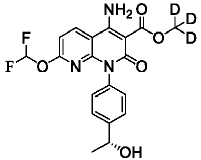

Methyl 4-amino-7-(difluoromethoxy)-1-(4-(1-(*R*)-hydroxyethoxy)phenyl)-2-oxo-1,2-dihydro-1,8-naphthyridine-3-carboxylate obtained in Example 372 was used as the starting material to obtain the product with reference to the preparation route of Example 331.

¹H NMR (400 MHz, CD₃OD+CDCl₃) δ 8.28 (d, *J* = 8.4 Hz, 1H), 7.36-7.46 (m, 2H), 7.06 (d, *J* = 7.6 Hz, 2H), 6.69 (t, *J* = 72.4 Hz, 1H), 6.64 (d, *J* = 8.4 Hz, 1H), 4.86 (q, *J* = 6.8 Hz, 1H), 1.43 (d, *J* = 6.8 Hz, 3H).

### Example 423. methyl-d₃ 4-amino-1-(4-aminophenyl)-7-(difluoromethoxy)-2-oxo-1,2-dihydro-1,8-naphthyridine-3-carboxylate

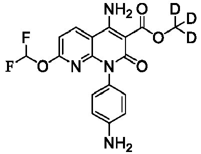

### Step A: 4-amino-1-(4-(((tert-butoxycarbonyl)amino)phenyl)-7-(difluoromethoxy)-2-oxo-1,2-dihydro-1,8-naphthyridine-3-carboxylic acid

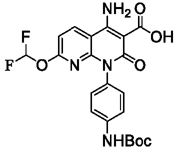

Methyl 4-amino-1-(4-(((*tert*-butoxycarbonyl)amino)phenyl)-7-(difluoromethoxy)-2-oxo-1,2-dihydro-1,8-naphthyridine-3-carboxylate (60 mg) was dissolved in a mixed solvent of tetrahydrofuran (5 mL) and water (5 mL), lithium hydroxide monohydrate (16 mg) was added, and the mixture was heated to 50 °C and stirred for 6 hours and concentrated under reduced pressure, 1 N hydrochloric acid was used to adjust pH to 6, a solid precipitated, and the mixture was filtered to collect the solid, which was dried to obtain the product (40 mg).

### Step B: methyl-d₃ 4-amino-1-(4-(((tert-butoxycarbonyl)amino)phenyl)-7-(difluoromethoxy)-2-oxo-1,2-dihydro-1,8-naphthyridine-3-carboxylate

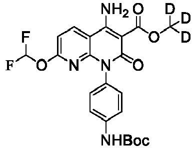

4-Amino-1-(4-(((*tert*-butoxycarbonyl)amino)phenyl)-7-(difluoromethoxy)-2-oxo-1,2-dihydro -1,8-naphthyridine-3-carboxylic acid (40 mg) and 2-(7-azabenzotriazol-1-yl)- *N,N,N',N'-*tetramethyluronium hexafluorophosphate (49 mg) were dissolved in a mixed solvent of DMF and methanol-*d₄* (10 mL/1 mL), diisopropylethylamine (22 mg) was added, the mixture was stirred at room temperature for 4 hours and concentrated under reduced pressure, and the residue was purified through column chromatography (dichloromethane/methanol = 13:1) to obtain the product (18 mg).

¹H NMR (400 MHz, CDCl₃) δ 9.64-10.13 (brs, 1H), 8.36-8.82 (brs, 1H), 7.49-7.53 (m, 2H), 7.40-7.45 (m, 1H), 7.18-7.24 (m, 3H), 6.63 (d, *J* = 8.4 Hz, 1H), 6.18 (d, *J* = 8.4 Hz, 1H), 4.03 (s, 3H), 3.86 (s, 3H).

### Step C: methyl-d₃ 4-amino-1-(4-aminophenyl)-7-(difluoromethoxy)-2-oxo-1,2- dihydro-1,8-naphthyridine-3 -carboxylate

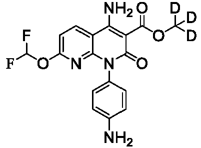

Methyl 4-amino-1-(4-(((*tert*-butoxycarbonyl)amino)phenyl)-7-(difluoromethoxy)-2-oxo-1,2-dihydro-1,8-naphthyridine-3-*d3*-carboxylate (18 mg) was dissolved in methanol (2 mL), 4 N hydrogen chloride ethyl acetate solution (2 mL) was added, the reaction solution was reacted at room temperature for 8 hours and concentrated under reduced pressure, 7 N ammonia methanol solution (1 mL) was added to adjust pH to 8, the mixed solution was concentrated under reduced pressure, and the residue was purified through column chromatography (dichloromethane/methanol = 8:1) to obtain the target compound (8 mg).

¹H NMR (400 MHz, CD₃OD+CDCl₃) δ 8.23 (d, *J* = 8.8 Hz, 1H), 6.87 (d, *J* = 8.8 Hz, 2H), 6.84 (t, *J* = 72.4 Hz, 1H), 6.73 (d, *J* = 8.8 Hz, 2H), 6.61 (d, *J* = 8.8 Hz, 1H).

### Example 424. methyl 5-amino-8-(4-chlorophenyl)-7-oxo-2-((2,2,2-trifluoroethyl)amino)-7H,8H-pyrido[2,3-d]pyrimidine-6-carboxylate

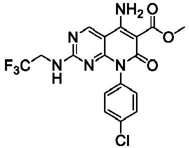

According to the preparation route of scheme 7, 4-chloro-2-((2,2,2-trifluoroethyl)amino) pyrimidine-5-carbonitrile and 4-chloroaniline were used as the starting material to obtain the product with reference to the preparation route of Example 165.

¹H NMR (400 MHz, CD₃OD) δ 8.77 (s, 1H), 7.31 (d, *J* = 8.0 Hz, 2H), 6.99 (d, *J* = 7.9 Hz, 2H), 3.70 (s, 3H), 3.55 (d, *J* = 10.5 Hz, 2H).

### Example 425. methyl 5-amino-8-(4-aminophenyl)-7-oxo-2-((2,2,2-trifluoroethyl)amino)-7H, 8H-pyrido[2,3 -d]pyrimidine-6-carboxylate

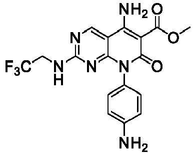

According to the preparation route of scheme 6, 4-chloro-2-((2,2,2-trifluoroethyl) amino) pyrimidine-5-carbonitrile and *tert*-butyl (4-aminophenyl)carbamate were used as the starting material to obtain the product with reference to the preparation route of Example 81.

¹H NMR (400 MHz, DMSO-*d₆*) δ 9.10 (s, 1H), 8.43 (s, 2H), 8.33 (s, 1H), 6.72 (d, *J* = 8.5 Hz, 2H), 6.59 (d, *J* = 8.3 Hz, 2H), 5.19 - 5.08 (m, 2H), 4.09 (s, 1H), 3.75 (s, 1H), 3.68 (s, 3H).

### Example 426. methyl-d₃ 4-amino-1-(1-chloroisoquinolin-5-yl)-2-oxo-7-(trifluoromethyl) pyrido [2,3 -b]pyridin-3 -carboxylate

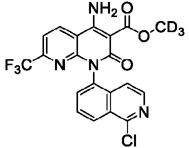

According to the preparation route of scheme 7, 2-chloro-6-(trifluoromethyl)pyridin-3-carbonitrile and 1-chloroisoquinolin-5-amine were used as the starting material to obtain the product with reference to the preparation route of Example 165 and Example 331.

¹H NMR (400 MHz, CD₃OD) δ 8.83 (d, *J* = 8.8 Hz, 1H), 8.51 (d, *J* = 8.4 Hz, 1H), 8.11 (d, *J* = 5.6 Hz, 1H), 7.89-7.93 (m, 1H), 7.79 (d, *J =* 7.6 Hz, 1H), 7.65 (d, J = 8.4 Hz, 1H), 7.33 (d, *J =* 5.6 Hz, 1H).

### Example 427. methyl-d₃ 4-amino-1-(4-((1R)-1-hydroxyethyl)phenyl)-2-oxo-7-(trifluoro methyl)pyrido [2,3 -b]pyridin-3 -carboxylate

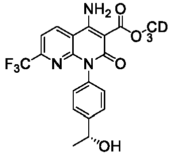

2-Chloro-6-(trifluoromethyl)pyridin-3-carbonitrile and *(1R)*-1-(4-aminophenyl)ethan-1-ol were used as the starting material to obtain the product with reference to the preparation route of Example 189 and Example 331.

¹H NMR (400 MHz, CD₃OD) δ 8.75 (d, *J* = 8.0 Hz, 1H), 7.62 (d, *J =* 8.0 Hz, 1H), 7.47-7.56 (m, 2H), 7.17 (d, *J* = 8.4 Hz, 2H), 4.92 (q, *J* = 6.8 Hz, 1H), 1.49 (d, *J* = 6.8 Hz, 3H).

### Example 428. methyl 4-amino-1-(2-methyl-4-aminophenyl)-7-(difluoromethoxy)-2-oxo-1,2-dihydroquinoline-3 -carboxylate

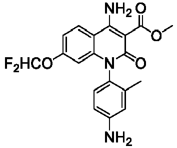

According to the preparation route of scheme 6, 2-chloro-4-difluoromethoxybenzonitrile and 4-nitro-2-methylaniline were used as the starting material to obtain the product with reference to the preparation route of Example 147.

¹H NMR (400 MHz, DMSO-*d₆*) δ 8.31 (s, 2H), 8.26 (d, *J =* 9.0 Hz, 1H), 7.42-6.96 (m, 2H), 6.68 (d, *J =* 8.3 Hz, 1H), 6.58-6.46 (m, 2H), 6.06 (d, *J =* 2.5 Hz, 1H), 5.23 (s, 2H), 3.69 (s, 3H), 1.70 (s, 3H).

### Example 429. methyl-d₃ 4-amino-1-(2-methyl-4-aminophenyl)-7-(difluoromethoxy)-2-oxo-1,2- dihydroquinolin-3-carboxylate

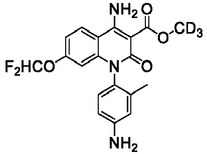

According to the preparation route of Example 311, methyl 4-amino-1-(2-methyl-4-amino phenyl)-7-(difluoromethoxy)-2-oxo-1,2-dihydroquinoline-3-carboxylate obtained in Example 428 was used as the starting material to obtain the product.

¹H NMR (400 MHz, DMSO-*d₆*) δ 8.31 (s, 2H), 8.26 (d, *J* = 9.0 Hz, 1H), 7.39-6.98 (m, 2H), 6.68 (d, *J* = 8.3 Hz, 1H), 6.58-6.46 (m, 2H), 6.06 (d, *J* = 2.4 Hz, 1H), 5.23 (s, 2H), 1.70 (s, 3H).

### Example 430. methyl-d₃ 4-amino-1-(4-atninophenyl)-2-oxo-7-(difluoromethyl)-1,2-dihydroquinoline-3 -carboxylate

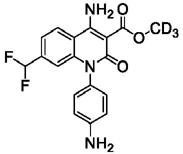

According to the preparation route of Example 331, methyl 4-atnino-1-(4-aminophenyl)-2-oxo-7-(difluoromethyl)-1,2-dihydroquinoline-3-carboxylate obtained in Example 327 was used as the starting material to obtain the product.

¹H NMR(400 MHz, CD₃OD+CDCl₃) δ 8.17 (d, *J* = 8.8 Hz, 1H), 7.35 (d, *J* = 8.8 Hz, 1H), 6.92 (d, *J* = 8.8 Hz, 2H), 6.87 (d, *J* = 8.8 Hz, 2H), 6.83 (s, 1H), 6.63 (t, *J* = 56 Hz, 1H).

### Example 431. methyl 4-amino-1-(6-(1-hydroxyethyl)pyridin-3-yl)-2-oxo-7-(trifluoromethoxy)-1,2-dihydroquinoline-3 -carboxylate

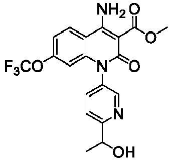

According to the preparation route of scheme 6, 4-(trifluoromethoxy)-2-bromobenzonitrile and 1-(5-aminopyridin-2-yl)ethan-1-one were used as the starting material to obtain the product with reference to the preparation route of Example 81 and 340.

¹H NMR (400 MHz, DMSO-*d₆*) δ 8.51 (s, 2H), 8.43-8.22 (m, 2H), 7.81-7.49 (m, 2H), 7.25 (d, *J =* 8.9 Hz, 1H), 6.20 (s, 1H), 5.51 (d, *J* = 4.7 Hz, 1H), 5.00-4.63 (m, 1H), 3.70 (s, 3H), 1.42 (d, *J* = 6.5 Hz, 3H).

### Example 432. methyl 4-amino-1-(4-(1-hydroxyethyl)-2-methylphenyl)-2-oxo-7-(trifluoromethoxy)-1,2-dihydroquinoline-3 -carboxylate

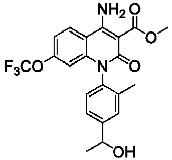

According to the preparation route of scheme 6, 2-amino-4-(trifluoromethoxy)benzonitrile and 1-(4-bromo-3-methylphenyl)ethan-1-one were used as the starting material to obtain the product with reference to the preparation route of Example 81 and 340.

¹H NMR (400 MHz, DMSO-*d₆*) δ 8.43 (s, 2H), 8.38 (d, *J =* 9.0 Hz, 1H), 7.41 (dd, *J =* 14.5, 2.0 Hz, 1H), 7.37-7.28 (m, 1H), 7.22 (dd, *J =* 9.0, 1.3 Hz, 1H), 7.07 (dd, *J =* 8.0, 3.9 Hz, 1H), 6.10-6.0 (m, 1H), 5.26 (d, *J =* 4.3 Hz, 1H), 4.77 (q, *J =* 6.3 Hz, 1H), 3.71 (s, 3H), 1.87 (s, 3H), 1.36 (d, *J* = 6.4 Hz, 3H).

### Example 433. methyl 4-amino-1-(4-amino-2-methylphenyl)-2-oxo-7-(trifluoro methoxy)-1,2-dihydroquinoline-3 -carboxylate

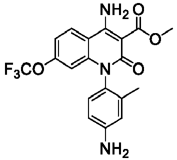

According to the preparation route of scheme 6, 2-bromo-4-[(trifluoromethyl)oxy] benzonitrile and 2-methyl-4-nitroaniline were used as the starting material to obtain the product with reference to the preparation route of Example 81.

¹H NMR (400 MHz, CD₃OD) δ 8.21 (d, *J* = 9.2 Hz, 1H), 7.13 (d, *J* = 9.2 Hz, 1H), 6.82 (d,*J* = 8.4 Hz, 1H), 6.77 (d, *J* = 2.0 Hz, 1H), 6.72 (dd, *J* = 8.4 Hz, 2.0 Hz, 1H), 6.41 (s, 1H), 3.82 (s, 3H), 1.84 (s, 3H).

### Example 434. methyl-d₃ 4-amino-1-(4-aminophenyl)-7-bromo-6-fluoro-2-oxo-1,2-dihydroquinoline-3 -carboxylate

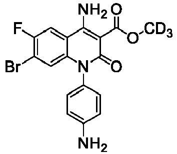

According to the preparation route of scheme 7, 2-amino-4-bromo-5-fluorobenzonitrile and 1-fluoro-4-nitrobenzene were used as the starting material to obtain the product with reference to the preparation route of Example 165.

¹H NMR (400 MHz, CD₃OD) δ 8.02 (d, *J* = 10.0 Hz, 1H), 6.86-6.93 (m, 5H).

### Example 435. methyl-d₃ 4-amino-1-(1-chloroisoquinolin-5-yl)-7-(difluoromethyl)-2-oxo-1,2-dihydroquinoline-3 -carboxylate

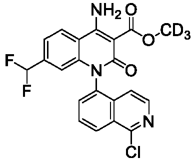

Compound methyl 4-amino-1-(1-chloroisoquinolin-5-yl)-7-(difluoromethyl)-2-oxo-1,2-dihydroquinoline-3-carboxylate obtained in Example 411 was used as the starting material to obtain the product with reference to the preparation route of Example 331.

¹H NMR (400 MHz, CDCl₃) δ 8.49 (dd, *J =* 8.5, 1.0 Hz, 1H), 8.12 (dd, *J =* 5.8, 0.8 Hz, 1H), 7.85 (m, 2H), 7.71 (dt, *J* = 7.4, 1.0 Hz, 1H), 7.24 (d, *J* = 8.4 Hz, 1H), 7.12 (dt, *J* = 5.9, 0.9 Hz, 1H), 6.39 (m, 2H).

### Example 436. methyl 4-amino-7-bromo-6-fluoro-1-(6-methylpyridin-3-yl)-2-oxo-1,2-dihydroquinoline-3 -carboxylate

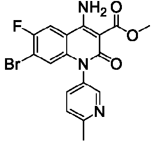

According to the preparation route of scheme 6, 2-amino-4-bromo-5-fluorobenzonitrile and 5-bromo-2-methylpyridine were used as the starting material to obtain the product with reference to the preparation route of Example 81.

¹H NMR (400 MHz, DMSO-*d₆*) δ 8.53 (s, 1H), 8.27 (brs, 2H), 7.79 (s, 1H), 7.71 (s, 1H), 7.55 (d, *J* = 8.1, 1H), 7.24 (d, *J* = 8.0 Hz, 1H), 3.75 (s, 3H), 2.51 (s, 3H).

### Example 437. methyl 4-amino-7-bromo-1-(4-chlorophenyl)-6-fluoro-2-oxo-1,2-dihydroquinoline-3 -carboxylate

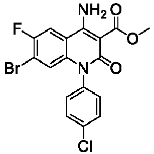

According to the preparation route of scheme 6, 2-amino-4-bromo-5-fluorobenzonitrile and 1-chloro-4-iodobenzene were used as the starting material to obtain the product with reference to the preparation route of Example 81.

¹H NMR (400 MHz, DMSO-*d₆*) δ 8.34 (brs, 2H), 7.79 (s, 1H), 7.72 (s, 1H), 7.51 (d, *J =* 8.6 Hz, 2H), 7.32 (d, *J* = 8.5 Hz, 2H), 3.73 (s, 3H)

### Example 438. methyl 4-amino-7-bromo-6-fluoro-1-(4-(1-hydroxyethyl)phenyl)-2-oxo-1,2-dihydroquinoline-3 -carboxylate

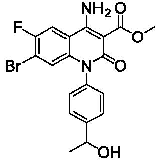

2-Amino-4-bromo-5-fluorobenzonitrile and 1-(4-iodophenyl)ethan-1-one were used as the starting material to obtain the product with reference to the preparation route of Example 120 and 122

¹H NMR (400 MHz, DMSO-*d₆*) δ 8.31 (brs, 2H), 7.78 (s, 1H), 7.69 (s, 1H), 7.39 *(d, J=* 8.7 Hz, 3H), 7.27 (d, *J* = 8.7 Hz, 1H), 5.28 (d, *J* = 4.8 Hz, 1H), 4.87 - 4.76 (m, 1H), 3.73 (s, 3H), 1.43 (d, *J* = 6.4 Hz, 3H).

### Example 439. methyl 4-amino-1-(6-aminopyridin-3-yl)-7-bromo-6-fluoro-2-oxo-1,2-dihydroquinoline-3 -carboxylate

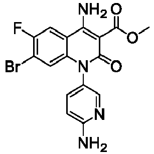

According to the preparation route of scheme 7, 2-amino-4-bromo-5-fluorobenzonitrile and 6-nitropyridin-3-amine were used as the starting material to obtain the product with reference to the preparation route of Example 165.

¹H NMR (400 MHz, DMSO-*d₆*) δ 8.37 (brs, 2H), 7.98 (s, 1H), 7.79 (s, 1H), 7.72 *(d, J=* 8.0 Hz, 1H), 7.71 (s, 1H), 6.58 (d, *J* = 8.1 Hz, 1H), 3.69 (s, 3H).

### Example 440. methyl 8-amino-5-(4-(1-(R)-hydroxyethyl)phenyl)-6-oxo-3-((2,2,2-trifluoro methylethyl)amino)- 5,6-dihydropyrido [2,3 -b]pyrazine-7-carboxylate

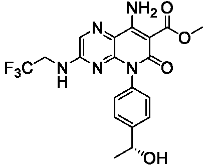

According to the preparation route of scheme 6, 3-chloro-5-((2,2,2-trifluoroethyl)amino) pyrimidine-5-carbonitrile and 4-(1-(*R)*-((*tert*-butyldimethylsilyl)oxy)ethyl)aniline were used as the starting material to obtain the product with reference to the preparation route of Example 81 and 340.

¹H NMR (400 MHz, CDCL3) δ 7.73 (s, 1H), 7.33-7.48 (m, 2H), 7.11 (d, *J =* 7.2 HZ, 2H), 4.84-4.94 (m, 1H), 3.68-3.76 (m, 2H), 3.88 (s, 3H), 1.41 (d, *J* = 6.8 Hz, 3H).

### Example 441. methyl 8-amino-5-(4-aminophenyl)-6-oxo-3-isopropoxypyrido[3,2-b]pyrazine-7-carboxylate

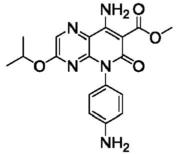

According to the preparation route of scheme 6, 3-chloro-5-isopropoxypyrazine-2-carbonitrile and 4-nitroaniline were used as the starting material to obtain the product with reference to the preparation route of Example 147, 165 and 387.

¹H NMR (400 MHz, DMSO-*d₆*) δ 8.18 (brs, 2H), 8.01 (s, 1H), 6.80 (d, *J* = 8.6 Hz, 2H), 6.59 (d, *J* = 8.6 Hz, 2H), 5.17 (brs, 2H), 4.75-4.63 (m, 1H), 3.70 (s, 3H), 1.13 (d, *J* = 6.2 Hz, 6H).

### Example 442. methyl 8-amino-5-(4-amino-2-methylphenyl)-6-oxo-3-isopropoxypyrido [3,2-b]pyrazine-7-carboxylate

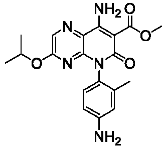

According to the preparation route of scheme 6, 3-chloro-5-isopropoxypyrazine-2-carbonitrile and 2-methyl-4-nitroaniline were used as the starting material to obtain the product with reference to the preparation route of Example 147, 165 and 387.

¹H NMR (400 MHz, DMSO-*d₆*) δ 7.70 (s, 1H), 7.65 (d, *J =* 7.4 Hz, 1H), 6.87-6.83 (m, 1H), 6.64 (d, *J* = 8.6 Hz, 1H), 6.52-6.45 (m, 2H), 5.07-4.93 (m, 1H), 4.79 (s, 2H), 3.80 (s, 3H), 2.20 (s, 3H), 1.15 (d, *J* = 6.8 Hz, 6H).

### Example 443. methyl 4-amino-1-(4-chlorophenyl)-7-cyclopropoxy-2-oxo-1,2-dihydro-1,8-naphthyridine-3 -carboxylate

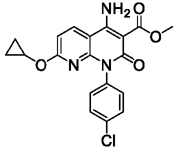

According to the preparation route of scheme 7, 2-chloro-6-cyclopropoxynicotinonitrile and 4-chloroaniline were used as the starting material to obtain the product with reference to the preparation route of Example 165.

¹H NMR (400 MHz, DMSO-*d₆*) δ 8.28 (brs, 2H), 7.93 (d, *J=* 9.4 Hz, 1H), 7.57 (d, *J* = 8.5 Hz, 2H), 7.33 (d, *J* = 8.5 Hz, 2H), 6.82 (s, 1H), 4.94 (m, 1H), 3.75 (s, 3H), 1.71-1.59 (m, 2H), 1.59-1.44 (m, 2H).

### Example 444. methyl 4-amino-1-(6-methylpyridin-3-yl)-2-oxo-7-(prop-2-yloxy)-1,2-dihydro-1,8-naphthyridine-3-carboxylate

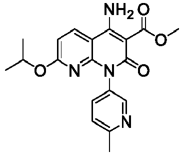

According to the preparation route of scheme 7, 2-chloro-6-(prop-2-yloxy)nicotinonitrile and 6-methylpyridin-3-amine were used as the starting material to obtain the product with reference to the preparation route of Example 165.

¹H NMR (400 MHz, DMSO-*d₆*) δ 8.75 (s, 1H), 8.34 (s, 2H), 7.93 (d, *J* = 9.4 Hz, 1H), 7.54 (d, *J* = 8.1 Hz, 1H), 7.17 (d, *J* = 9.0 Hz, 1H), 6.82 (d, *J* = 9.3 Hz, 1H), 5.06 (m, 1H), 3.73 (s, 3H), 2.51 (s, 3H), 1.35 (s, 6H).

### Example 445. methyl 4-amino-7-cyclopropoxy-1-(6-methylpyridin-3-yl)-2-oxo-1,2-dihydro-1,8-naphthyridine-3 -carboxylate

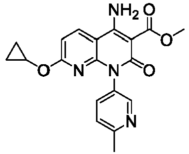

According to the preparation route of scheme 7, 2-chloro-6-cyclopropoxynicotinonitrile and 6-methylpyridin-3-amine were used as the starting material to obtain the product with reference to the preparation route of Example 165.

¹H NMR (400 MHz, DMSO-*d₆*) δ 8.78 (s, 1H), 8.37 (s, 2H), 7.91 (d, *J* = 9.2 Hz, 1H), 7.53 (d, *J* = 8.1 Hz, 1H), 7.14 (d, *J* = 7.3 Hz, 1H), 6.81 (d, *J* = 9.2 Hz, 1H), 4.64 (m, 1H), 3.76 (s, 3H), 2.53 (s, 3H), 1.70-1.61 (m, 2H), 1.58-1.48 (m, 2H).

### Example 446. methyl 4-amino-8-(4-aminophenyl)-7-oxo-2-(trifluoromethyl)-7H,8H-pyrido [2,3-d]pyrimidine-6-carboxylate

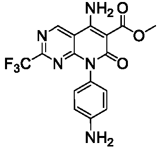

According to the preparation route of scheme 7, 4-chloro-2-(trifluoromethyl)pyrimidine-5-carbonitrile and 4-nitroaniline were used as the starting material to obtain the product with reference to the preparation route of Example 165 and Example 147.

¹H NMR (400 MHz, DMSO-*d₆*) δ 8.74 (s, 1H), 8.30 (s, 2H), 7.49 (d, *J* = 8.4 Hz, 1H), 6.57 (d, *J* = 8.3 Hz, 1H), 4.94 (s, 2H).

### Example 447. methyl 5-amino-8-(4-(1-(R)-hydroxyethyl)phenyl)-7-oxo-2-(trifluoromethyl)-7H, 8H-pyrido[2,3 -d]pyrimidine-6-carboxylate

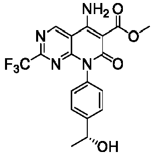

According to the preparation route of scheme 7, 4-chloro-2-(trifluoromethyl)pyrimidine-5-carbonitrile and 1-(4-aminophenyl)ethan-1-one were used as the starting material to obtain the product with reference to the preparation route of Example 81 and Example 340.

¹H NMR (400 MHz, DMSO-*d₆*) δ 8.73 (s, 1H), 8.34 (s, 2H), 7.44 (d, *J* = 8.6 Hz, 2H), 7.27 (d, *J =* 8.6 Hz, 2H), 5.30 (d, *J =* 4.8 Hz, 1H), 4.86-4.77 (m, 1H), 3.73 (s, 3H), 1.43 (d, *J =* 6.4 Hz, 3H).

### Example 448. methyl 8-amino-5-(6-methylpyridin-3-yl)-6-oxo-3-((2,2,2-trifluoroethyl) amino)-5,6-dihydropyrido[2,3-b]pyrazine-7-carboxylate

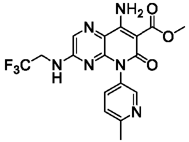

3-Chloro-5-((2,2,2-trifluoroethyl)amino)pyrazine-2-cyano and 6-methylpyridin-3-amine were used as the starting material to obtain the product with reference to the preparation route of Example 81 and Example 340.

¹H NMR (400 MHz, DMSO-d6) δ 8.75 (d, *J* = 1.9 Hz, 1H), 7.90 (t, *J* = 5.5 Hz, 1H), 7.55 (s, 1H), 7.54 (dd, *J* = 8.1, 2.0 Hz, 1H), 7.17 (d, *J* = 8.1 Hz, 1H), 6.95 (s, 2H), 4.05-4.09 (m, 2H), 3.73 (s, 3H), 2.50 (s, 3H).

### Example 449. methyl 8-amino-5-(4-chlorophenyl)-6-oxo-3-((2,2,2-trifluoroethyl)amino)-5H,6H- pyrido[2,3-b]pyrazine-7-carboxylate

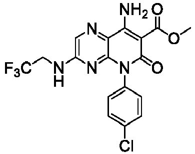

According to the preparation route of scheme 7, 3-chloro-5-[(2,2,2-trifluoroethyl) amino]pyrazine-2-carbonitrile and 4-chloroaniline were used as the starting material to obtain the product with reference to the preparation route of Example 165.

¹H NMR (400 MHz, DMSO-*d₆*) δ 8.59 (s, 1H), 7.92 (s, 1H), 7.52 (d, *J=* 8.6 Hz, 2H), 7.22 (d, *J* = 8.6 Hz, 2H), 3.81 (s, 2H), 3.70 (s, 3H).

### Example 450. methyl-d₃ 4-amino-1-(4-amino-2-methylphenyl)-7-bromo-2-oxo-1,2-dihydroquinoline-3 -carboxylate

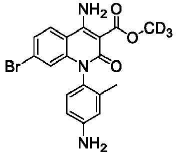

Methyl 4-amino-1-(4-amino-2-methylphenyl)-7-bromo-2-oxo-1,2-dihydroquinoline-3 - carboxylate obtained in Example 238 was used as the starting material to obtain the product with reference to the preparation route of Example 311.

¹H NMR (400 MHz, DMSO-*d₆*) δ 8.28 (s, 2H), 8.13 (d, *J* = 8.4 Hz, 1H), 7.36 (dd, *J* = 8.8 Hz, 2.0 Hz, 1H), 6.70 (d, *J* = 8.0 Hz, 1H), 6.49-6.56 (m, 3H), 5.25 (s, 2H), 1.70 (s, 3H).

### Example 451. methyl-d₃ 4-amino-1-(4-aminophenyl)-2-oxo-7-(trifluoromethyl)-1,2-dihydro-1,8-naphthyridine-3 -carboxylate

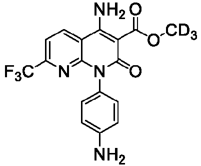

Methyl 4-amino-1-(4-aminophenyl)-2-oxo-7-(trifluoromethyl)-1,2-dihydro-1,8-naphthyridine-3-carboxylate obtained in Example 274 was used as the starting material to obtain the product with reference to the preparation route of Example 311.

¹H NMR (400 MHz, DMSO-*d₆*) δ 8.84 (d, *J* = 8.4 Hz, 1H), 8.31 (br, 2H), 7.68 (d, *J* = 8.4 Hz, 1H), 6.74 (d, *J* = 8.8Hz, 2H), 6.58 (d, *J* = 8.4 Hz, 2H), 5.14 (br, 2H).

### Example 452. methyl 4-amino-1-(4-aminophenyl)-7-((2,2,2-trifluoroethyl) amino)-2-oxo-1,2-dihydroquinoline-3 -carboxylate

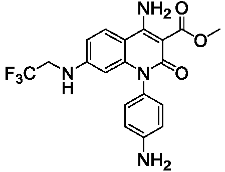

According to the preparation route of scheme 7, 2-chloro-4-((2,2,2-trifluoroethyl)amino) benzonitrile and 4-nitroaniline were used as the starting material to obtain the product with reference to the preparation route of Example 165 and Example 147.

¹H NMR (400 MHz, DMSO-*d₆*) δ 8.31 (d, *J* = 8.8 Hz, 1H), 8.29 (s, 2H), 7.17 (d, *J* = 8.8 Hz, 1H), 6.80 (d, *J* = 8.8 Hz, 2H), 6.67 (d, *J* = 8.8 Hz, 2H), 6.45 (d, *J* = 8.7 Hz, 1H), 6.33 (s, 1H),5.19 (m, 2H), 3.68 (s, 3H).

### Example 453. methyl-d₃ 4-amino-1-(4-aminophenyl)-7-((2,2,2-trifluoroethyl)amino)-2-oxo-1,2-dihydroquinoline-3 -carboxylate

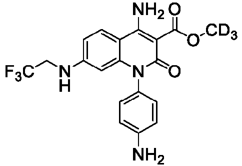

Methyl 4-amino-1-(4-aminophenyl)-7-((2,2,2-trifluoroethyl)amino)-2-oxo-1,2-dihydroquinoline-3-carboxylate obtained in Example 274 was used as the starting material to obtain the product with reference to the preparation route of Example 311.

¹H NMR (400 MHz, DMSO-*d₆*) δ 8.31 (d, *J* = 8.8 Hz, 1H), 8.29 (s, 2H), 7.17 (d, *J* = 8.8 Hz, 1H), 6.80 (d, *J* = 8.8 Hz, 2H), 6.67 (d, *J* = 8.8 Hz, 2H), 6.45 (d, *J* = 8.7 Hz, 1H), 6.33 (s, 1H),5.19 (m, 2H).

### Example 454. methyl 4-amino-1-(4-aminophenyl)-7-((2,2,2-trifluoroethyl) amino)-2-oxo-1,2-dihydro-1,8-naphthyridine-3-carboxylate

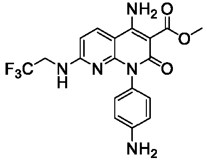

According to the preparation route of scheme 7, 2-chloro-6-((2,2,2-trifluoroethyl) amino)-3-nicotinonitrile and 4-nitroaniline were used as the starting material to obtain the product with reference to the preparation route of Example 165 and Example 147.

¹H NMR (400 MHz, CD₃OD+CDCl₃) δ 8.23 (d, *J* = 8.8 Hz, 1H), 6.87 (d, *J* = 8.8 Hz, 2H), 6.84 (t, *J =* 72.4 Hz, 1H), 6.73 (d, *J =* 8.8 Hz, 2H), 5.19 (m, 2H), 3.68 (s, 3H).

### Example 455. methyl-d₃ 4-amino-1-(4-aminophenyl)-7-((2,2,2-trifluoroethyl) amino)-2-oxo-1,2-dihydro-1,8-naphthyridine-3 -carboxylate

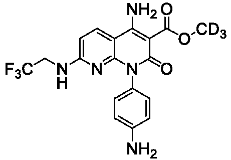

Methyl 4-amino-1-(4-aminophenyl)-7-((2,2,2-trifluoroethyl)amino)-2-oxo-1,2-dihydro-1,8-naphthyridine-3-carboxylate obtained in Example 454 was used as the starting material to obtain the product with reference to the preparation route of Example 311.

¹H NMR (400 MHz, CD₃OD+CDCl₃) δ 8.23 (d, *J* = 8.8 Hz, 1H), 6.87 (d, *J* = 8.8 Hz, 2H), 6.84 (t, *J =* 72.4 Hz, 1H), 6.73 (d, *J =* 8.8 Hz, 2H), 5.19 (m, 2H).

### Example 456. methyl 4-amino-1-(4-aminophenyl)-7-(ethoxy)-2-oxo-1,2-dihydro-1,8-naphthyridine-3 -carboxylate

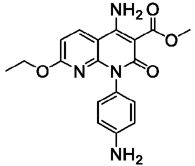

According to the preparation route of scheme 7, 4-ethoxy-2-chloro3-nicotinonitrile and 4-nitroaniline were used as the starting material to obtain the product with reference to the preparation route of Example 165 and 147.

¹H NMR (400 MHz, CD₃OD+CDCl₃) δ 8.20 (d, *J* = 8.8 Hz, 1H), 6.84 (d, *J* = 8.8 Hz, 2H), 6.81 (t, *J* = 72.4 Hz, 1H), 6.70 (d, *J* = 8.8 Hz, 2H), 3.85-3.90 (m, 2H), 3.66 (s, 3H), 1.22 (t, *J =* 7.2 Hz, 3H).

### Example 457. methyl-d₃ 4-amino-1-(4-aminophenyl)-7-(ethoxy)-2-oxo-1,2-dihydro-1,8-naphthyridine-3 -carboxylate

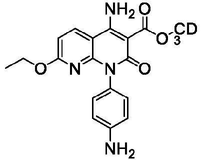

Methyl 4-amino-1-(4-aminophenyl)-7-(ethoxy)-2-oxo-1,2-dihydro-1,8-naphthyridine-3-carboxylate obtained in Example 456 was used as the starting material to obtain the product with reference to the preparation route of Example 311.

¹H NMR (400 MHz, CD₃OD+CDCl₃) δ 8.20 (d, *J* = 8.8 Hz, 1H), 6.84 (d, *J* = 8.8 Hz, 2H), 6.81 (t, *J* = 72.4 Hz, 1H), 6.70 (d, *J* = 8.8 Hz, 2H), 3.85-3.90 (m, 2H), 1.22 (t, *J* = 7.2 Hz, 3H).

### Example 458. methyl-d₃ 4-atnino-1-(4-aminophenyl)-7-ethoxy-2-oxo-1,2-dihydroquinoline-3-carboxylate

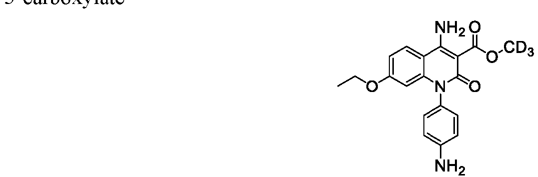

Methyl 4-amino-1-(4-aminophenyl)-7-ethoxy-2-oxo-1,2-dihydroquinoline-3-carboxylate obtained in Example 276 was used as the starting material to obtain the product with reference to the preparation route of Example 311.

¹H NMR (400 MHz, DMSO-*d₆*) δ 8.26 (br, 2H), 8.08 (d, *J* = 8.8 Hz, 1H), 6.74-6.80 (m, 3H), 6.65 (d, J = 8.4 Hz, 2H), 5.89 (d, *J* = 2.8 Hz, 1H), 5.29 (br, 2H), 3.85-3.90 (m, 2H) 1.22 (t, *J* = 7.2 Hz, 3H).

### Example 459. methyl 4-amino-1-(4-amino-2-methylphenyl)-7-((2,2,2-trifluoroethyl)amino)-2-oxo-1,2-dihydroquinoline-3-carboxylate

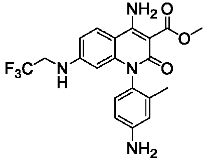

According to the preparation route of scheme 7, 2-chloro-4-((2,2,2-trifluoroethyl) amino)benzonitrile and 4-nitro-2-methylaniline were used as the starting material to obtain the product with reference to the preparation route of Example 165 and Example 147.

¹H NMR (400 MHz, DMSO-*d₆*) δ 8.13 (d, *J* = 8.4 Hz, 1H), 7.36 m, 1H), 6.70 (d, *J =* 8.0 Hz, 1H), 6.49-6.56 (m, 3H), 5.19 (m, 2H), 3.70 (s, 3H), 1.70 (s, 3H).

### Example 460. methyl-d₃ 4-amino-1-(4-amino-2-methylphenyl)-7-((2,2,2-trifluoroethyl) amino)-2-oxo-1,2-dihydroquinoline-3-carboxylate

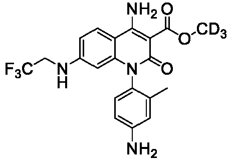

Methyl 4-amino-1 -(4-amino-2-methylphenyl)-7-((2,2,2-trifluoroethyl)amino)-2-oxo-1,2-dihydroquinoline-3-carboxylate obtained in Example 459 was used as the starting material to obtain the product with reference to the preparation route of Example 311.

¹H NMR (400 MHz, DMSO-*d₆*) δ 8.13 (d, *J* = 8.4 Hz, 1H), 7.36 m, 1H), 6.70 (d, *J* = 8.0 Hz, 1H), 6.49-6.56 (m, 3H), 5.19 (m, 2H),1.70 (s, 3H).

### Example 461. methyl 4-amino-1-(4-amino-2-methylphenyl)-7-((2,2,2-trifluoroethyl)amino)-2-oxo-1,2-dihydro-1,8-naphthyridine-3-carboxylate

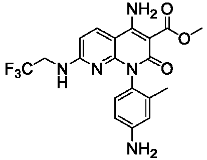

According to the preparation route of scheme 7, 2-chloro-6-((2,2,2-trifluoroethyl) amino)-3-nicotinonitrile and 4-nitro-2-methylaniline were used as the starting material to obtain the product with reference to the preparation route of Example 165 and Example 147.

¹H NMR (400 MHz, DMSO-*d₆*) δ 8.53 (d, *J* = 8.8 Hz, 1H), 7.49 (d, *J* = 8.8 Hz, 1H), 6.89 (d, *J* = 8.4 Hz, 1H), 6.70 (d, *J* = 2.4 Hz, 1H), 6.55 (dd, *J* = 8.8 Hz, 2.4 Hz, 1H), 5.19 (m, 2H), 3.70 (s, 3H), 1.83 (s, 3H).

### Example 462. methyl-d₃ 4-amino-1-(4-amino-2-methylphenyl)-7-((2,2,2-trifluoroethyl) amino)-2-oxo-1,2-dihydro-1,8-naphthyridine-3-carboxylate

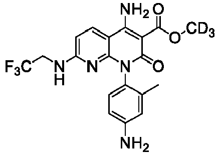

Methyl 4-amino-1-(4-amino-2-methylphenyl)-7-((2,2,2-trifluoroethyl)amino)-2-oxo-1,2-dihydro-1,8-naphthyridine-3-carboxylate obtained in Example 461 was used as the starting material to obtain the product with reference to the preparation route of Example 311.

¹H NMR (400 MHz, DMSO-*d₆*) δ 8.53 (d, *J* = 8.8 Hz, 1H), 7.49 (d, *J* = 8.8 Hz, 1H), 6.89 (d, *J* = 8.4 Hz, 1H), 6.70 (d, *J* = 2.4 Hz, 1H), 6.55 (dd, *J* = 8.8 Hz, 2.4 Hz, 1H), 5.19 (m, 2H), 1.83 (s, 3H).

### Biological testing

### 1. In vitro enzymatic inhibition activity assay of compounds on MAT2A

Enzymatic activity assays of compounds of this patent against MAT2A (to detect half inhibitory concentration IC50 values of the compounds) were performed using the phosphate detection kit PiColorLockTM (ab270004, purchased from Abcam). This method utilized the principle that purified human MAT2A enzyme catalyzes the formation of S-adenosylmethionine (SAM) from L-methionine and ATP to release free phosphate, and used the spectrophotometric method to quantify phosphate, so as to determine the enzymatic activity of MAT2A. Compounds were diluted in a 10-fold gradient starting at 1 mM with 100% DMSO (for a total of 8 concentrations) and 2 µL of each concentration was added to 48 µL of reaction buffer (50 mM Tris-HCl PH 7.5, 50 mM KCl, 10 mM MgCl₂, 0.01% Brij-35, 0.01% BSA, 1 mM DTT) and mixed well. The final diluted compound was prepared, and 5 µL of MAT2A enzyme (final concentration of 10 nM), 5 µL of ATP (final concentration of 50 µM), and 5 µL of L-methionine (final concentration of 100 µM) were added to each well of a 384-well plate (#3701, purchased from CORNING). After the 384-well plate was placed in the incubator at 23°C for 6 h, 5 µL of PiColorLock reagent diluted Accelerator (dilution ratio 1:100) was added to each well, and the reaction was stopped by incubating in the incubator at 23°C for 5 min. 2 µL of Stabiliser was added, the absorbance was read at 630 nm on BMG CLARIOstar, and the IC50 value of the compound was calculated by using GraphPad Prism software.

### Experimental results:

Under the conditions of this experiment, the inhibition of MAT2A by test compounds can be expressed as an IC₅₀ value for the inhibition of the level of phosphate production during the enzymatic reaction. The MAT2A inhibitory activity of some of the examples is shown in Table 1.

**Table 1. MAT2A inhibitory activity**

| Compound | IC₅₀ (nM) | Compound | IC₅₀ (nM) | Compound | IC₅₀ (nM) |
|---|---|---|---|---|---|
| Example 4 | 2.28 | Example 25 | 0.43 | Example 59 | 5.12 |
| Example 16 | 1.23 | Example 26 | 1.04 | Example 63 | 1.58 |
| Example 17 | 3.58 | Example 28 | 3.62 | Example 66 | 6.76 |
| Example 19 | 2.34 | Example 29 | 3.79 | Example 69 | 6.39 |
| Example 20 | 5.98 | Example 30 | 6.10 | Example 73 | 1.67 |
| Example 21 | 6.58 | Example 49 | 8.58 | Example 76 | 1.71 |
| Example 22 | 0.54 | Example 50 | 4.57 | Example 78 | 2.72 |
| Example 23 | 0.91 | Example 51 | 2.62 | Example 79 | 0.61 |
| Example 24 | 2.34 | Example 52 | 2.38 | Example 80 | 4.03 |

| Compound | IC₅₀ (nM) | Compound | IC₅₀ (nM) | Compound | IC₅₀ (nM) |
|---|---|---|---|---|---|
| Example 81 | 1.68 | Example 128 | 3.49 | Example 154 | 3.00 |
| Example 83 | 1.21 | Example 130 | 5.99 | Example 155 | 1.93 |
| Example 86 | 2.37 | Example 132 | 4.91 | Example 158 | 3.83 |
| Example 87 | 1.86 | Example 133 | 2.79 | Example 159 | 2.87 |
| Example 88 | 7.30 | Example 134 | 2.01 | Example 161 | 4.67 |
| Example 91 | 0.93 | Example 135 | 8.85 | Example 164 | 7.58 |
| Example 92 | 0.83 | Example 137 | 3.34 | Example 173 | 1.18 |
| Example 93 | 7.39 | Example 145 | 4.60 | Example 174 | 5.12 |
| Example 97 | 9.95 | Example 147 | 0.78 | Example 175 | 6.25 |
| Example 101 | 0.59 | Example 148 | 2.83 | Example 176 | 2.74 |
| Example 107 | 1.52 | Example 149 | 3.12 | Example 178 | 2.59 |
| Example 111 | 4.06 | Example 150 | 1.66 | Example 180 | 0.76 |
| Example 115 | 2.89 | Example 151 | 2.86 | Example 182 | 1.16 |
| Example 122 | 2.17 | Example 152 | 1.64 | / | / |
| Example 125 | 2.67 | Example 153 | 2.62 | / | / |

| Compound | IC₅₀ (nM) | Compound | IC₅₀ (nM) | Compound | IC₅₀ (nM) |
|---|---|---|---|---|---|
| Example 185 | 8.94 | Example 215 | 2.50 | Example 248 | 1.69 |
| Example 187 | 0.76 | Example 216 | 2.34 | Example 249 | 2.99 |
| Example 189 | 0.32 | Example 219 | 1.87 | Example 250 | 6.06 |
| Example 192 | 6.47 | Example 220 | 2.07 | Example 252 | 0.14 |
| Example 195 | 4.91 | Example 221 | 2.71 | Example 253 | 1.76 |
| Example 196 | 0.81 | Example 222 | 8.38 | Example 257 | 2.09 |
| Example 199 | 3.57 | Example 224 | 2.81 | Example 258 | 1.31 |
| Example 200 | 1.63 | Example 225 | 3.11 | Example 259 | 1.08 |
| Example 201 | 5.38 | Example 227 | 1.96 | Example 263 | 1.41 |
| Example 203 | 1.26 | Example 229 | 4.18 | Example 265 | 0.94 |
| Example 204 | 4.62 | Example 231 | 2.36 | Example 266 | 2.37 |
| Example 205 | 4.47 | Example 237 | 1.64 | Example 267 | 7.38 |
| Example 207 | 2.17 | Example 238 | 1.24 | Example 268 | 3.39 |
| Example 208 | 4.09 | Example 240 | 1.72 | Example 269 | 5.95 |
| Example 211 | 1.84 | Example 241 | 1.42 | Example 271 | 1.83 |
| Example 212 | 2.83 | Example 242 | 1.26 | Example 272 | 0.78 |
| Example 214 | 2.89 | Example 244 | 0.86 | / | / |

| Compound | IC₅₀ (nM) | Compound | IC₅₀ (nM) | Compound | IC₅₀ (nM) |
|---|---|---|---|---|---|
| Example 273 | 0.92 | Example 296 | 2.00 | Example 322 | 1.84 |
| Example 274 | 0.53 | Example 297 | 2.04 | Example 325 | 2.29 |
| Example 275 | 0.15 | Example 298 | 0.43 | Example 327 | 1.25 |
| Example 280 | 1.72 | Example 303 | 0.92 | Example 328 | 2.59 |
| Example 282 | 2.20 | Example 304 | 2.28 | Example 329 | 2.65 |
| Example 283 | 0.70 | Example 307 | 1.14 | Example 330 | 0.92 |
| Example 284 | 2.44 | Example 308 | 2.42 | Example 331 | 2.49 |
| Example 287 | 1.45 | Example 309 | 2.02 | Example 332 | 2.28 |
| Example 289 | 1.04 | Example 310 | 0.74 | Example 335 | 2.06 |
| Example 290 | 0.79 | Example 311 | 0.6 | Example 336 | 1.94 |
| Example 291 | 2.18 | Example 318 | 2.34 | Example 338 | 1.45 |
| Example 294 | 1.08 | Example 319 | 1.20 | Example 339 | 2.30 |
| Example 295 | 0.89 | Example 320 | 1.19 | Example 340 | 1.44 |

| Compound | IC₅₀ (nM) | Compound | IC₅₀ (nM) | Compound | IC₅₀ (nM) |
|---|---|---|---|---|---|
| Example 343 | 2.39 | Example 353 | 1.25 | Example 358 | 1.36 |
| Example 348 | 1.33 | Example 354 | 0.46 | Example 360 | 1.27 |
| Example 351 | 1.05 | Example 356 | 1.19 | Example 365 | 2.02 |
| Example 352 | 2.58 | Example 357 | 0.54 | Example 366 | 1.00 |

| Compound | IC₅₀ (nM) | Compound | IC₅₀ (nM) | Compound | IC₅₀ (nM) |
|---|---|---|---|---|---|
| Example 371 | 1.45 | Example 379 | 1.41 | Example 401 | 1.49 |
| Example 372 | 1.96 | Example 382 | 1.88 | Example 412 | 0.97 |
| Example 373 | 1.05 | Example 388 | 1.55 | Example 429 | 1.15 |
| Example 377 | 1.89 | Example 391 | 1.31 | / | / |
| Example 378 | 1.20 | Example 393 | 1.52 | / | / |

### 2. Activity assay of compound inhibiting the proliferation of HCT116 MTAP-/- cells

The MTAP deficient human colorectal cancer cell line HCT116 MTAP-/- was constructed using CRISPR Cas9 technology and cultured in DMEM medium supplemented with 10% fetal bovine serum (FBS, purchased from Gibco) and 1% penicillin/streptomycin (P/S, purchased from Thermo Fisher Scientific) at 37°C with 5% CO₂. HCT116 MTAP-/- cells were plated in 96 well plates (# 3917, purchased from CORNING) at a concentration of 200 cells/195 µL/well. After 24 h, the compounds were diluted in a 3-fold gradient starting at 10 mM with 100% DMSO (for a total of 10 concentrations), then 4 µL of each concentration was added to 96 µL of serum-free DMEM medium for dilution. 5 µL of diluted compound at each concentration was added to the plated cell suspension and the compound was incubated with the cells for 168 h (7 days) in a cell culture incubator. The medium was then removed and 25 µL of Cell-Titer Glo ^{®} (G7570, purchased from Promega) reagent was added. Chemiluminescence values were read on a BMG and data were processed using GraphPad Prism software to calculate IC50 values for inhibition of cell proliferation by the compounds.

The experimental results of some examples are shown in Table 2.

**Table 2. HCT116 MTAP-/-antiproliferative activity**

| **Compound No.** | **IC₅₀ (nM)** | **Compound No.** | **IC₅₀ (nM)** |
|---|---|---|---|
| Example 86 | 85.2 | Example 207 | 53.2 |
| Example 92 | 87.2 | Example 208 | 70.9 |
| Example 122 | 50.0 | Example 221 | 41.4 |
| Example 147 | 81.5 | Example 240 | 61.2 |
| Example 154 | 35.1 | Example 244 | 46.4 |
| Example 173 | 30.9 | Example 252 | 14.2 |
| Example 174 | 79.0 | Example 265 | 47.3 |
| Example 175 | 53.5 | Example 266 | 32.7 |
| Example 176 | 39.2 | Example 272 | 16.6 |
| Example 199 | 48.5 | Example 340 | 2.1 |
| Example 200 | 23.4 | Example 378 | 1.5 |
| Example 203 | 25.0 | / | / |

### 3. Animal pharmacokinetic studies of the examples:

In vivo pharmacokinetic study in CD-1 mice

Twenty-four healthy adult male mice from Beijing Vital River Laboratory Animal Technology Co., Ltd. were used in the study.

CD-1 mice were administered with a single oral gavage dose of 10 mg/kg in a volume of 10 mL/kg at a concentration of 1 mg/mL.

The test article in this project is generally suspended in 0.5% hydroxypropylmethyl cellulose, 0.1% Tween 80 (W/V/V) aqueous solution at the concentration of 1 mg/mL.

Animals dosed by gavage were fasted overnight before the experiment from 16 h before dosing to 4 h after dosing. Blood was collected at 0.25, 0.5, 1, 2, 4, 6, 8, and 24 h after administration successively. The animals were lightly anesthetized with isoflurane. About 0.2 mL of whole blood was collected from the orbital venous plexus using a glass blood collection tube and placed in a sodium heparin anticoagulant tube. The sample was centrifuged at 5200 rpm for 5 min at 4°C. The plasma was transferred to a centrifuge tube and stored at -80°C until analysis.

Analysis of plasma samples Test compounds and internal standards (warfarin or propranolol) were extracted from mouse plasma using an acetonitrile protein precipitation method and the extracts were analyzed by LC/MS/MS. The measured plasma concentration-time data of individual animals were analyzed using the non-compartmental model of WinNonlin (version 5.2.1, Pharsight) software to obtain the corresponding pharmacokinetic parameters: maximum (peak) plasma drug concentration Cₘₐₓ; Time to peak Tₘₐₓ; Half-life T_{1/2} and area under the plasma concentration-time curve extrapolated to infinite time AUC0-inf.
The experimental results of some examples are shown in Table 3.

**Table 3**

| Compound No. | AUCall (hr*ng/mL) | T_{1/2} (hr) | Tₘₐₓ (hr) | Cₘₐₓ (ng/mL) |
|---|---|---|---|---|
| Example 15 | 44186 | 8.88 | 6.00 | 3081 |
| Example 22 | 25306 | 6.82 | 6.00 | 1925 |
| Example 23 | 37032 | 6.51 | 6.00 | 2640 |
| Example 88 | 24844 | 3.88 | 0.42 | 6689 |
| Example 147 | 24067 | 8.44 | 1.67 | 2316 |
| Example 174 | 24483 | 3.08 | 0.5 | 9543 |
| Example 180 | 32806 | 4.09 | 2.08 | 5061 |
| Example 199 | 24602 | 2.98 | 2.50 | 4268 |
| Example 214 | 231892 | 2.53 | 1.00 | 28602 |
| Example 238 | 591218 | 9.25 | 4.00 | 37488 |
| Example 325 | 452267 | 6.81 | 1.00 | 52767 |
| Example 379 | 468321 | 16.50 | 4.00 | 18551 |

## Claims

1. A compound of Formula (I) or a pharmaceutically acceptable salt, solvate, polymorph or tautomer thereof 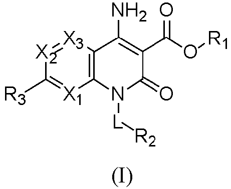 wherein,
L is bond or CH₂, CH₂ can be optionally substituted with C₁₋₆ alkyl or 3-8 membered cycloalkyl,
X₁, X₂ and X₃ are each independently selected from N and CR₄,
R₁ is selected from C₁₋₆ alkyl, 3-8 membered cycloalkyl, 3-8 membered heterocycloalkyl, 6-10 membered aryl and 5-12 membered heteroaryl, the alkyl, cycloalkyl, heterocycloalkyl, aryl, or heteroaryl can be optionally substituted with halogen, -CN, -OH, -NH₂, C₁₋₆ alkyl, 3-8 membered cycloalkyl, 3-8 membered heterocycloalkyl, 6-10 membered aryl, 5-12 membered heteroaryl, -O-C₁₋₆ alkyl, or -N(R)-C₁₋₆ alkyl,
R₂ is 3-8 membered carbocycle, 3-8 membered heterocycle, benzene ring, or 5-6 membered heteroaromatic ring, said carbocycle, heterocycle, benzene ring and heteroaromatic ring can be optionally fused with 3-8 membered carbocycle, 3-8 membered heterocycle, benzene ring, or 5-6 membered heteroaromatic ring, said carbocycle, heterocycle, benzene ring, or heteroaromatic ring can be optionally substituted with 1-3 R₁₂,
R₁₂ are each independently selected from halogen, NO₂, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -(CO)-C₁₋₆ alkyl, -(CO)-C₃₋₈ cycloalkyl, -COOH, -(CO)-O-C₁₋₆ alkyl, -(CO)-NH₂, -(CO)-NH-C₁₋₆ alkyl, -O-C₁₋₆ alkyl and -N(R)-C₁₋₆ alkyl, the alkyl, cycloalkyl and heterocycloalkyl can be optionally substituted with halogen, -CN, -OH, -NH₂, -NH-(CO)-O-*tert-*butyl, =N-OH, C₁₋₆ alkyl, 3-8 membered cycloalkyl, 3-8 membered heterocycloalkyl, -O-C₁₋₆ alkyl, or -N(R)-C₁₋₆ alkyl,
R₃ is selected from H, halogen, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl, -S-C₁₋₆ alkyl, -NH(R), -N(R)-C₁₋₆ alkyl, -N(R)-(3-8 membered cycloalkyl), -N(R)-(3-8 membered heterocycloalkyl) and 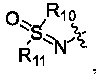 the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl,
R₄ are each independently selected from H, halogen, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl and -N(R)-C₁₋₆ alkyl, the alkyl, cycloalkyl and heterocycloalkyl can be optionally substituted with halogen, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl, or -N(R)-C₁₋₆ alkyl,
R are each independently selected from H, C₁₋₆ alkyl, 3-8 membered cycloalkyl and 3-8 membered heterocycloalkyl, the alkyl, cycloalkyl and heterocycloalkyl can be optionally substituted with halogen, -CN, -OH, -NH₂, C₁₋₆ alkyl, 3-8 membered cycloalkyl, or 3-8 membered heterocycloalkyl,
R₁₀ and R₁₁ are each independently selected from C₁₋₆ alkyl and 3-8 membered cycloalkyl, the alkyl and cycloalkyl can be optionally substituted with halogen, -CN, -OH, or -NH₂,
with the proviso that the following compound is not included :

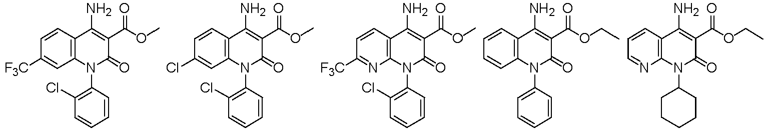

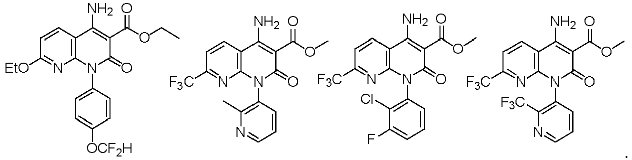

2. The compound according to claim 1 or a pharmaceutically acceptable salt, solvate, polymorph or tautomer thereof, wherein R₃ is selected from H, halogen, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl, -NH(R), -N(R)-C₁₋₆ alkyl, -N(R)-(3-8 membered cycloalkyl), -N(R)-(3-8 membered heterocycloalkyl) and 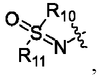 the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl, R, R₁₀ and R₁₁ are as defined in claim 1.

3. The compound according to claim 1 or a pharmaceutically acceptable salt, solvate, polymorph or tautomer thereof, wherein R₃ is selected from H, halogen, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl, -N(R)-C₁₋₆ alkyl and 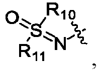 the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl, R, R₁₀ and R₁₁ are as defined in claim 1.

4. The compound according to claim 3 or a pharmaceutically acceptable salt, solvate, polymorph or tautomer thereof, wherein R₁₂ are each independently selected from halogen, NO₂, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -(CO)-C₁₋₆ alkyl, -(CO)-C₃₋₈ cycloalkyl, -COOH, -(CO)-O-C₁₋₆ alkyl, -(CO)-NH₂, -(CO)-NH-C₁₋₆ alkyl, -O-C₁₋₆ alkyl and -N(R)-C₁₋₆ alkyl, the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl, R is as defined in claim 3.

5. The compound according to claim 3 or a pharmaceutically acceptable salt, solvate, polymorph or tautomer thereof, wherein
R₁₂ are each independently selected from halogen, CF₃, -CN, -OH, -NH₂, C₁₋₆ alkyl, C₂₋₆ alkenyl, C₂₋₆ alkynyl, 3-8 membered cycloalkyl, 3-8 membered heterocycloalkyl, -O-C₁₋₆ alkyl and -N(R)-C₁₋₆ alkyl, the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, -O-C₁₋₆ alkyl, or -N(R)-C₁₋₆ alkyl,
R are each independently selected from C₁₋₆ alkyl, 3-8 membered cycloalkyl and 3-8 membered heterocycloalkyl, the alkyl, cycloalkyl and heterocycloalkyl can be optionally substituted with halogen, -CN, -OH, -NH₂, C₁₋₆ alkyl, 3-8 membered cycloalkyl, or 3-8 membered heterocycloalkyl.

6. The compound according to claim 1 or a pharmaceutically acceptable salt, solvate, polymorph or tautomer thereof, wherein L is bond, R₁ is selected from C₁₋₆ alkyl, 3-8 membered cycloalkyl and 3-8 membered heterocycloalkyl, the alkyl, cycloalkyl and heterocycloalkyl can be optionally substituted with halogen, -CN, -OH, -NH₂, C₁₋₆ alkyl, 3-8 membered cycloalkyl, 3-8 membered heterocycloalkyl, 6-10 membered aryl, 5-12 membered heteroaryl, -O-C₁₋₆ alkyl, or -N(R)-C₁₋₆ alkyl, R is as defined in claim 1.

7. The compound according to claim 1 or a pharmaceutically acceptable salt, solvate, polymorph or tautomer thereof, wherein R₃ is selected from H, halogen, CF₃, -CN, -OH, -NH₂, C₁₋₆ alkyl, C₂₋₆ alkenyl, C₂₋₆ alkynyl and 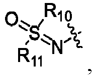 R₁₀ and R₁₁ are as defined in claim 1.

8. The compound according to claim 1 or a pharmaceutically acceptable salt, solvate, polymorph or tautomer thereof, wherein R₂ is benzene ring or 5-6 membered heteroaromatic ring, the benzene ring and heteroaromatic ring can be optionally fused with 3-8 membered carbocycle, 3-8 membered heterocycle, benzene ring, or 5-6 membered heteroaromatic ring, said carbocycle, heterocycle, benzene ring, or heteroaromatic ring can be optionally substituted with 1-3 R₁₂, R₁₂ is as defined in claim 1.

9. The compound below or a pharmaceutically acceptable salt, solvate, polymorph or tautomer thereof

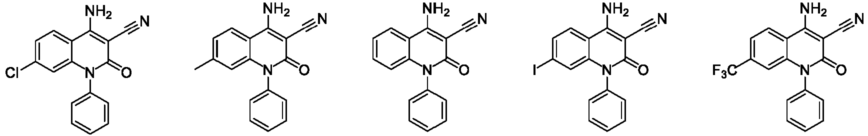

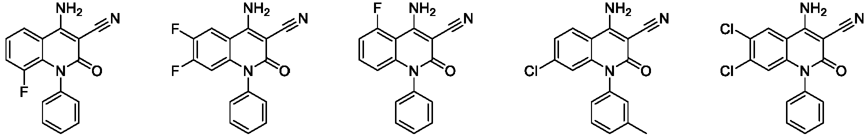

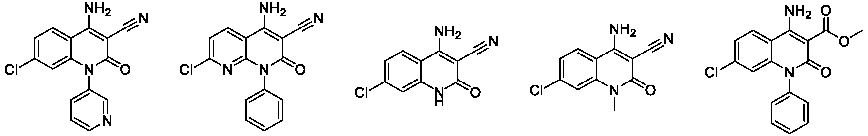

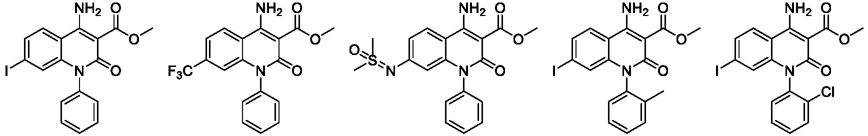

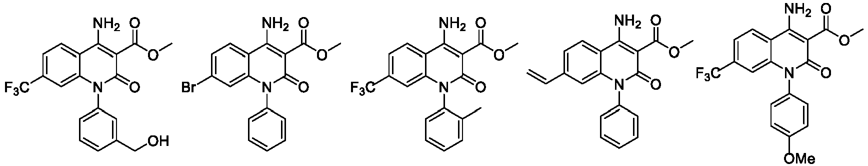

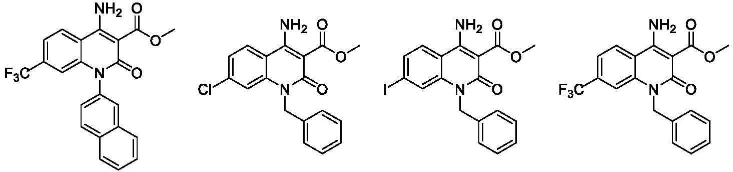

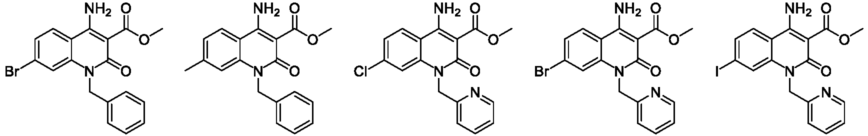

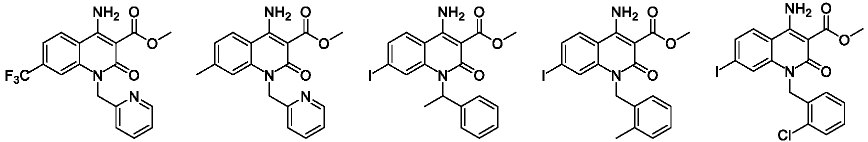

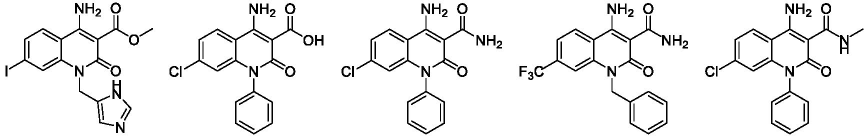

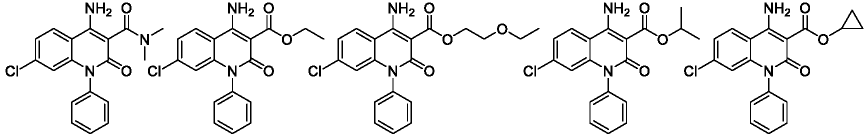

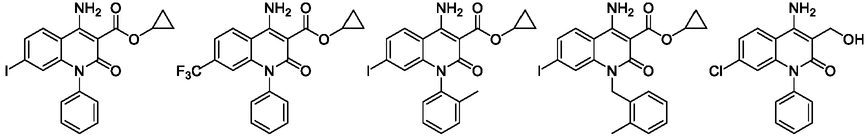

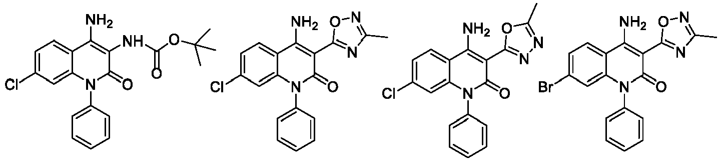

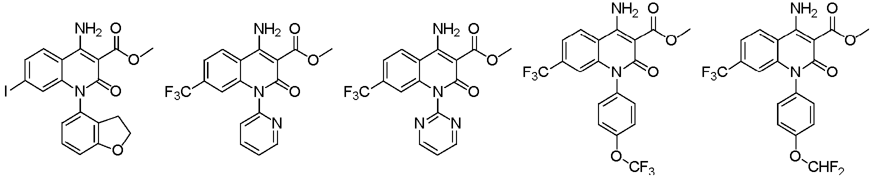

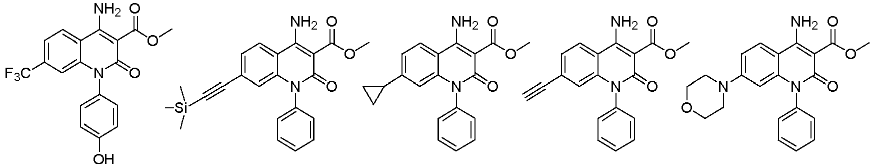

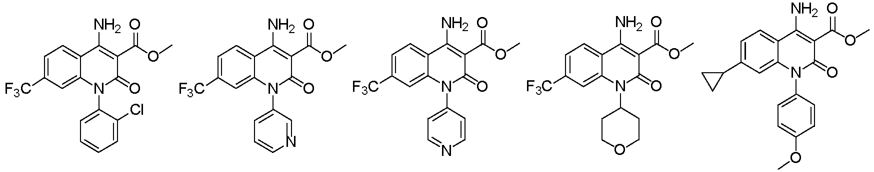

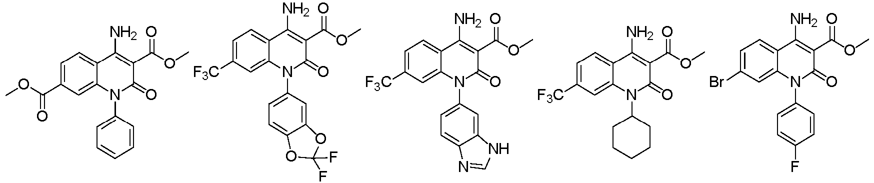

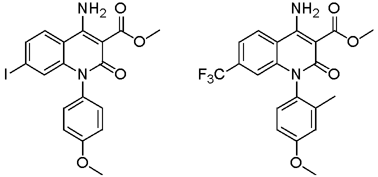

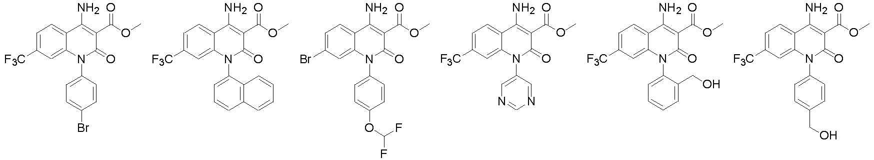

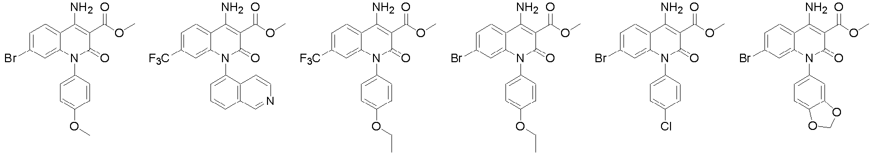

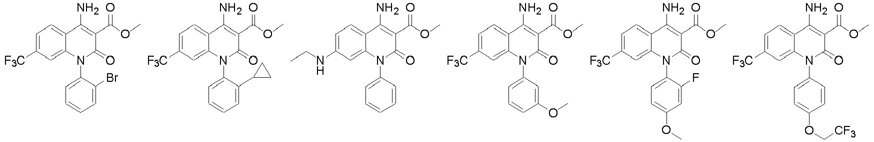

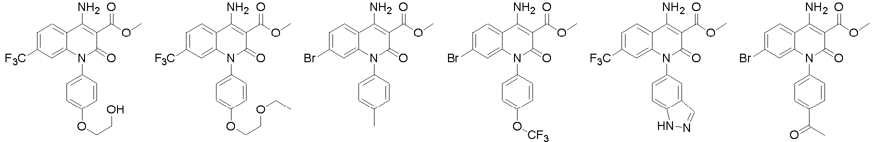

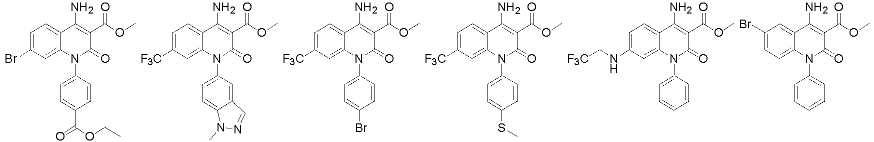

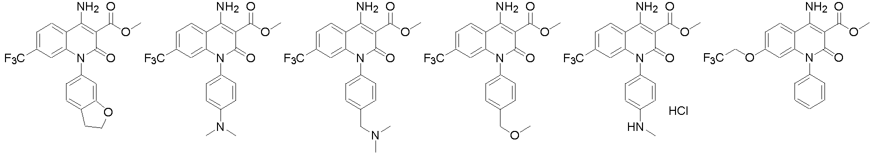

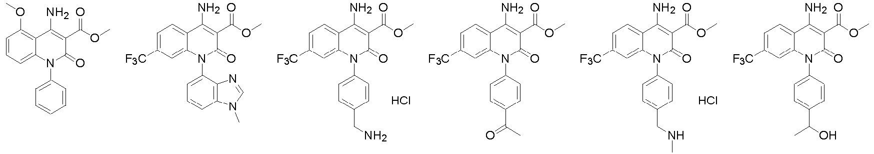

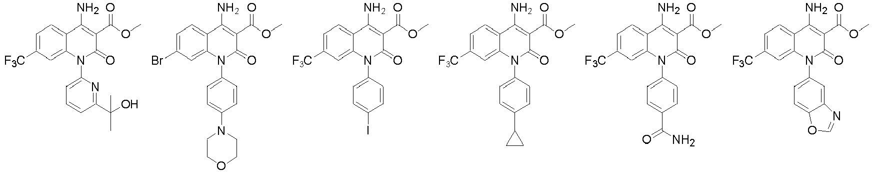

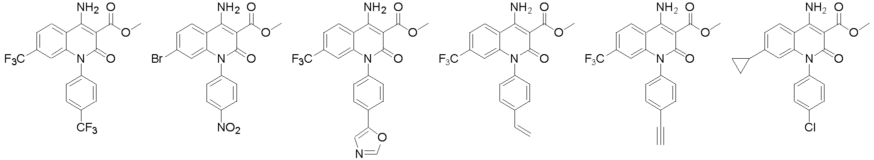

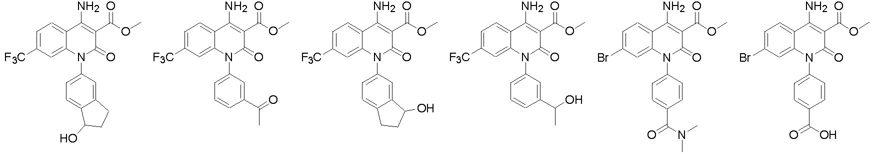

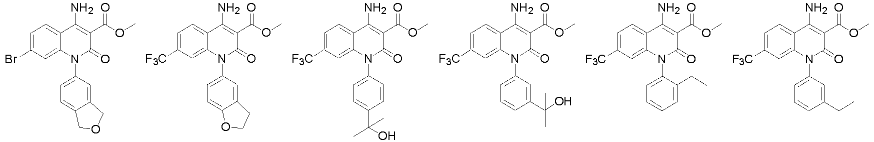

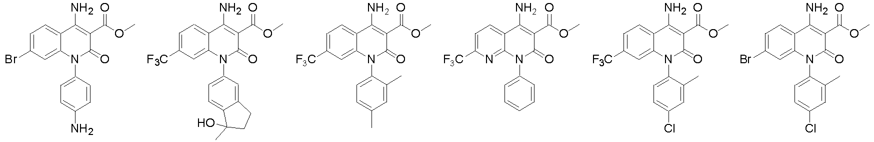

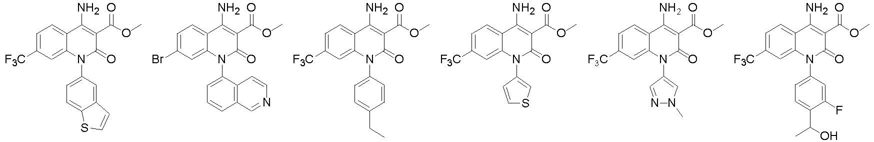

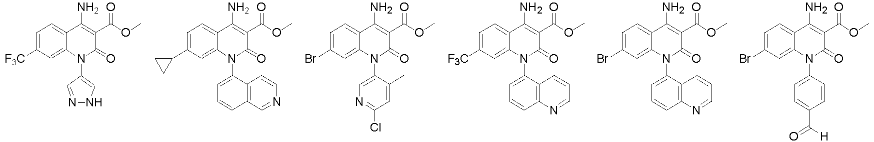

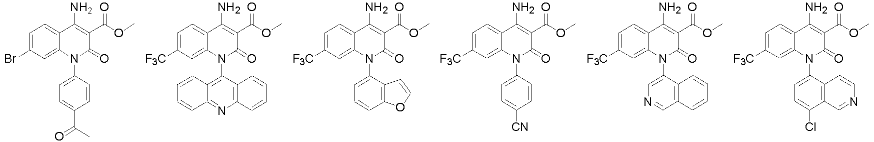

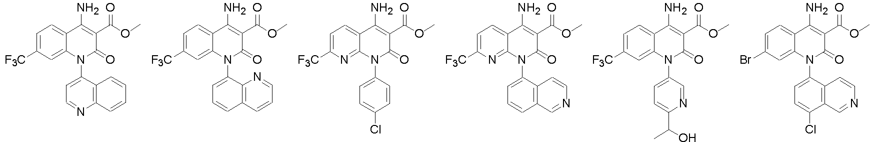

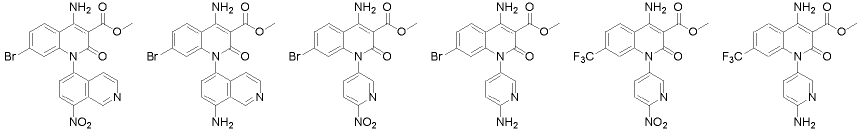

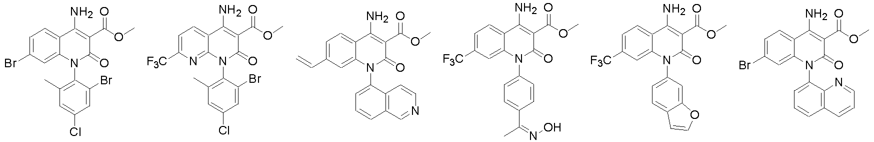

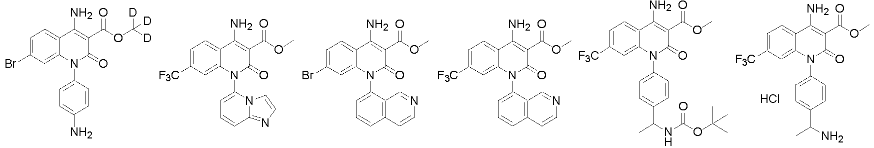

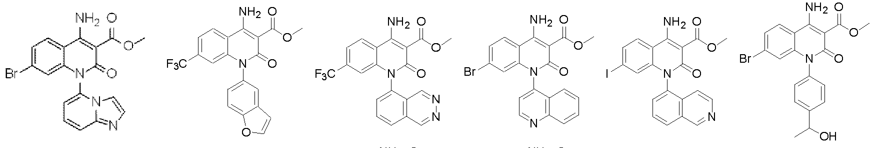

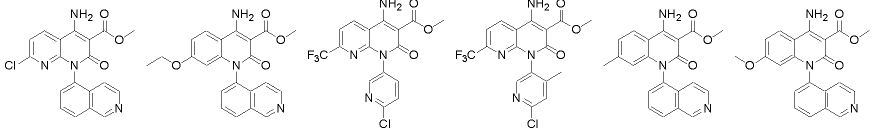

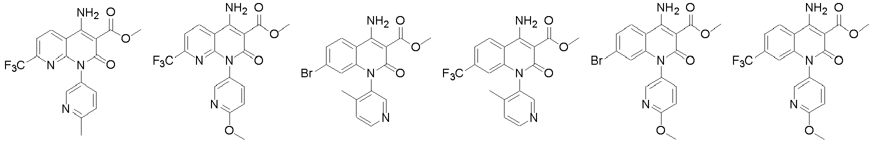

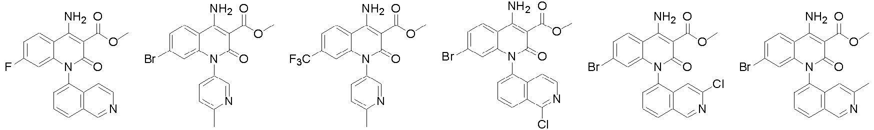

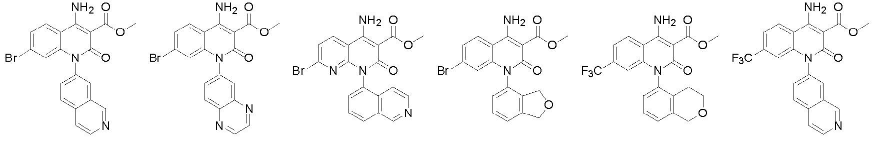

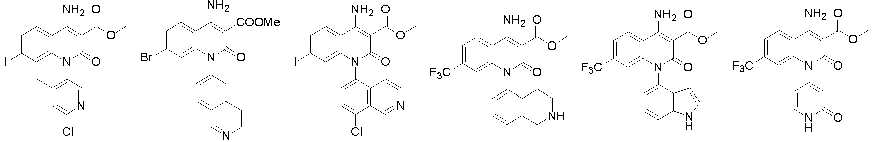

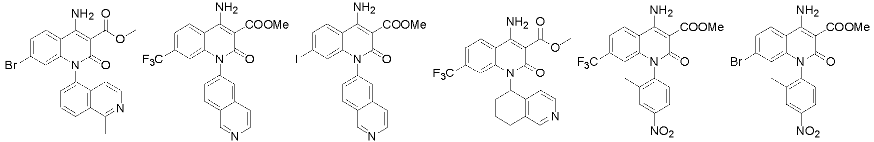

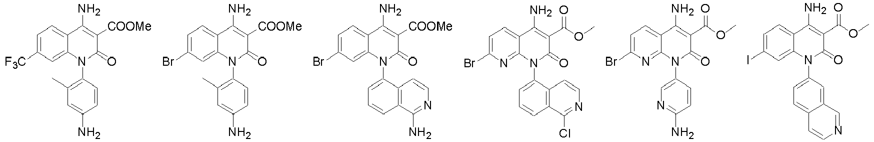

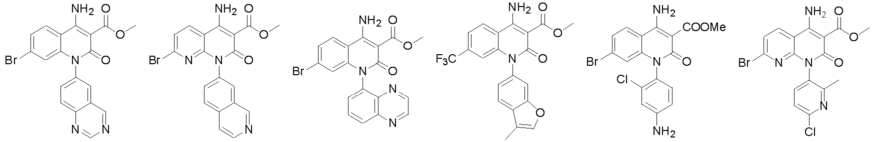

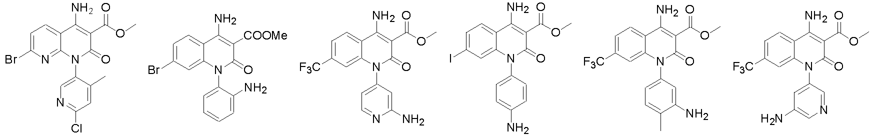

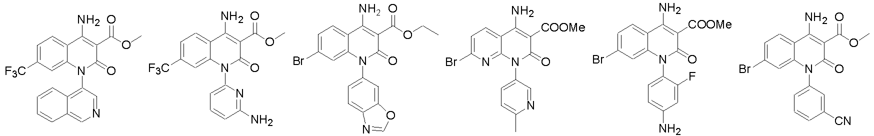

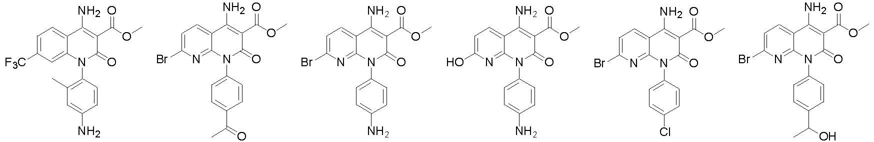

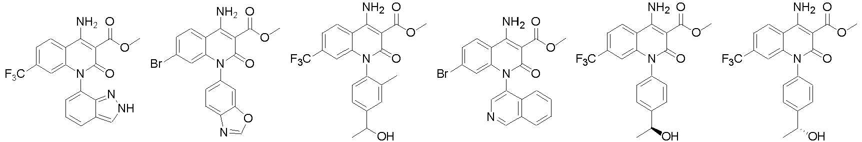

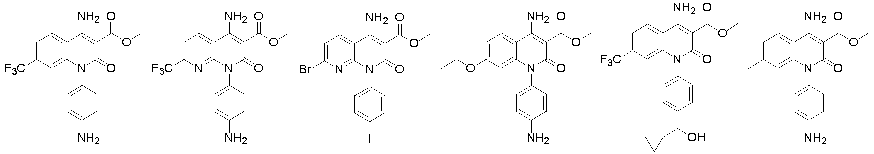

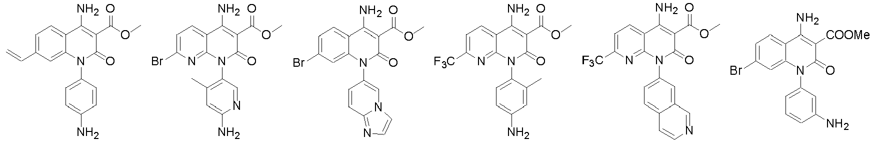

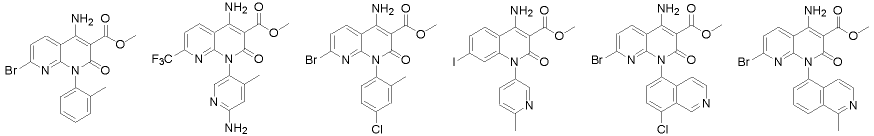

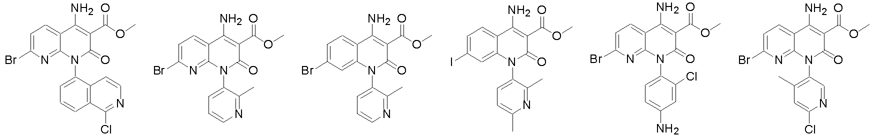

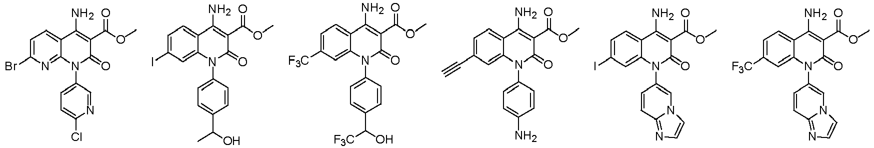

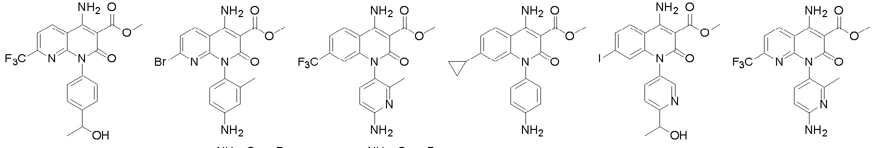

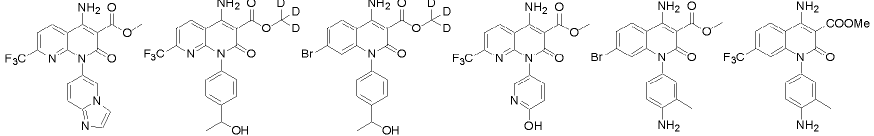

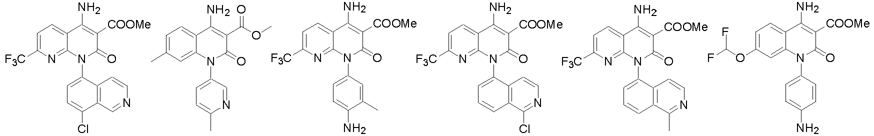

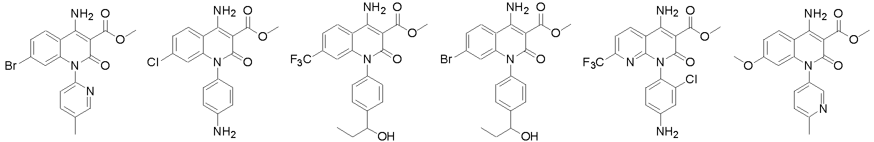

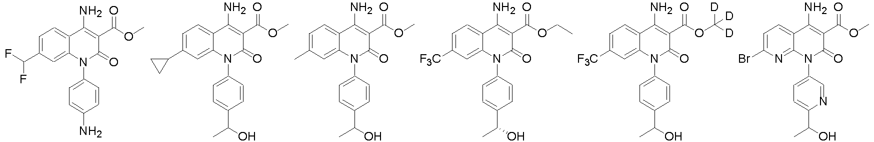

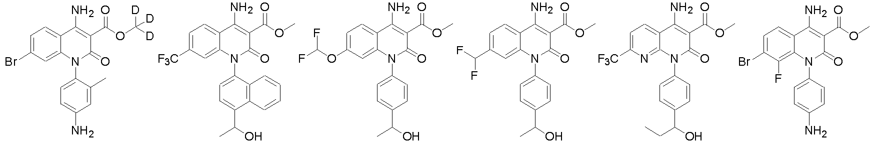

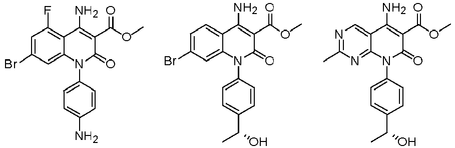

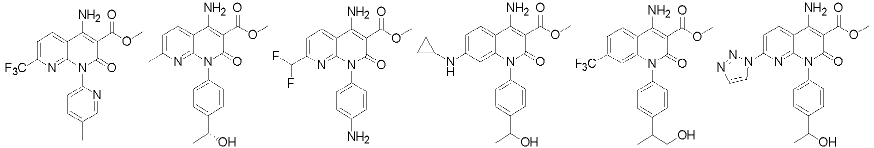

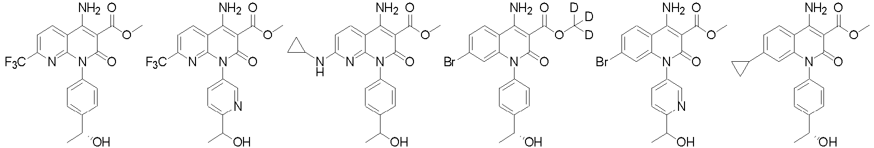

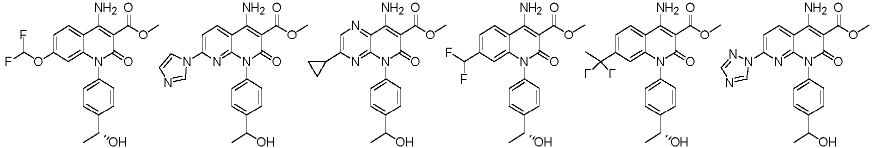

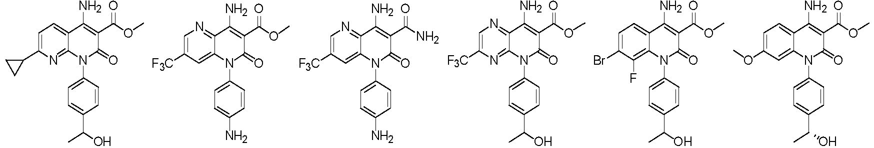

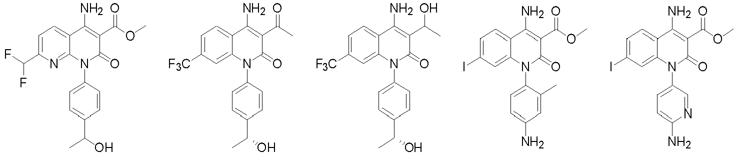

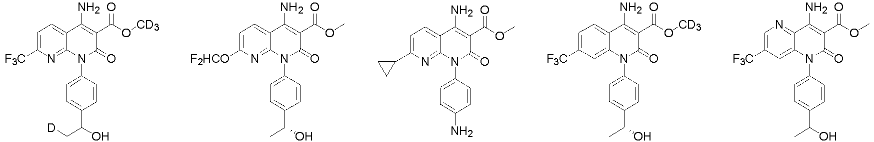

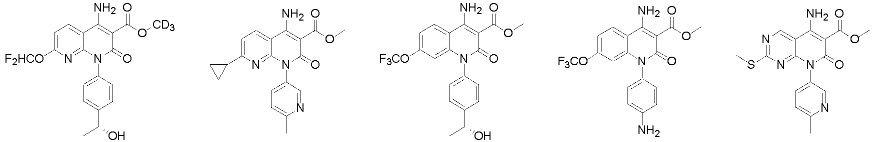

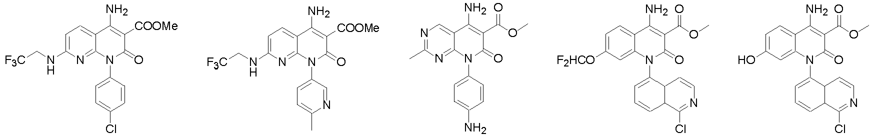

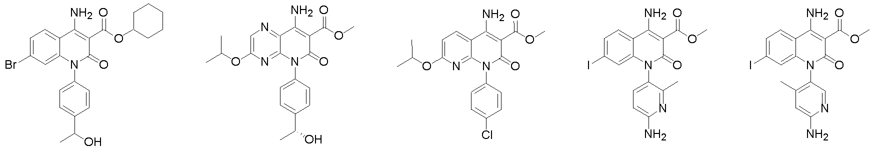

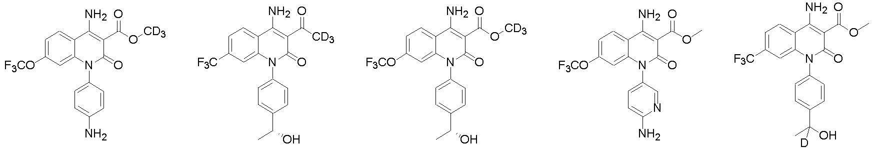

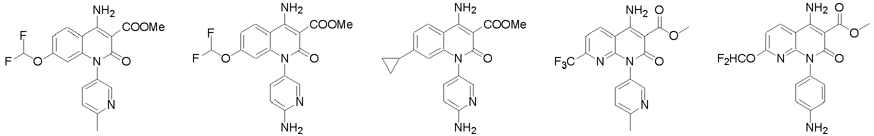

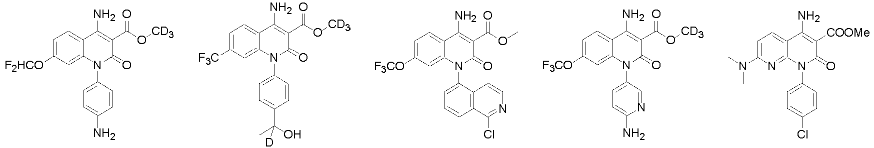

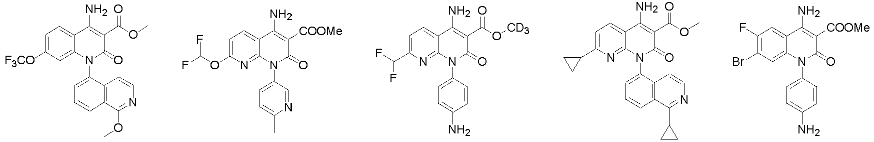

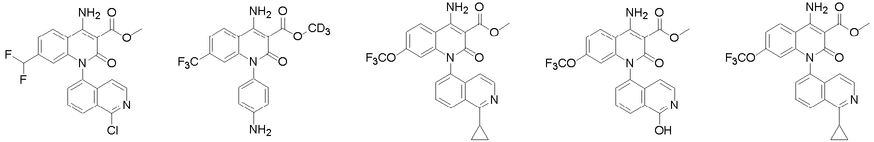

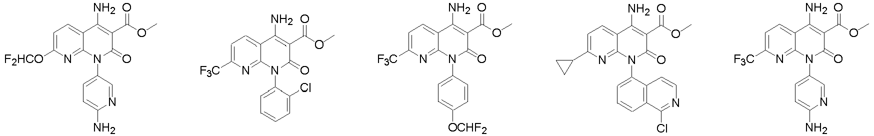

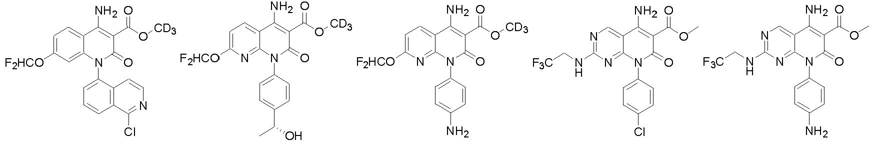

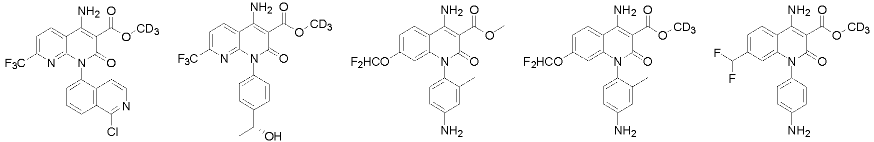

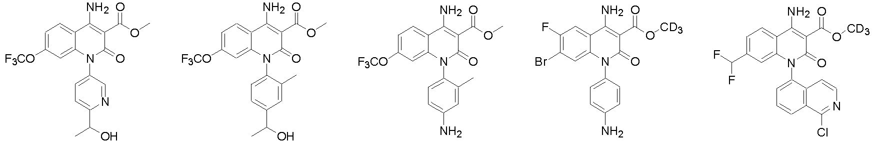

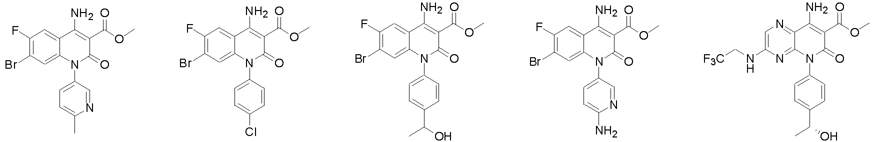

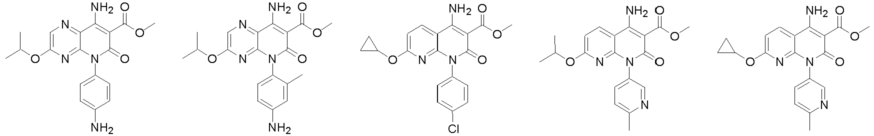

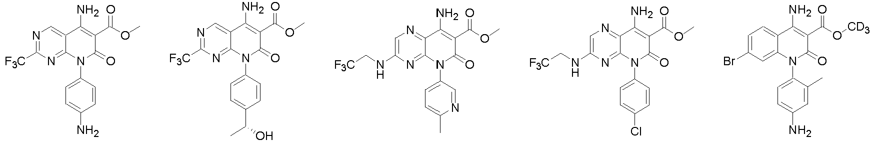

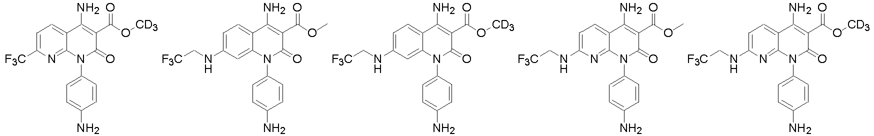

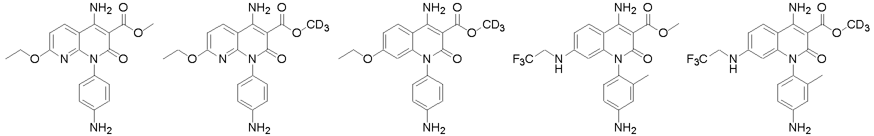

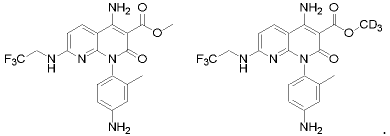

10. A pharmaceutical composition comprising the compound according to anyone of claims 1-9 or a pharmaceutically acceptable salt, solvate, polymorph or tautomer thereof, and optionally comprising a pharmaceutically acceptable excipient.

11. Use of the compound according to anyone of claims 1-9 or a pharmaceutically acceptable salt, solvate, polymorph or tautomer thereof or the pharmaceutical composition according to claim 10 in the manufacture of a medicament for the treatment of a disease related to MAT2A.

12. The use according to claim 11 wherein the disease related to MAT2A is tumor.

13. The use according to claim 11 wherein the disease related to MAT2A is non-small cell lung cancer, esophageal cancer, pancreatic cancer, or bladder cancer.
